# EUROPEAN PATENT SPECIFICATION

(11) **EP 1 390 488 B1**
(45) Date of publication and mention of the grant of the patent: **29.08.2007**
(21) Application number: 02748701.6
(22) Date of filing: 13.05.2002
(51) Int. Cl.: C12N 9/88, G06F 17/50, G06F 19/00

(54) **CRYSTAL STRUCTURE OF 2C-METHYL-D-ERYTHRITOL 2,4-CYCLODIPHOSPHATE SYNTHASE**
KRISTALLSTRUKTUR DER 2C-METHYL-D-ERYTHRITOL-2,4-CYCLODIPHOSPHAT-SYNTHASE
STRUCTURE CRISTALLINE DE 2C-METHYL-D-ERYTHRITOL 2,4-CYCLODIPHOSPHATE SYNTHASE

(30) Priority: 15.05.2001 DE 10123597; 25.05.2001 US 293875 P
(43) Date of publication of application: 25.02.2004
(73) Proprietor: Bacher, Adelbert, 85748 Garching (DE); Max-Planck-Gesellschaft zur Förderung der Wissenschaften e.V., 80539 München (DE)
(72) Inventor: BACHER, Adelbert, 85748 Garching (DE); HECHT, Stefan, 85386 Dietersheim (DE); HUBER, Robert, 82110 Germering (DE); KAISER, Johannes, 85748 Garching (DE); ROHDICH, Felix, 85406 Zolling (DE); STEINBACHER, Stefan, 81375 München (DE)
(74) Representative: Hartz, Nikolai
(86) International application number: PCT/EP2002/005238
(87) International publication number: WO 2002/092800

(56) References cited:
- WO-A-02/102991
- DATABASE SWISSPROT [Online] 1 June 1994 (1994-06-01) "ISPF_ECOLI" retrieved from EBI Database accession no. P36663 XP002242322
- SKELLY J V ET AL: "Overexpression, isolation, and crystallization of proteins" METHODS IN MOLECULAR BIOLOGY, HUMANA PRESS INC., CLIFTON, NJ, US, vol. 56, 1996, pages 23-53, XP002218318
- ABOLA E ET AL: "AUTOMATION OF X-RAY CRYSTALLOGRAPHY" NATURE STRUCTURAL BIOLOGY, NEW YORK, NY, US, vol. 7, no. SUPPL, November 2000 (2000-11), pages 973-977, XP001062873 ISSN: 1072-8368
- STEINBACHER STEFAN ET AL: "Structure of 2C-methyl-D-erythritol-2,4-cyclodiphosphat e synthase involved in mevalonate-independent biosynthesis of isoprenoids." JOURNAL OF MOLECULAR BIOLOGY, vol. 316, no. 1, 2002, pages 79-88, XP002242320 8 February, 2002 ISSN: 0022-2836
- RICHARD S B ET AL: "Structure and mechanism of 2-C-methyl-D-erythritol 2,4-cyclodiphosphate synthase: An enzyme in the mevalonate-independent isoprenoid biosynthetic pathway" JOURNAL OF BIOLOGICAL CHEMISTRY 08 MAR 2002 UNITED STATES, vol. 277, no. 10, 8 March 2002 (2002-03-08), pages 8667-8672, XP002242321 ISSN: 0021-9258
- RIDLEY R G: "Planting the seeds of new antimalarial drugs." SCIENCE. UNITED STATES 3 SEP 1999, [Online] vol. 285, no. 5433, 3 September 1999 (1999-09-03), pages 1502-1503, XP002242346 ISSN: 0036-8075 Retrieved from the Internet: <URL:http://www.sciencemag.org/cgi/content /full/285/5433/1502> [retrieved on 2003-05-23]

## Description

### Field of the invention

The present invention relates to isoprenoid biosynthesis and notably to 2C-methyl-D-erythritol 2,4-cyclodiphosphate synthases involved in that pathway and inhibitors of 2C-methyl-D-erythritol 2,4-cyclodiphosphate synthases, that may be used as antibiotics against pathogenic eubacteria and the protozoon *Plasmodium falciparum.* More specifically, the present invention relates to the crystal structure of 2C-methyl-D-erythritol 2,4-cyclodiphosphate synthase of *E. coli* in complex with substrate, substrate analogs and products and to methods of designing and identifying inhibitors of this enzyme. Moreover, the present invention relates to novel inhibitors detectable by said methods as well as compositions and processes for inhibiting the synthesis of isoprenoids and for controlling the growth of organisms based on said inhibitors. The

### Background of the invention

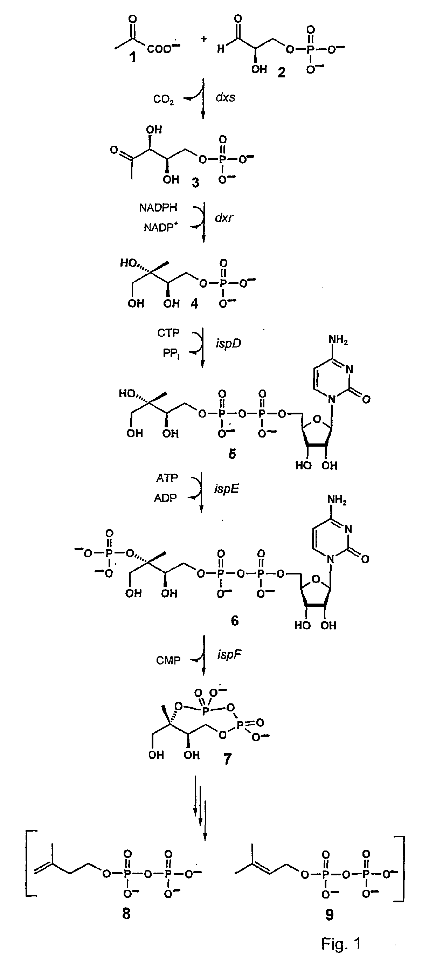

By the classical research of Bloch, Cornforth, Lynen and co-workers, isopentenyl pyrophosphate (IPP) and dimethylallyl pyrophosphate (DMAPP) have become established as key intermediates in the biosynthesis of isoprenoids via mevalonate. Bacterial, plants and the protozoon *Plasmodium falciparum* synthesize isoprenoids by an alternative pathway via 1-deoxy-D-xylulose 5-phosphate. This non-mevalonate pathway has so far only been partially explored (Fig. 1). For a better understanding of these aspects of the invention, the pathway shall be briefly explained. It can be conceptualized to consist of three segments:

In a first pathway segment shown in Fig. 1 pyruvate (1) is condensed with glyceraldehyde 3-phosphate (2) to 1-deoxy-D-xylulose 5-phosphate (DXP) (3). Subsequently, DXP is converted into 2*C*methyl-D-erythritol 4-phosphate (MEP) (4) by a two-step reaction comprising a rearrangement and a reduction. This established the 5-carbon isoprenoid skeleton.

In the subsequent segment of the non-mevalonate pathway (Fig. 1), MEP (4) is first condensed with CTP to 4-diphosphocytidyl-2C-methyl-D-erythritol (CDP-ME) (5) by 4-diphosphocytidyl-2C-methyl-D-erythritol synthase (PCT/EPOO/07548). CDP-ME (5) is subsequently ATP-dependent phosphorylated by 4-diphosphocytidyl-2C-methyl-D-erythritol kinase yielding 4-diphosphocytidyl-2C-methyl-D-erythritol 2-phosphate (CDP-MEP) (6). The intermediate is subsequently converted into 2C-methyl-D-erythritol 2,4-cyclodiphosphate (cMEPP) (7), by 2C-methyl-D-erythritol 2,4-cyclodiphosphate synthase. These three enzymatic steps form a biosynthetic unit which activates the isoprenoid C₅-skeleton for the third pathway segment (Rohdich *et al.,* 1999; Lüttgen *et al.,* 2000; Herz *et al.,* 2000).

For numerous pathogenic eubacteria as well as for the malaria parasite *P*. *falciparum,* the enzymes involved in the non-mevalonate pathway are essential. The intermediates of the non-mevalonate pathway can not be assimilated from the environment by pathogenic eubacteria and *P. falciparum.* The enzymes of the alternative isoprenoid pathway do not occur in mammalia which synthesize their isoprenoids and terpenoids exclusively via the mevalonate pathway. Moreover, the idiosyncratic nature of the reactions in this pathway reduces the risk of cross-inhibitions with other, notably mammalian enzymes. Therefore, the enzymes of the alternative pathway seemed to be specially suited as targets for novel agents against pathogenic bacteria and protozoa. Among the enzymes of the non-mevalonate pathway, 2C-methyl-D-erythritol 2,4-cyclodiphosphate synthase seems to be especially suited for the development of new inhibitors, because its reaction mechanism and reaction product are unique in animal and plant kingdoms and numerous possibilities for the design of transition state and intermediate analogues are opened. To aid in finding inhibitors of the non-mevalonate pathway, a crystal structure of 2*C-*methyl-D-erythritol 2,4-cyclodiphosphate synthase is highly desired.

Therefore, it is an object of this invention to provide crystals comprising 2C-methyl-D-erythritol 2,4-cyclodiphosphate synthase suitable for three-dimensional structure elucidation by crystallographic means and methods for obtaining such crystals.

Further, it is an object of this invention to provide methods for identifying inhibitors of 2*C-*methyl-D-erythritol 2,4-cyclodiphosphate synthase based on the 3D structure of said synthase.

It is another object of this invention to provide compounds which are inhibitors of 2*C-*methyl-D-erythritol 2,4-cyclodiphosphate synthase.

### Summary of the invention

These objects are achieved by:

A crystal which comprises the protein 2C-methyl-D-erythritol 2,4-cyclodiphosphate synthase with or without zinc. The crystal has the cubic space group I 2(1)3 and a unit cell with a= 144.5 ± 2 Å. This crystal preferably diffracts x-rays effectively for the determination of the atomic coordinates of the protein to a resolution better than 5 Å, more preferably better than 3.5 Å. The crystal may comprise an organic compound selected from the group of 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol 2,4-cyclodiphosphate or a combination of cytidine monophosphate and 2C-methyl-D-erythritol 2,4-cyclodiphosphate.

A method of growing a crystal comprising the protein 2C-methyl-D-erythritol 2,4-cyclodiphosphate synthase and zinc by vapor diffusion uses a reservoir solution containing 0.1 M HEPES pH 7.5 and 2 M ammonium formate.

Use of a crystal as described above for the determination of the three-dimensional structure of the protein 2C-methyl-D-erythritol 2,4-cyclodiphosphate synthase or the three-dimensional structure of said synthase in complex with a compound selected from the group of 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol 2,4-cyclodiphosphate or a combination of cytidine monophosphate and 2C-methyl-D-erythritol 2,4-cyclodiphosphate; with or without zinc.

A data storage device is described having stored thereon atomic coordinates of the three-dimensional structure of the protein 2C-methyl-D-erythritol 2,4-cyclodiphosphate synthase or of the three-dimensional structure of said protein in complex with a compound selected from the group of 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol 2,4-cyclodiphosphate or a combination of cytidine monophosphate and 2C-methyl-D-erythritol 2,4-cyclodiphosphate; with or without zinc.

A method of using 2*C*-methyl-D-erythritol 2,4-cyclodiphosphate synthase and atomic coordinates of the three-dimensional structure of said synthase or of a complex of said synthase with a compound selected from the following group: 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol 2,4-cyclodiphosphate or a combination of cytidine monophosphate and 2C-methyl-D-erythritol 2,4-cyclodiphosphate, with or without zinc, derived from a crystal structure determination in an inhibitor-screening assay, comprising:
(a) selecting a potential inhibitor by performing rational drug design using said atomic coordinates in conjunction with computer modelling;
(b) contacting the potential inhibitor with said synthase with or without zinc; and
(c) detecting binding of the potential inhibitor to said synthase or detecting inhibition of enzymatic activity of said synthase by the potential inhibitor; according to claim 7.
Binding may be detected by soaking the crystal with the potential inhibitor or by growing the crystal in the presence of the potential inhibitor and determining the three-dimensional structure of the complex comprising the synthase and the potential inhibitor with or without zinc.

A method of identifying a potential inhibitor of 2C-methyl-D-erythritol 2,4-cyclodiphosphate synthase by determining binding interactions between the potential inhibitor and a set of binding interaction sites in a binding cavity of said synthase complexed with a compound selected from the group consisting of 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol 2,4-cyclodiphosphate or a combination of cytidine monophosphate and 2C-methyl-D-erythritol 2,4-cyclodiphosphate, with or without zinc, comprising
(a) generating the binding cavity on a computer screen,
(b) generating potential inhibitors with their spatial structure on the computer screen, and
(c) selecting potential inhibitors that can bind to at least 3 amino acid residues without steric interference; according to claim 9.

A computer-assisted method for identifying potential inhibitors of the protein 2C-methyl-D-erythritol 2,4-cyclodiphosphate synthase using a programmed computer comprising a processor, a data storage system, a data input device, and a data output device, comprising the following steps:
(a) inputting into the programmed computer through said input device data comprising:
   atomic coordinates of a subset of the atoms of a complex of said protein with a compound selected from the following group: 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol 2,4-cyclodiphosphate or a combination of cytidine monophosphate and 2C-methyl-D-erythritol 2,4-cyclodiphosphate, with or without zinc, thereby generating a criteria data set;
(b) comparing, using said processor, the criteria data set to a computer data base of low-molecular weight organic chemical structures stored in the data storage system; and
(c) selecting from said data base, using computer methods, a chemical structure having a portion that is structurally complementary to the criteria data set pertaining to the protein and/or structurally similar to the criteria data set pertaining to a compound of said group and being free of steric interference with the protein; according to claim 10.

A computer-assisted method for identifying potential inhibitors of the protein 2C-methyl-D-erythritol 2,4-cyclodiphosphate synthase using a programmed computer comprising a processor, a data storage system, a data input device, and a data output device, comprising the following steps:
(a) inputting into the programmed computer through said input device data comprising: atomic coordinates of a subset of the atoms of a complex of said protein with a compound selected from the following group: 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol 2,4-cyclodiphosphate or a combination of cytidine monophosphate and 2C-methyl-D-erythritol 2,4-cyclodiphosphate with or without zinc, thereby generating a criteria data set; and
(b) constructing, using computer methods, a model of a chemical structure having a portion that is structurally complementary to the criteria data set pertaining to the protein and/or structurally similar to the criteria data set pertaining to a compound of said group and being free of steric interference with the protein; according to claim 11.

A method of identifying a candidate inhibitor capable of binding to and inhibiting the enzymatic activity of 2C-methyl-D-erythritol 2,4-cyclodiphosphate synthase, said method comprising the following steps:
(a) introducing into a computer information derived from atomic coordinates defining a conformation of the active site of said synthase or a complex of said synthase with a compound selected from the following group: 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol or a combination of cytidine monophosphate and 2C-methyl-D-erythritol 2,4-cyclodiphosphate; with or without zinc, based on three-dimensional structure determination, whereby said program utilizes or displays on the computer screen the structures of said conformation;
(b) generating a three-dimensional representation of at least one of the three pockets of the active site of said synthase and/or a compound of said group by said computer program on a computer screen;
(c) superimposing a model of a candidate inhibitor on the representation of at least one pocket of the active site and/or a compound of said group;
(d) assessing the possibility of bonding and the absence of steric interference of the candidate inhibitor with the active site of the protein;
(e) incorporating said candidate compound in an activity assay of said synthase; and
(f) determining whether said candidate compound inhibits enzymatic activity of said synthase,
according to claim 12.
In the above methods, the atomic coordinates are preferably determined to a resolution of at least 4 Å, more preferably better than 3 Å, and potential inhibitors are selected that can bind to at least 5 binding sites of the synthase.

Finally, a compound is provided having a chemical structure obtained or obtainable by the above methods, said compound being an inhibitor of 2C-methyl-D-erythritol 2,4-cyclodiphosphate synthase.

It has been surprisingly found that protein crystals of 2C-methyl-D-erythritol 2,4-cyclodiphosphate synthase from *E. coli* can be obtained using the procedures disclosed herein. These crystals are of high quality and are suitable for crystallographic structure determination. According to methods known in the art, the three-dimensional structure of said synthase has been determined (see examples) from a crystal containing zinc and designated "nat2".

It has been surprisingly found that 2C-methyl-D-erythritol 2,4-cyclodiphosphate synthase tightly binds one zinc ion per monomer of the synthase. This fact provides invaluable information for the understanding of the reaction mechanism of the synthase which in turn is helpful for the rational design of potential inhibitors. Moreover, said synthase forms trimers and the regions on the surface of the monomers involved in interaction with other monomers have been identified.

Furthermore, the 3D structure of 2C-methyl-D-erythritol 2,4-cyclodiphosphate synthase has been determined from crystals soaked with
(a) cytidine diphosphate (CDP) and magnesium ions giving a crystal named "mgcdp2";
(b) 4-diphosphocytidyl-2C-methyl-D-erythritol giving a crystal named "subop";
(c) 4-diphosphocytidyl-2C-methyl-D-erythritol 2-phosphate and EDTA giving a crystal named "subs";
(d) 4-diphosphocytidyl-2C-methyl-D-erythritol 2-phosphate in the presence of zinc, which is converted by the synthase to cytidine monophosphate (CMP) and 2C-methyl-D-erythritol 2,4-cyclodiphosphate. This crystal is named "sub2".
(e) cytidine giving a crystal named "cyt".

In all these cases, the indicated compounds were identified in electron densities of the respective structures.

Surprisingly, the substrate 4-diphosphocytidyl-2C-methyl-D-erythritol 2-phosphate has been found to bind well-ordered to the synthase without conversion to the products under conditions where bivalent metal ions are complexed by EDTA (crystal subs), thereby identifying the active site of the enzyme. Moreover, the substrate analogs, fragments and products CMP, CDP, cytidine and 4-diphosphocytidyl-2C-methyl-D-erythritol were found to bind to the crystallised synthase, allowing a detailed mapping of active site regions and residues which are responsible for binding certain substrate of product moieties. Specific functions could be -assigned to specific active site amino acid residues. Even more surprisingly, it has been found that the substrate is coordinated by amino acid residues from two monomers of the trimeric synthase. Magnesium ions are known to be required for the catalytic activity of said synthase. Herein, the location of a magnesium ion has been identified in the complex of the synthase and CDP.

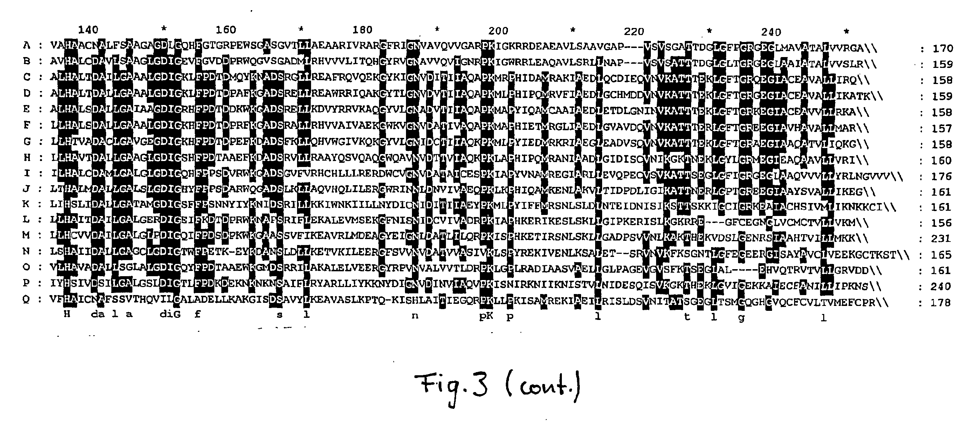

On the basis of the crystal structure data and the alignment of putative 2C-methyl-D-erythritol 2,4-cyclodiphosphate synthases from various organisms (Fig. 3), site-directed mutants of the *E. coli* synthase were prepared and the essential function of certain amino acids could be confirmed. This further highlights the usefulness of knowing the three-dimensional structure of a protein for understanding and manipulating the function of a protein in a rational way.

Taken together, the three-dimensional structure information disclosed herein has allowed to design methods for identifying potential inhibitors of the synthase employing computer methods of rational drug design and computer modeling. Preferably, structural information of active site residues and/or of bound substrate and/or substrate fragments and products are used for the rational design/computer modelling of potential inhibitors. Potential inhibitors may then be synthesized and their inhibitory potential be tested experimentally. This approach allows the direct design or identification of an inhibitor or reduces the number of compounds which have to be synthesized and to be tested for their inhibitory potential experimentally, since only structures found to be promising in silico are further pursued experimentally. Inhibitors obtained by the methods of this invention may be used as antibiotics against bacteria or protozoa, notably the malaria parasite *P. falciparum* or as herbicides.

Once a suitable inhibitor of the synthase has been found, inhibitor-resistant mutants of the synthase may be designed using the 3D structures disclosed herein.

The accuracy of the coordinates of a protein crystal structure depends on the resolution of the diffraction data used in refinement. The resolution should be such that amino acid side chains in well-ordered regions of the protein can be seen in the electron density maps. The resolution should be at least 5 Å, preferably better than 4 Å, and most preferably at least 3.0 Å. The coordinates provided herein contain experimental error and are limited by the resolution of the diffraction data. Crystallisation conditions may be further improved according to known approaches in protein crystallisation, diffraction data to better resolution may be measured and more accurate coordinates may be obtained. This may e.g. be achieved by using synchrotron radiation, optionally in combination with cryo-crystallography. It has been found that the crystals used herein can easily be frozen by liquid nitrogen. Using the atomic coordinates disclosed herein as starting structure, such an improved structure may be easily obtained. This will change the numerical values of the coordinates in table 2 to some extent but the fold of 2*C*-methyl-D-erythritol 2,4-cyclodiphosphate synthase will remain the same. All 3D structures of the synthase with atomic coordinates the numerical values of which differ only within experimental or computational error, due to a different choice of the coordinate system, due to different experimental conditions or due to a different quality of experimental data are also comprised by the present invention. The same applies to 3D structures derived from different crystal forms or to 3D structures determined by experimental approaches different from crystallography such as NMR or electron microscopy.

Upon binding of a molecule to said synthase, the structure of the synthase may undergo changes. Often, such changes are limited to amino acid side chain conformations but whole groups of amino acids including their peptide back bone may move as well, particularly amino acids in a flexible loop. Such altered conformations are also comprised by this invention as long as the overall fold of the protein remains the same. The six structures disclosed herein provide a framework of conformational states assumable by the synthase in the absence and presence of substrate, substrate analogs and products. When performing rational drug design or computer modelling, at least the conformation of amino acid side chains will also be varied in the process of finding a potential inhibitor. Preferred and less-preferred side chain conformations (torsion angles) are known in the art.

Herein, the *E*. *coli* protein has been used to determine the structure of 2C-methyl-D-erythritol 2,4-cyclodiphosphate synthase. This invention relates also to said synthases from other organisms. Although orthologous proteins from various organisms may differ considerably in the primary structure (compare sequence alignment of Fig. 3), the fold and active site architecture typically remain essentially the same. Active site residues necessary for the function of a protein are conserved. As a consequence, inhibitors found according to the methods of this invention using the 3D structure of 2C-methyl-D-erythritol 2,4-cyclodiphosphate synthase from *E*. *coli* will also be inhibitors of orthologous synthases from organisms other than *E*. *coli.* This invention therefore comprises proteins with root mean square deviations from the *E. coli* synthase structure over protein backbone atoms or better over all non-hydrogen atoms of not more than 3 Å, preferably not more than 2 Å and most preferably not more than 1 Å. The 3D structures of such related proteins may easily be obtained by homology modeling using the 3D structures of this invention. Practically, other proteins comprised by this invention may be those whose crystal structures can be solved by molecular replacement using the coordinates of the *E. coli* synthase of this invention.

The crystals used herein belong to the cubic space group I2(1)3 with unit cell parameter a≈144.5 Å. However, other crystal forms may also be used to determine the 3D structure of the synthase if they are of sufficient quality for diffraction experiments. A structure from another crystal form may preferably be solved using molecular replacement with the atomic coordinates disclosed herein as a starting model. The structure of the synthase as determined from another crystal form may differ to some extent from the structures disclosed herein. Such differences will however be limited essentially to surface amino acid residues involved in crystal packing. The crystals of space group 12(1)3 used herein feature the important advantage that the active site of the synthase is easily accessible to substrate and analogs thereof or inhibitors, which allows soaking experiments and the determination of the synthase in complex with low molecular weigth organic compounds. The synthase used for crystallisation optionally contains zinc in order to be in an active form. Extra zinc may be added during crystallisation.

Crystallisation may be done by any method known in the art like batch methods or vapour diffusion methods. Hanging- or sitting-drop vapour diffusion methods are preferred. The 3D structure of the synthase in complex with an inhibitor may be solved by preparing a crystal containing the synthase in complex with the inhibitor. This may be achieved by co-crystallizing the synthase with the inhibitor or by soaking a crystal of the synthase not containing an inhibitor in mother liquor containing an excess of the inhibitor of interest for a suitable time. Prior to collection of diffraction data, crystals may be frozen according to known methods of cryo-crystallography, preferably after treatment of the crystal with a suitable cryo protectant.

After the structure of the synthase has been solved and refined, the atomic coordinates may be stored on a computer-readable data storage device for further use.

### Short description of the figures, tables, enclosed CD-ROM and annexes

Figure 1:
   Biosynthesis of both isoprenoid precursors isopentenyl pyrophosphate and dimethylallyl pyrophosphate via the alternative pathway.
Figure 2:
   Enzyme activities of 2C-methyl-D-erythritol 2,4-cyclodiphosphate synthase of *E*. *coli.*
Figure 3:
   Alignment of amino acid sequences of putative 2C-Methyl-D-erthritol-2,4-cyclodiphosphat synthases from various organisms. A, *Streptomyces* sp.; B, *Mycobacterium tuberculosis;* C, *Haemophilus influenzae;* D*, . coli;* E*, Vibrio cholerae;* F*, Pseudomonas aeruginosa;* G*, B. subtilis;* H*, Neisseria meningitidis;* I, *Xylella fastidiosa;* J*, Synechocystis* sp.; K, *Buchnera* sp.; L, *Aquifex aeolicus;* M*, A. thaliana;* N*, Thermotoga maritima;* O, *Deinococcus radiodurans;* P*, P. falciparum;* Q, *Chlamydia muridarum.*

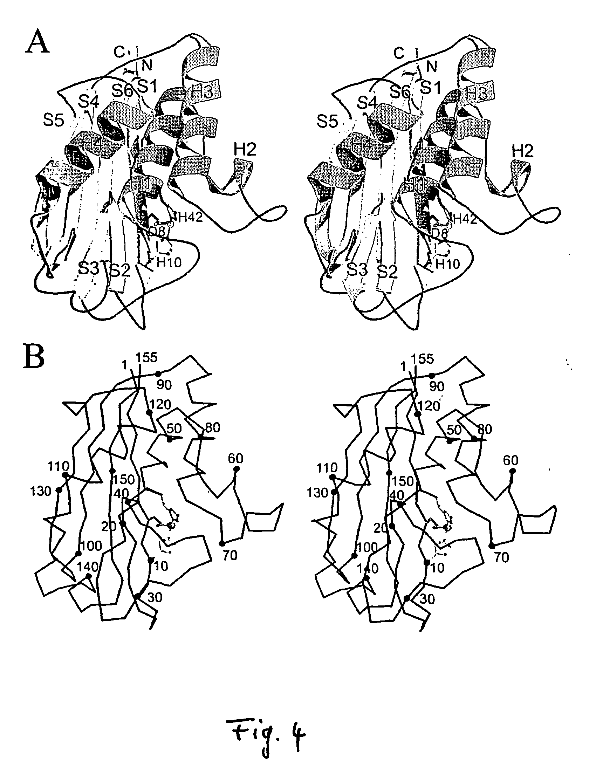

Figure 4: Overall fold of the monomer
   (A) Stereo ribbon representation of the structure of the 2C-methyl-D-erythritol 2,4-cyclodiphosphate synthase monomer. The four-stranded β-sheet is shown in yellow, the smaller two-stranded β-sheet in orange, α-helices are depicted in red. The bound zinc ion is shown as a pink ball including the co-ordinating side chains of Asp8, His 10 and His42. (B) Cα-trace of the 2C-methyl-D-erythritol 2,4-cyclodiphosphate synthase monomer. Every tenth amino acid is shown as a black ball.

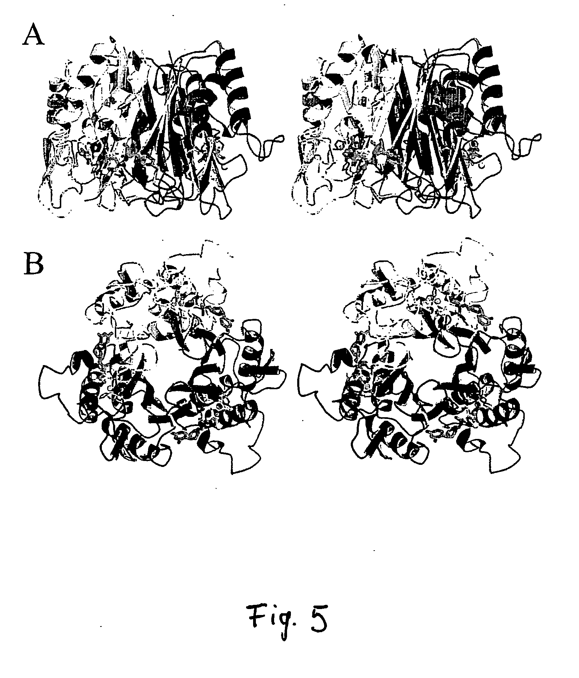

Figure 5: Overall fold of the trimer
   (A) Stereo side view of the 2C-methyl-D-erythritol 2,4-cyclodiphosphate synthase trimer perpendicular to the trimer axis. The view goes onto the CDP binding site. The monomers are shown in blue, green and ochre. CDP is depicted as a stick model in red. (B) View along the threefold axis. The N- and C-termini point away from the viewer.

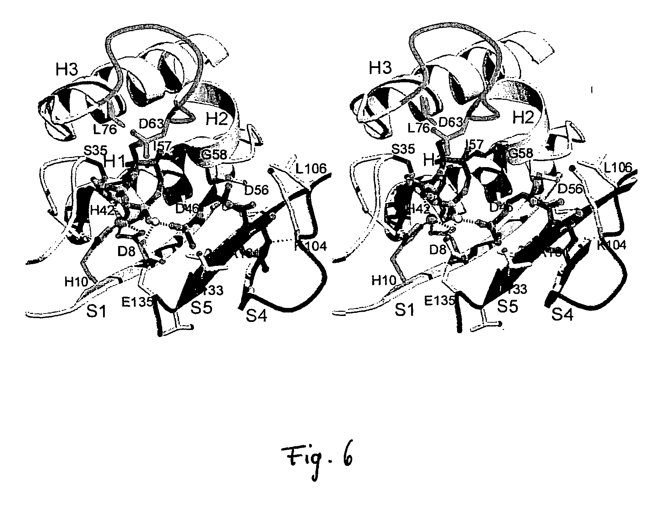

Figure 6: Active site structure
   The substrate binding site/active site is composed of two subunits (in ochre and green). CDP is shown as a ball and stick model in dark green. A magnesium ion depicted as a gray ball is liganded by Glul135 and bridges the α- and β-phosphate of CDP. The β-phosphate of CDP is bound to a zinc ion (pink ball) co-ordinated by Asp8, His10 and His42. Two sequence motifs are involve in substrate binding: the KATTTE-motif (residues 130 to 135) at the C-terminal half of β-strand S5 that contributes Ala131, Thr133 and Glu135 to substrate binding and the DIG-motif (residues 56 to 58) at the N-terminus of α-helix H2 where Asp56 and Gly58 contact the ribose. The presumable binding site for the 2*C*-methyl-D-erythritol 2-phosphate moiety is formed by Ile57, Leu76, Ser35 adjacent main chain atoms and completed by the loop from Pro62 to Ala71 including Asp63 (shown in red).

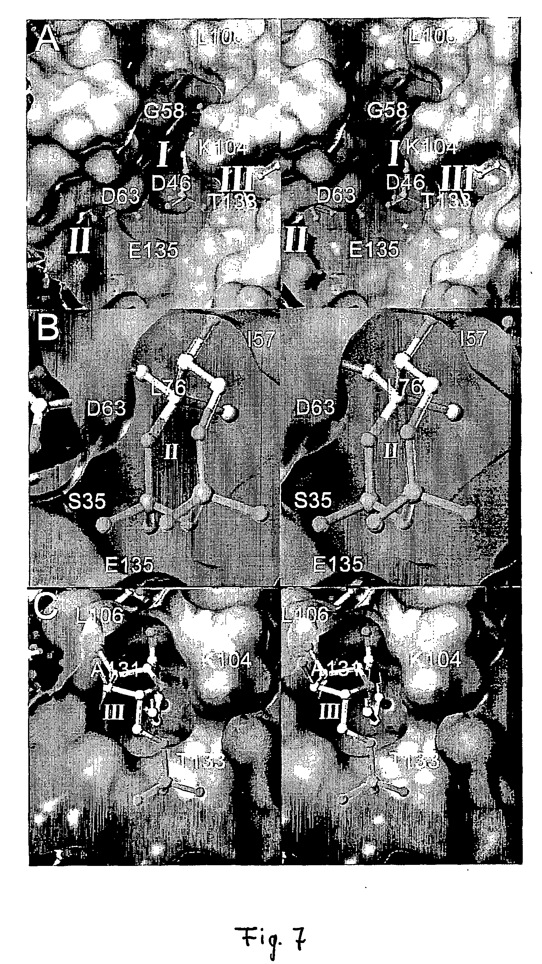

Figure 7: Electrostatic and surface properties of the active site. The colour scale goes from blue (negative potential) via green to red (positive potential).
   (A) The active site/ substrate binding site consists of three subsites designated I, II and III. The central subsite I accommodates the ribosyl 5'-diphosphate of CDP. (B) The potential subsite for the 2C-methyl-*D*-erythritol 2-phosphate-moiety is flanked by the highly conserved IIe57 and Ser35. (C) The subsite for the cytidyl-moiety is formed by Lys104, Leu106, Ala131 and Thr133.
Fig. 8
   Schematic two-dimensional representation of product binding from the model of complex structure sub2. Interactions of protein residues with CMP and 2C-methyl-D-erythritol 2,4-cyclodiphosphate are depicted as dotted lines along with the nomenclature of product atoms as used in the coordinate file of structure sub2. Distances are given in A.
Fig. 9:
   DNA and deduced amino acid sequences of the *ispF* gene of *Escherichia coli.* The positions and directions of oligonucleotides used in PCR reactions for the construction of expression vectors for the expression of the site directed 2C-methyl-D-erythritol 2,4-cyclodiphosphate synthase mutant proteins D8S, H10S and H42S are indicated by arrows.

Table 1 shows statistics of data collection and refinement of the six structures disclosed herein.
Table 2 gives coordinates of atoms within 10 Å of the bound ligands of structure sub2.

Annexes 1 to 6 are printouts of coordinate files of structures cyt, mgcdp2, nat2, sub2, subop and subs, respectively.
Structure sub2 was deposited with the protein data bank (PDB) and can be accessed via www.rcsb.org/pdb using entry number 1JY8.

### Detailed description of the invention

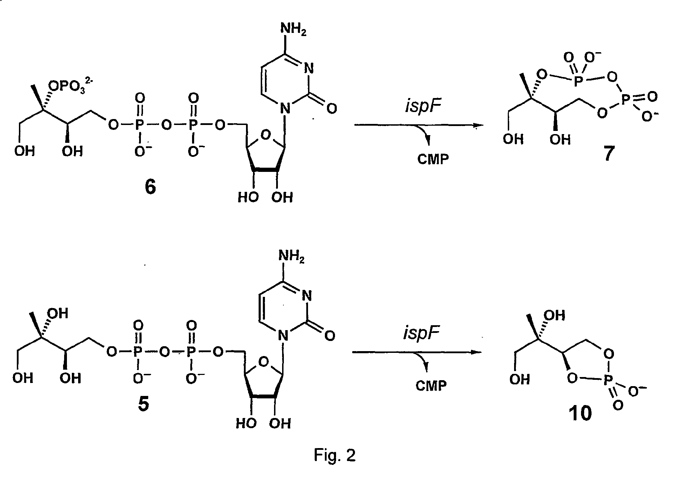

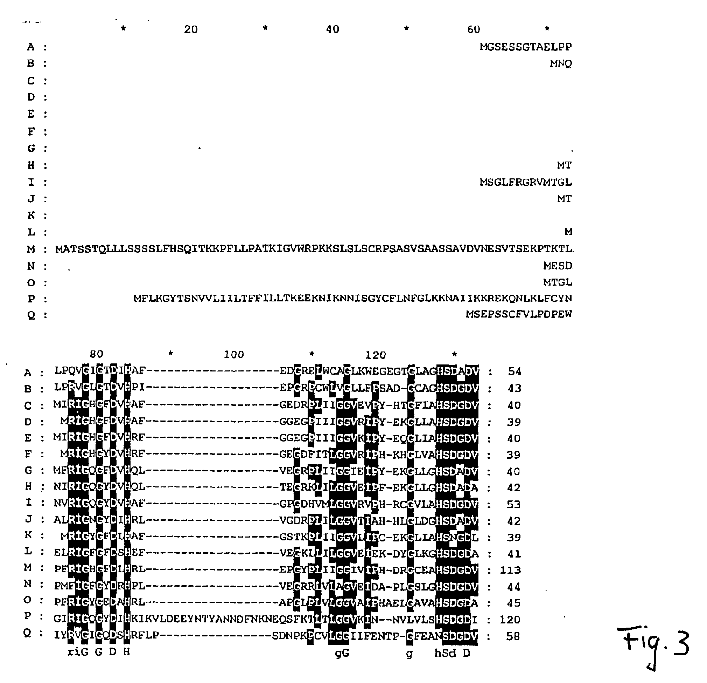

2C-methyl-D-erythritol 2,4-cyclodiphosphate synthase specified by the *ispF* gene from *E*. *coli* catalyzes the cyclization of 4-diphosphocytidyl-2C-methyl-D-erythritol 2-phosphate **6** to 2*C-*methyl-D-erythritol 2, 4-cyclodiphosphate 7 (Fig. 2). With lower rate, 2C-methyl-D-erythritol 2,4-cyclodiphosphate synthase also converts 4-diphosphocytidyl-2C-methyl-D-erythritol **5** into 2*C-*methyl-D-erythritol 3,4-cyclomonophosphate **10** (Herz et al. 2000). Monomeric 2C-methyl-D-erythritol 2,4-cyclodiphosphate synthase from *E*. *coli* has a molecular weight of 17 kDa (corresponding to 159 amino acids) (Herz *et al.* 2000). Magnesium ions are necessary for the catalytic activity. The K_{M} and *vₘₐₓ* values of the *E. coli* enzyme are 37 µM and 76 µmol mg⁻¹ min⁻¹, respectively, with 4-diphosphocytidyl-2C-methyl-D-erythritol 2-phosphate as substrate.

### 3D structure solution

2C-methyl-D-erythritol 2,4-cyclodiphosphate synthase was crystallized in the cubic space group l2(1)3 with a = 144.2 Å (in structure nat2). The solvent content is about 84 %. A native data set was collected on a rotating anode generator equipped with an *MARresearch* Image Plate detector up to a resolution of 2.85 Å. Details and statistics of data collection are given in Table 1. For structure solution, a native crystal was incubated with 2 mM mercury(II) acetate in mother liquor for one hour yielding an isomorphous derivative crystal with a single heavy atom site in the asymmetric unit. Heavy atom parameters were refined and phases calculated with the program MLPHARE (Collaborative Computational Project No. 4,1994). The phasing power was 1.0 (25 to 3.6Å) with a figure of merit of 0.27 in a resolution range from 20 to 3.6 Å. Phases were improved by solvent flattening using the program DM (Collaborative Computational Project No. 4, 1994). An atomic model was built with the program MAIN (Turk, 1992) and refined with the program X-PLOR (Brünger *et al.*, 1998). The model comprises residues Met1 to IIe155. The electron density is continuous throughout the model with the exception of a short break between Phe61 and Pro62 in a loop structure comprising Phe61 to Lys69. The model of structure nat2 has been refined to an R-value of 22.6% and a free R value of 23.7% calculated with 5% of the reflections in the resolution range 20.0 to 2.85Å (Table 1).

The following substrate, substrate analoge and product complexes were determined in order to determine binding of these molecules to the synthase:
- subop: a crystal soaked with 10mM 4-diphosphocytidyl-2C-methyl-D-erythritol. The zinc atom the water or hydroxyl-group at the zinc is replaced by an oxygen of the beta-phosphate of the diphosphocytidyl-moiety.
- subs: a crystal soaked with 10mM 4-diphosphocytidyl-2C-methyl-D-erythritol 2-phosphate and 10mM EDTA, that contains no zinc.
- sub2: a crystal soaked with 10mM 4-diphosphocytidyl-2C-methyl-D-erythritol 2-phosphate, that containes zinc and the products CMP and 2C-methyl-D-erythritol 2,4-cyclodiphosphate.
- mgcdp2: a crystal soaked with 2mM CDP and 5mM MgCl2
- cyt: a crystal soaked with 10mM cytidine

Statistics on data collection and refinement for these crystals/structures of the invention are given in Table 1. These structures were determined using the nat2 structure as starting model. Initially a rigid body refinement was carried out followed by positional refinement. Then the substrate, substrate analoge or product molecules were build into the electron density of difference electron density maps, again followed by refinement of atom positions and B-factors.

### Overall description of the 3D structure

2*C*-methyl-D-erythritol 2,4-cyclodiphosphate synthase forms bell-shaped homotrimers with overall dimensions of about 40Å in height and between 40 and 60 Å in diameter. These molecular trimers are generated by the crystallographic threefold axis from monomers of 17 kDa that represent the asymmetric unit of the crystal. The overall appearance of the trimer is compact but the molecule shows pronounced loop structures surrounding a zinc binding site at the wider end of the trimer opposite to the location of the N- and C-termini. The latter are in direct neighbourhood within each monomer. Analysis of the secondary structure of 2Gmethyl-D-erythritol 2,4-cyclodiphosphate synthase with the program STRIDE (Frishman & Argos, 1995) shows a content of regular secondary structure of 26.5% β-strand and 28.4% α-helix. The secondary structural elements are depicted in Fig. 4 along with the used nomenclature. In the Ramachandran plot, 72.2% of the residues were found in the most favourable, 19.0% in the favourable and 4.8% in the generously allowed region as indicated by the program PROCHECK (Laskowski *et al.,* 1993). The general correctness of the model was further attested by the 'omit' density which appeared for the zinc ion and the substrate-like CDP molecule.

### Detailed description of the monomer

Each monomer consists of a large four-stranded β-sheet comprised of β-strands S1 and S4 to S6, a small two-stranded β-sheet formed by S2 and S3 and four α-helices H1 to H4 (Fig. 4). The four-stranded β-sheet with the topology 1-4-2-3 and strand directions up-down-up-up is located towards the trimer contacts with strands that run parallel to the trimer axis. α-helices H1 and H4 pack onto this β-sheet which in turn serve as support for α-helix H3. The small β-sheet which is inserted between β-strand S1 and α-helix H1 is oriented towards the solvent and packs on one end of the large β-sheet and between α-helices H1 and H4. α-helix H2 constitutes a single α-helical turn within a loop structure connecting α-helices H1 and H3. The coil region between α-helices H2 and H3 caps a pronounces cavity above the zinc binding site and displays considerable flexibility a shown by the relatively weak electron density and elevated temperature factors.

2C-methyl-D-erythritol 2,4-cyclodiphosphate synthase shares a structural module comprising the four-stranded β-sheet and two α-helices in the same topology with a number of proteins including the YjgF gene product (Volz, 1999)(PDB entry: 1QU9) (rmsd 3.1Å fpr 82 Cα-positions), phosphoribosyl-aminoimidazole synthetase (Li *et al.,* 1999)(1CLI) (rmsd 3.1Å for 94 Cα-positions) or chorismate mutase (Chook *et al.,* 1993)(2CHS) (rmsd 4.0Å for 73 Cα-positions). YjgF shows an extension of 25 amino acids at the N-terminus compared to 2*C-*methyl-D-erythritol 2,4-cyclodiphosphate synthase that form two additional β-strands, whereas chorismate mutase has a C-terminal extension that creates only one additional β-strand. Interestingly, 2C-methyl-D-erythritol 2,4-cyclodiphosphate synthase, YjgF and chorismate mutase form trimers where the monomer orientation displays a significant tilt due to packing of the additional b-strands.

In addition, glutamine phosphoribosylpyrophosphate amidotransferase (Muchmore *et al.,* 1998)(1ECF), 5-carboxymethyl-2-hydroxymuconate isomerase (Subramanya *et al.,* 1996)(1OTG) or zinc-dependent cytidine deaminase (Xiang *et al.,* 1995)(1CTT) show also a four-stranded β-sheet with two α-helices packed on one side, however with different topologies within the β-sheet, or a three-stranded β-sheet as in the case of the ribosomal protein L22 (Unge *et al.,* 1998)(1 BXE).

### Detailed description of the trimer

2C-methyl-D-erythritol 2,4-cyclodiphosphate synthase forms trimers that are generated by an exact crystallographic three-fold axis from the monomers (Fig. 5). The main trimer contacts are formed between the backside of the central four-stranded β-sheets, especially the terminal β-strands S1 and S5. They pack edge on against those of the neighbouring molecules in an almost perpendicular fashion so that no continuous β-barrel is formed from the β-sheets. The edge of β-strand S5 is exposed to the solvent where its C-terminal half contributes to binding of the CDP-moiety of the substrate. The contact between the β-sheets involves hydrophobic interactions (IIe3, Phe7, Val9, Ile99, Phe139, Ile146, Val151, Leu153) but also three internal salt bridges formed between Glul49 and His5 are present in the centre of each β-sheet. These contacts are closer at the C- and N-termini of the trimer whereas the contacts on the opposite side are mainly mediated by the loop that connects β-strands S5 and S6.

### The zinc binding site

During structure analysis of the nat2 structure, it became apparent that a metal ion was co-ordinated in a distorted tetrahedral geometry by Asp8, His10 and His42 which represent highly conserved residues within the protein family (Fig. 3). Asp8 appears as a bidentate ligand, His10 binds via N_{ε} and His42 via N_{δ}. The fourth ligand, presumably a water molecule (or hydroxyl ion) in the absence of substrate, can be replaced by chloride as observed in crystals grown from NaCl at pH 9.0 or by the β-phosphate of the substrates CDP-moiety. This ion was identified as tightly bound zinc by atomic absorption spectroscopy as described in the example section. About 0.9 mol zinc were found per mol of synthase monomer. The importance of this zinc ion and of its ligands for the catalytic activity of the synthase was demonstrated by the site-directed mutants Asp8Ser, His10Ser and His42Ser which were all enzymatically inactive. It should be noted that no extra zinc was added in any step from bacterial growth, to protein purification to crystallization for the preparation of the native nat2 crystal.

### Identification of the active site - complex with Mg-CDP (structure mgcdp2)

The active site was first identified in the structure of the complex between the synthase and cytidine diphosphate (CDP) both in the presence and the absence of magnesium ions (Fig. 6). CDP is a substructure of the substrate 4-diphosphocytidyl-2C-methyl-D-erythritol 2-phosphate. In the crystal form used herein, there are no crystal contacts to symmetry related trimers. This renders this crystal forms particularly suitable for soaking in or cocrystallizing the synthase with potential inhibitors, substrates or substrate analogs without disturbing crystal packing. In this respect, the high solvent content is advantageous as well.

The binding site for the elongated substrate molecule extends over two adjacent monomers. A pronounced and richly structured cavity that accommodates the substrate is formed opposite to the location of the N- and C-termini of the synthase. It involved the α-helices H1, H2 and adjacent residues, the N-terminus of H3 and parts of the loop connecting H2 and H3 of one molecule and the C-termini of β-strands S4 and S5 and the loop connecting S4 and α-helix H4 of another molecule. The active site itself is located in the vicinity of the zinc-ion where the β-phosphate of CDP is bound. This phosphate has replaced the water (or hydroxide) as the fouth ligand to the zinc ion, which renders it more nucleophilic. It follows that in the reaction of the synthase with its natural substrate, it will be the phosphate corresponding to the β-phosphate group of CDP that will be attacked by the terminal phosphate group of the substrate as a nucleophile to form the cyclic diphosphate. Therefore, residues in proximity are expected to directly contribute to the enzymatic reaction. Residues near the zinc-ion include the highly conserved residues Ser35, Asp46 and lle57. The active site is capped by a rather flexible segment comprising residues Pro62 to Ala71 including Asp63 which points towards the β-phosphate of CDP and to Ser35, suggesting a role in catalysis although it is not strictly conserved. Consequently, this flexible segment is better ordered in the complex than in the apo structure. From the position of the β-phosphate of CDP it can be predicted that the 2C-methyl-D-erythritol 2-phosphate-moiety of the substrate will be bound in the vicinity of Ser35 and Ile57 and will be in contact to the above mentioned flexible loop.

### The KATTTE-motif

The β-phosphate of CDP is anchored to the zinc-ion and completes its co-ordination sphere. The α-phosphate is bound by Thr133# (#denotes from another subunit, generated by threefold symmetry) of the KATTTE sequence motif (residues 130 to 135) located at the C-terminal end of β-strand S6. Lys130# forms an internal salt-bridge to Asp95# of the neighbouring β-strand S5 but has no contact to the substrate. The side-chain of Ala131 # points onto the side-face of the cytidyl-moiety and thereby helps to position the substrate. The side chain of Thr133# contacts the α-phosphate of the CDP molecule whereas Thr134# is buried in the interior of the protein. In addition, Thr133# supports the cytidyl-moiety, which is further bound by contacts to the side-chains of Lys104# and Leu106#, the latter being badly defined by electron density. N3 forms an H-bond to the peptide amide between Lys104# and Met105#. Glu135# functions as the protein ligand to the magnesium ion that bridges the α- and β-phosphates of CDP.

### The DIG-motif

The 2'- and 3'-hydroxyl groups of the CDP-ribose are bound to the carbonyl oxygen of Ala131# and by polar contacts to the carboxylate group Asp56 and van der Waals contacts to Gly58. Residue Ile57 forms one side of the cavity surrounding the zinc-ion where the 2C-methyl-D-erythritol 2-phosphate-moiety of substrate will presumably be located. Together these residues form the conserved DIG-motif (residues 56 to 58). The mutant Asp56Ser shows a decreased activity of 35% as compared to the native enzyme which indicates the importance of that contact to the CDP-ribose for binding but also that Asp56 is not directly involved in the enzymatic mechanism.
The cytidyl-moiety binds to a pocket formed by the C-terminal ends of the β-strands S4 and S5 and the loop structure that connects β-strand S4 and α-helix H4.

### Properties of the active site/substrate binding site

Based on the mgcdp2 structure, the active site/ substrate binding site can be subdivided into three distinct pockets (Fig. 7): a central pocket that surrounds the ribosyl 5'-diphosphate of CDP (pocket I), a pocket where the 2*C*-methyl-*D*-erythritol 2-phosphate-moiety of substrate will bind presumalby (pocket II), and a pocket for the cytidyl-moiety (pocket lll). Pocket II is capped by a relatively flexible loop (Pro62 to Ala71) which suggests an induced fit mechanism for the binding of that substrate part. This pocket shows both hydrophobic (lle57, Leu76) and hydrophilic (Ser35, Ser73 and Asp63) side chains in addition to polar backbone atoms of the contributing amino-acid chain. In contrast, the central and cytidyl- pocket appear rather static and show only minor changes in side chain orientation upon CDP binding for Glu136 and Leu106. The central pocket (I) is deep with the highly conserved Asp46 at its base which is surrounded by Asp56, Gly58 and lle57. The front entrance to the central cavity is framed by Lys104, The133, Glu135 and Asp63. The cytidyl-moiety is bound to pocket III formed by Ala131, Thr133, Lys104 and Leu106. It is not stacked between side chains but packs only with one face against Ala131.

Further, important information is drawn from the other complex structures. The complexes allow the description of interaction sites in pocket II which binds 2C-methyl-D-erythritol 2,4-cyclodiphosphate coordinated to zinc and in pocket III where the cytidyl-moiety is anchored. The central pocket I adjacent to the ribose and the diphosphate moiety of CDP is filled by three water molecules (number 506, 507 and 511 in structure sub2).

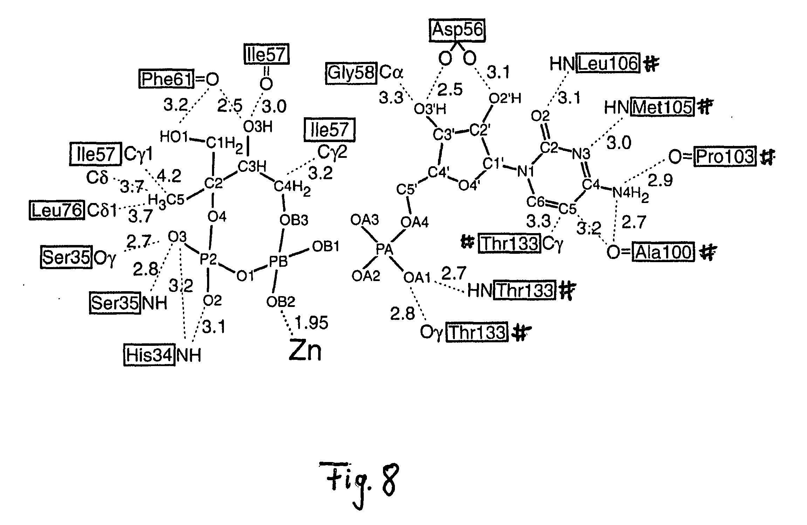

Based on the interactions observed in the product complex sub2 a pharmacophore can be described that mimics parts of the substrate and/or product. In detail, the following interaction sites of a potential inhibitor for substrate-like inhibitor molecules can be deduced. These interaction sites may interact with interaction sites of the active site of the synthase. Some of the interactions involved are schematically depicted in Fig. 8.
Cytosine (provides four interaction sites):
- carbonyl oxygen at positions 2,
- nitrogen N3,
- the amino group in position 4 and
- the carbon C5.
These interaction sites interact in a precise H-bonded network and van der Waals contacts with Leu106, Met105, Pro103, Ala100 and Thr133.

### Ribose (two interaction sites):

- C2 and C3 hydroxyl groups bind to the carboxylate group of Asp56, the C3 hydroxyl group forms a van der Waals contact to Cα of Gly58

### CMP alpha-phosphate (one interaction site):

- contacts the side-chain of Thr133 and a solvent molecule (507) in subsite I.

### Cyclodiphosphate product (six interaction sites, see also Fig. 8).

- hydrophobic sites at C4 and C5 interacting with the side chains of Ile57 and Leu76 (Fig. 8). Ile57 is highly conserved.
- hydrophilic sites at the 1-hydroxyl and 3-hydroxyl-groups interacting with Phe61, Ile57
- a hydrophilic/charged site at the P2 phosphate forming hydrogen bonds to His34 and Ser35. In the absence of substrate this site is occupied by a water molecule.
- the beta-phosphate PB which is a ligand to the zinc ion.

A potential inhibitor molecule may have moieties corresponding to at least three of these interaction sites, especially the hydrogen-bonding network of the cytidyl-moiety, a zinc ligand like the PB phosphate (or similar ligands like carboxylate or a hydroxamic acid moiety) and hydrophobic sites binding the C5-methyl group.

In addition to these interactions, subsite I that is not occupied by any of the analysed ligand molecules but filled with three water molecules may be used by an inhibitor molecule for interactions.

The crystals/structures subs, sub2 and cyt demonstrate that coordination to the zinc ioin is not essential for binding as the substructures cytidine or CMP have considerable affinity for the protein. The bound 2C-methyl-D-erythritol 2,4-cyclodiphosphate in the crystal sub2 shows that coordination of the zinc ion and the interactions described above in subsite II are also sufficient for binding.

### Properties of the site-directed mutants IspF-D8S, IspF-H10S and IspF-H42S

For confirmation of the crystal structure data the recombinant His-tagged site-directed mutant proteins IspF-D8S, IspF-H10 and lspF-H42S were prepared as described in the example section. As already described above, the amino acid residues Asp8, His10 and His42 are the co-ordinating ligands for the zinc-ion in the active site. The three site-directed mutants show less than 1 % activitity as compared to the recombinant His-tagged wild-type protein. Moreover, atomic absorption spectroscopy experiments show that these mutants contain less than 0.2 mol Zn per monomer of the synthase. These results clearly demonstrate for the first time that zinc is essential for the catalytic activity of this synthase.

### Methods of selecting or identifying potential inhibitors of 2C-methyl-D-erythritol 2,4-cyclodiphosphate synthase

The binding mode of CDP and of the other substrate analogs to the synthase indicates that they might be competitive inhibitors of the synthase. Other potential inhibitors may be identified using the structural information and the methods provided herein. Preferably, potential inhibitors are selected by their potential of binding to the active site. The active site comprises the three binding pockets I, II and III described above. Compounds which bind to at least one of these pockets can be expected to compete with binding of the substrate thus functioning as competitive inhibitors of the synthase. When selecting a potential inhibitor by rational drug design or computer modeling, the 3D structure of the synthase is loaded from a data storage device into a computer memory and may be displayed (generated) on a computer screen using a suitable computer program. Preferably, only a subset of interest of the coordinates of the whole structure of the synthase is loaded in the computer memory or displayed on the computer screen. This subset of interest may comprise the coordinates of active site residues and/or those which make up a binding cavity (pocket) of the synthase and the above mentioned zinc ion. This subset may be called a criteria data set; this subset of atoms may be used for designing an inhibitor. It may contain amino acid residues of more than one synthase monomer and may comprise at least some of the following amino acid residues:
Ala131# contacting the face of the cytidyl moiety;
Ala131# bonding with its carbonyl oxygen to at least one of the 2'-and 3'-hydroxyl groups of the cytidyl moiety;
Asp56 making a hydrogen bond with its carboxyl group to at least one of the 2'-and 3'-hydroxyl groups of the cytidyl moiety;
Gly58 making van der Waals contact with its Cα to at least one of the the 2'-and 3'-hydroxyl groups of the cytidyl moiety;
peptide group between Lys104# and Met105# hydrogen bonding to N3 of the cytidyl moiety;
Thr133# supporting the cytidyl moiety and hydrogen bonding with its γ-O or its backbone NH to the α-phosphate;
Lys104# contacting with its side chain the cytidyl moiety;
Leu106# contacting with its side chain the cytidyl moiety;
Leu106# hydrogen bonding with its NH to the carbonyl oxygen of the cytidyl moiety;
Asp63 binding to the β-phosphate of cytidine diphosphate;
His34 hydrogen bonding with its backbone NH group to at least one oxygen atom of the P2 phosphate group of 2C-methyl-D-erythritol 2,4-cyclodiphosphate;
Ser35 hydrogen bonding with its backbone NH group to one oxygen atom of the P2 phosphate group;
Ser35 hydrogen bonding with its γ-OH to one of the oxygen atoms of the P2 phosphate group;
Leu76 making a van der Waals contact with its δ-C to the 2-methyl group;
Ile57 making a van der Waals contact with δ-C to the 2-methyl group;
Ile57 making a van der Waals contact with γ-C to the 2-methyl group;
Phe61 hydrogen bonding with its backbone carbonyl oxygen to the 1-hydroxyl group;
Phe61 hydrogen bonding with its backbone carbonyl oxygen to the 3-hydroxyl group;
Ile57 hydrogen bonding with its backbone carbonyl oxygen to the 3-hydroxyl group;
Ile57 making van der Waals contact with its γ-C to the carbon at the 4-position;
Pro100# hydrogen bonding with its backbone carbonyl oxygen to the amino group of the cytidyl moiety;
Ala100# hydrogen bonding with its backbone carbonyl oxygen to the amino group of the cytidyl moiety;
Ala100# supporting with its backbone carbonyl oxygen the C5 position of the cytidyl moiety.
Amino acids not denoted by # belong to one subunit and those denoted by # belong to another subunit.

A potential inhibitor may then be designed de novo by rational drug design in conjunction with computer modelling. Models of chemical structures or molecule fragments may be generated on a computer screen using information derived from known low-molecular weight organic chemical structures stored in a computer data base or are built using the general knowledge of an organic chemist regarding bonding types, conformations etc. Suitable computer programs may aid in this process in order to build chemical structures of realistic geometries. Chemical structures or molecule fragments may be selected and/or used to construct a potential inhibitor such that favorable interactions to said subset or criteria data set become possible. The more favorable interactions become possible, the stronger the potential inhibitor will bind to the synthase. Preferably, favorable interactions to at least three amino acid residues should become possible. Favorable interactions are any non-covalent attractive forces which may exist between chemical structures such as hydrophobic or van-der-Waals interactions and polar interactions such as hydrogen bonding, salt-bridges etc. Unfavorable interactions such as hydrophobic-hydrophilic interactions should be avoided but may be accepted if they are weaker than the sum of the attractive forces. Steric interference such as clashes or overlaps of portions of the inhibitor being selected or constructed with protein moieties will prevent binding unless resolvable by conformational changes. The binding strength of a potential inhibitor thus created may be assessed by comparing favorable and unfavorable interactions on the computer screen or by using computational methods implemented in commercial computer programs.

Conformational freedom of the potential inhibitor and amino acid side chains of the synthase should be taken into account. Accessible conformations of a potential inhibitor may be determined using known rules of molecular geometry, notably torsion angles, or computationally using computer programs having implemented procedures of molecular mechanics and/or dynamics or quantum mechanics or combinations thereof.

A potential inhibitor is at least partially complementary to at least a portion of the active site of the synthase in terms of shape and in terms of hydrophilic or hydrophobic properties.

Databases of chemical structures (e.g. cambridge structural database or from Chemical Abstracts Service; for a review see: Rusinko (1993) Chem. Des. Auto. News 8, 44-47) may be used to varying extents. In a totally automatic embodiment, all structures in a data base may be compared to the active site or to the binding pockets of the synthase for complementarity and lack of steric interference computationally using the processor of the computer and a suitable computer program. In this case, computer modeling which comprises manual user interaction at a computer screen may not be necessary. Alternatively, molecular fragments may be selected from a data base and assembled or constructed on a computer screen e.g. manually. Also, the ratio of automation to manual interaction by a person skilled in the art in the process of selecting may vary a lot. As computer programs for drug design and docking of molecules to each other become better, the need for manual interaction decreases.

A preferred approach of selecting or identifying potential inhibitors of the synthase makes use of the structure of the synthase-CDP complex of this invention. CDP is a fragment of the natural substrate and is a competitive inhibitor of the synthase as it binds to a portion of the active site. A potential inhibitor may be more easily found based on the structure and conformation of CDP bound to the active site of the synthase than based on complementarity to the active site. Thus, CDP may be a determinant of the structure of an inhibitor. Analogously to the principles of drug design and computer modeling outlined above, chemical structures or fragments thereof may be selected or constructed based on similarity to the structure of CDP bound to the synthase. CDP may even be used as a starting inhibitor. Models of chemical structures taken from a data base or constructed by modeling on the computer screen may be added to CDP. Alternatively, fragments of CDP may be exchanged by other fragments or chemical structures in order to improve the inhibitory function and/or in order to improve the suitability of the potential inhibitor thus obtained for pharmaceutical purposes. Putative inhibitors so obtained are 4-diphosphocytidyl-erythritol or 5-diphosphocytidyl-ribitol and derivatives, notably the 2-phosphates from these compounds.

The binding mode of CDP to the synthase and the information derivable therefrom regarding the mechanism of the reaction catalyzed by the synthase may be used to design inhibitors. Preferably, the zinc ion or the magnesium ion are used in such considerations.

Potential inhibitors may be selected or designed such that they interfere with said zinc or said magnesium ion. Such inhibitors may e.g. prevent binding of these metal ions or they may chelate them out of the synthase.

Another preferred approach is the design of mechanism-based inhibitors like suicide inhibitors which modify the synthase when turning over with the inhibitor.

Possible approaches for rational drug design are described extensively in the literature. See e.g. Meng et al. (1992) J. Comp. Chem. 505-524; Cohen et al. (1990) J. Med. Chem. 33, 883-894; Navia and Murcko (1992) Current Opinion in Structural Biology, 202-210; A. Parrill: rational drug design, ACS symposium series 719, American Chemical Society, 1999, Washington DC; Chemical and structural approaches to rational drug design, eds.: D. Weiner and W. Williams, CRC Press, 1995, Boca Raton.

Programs usable for computer modelling include Quanta (Molecular Simulations, Inc.) and Sibyl (Tripos Associates). Other useful programs are Autodock (Scripps Research Institute, La Jolla, described in Goodsell and Olsen (1990) Proteins: Structure, Function and Genetics, 8, 195-201), Dock (University of California, San Francisco, described in: Kuntz et al. (1982) J. Mol. Biol. 161, 269-288.

### Experimental assessment of potential inhibitors

Potential inhibitors may be assessed experimentally for binding to the synthase and/or for their inhibitory action on the catalytic activity of the synthase. The potential inhibitors can be synthesized according to the methods or organic chemistry. Preferably, compounds from a database have been selected without remodelling, since their synthesis may already be known. In any event, the synthetic effort needed to find an inhibitor is greatly reduced by the achievements of this invention due to the preselection of promising inhibitors by the above methods. Binding of a potential inhibitor may be determined after contacting the potential inhibitor with the synthase. This may be done crystallographically by soaking a crystal of the synthase with the potential inhibitor or by cocrystallisation and determining the crystal structure of the complex. Preferably, binding may be measured in solution according to methods known in the art. More preferably, inhibition of the catalytic activity of the synthase by the inhibitor is determined e.g. using the assays described in the examples section.

The activity of the synthase may be measured by determining the consumption of 4-diphosphocytidyl-2C-methyl-D-erythritol 2-phosphate and/or formation of 2C-methyl-D-erythritol 3,4-cyclopyrophosphate. Alternatively, consumption of 4-diphosphocytidyl-2C-methyl-D-erythritol and/or production of 2C-methyl-D-erythritol 3,4-cyclomonophosphate may be determined. The measurement may be carried out either directly with the reaction mixture or after the separation of the reaction mixture by chromatography, such as HPLC. The reaction should preferably be carried out at a pH of 5.5 to 9, preferably 7 to 8.5, in the presence of Mn²⁺ or Mg²⁺. Extra zinc may also be added. The temperature is preferably in the range of ±10 °C from the optimum temperature. The start of this reaction can be timed by the addition of the last of the essential components e.g. the synthase or substrate. The reaction can be stopped by methanol, chelating agents, like EDTA or acids like trichloro acetic acid. The activity of the synthase may be expressed as the amount of substrate comsumed or product produced in a specified period of time and under specified conditions. It may be advantageous to label the substrate by 32-phosphorous, 14-carbon, 13-carbon, deuterium or tritium in order to measure substrate consumption or product formation by a radio detector. These labeling types may be used alone or in any combination. Preparation of labeled substrates and their use in activity assays is described in detail in WO 01/11055.

Inhibition by potential inhibitor may be determined by repeating an activity assay in the presence of a predetermined concentration of a potential inhibitor and comparing the obtained activities of the synthase. An inhibitor may be tested at several different concentrations. Further, the type of inhibition e.g. competitive, non-competitive, un-competitive or irreversible may be determined according to known methods. Assays are known from WO 01/11055.

The asymmetric unit of the crystal of space group 12(1)3 of this invention contains one monomer of the synsthase. The functional trimer which is also present in solution is generated by the symmetry operations (x,y,z), (y,z,x) and (z,x,y). Therefore three substrate binding sites/active sites are present in the trimer. Two subunits contribute to each substrate binding site.

The invention will now be described in detail with reference to specific examples.

### Example 1

### Production of an expression clone and construction of an expression vector for the expression of 2C-methyl-D-erythritol 2,4-cyclodiphosphate synthase (IspF)

The *E. coli* ORF *ispF* (accession no. gb AE000358) from bp position 6231 to 6754 is amplified by PCR using chromosomal *E. coli* DNA as template. Chromosomal DNA from *Escherichia coli* strain XL1-Blue (Bullock et al. 1987; commercial source: Stratagene, LaJolla, CA, USA) is isolated according to a method described by Meade *et al.,* 1982.

The reaction mixture contains 10 pmol of the primer 5'-GAGAAGGATCCATGCGAATTGGACACGGTTTTGACG-3', 10 pmol of the primer 5'-TATTATCTGCAGCCTTGCGGTTTACCGTGGAGG-3', 20 ng of chromosomal DNA, 2 U of Taq DNA polymerase (Eurogentec, Seraing, Belgium) and 20 nmol of dNTPs in a total volume of 100 µl containing 1.5 mM MgCl₂, 50 mM KCl, 10 mM Tris-hydrochloride, pH 8.8 and 0.1 % (w/w) Triton X-100.

The mixture is denaturated for 5 min at 94 °C. Then 30 PCR cycles for 30 sec at 94 °C, 45 sec at 50 °C and 45 sec at 72 °C followed. After further incubation for 7 min at 72 °C, the mixture is cooled to 4 °C. An aliquot of 2 µl is subjected to agarose gel electrophoresis.

The PCR amplificate is purified with the PCR purification kit from Qiagen (Hilden, Germany).

1.0 µg of the vector pQE30 (Qiagen) and 0.5 µg of the purified PCR product are digested in order to produce DNA fragments with overlapping ends. Each restriction mixture contains 10 µl of NEB3 buffer from New England Biolabs (NEB), 100 U of *BamHI* (NEB), 100 U of *Pst*l (NEB) in a total volume of 100 µl and is incubated for 3 h at 37°C. Digested vector DNA and PCR product are purified using the PCR purification kit from Qiagen.

20 ng of the vector DNA and 13 ng of the purified PCR product are ligated together with 1 U of T4-Ligase (Gibco), 2 µl of T4-Ligase buffer (Gibco) in a total volume of 10 µl, yielding the plasmid pQEispF. The ligation mixture is incubated for 2 h at 25°C. 1µl of the ligation mixture is transformed into electrocompetent *E*. *coli* XL1-Blue cells according to a method described by Dower *et al.,* 1988. The plasmid pQEispF is isolated with the plasmid isolation kit from Qiagen.

The DNA insert of the plasmid pQEispF is sequenced by the automated dideoxynucleotide method (Sanger *et al*., 1992) using an ABI Prism 377^{™} DNA sequencer from Perkin Elmer (Norwalk, USA) with the ABI Prism^{™} Sequencing Analysis Software from Applied Biosystems Divisions (Foster city, USA). It is identical with the DNA sequence of the database entry (gb AE000358). The 5'-end of the DNA insert carries the coding region for 6 histidine residues.

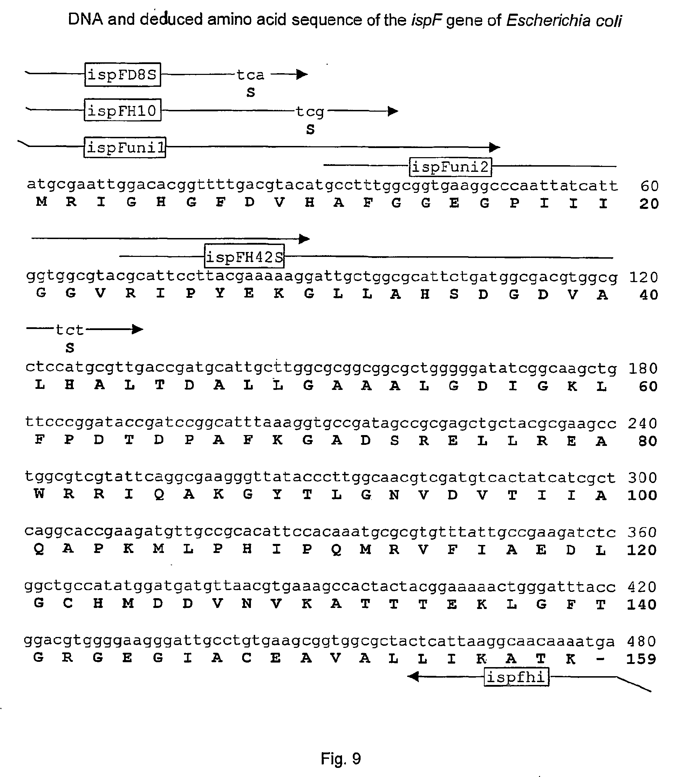

The DNA sequence and corresponding amino acid sequence of the *ispF* gene of *E. coli* are shown in Fig. 9.

### Example 2

### Production of an expression clone and construction of an expression vector for the expression of the site directed 2C-methyl-D-erythritol 2,4-cyclodiphosphate synthase mutant Asp8Ser

A DNA fragment containing the *ispF* gene carrying the D8S mutation is generated by PCR using the plasmid pQEispF as template (Fig. 9).
The reaction mixture contains 10 pmol of primer ispFD8S 5'-CCTGACGGATCCATGCGAATTGGACACGGTTTTTCAGTAC-3', 10 pmol of the primer ispFhi 5'-TATCAACTGCAGTCATTTTGTTGCCTTAATGAG-3', 2 ng of pQEispF DNA, 2 U of Taq DNA polymerase (Eurogentec, Seraing, Belgium) and 20 nmol of dNTPs in a total volume of 100 µl containing 1.5 mM MgCl₂,50 mM KCI, 10 mM Tris-hydrochloride, pH 8.8 and 0.1 % (w/w) Triton X-100.

The mixture is denaturated for 5 min at 94 °C. Then 30 PCR cycles for 30 sec at 94 °C, 45 sec at 50 °C and 45 sec at 72 °C follow. After further incubation for 7 min at 72 °C, the mixture is cooled to 4 °C. An aliquot of 2 µl is subjected to agarose gel electrophoresis.

The PCR amplificate is purified with the PCR purification kit from Qiagen (Hilden, Germany).
1.0 µg of the vector pQE30 (Qiagen) and 0.5 µg of the purified PCR product are digested in order to produce DNA fragments with overlapping ends. Each restriction mixture contains 10 µl of NEB3 buffer from New England Biolabs (NEB), 100 U of *BamHI* (NEB), 100 U of *Pst*I (NEB) in a total volume of 100 µl and is incubated for 3 h at 37 °C. Digested vector DNA and PCR product are purified using the PCR purification kit from Qiagen.

20 ng of the vector DNA and 12 ng of the purified PCR product are ligated together with 1 U of T4-Ligase (Gibco), 2 µl of T4-Ligase buffer (Gibco) in a total volume of 10 µl, yielding the plasmid pQEispFD8S. The ligation mixture is incubated for 2 h at 25 °C. 1 µl of the ligation mixture is transformed into electrocompetent *E. coli* XL1-Blue cells. The plasmid pQEispFD8S is isolated with the plasmid isolation kit from Qiagen.

The DNA insert of the plasmid pQEispFD8S is sequenced and is found to be as expected (Fig. 9). The 5'-end of the DNA insert carries the coding region for 6 histidine residues.

### Example 3

### Production of an expression clone and construction of an expression vector for the expression of the site directed 2C-methyl-D-erythritol 2,4-cyclodiphosphate synthase mutant His10Ser

A DNA fragment containing the *ispF* gene carrying the H10S mutation is generated by PCR using the plasmid pQEispF as template (Fig. 9).

The reaction mixture contains 10 pmol of primer ispFH10S 5'-CCTGACGGATCCATGCGAATTGGACACGGTTTTGACGTATCGGCCTTTGG-3', 10 pmol of the primer ispFhi 5'-TATCAACTGCAGTCATTTTGTTGCCTTAATGAG-3', 2 ng of pQEispF DNA, 2 U of Taq DNA polymerase (Eurogentec, Seraing, Belgium) and 20 nmol of dNTPs in a total volume of 100 µl containing 1.5 mM MgCl₂, 50 mM KCl, 10 mM Tris-hydrochloride, pH 8.8 and 0.1 % (w/w) Triton X-100.

The mixture is denaturated for 5 min at 94 °C. Then 30 PCR cycles for 30 sec at 94 °C, 45 sec at 50°C and 45 sec at 72 °C follow. After further incubation for 7 min at 72 °C, the mixture is cooled to 4 °C. An aliquot of 2 µl is subjected to agarose gel electrophoresis.

The PCR amplificate is purified with the PCR purification kit from Qiagen.

1.0 µg of the vector pQE30 (Qiagen) and 0.5 µg of the purified PCR product are digested in order to produce DNA fragments with overlapping ends. Each restriction mixture contains 10 µl of NEB3 buffer from New England Biolabs (NEB), 100 U of *BamH*I (NEB), 100 U of *Pst*l (NEB) in a total volume of 100 µl and is incubated for 3 h at 37 °C. Digested vector DNA and PCR product are purified using the PCR purification kit from Qiagen.

20 ng of the vector DNA and 12 ng of the purified PCR product are ligated together with 1 U of T4-Ligase (Gibco), 2 µl of T4-Ligase buffer (Gibco) in a total volume of 10 µl, yielding the plasmid pQEispFH10S. The ligation mixture is incubated for 2 h at 25 °C. 1 µl of the ligation mixture is transformed into electrocompetent *E. coli* XL1-Blue cells. The plasmid pQEispFH10S is isolated with the plasmid isolation kit from Qiagen.

The DNA insert of the plasmid pQEispFH10S is sequenced and is found to be as expected (Fig. 9). The 5'-end of the DNA insert carries the coding region for 6 histidine residues.

### Example 4

### Production of an expression clone and construction of an expression vector for the expression of the site directed 2C-methyl-D-erythritol 2,4-cyclodiphosphate synthase mutant His42Ser

A DNA fragment containing the *ispF* gene carrying the H42S mutation is generated by PCR using the plasmid pQEispF as template (Fig. 9).

The reaction mixture contains 10 pmol of primer ispFH42S 5'-CGCATTCCTTACGAAAAAGGATTGCTGGCGCATTCTGATGGCGACGTGGCGCTCTCTGC GTTG-3', 10 pmol of the primer ispFhi 5'-TATCAACTGCAGTCATTTTGTTGCCTTAATGAG-3', 2 ng of pQEispF DNA, 2 U of Taq DNA polymerase (Eurogentec, Seraing, Belgium) and 20 nmol of dNTPs in a total volume of 100 µl containing 1.5 mM MgCl₂, 50 mM KCl, 10 mM Tris-hydrochloride, pH 8.8 and 0.1 % (w/w) Triton X-100.

The mixture is denaturated for 5 min at 94 °C. Then 30 PCR cycles for 30 sec at 94 °C, 45 sec at 50 °C and 45 sec at 72 °C follow. After further incubation for 7 min at 72 °C, the mixture is cooled to 4 °C. An aliquot of 2 µl is subjected to agarose gel electrophoresis.

The PCR amplificate is purified with the PCR purification kit from Qiagen.

The PCR amplificate is used as template for a second PCR reaction. The reaction mixture contained 25 pmol of primer ispFuni2 5'-GCCTTTGGCGGTGAAGGCCCAATTATCATTGGTGGCGTACGCATTCCTTACGAAAAAGG-3', 25 pmol of primer ispFhi 5'-TATCAACTGCAGTCATTTTGTTGCCTTAATGAG-3', 2 µl of the first PCR amplification , 2U of Taq DNA polymerase (Eurogentec, Seraing, Belgium) and 20 nmol of dNTPs in a total volume of 100 µl containing 1.5 mM MgCl₂, 50 mM KCl, 10 mM Tris-hydrochloride, pH 8.8 and 0.1 % (w/w) Triton X-100.

The mixture is denaturated for 3 min at 95 °C. Then 40 PCR cycles for 45 sec at 94 °C, 45 sec at 50 °C and 60 sec at 72 °C follow. After further incubation for 20 min at 72 °C, the mixture is cooled to 4 °C. An aliquot of 2 µl is subjected to agarose gel electrophoresis. An aliquot of 2 µl is subjected to agarose gel electrophoresis.

The PCR amplificate is purified with the PCR purification kit from Qiagen.

The PCR amplificate is used as template for a second PCR reaction. The reaction mixture contained 25 pmol of primer ispFuni1 5'-CCTGACGGATCCATGCGAATTGGACACGGTTTTGACGTACATGCCTTTGGCGGTGAA-3', 25 pmol of primer ispFhi 5'-TATCAACTGCAGTCATTTTGTTGCCTTAATGAG-3', 2 µl of the second PCR amplification , 2U of Taq DNA polymerase (Eurogentec, Seraing, Belgium) and 20 nmol of dNTPs in a total volume of 100 µl containing 1.5 mM MgCl₂, 50 mM KCl, 10 mM Tris-hydrochloride, pH 8.8 and 0.1 % (w/w) Triton X-100.

The mixture is denaturated for 3 min at 95 °C. Then 40 PCR cycles for 45 sec at 94°C, 45 sec at 50 °C and 60 sec at 72 °C follow. After further incubation for 20 min at 72°C, the mixture is cooled to 4 °C. An aliquot of 2 µl is subjected to agarose gel electrophoresis. An aliquot of 2 µl is subjected to agarose gel electrophoresis.

The PCR amplificate is purified with the PCR purification kit from Qiagen.

1.0 µg of the vector pQE30 (Qiagen) and 0.5 µg of the purified PCR product are digested in order to produce DNA fragments with overlapping ends. Each restriction mixture contains 10 µl of NEB3 buffer from New England Biolabs (NEB), 100 U of *BamH*l (NEB), 100 U of *Pst*l (NEB) in a total volume of 100 µl and is incubated for 3 h at 37 °C. Digested vector DNA and PCR product are purified using the PCR purification kit from Qiagen.

20 ng of the vector DNA and 12 ng of the purified PCR product are ligated together with 1 U of T4-Ligase (Gibco), 2 µl of T4-Ligase buffer (Gibco) in a total volume of 10 µl, yielding the plasmid pQEispFH42S. The ligation mixture is incubated for 2 h at 25 °C. 1 µl of the ligation mixture is transformed into electrocompetent *E*. *coli* XL1-Blue cells. The plasmid pQEispFH42S is isolated with the plasmid isolation kit from Qiagen.

The DNA insert of the plasmid pQEispFH42S is sequenced and is found to be as expected (Fig. 9). The 5'-end of the DNA insert carries the coding region for 6 histidine residues.

### Example 5

### Determination of 2C-methyl-D-erythritol 2,4-cyclodiphosphate synthase activity

Assay mixtures contain 50 mM potassium phosphate, pH 7.0, 2 mM DTT, 2 mM MgCl₂, 1 mM 4-diphosphocytidyl-2C-methyl-D-erythritol 2-phosphate and protein in a total volume of 100 µl. The mixtures are incubated at 37 °C for 20 min. The reactions are terminated by adding EDTA at a final concentration of 4 mM. The samples are centrifuged and the supernatant is analyzed by HPLC using a column of Multospher 120 RP 18-AQ-5 (4.6 x 250 mm, CS-Chromatographic Service GmbH) that has been equilibrated and run with Gradient No. 3 at a flow rate 1 ml min⁻¹ (see below). The effluent is monitored photometrically (270 nm).

One unit of 2C-methyl-D-erythritol 2,4-cyclodiphosphate synthase activity is defined as the amount of protein which produced 1 µmol min⁻¹ of CMP.

| | | |
|---|---|---|
| Column: | Multospher 120 RP 18-5 AQ, 250 x 4.6 mm, particle size 5 µm (CS: Chromatographic Service GmbH, Langerwehe, Germany) | |
| Mobile phase: | Eluent A | 10 mM TBAS in distilled H₂O |
| | Eluent B | 10 mM TBAS in 70 % (v/v) Methanol |

### Gradient No.3

| Time (min) | A [%] | B [%] |
|---|---|---|
| 0 | 100 | 0 |
| 20 | 40 | 60 |
| 25 | 0 | 100 |
| 26 | 100 | 0 |
| 30 | 100 | 0 |

### Example 6

### Purification of 2C-methyl-D-erythritol 2,4-cyclodiphosphate synthase and site-directed mutants thereof

The procedure described herein is used for the purification of 2C-methyl-D-erythritol 2,4-cyclodiphosphate synthase and the site directed mutant proteins D8S, H10S and H42S.
Cells of the recombinant *E*. *coli* strain XL1-pQEispF (7.3 g) are suspended in 35 ml of standard buffer (20 mM potassium phosphate pH 7.0, 0.5 M NaCl) containing 20 mM imidazole. Cell extract is performed by ultrasonication. The suspension is centrifuged at 16,000 rpm for 30 min. The supernatant was collected. The cell extract (806 mg) was loaded on top of a Ni²⁺-Chelating Sepharose column (1.6 x 6.0 cm, Amersham Pharmacia Biotech) which has been equilibrated with 20 mM imidazole in standard buffer at a flow rate of 3 ml min-¹. The column is washed with 100 ml of 20 mM imidazole in standard buffer. The enzyme is eluted by a linear gradient of 20-500 mM imidazole in buffer D (total volume 300 ml). The enzyme is eluted at 100-200 mM imidazole. Fractions are combined according to SDS-PAGE. The 2C-methyl-D-erythritol 2,4-cyclodiphosphate synthase protein migrates as a single band at a molecular mass of about 17 kDa. The pooled fractions are desalted on HiPrep desalting column (size 2.6 x 10 cm, Amersham Pharmacia Biotech) at a flow rate of 5 ml min⁻¹. The protein is eluted at a volume of 15 ml. Protein fractions are pooled.

### Example 7

### Flame Atomic Absorption Spectrometry (Flame-AAS) of 2C-methyl-D-erythritol 2,4-cyclodiphosphate synthase and site-directed mutants thereof

The protein mixture is diluted with water and 6 M HCl to a final concentration of 200 nM and a final concentration of 2 M. The aqueous solution is then incubated at 90 °C for 5 hours. 2 ml of this solution per measurement are then taken up by an Unicam 919 Flame-AAS. For the acquiration of one datapoint usually 3 measurments are averaged. To determine the background emission of amino acids, a peptone solution has been prepared and measured using similar procedures.

### Example 8

### Crystallization of 2C-methyl-D-erythritol 2,4-cyclodiphosphate synthase

The protein solution contains 20 mM potassium phosphate pH 7.0 and 10 mg ml⁻¹ of purified recominant 2C-methyl-D-erythritol 2,4-cyclodiphosphate synthase from recombinant *E*. *coli*. This solution is then applied onto a crystallization plate (CCLEAR-D/1, Douglas instruments, UK) and mixed 1:1 with a crystallization buffer containing 0.1 M HEPES pH 7.5 and 2 M ammonium formate. This mixture is then supported with ten times of volume of a base buffer, similar to the precipitation buffer. The crystallization plate is then sealed with Crystal-Clear^{®} tape and stored in a climate regulated room.
Micro and macro seeding techniques were then employed in order to produce more crystals.

### Example 9

### Cryo-protection and freezing of crystals and data collection

Crystals containing the snythase and zinc were incubated for one hour with about 5mM of a ligand in mother liquor containing 2M ammonium formate, 0.1M HEPES/NaOH at pH 7.0 and 20% D-(-)-2,3-butanediole (purchased from FLUKA) as a cryo-protectant. Crystals were mounted in a nylon-loop and flash frozen in a stream of nitrogen at 100Kelvin with an Oxford cryo stream. The quality of the crystals was not reduced by freezing and the mosaicity was only slightly increased from about 0.3° to 0.5°. Diffraction data were measured at that temperature without any indication of radiation damage.

### Example 10

### Soaking of 2C-methyl-D-erythritol 2,4-cyclodiphosphate synthase crystals with Mg-CDP

In order to obtain a crystal structure of 2*C*-methyl-*D*-erythritol 2,4-cyclodiphosphate synthase in complex with CDP, the synthase was crystallized according to example 8. A crystal of suitable size was then soaked in crystallization buffer (see example 8) further containing 5 mM CDP and 5 mM MgCl₂.

### Example 11

### Structure solution of 2C-methyl-D-erythritol 2,4-cyclodiphosphate synthase

2C-methyl-D-erythritol 2,4-cyclodiphosphate synthase was crystallized in the cubic space group 12(1)3 with a = 144.15Å. A native data set was collected on a rotating anode generator equipped with an *MARresearch* Image Plate detector at room temperature. The overall R_{merge} was 7.4% in the resolution range 20.0 to 2.85Å and 40.1 % in the outermost resolution shell. These date were 99.7% complete with a mean redundancy of 4.3 (Table 1). For structure solution a crystal was incubated with 2 mM mercury-(II)-acetate in mother liquor for one hour yielding an isomorphous derivative with a single heavy atom binding site in the asymmetric unit. The isomorphous difference was 25.3% in the resolution range 25.0 to 3.6Å. This derivative was interpreted with the program SHELXS (Sheldrick *et al.,* 1993). Heavy atom parameters were refined and phases calculated with the program MLPHARE (Collaborative Computational Project No. 4, 1994). The phasing power was 1.0 (25 to 3.6Å) with a figure of merit of 0.27. Phases were improved by solvent flattening using the program DM (Collaborative Computational Project No. 4, 1994) along with phase extension. An atomic model was built with the program MAIN (Turk, 1992) and refined with the program XPLOR (Brünger *et al.,* 1998). The model comprises residues Met1 to Ile155. The electron density is continuous throughout the model with the exception of a short break between Phe61 and Pro62 in a loop structure comprising Phe61 to Lys69. Refinement data and statistics are given in Table 1.

### Example 12

### Structure solution of 2C-methyl-D-erythritol 2,4-cyclodiphosphate synthase in complex with CDP

The starting model for the determination of the structure of the complex was the structure of the synthase without a substrate analoge. Initially a rigid body refinement of the starting model using F_{obs} from a crystal cocrystallized with CDP was carried out followed by several cycles of positional refinement with X-PLOR against the new F_{obs}. In electron density maps, the elongated structure of CDP was discernible and was modeled into the density. After further refinement cycles, a spherical electron density was found in coordinating distance to the phosphate groups of CDP and was interpreted as a magnesium ion.

### Example 13

### Screening for 2C-methyl-D-erythritol 2,4-cyclodiphosphate synthase activity

Synthase activity is screened for by a radiochemical method. Assay mixtures contained 100 mM tris hydrochloride pH 8.0, 10 mM MnCl₂, 14 nCi of [2-¹⁴C]4-diphosphocytidyl-2C-methyl-D-erythritol and 2 µg of the synthase from recombinant *E. coli.* They are incubated at 37°C for 30 min. After centrifugation, aliquots are spotted on Sil-NHR thin layer plates which are developed with a mixture of n-propanol/ethyl acetate/H₂O (6:1:3, v/v). The radiochromatogram is monitored and evaluated by Phosphor Imager (Storm 860, Molecular Dynamics,USA). The R_{f} value of the synthase product is 0.5. This screening method can be carried out in the presence or absence of prospective inhibitors.

### Screening of synthase activity by NMR

A solution containing 100 mM Tris HCl pH 8.0, 10 mM MnCl₂, 5 mM of 4-diphosphocytidyl-2C-methyl-D-erythritol and 0.1 mg of synthase from recombinant *E. coli* are incubated at 37° C for 1 h. The reaction is monitored by ³¹P-NMR. ³¹P-NMR spectra are recorded using a AC 250 spectrometer from Bruker at a transmitter frequency of 101.3 MHz. The chemical shifts are referenced to external 85 % H₃PO₄. The screening method is carried out in the presence or absence of prespective inhibitors by measuring the residual starting material and comparing the results.

The product 2C-methyl-D-erythritol 3,4-cyclomonophosphate displays one ³¹P singlet at +21.7 (see WO 01/11055 for an NMR characterization of this product). Synthase activity can therefore also be determined by measuring this signal for determining the amount of product.

### Literature

Brünger, A. T., Adams, P. D., Clore, G. M., DeLano, W. L., Gros, P., Grosse-Kunstleve, R. W., Jiang, J. S., Kuszewski, J., Nilges, M., Pannu, N. S., Read, R. J., Rice, L. M., Simonson, T. & Warren, G. L. (1998). Crystallography & NMR system: A new software suite for macromolecular structure determination. Acta Crystallogr. D54, 905-921.
Bullock, W. O.; Fernandez, J. M., & Short, J. M. (1987). XL1-Blue: a high efficiency plasmid transforming recA Escherichia coli with β-galactosidase selection. BioTechniques 5, 376-379.
Chook, Y. M., Ke, H. & Lipscomb, W. N. (1993). Crystal structures of the monofunctional chorismate mutase from Bacillus subtilis and its complex with a transition state analog. Proc. Natl. Acad. Sci. U S A 90, 8600-8603.
Collaborative Computational Project No. 4. (1994). The CCP4 suite: programs for protein crystallography. Acta Crystalogr. D50, 760-763.
Dower, W. J., Miller, J. F., & Ragsdale, C. (1988) High efficiency transformation of E. coli by high voltage electroporation. Nucleic Acids Res. 16, 6127-6145.
Frishman, D. & Argos, P. (1995). Knowledge-based protein secondary structure assignment. Proteins 23, 566-579.
Herz, S., Wungsintaweekul, J., Schuhr, C. A., Hecht, S., Lüttgen, H., Sagner, S., Fellermeier, M., Eisenreich, W., Zenk, M. H., Bacher, A. & Rohdich, F. (2000). Biosynthesis of terpenoids: YgbB protein converts 4-diphosphocytidyl-2C-methyl-D-erythritol 2-phosphate to 2C-methyl-D-erythritol 2,4-cyclodiphosphate. Proc. Natl. Acad. Sci. USA 97, 2486-2490.
Laskowski, R. A., MacArthur, M. W., Moss, D. S. & Thornton, J. M. (1993). PROCHECK: a program to check the stereochemical quality of protein structures. J. Appl. Crystallogr. 26, 283-291.
Li, C., Kappock, T. J., Stubbe, J., Weaver, T. M. & Ealick, S. E. (1999). X-ray crystal structure of aminoimidazole ribonucleotide synthetase (Purm), from the Escherichia coli purine biosynthetic pathway at 2.5 A Resolution. Structure 7, 1155-166.
Lüttgen, H., Rohdich, F., Herz, S., Wungsintaweekul, J., Hecht, S., Schuhr, C. A., Fellermeier, M., Sagner, S., Zenk, M. H., Bacher, A. & Eisenreich, W. (2000). Biosynthesis of terpenoids: YchB protein of Escherichia Coli phosphorylates the 2-hydroxy group of 4-diphosphocytidyi-2Gmethyl-D-erythritol. Proc. Natl. Acad. Sci. USA 97, 1062-1067.
Meade, H. M., Long, S. R., Ruvkun, C. B., Brown, S. E., & Auswald, F. M. (1982). Physical and genetic characterization of symbiotic and auxotrophic mutants of Rhizobium meliloti induced by transposon Tn5 mutagenis. J. Bacteriol. 149, 114-122.
Muchmore, C. R., Krahn, J. M., Kim, J. H., Zalkin, H. & Smith, J. L. (1998). Crystal structure of glutamine phosphoribosylpyrophosphate amidotransferase from Escherichia coli. Protein Science 7, 39-51.
Rohdich, F., Wungsintaweekul, J., Fellermeier, M., Sagner, S., Herz, S., Kis, K., Eisenreich, W., Bacher, A & Zenk, M. H. (1999). Cytidine 5'-triphosphate biosynthesis of isoprenoids: YgbP protein of Escherichia coli catalyzes the formation of 4-diphosphocytidyl-2C-methylerythritol. Proc. Natl. Acad. Sci. USA 96, 11758-11763.
Sanger, F., Nicklen, S., & Coulson, A. R. (1992). DNA sequencing with chain-terminating inhibitors. 1977. Biotechnology 24,104-8.
Sheldrick, G. M., Dauter, Z., Wilson, K. S., Hope, H. & Sieker, L. C. (1993). The application of direct methods of patterson interpretation to high-resolution native protein data. Acta Crystallogr. D49, 18-23.
Subramanya, H. S., Roper, D. I., Dauter, Z., Dodson, E. J., Davies, G. J., Wilson, K. S. & Wigley, D. B. (1996). Enzymatic ketonization of 2-hydroxymuconate: specificity and mechanism investigated by the crystal structures of two isomerases. Biochemistry 35, 792-802.
Turk, D. (1992). Weiterentwicklung eines Programms für Molekülgraphik und Elektronendichte-Manipulation und seine Anwendung auf verschiedene Protein-Strukturaufklärungen, TU München.
Unge, J., Berg, A., Al-Kharadaghi, S., Nikulin, A., Nikonov, S., Davydova, N., Nevskaya, N., Garber, M. & Liljas, A. (1998). The crystal structure of ribosomal protein L22 from Thermus thermophilus: insights into the mechanism of erythromycin resistance. Structure 6, 1577-1586.
Volz, K. (1999). A test case for structure-based functional assignment: The 1.2 Å crystal structure of the yjgF gene product from Escherichia coli. Protein Science 8, 2428-2437. Xiang, S., Short, S. A., Wolfenden, R. & Carter, R. C. (1995). Transition-state selectivity for a single hydroxyl group during catalysis by cytidine deaminase. Biochemistry 34(34), 4516-4.523.

**Table 1**

| Statistics for data collection and refinement | | | | | | |
|---|---|---|---|---|---|---|
| **Crystal** | nat2 | mgcdp2 | subop | subs | sub2 | cyt |
| **Data collection** | | | | | | |
| Cell constant a (Å) (a=b=c, α=β=γ=90°) | 144.15 | 144.66 | 144.81 | 145.01 | 144.63 | 144.70 |
| Resolution range (Å) | 20 to 2.85 | 25 to 2.5 | 20 to 2.8 | 20 to 3.2 | 25 to 2.5 | 25 to 2.9 |
| Reflections observed | 49159 | 54965 | 46385 | 20929 | 66701 | 31246 |
| Unique reflections | 11403 | 17015 | 12586 | 8183 | 17106 | 10634 |
| R_{merge}^{a} overall (%) | 7.4 | 6.4 | 7.5 | 10.6 | 6.3 | 6.7 |
| R_{merge}^{a} outermost shell (%) | 40.1 | 39.4 | 42.6 | 44.6 | 40.9 | 37.7 |
| Completeness overall (%) | 97.4 | 97.3 | 99.8 | 96.7 | 98.1 | 98.8 |
| Completeness outermost shell (%) | 99.7 | 97.1 | 99.9 | 96.4 | 99.8 | 99.0 |
| Temperature factor from Wilson plot (Å²) | 77.8 | 50.8 | 73.2 | 64.3 | 60.3 | 84.7 |
| **Refinement** | | | | | | |
| Reflections used for refinement (8.0Å to high resolution limit) | 10861 | 16531 | 12023 | 7672 | 16521 | 10075 |
| R value ^{b} final model (%) | 22.6 | 24.0 | 23.3 | 20.6 | 20.1 | 22.1 |
| R_{free} ^{c} value final model | 23.7 | 26.0 | 26.4 | 25.4 | 25.5 | 25.9 |
| R*m*s deviations | | | | | | |
| - bond lengths (Å) | 0,012 | 0.011 | 0.010 | 0.011 | 0.009 | 0.011 |
| - bond angles (°) | 1.84 | 1.88 | 1.85 | 1.84 | 1.81 | 1.91 |
| - dihedral angles (°) | 22.6 | 23.0 | 22.40 | 22.0 | 22.4 | 21.97 |
| - improper angles (°) | 1.23 | 1.34 | 1.23 | 1.24 | 1.18 | 1.25 |
| Ramachandran-plot: residues in | | | | | | |
| most favoured: | 93.0 | 89.8 | 93.7 | 86.6 | 92.1 | 88.2 |
| additional allowed: | 6.3 | 10.2 | 6.3 | 13.4 | 7.1 | 11.0 |
| generously allowed: | 0.8 | 0.0 | 0.0 | 0.0 | 0.8 | 0.8 |
| disallowed: | 0.0 | 0.0 | 0.0 | 0.0 | 0.0 | 0.0 |
| regions | | | | | | |
| RMSD for bond B restraints (Å²) | 2.85 | 2.7 | 2.8 | 2.6 | 2.3 | 2.9 |
| Temperature factors (Å²) | protein: | protein: 32.7 | protein: 38.4 | protein: 43.0 | protein: 36.1 | protein: 46.7 |
| | 41.5 | Zn: 36.9 | Zn: 46.5 | sub:59.7 | Zn:36.1 | Zn: 57.7 |
| | Zn:51.3 | Mg: 50.2 | subs:52.5 | | subs:46.7 | cytidine:43.2 |
| | | CDP: 32.0 | | | solvent: 54.5 (72 | |
| | | | | | water molecules) | |

| | | | | | | |
|---|---|---|---|---|---|---|
| ^{a}R_{merge}=Σ(I-<I>)/ΣI ^{b}R=Σ(\|F_{obs}\|-\|Fcalc\|)/Σ\|Fobs\| ^{c}R_{free} was calculated with 5% of the observed reflections which were omitted from the refinement and F-value calculation | | | | | | |

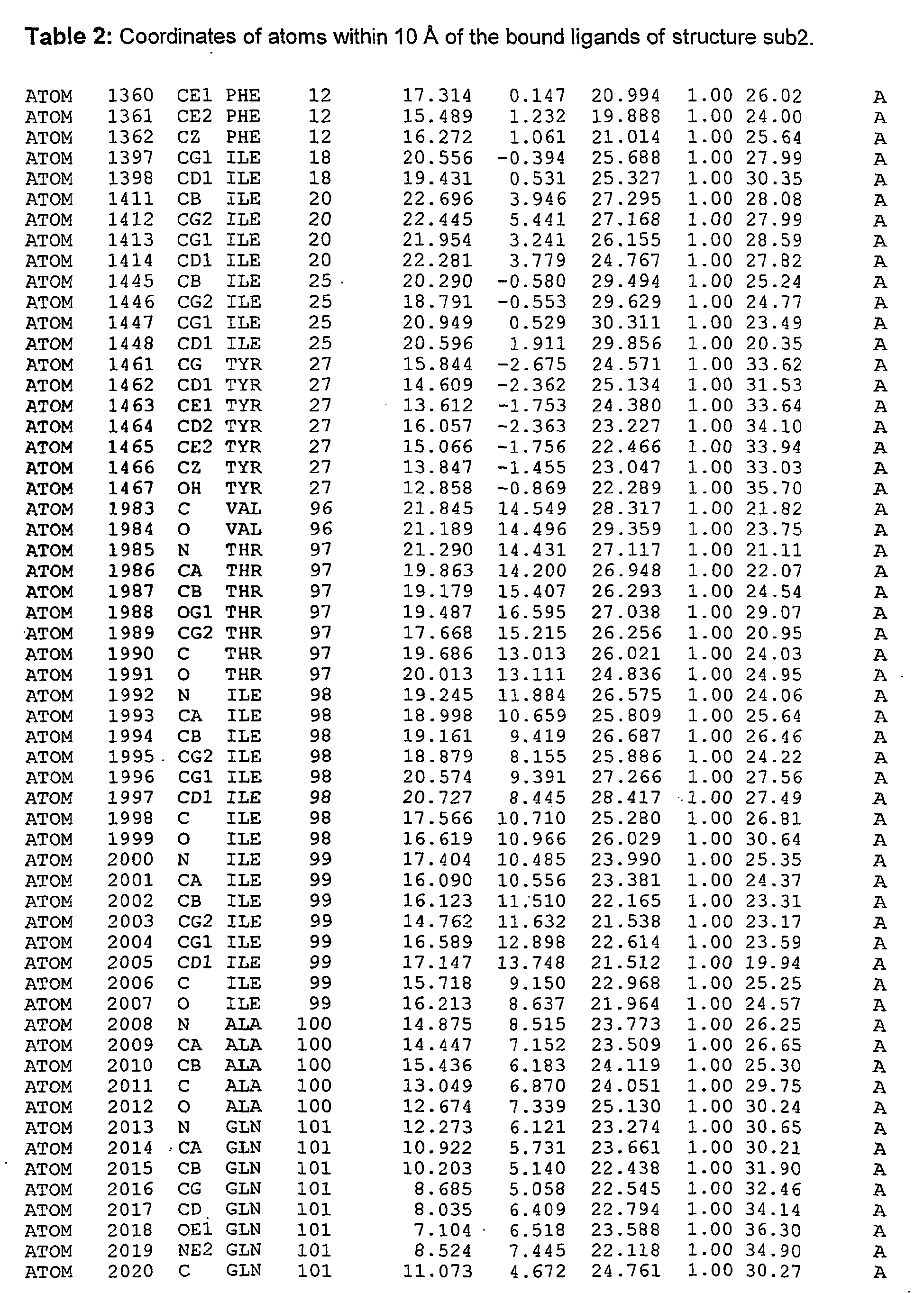

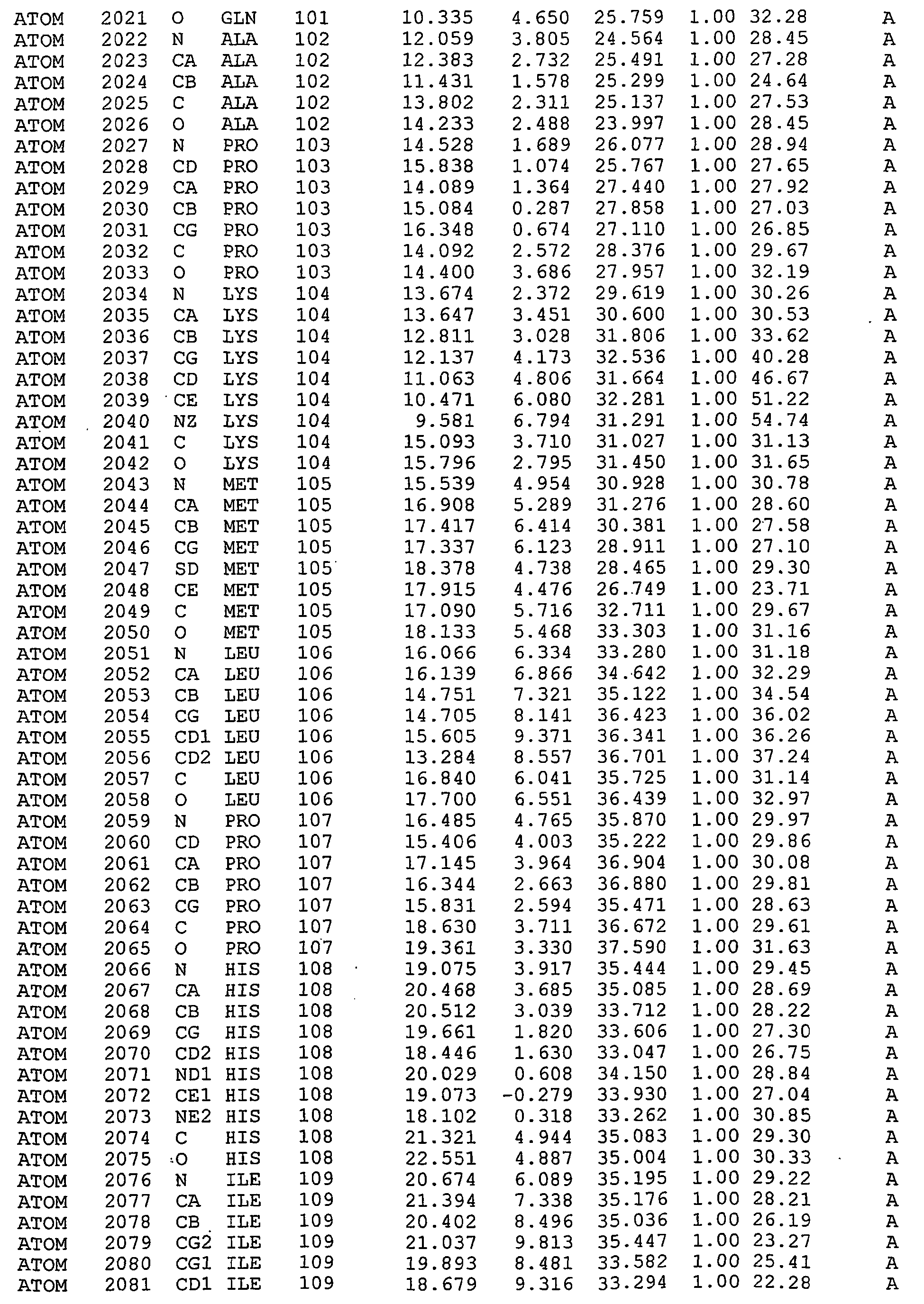

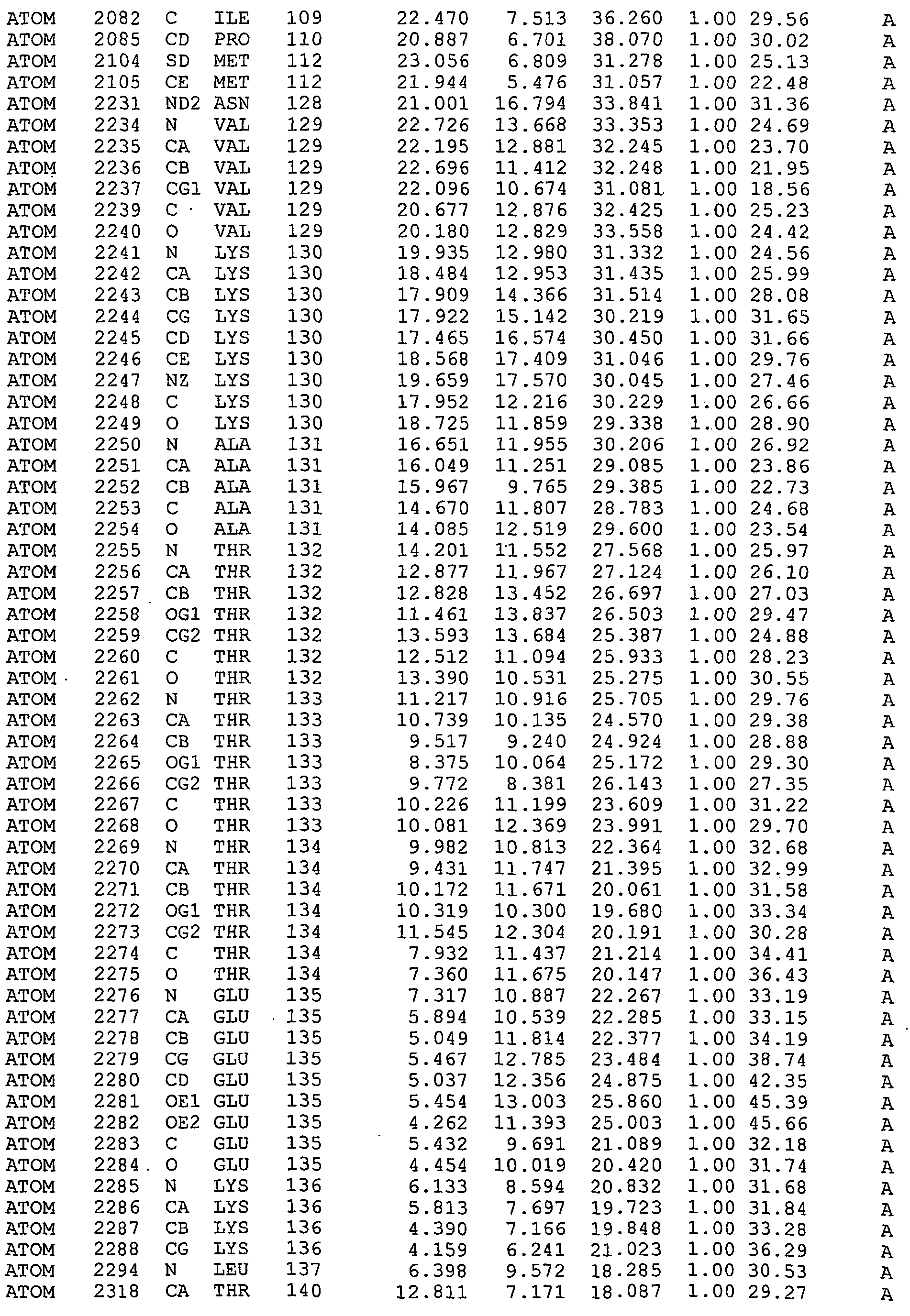

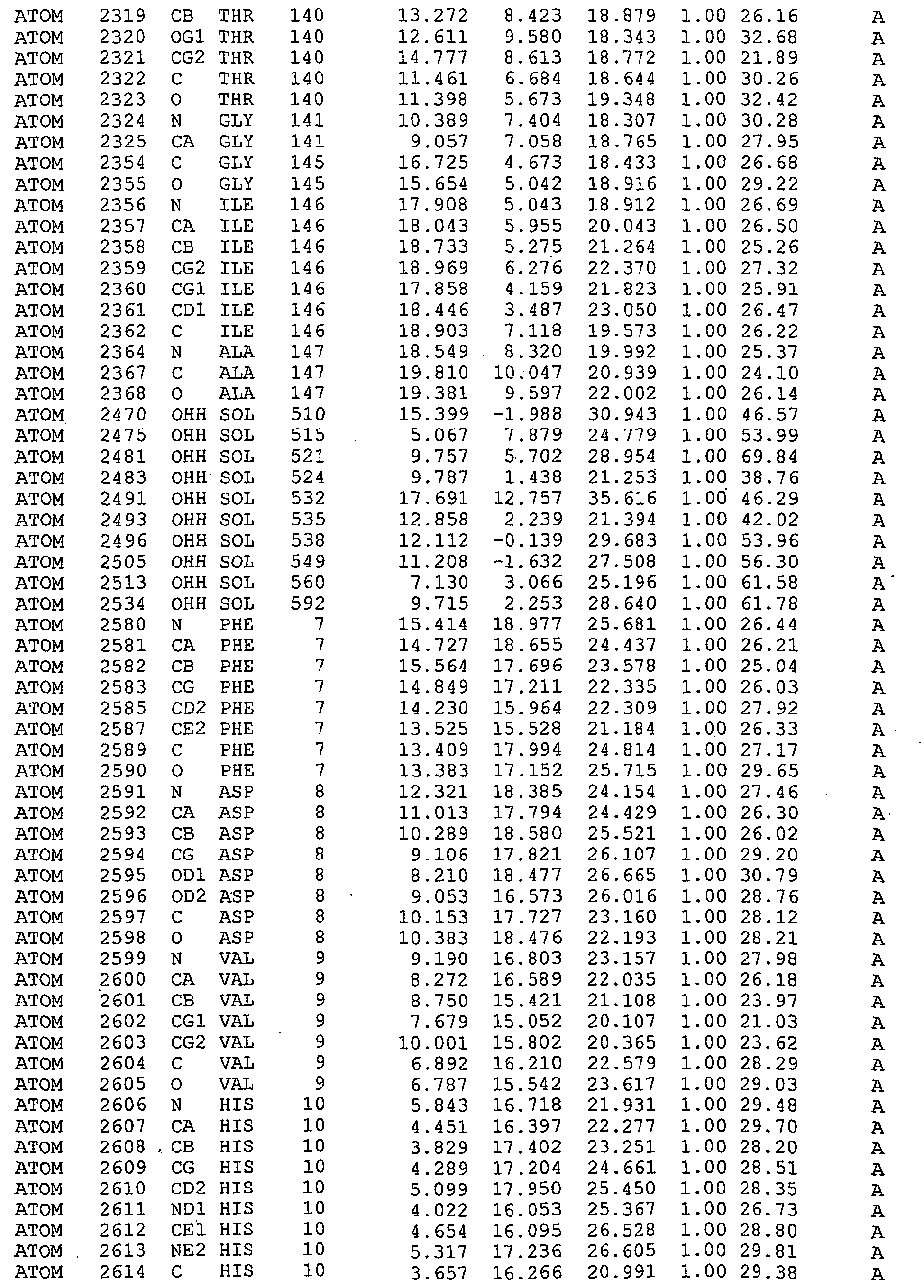

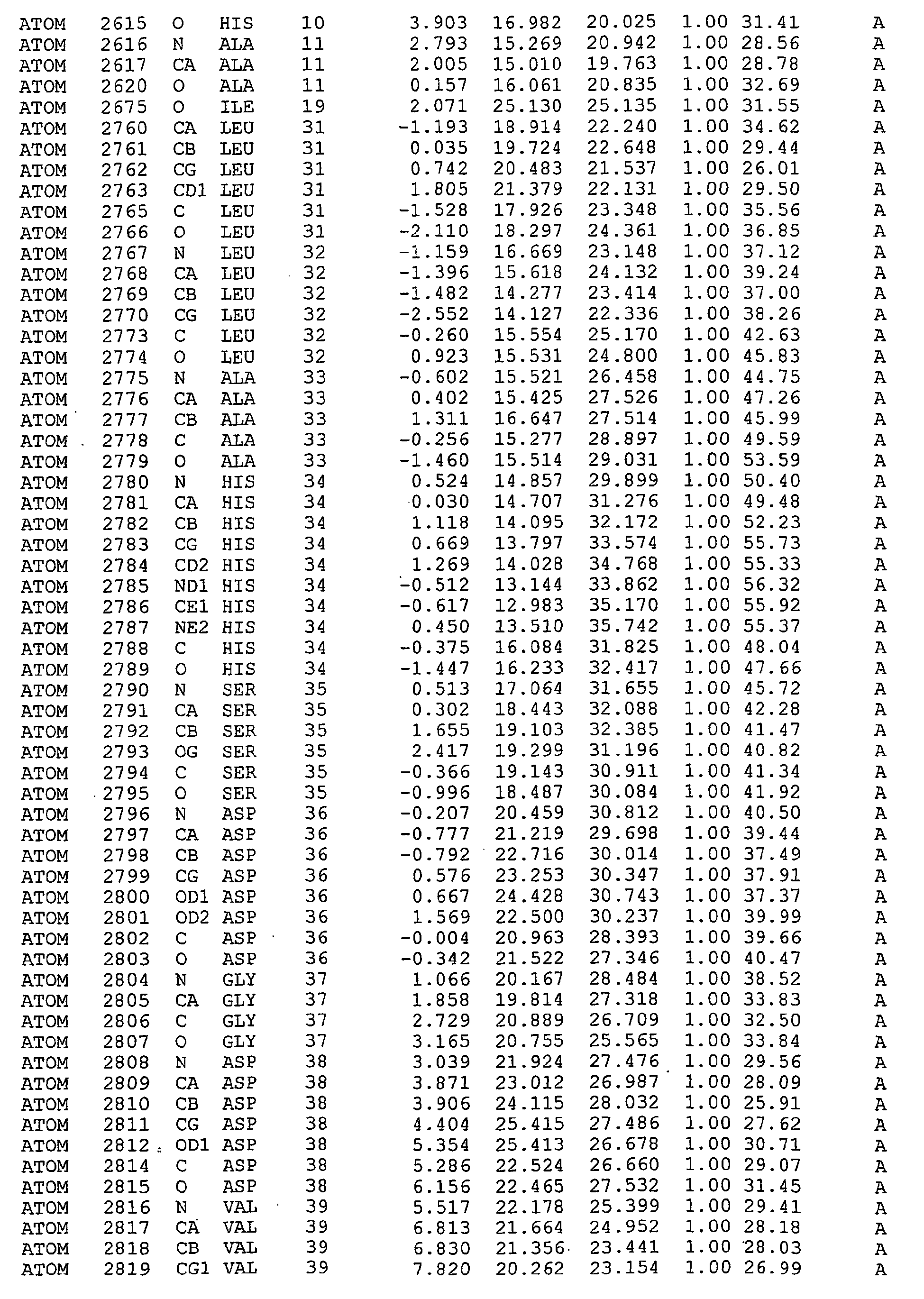

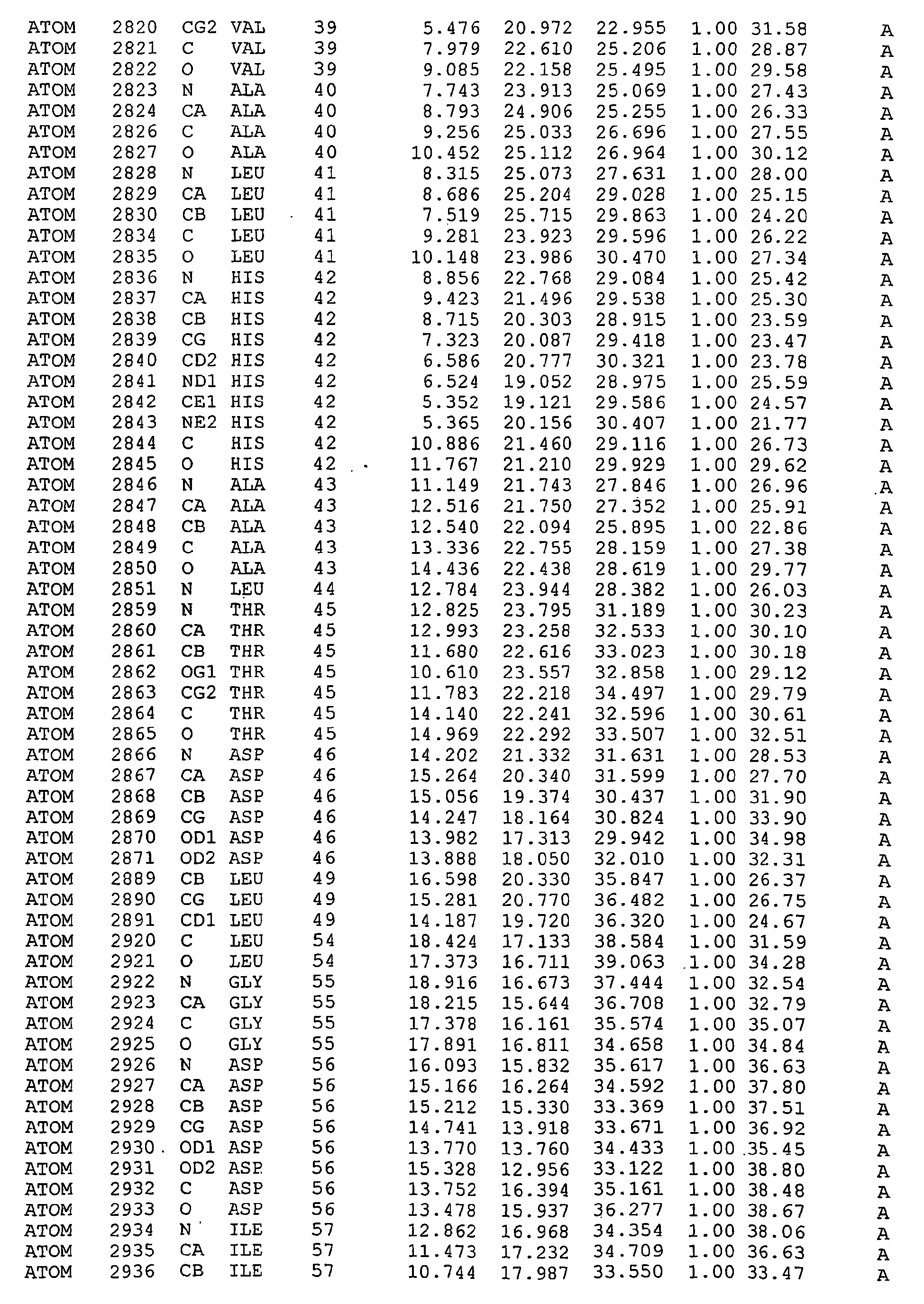

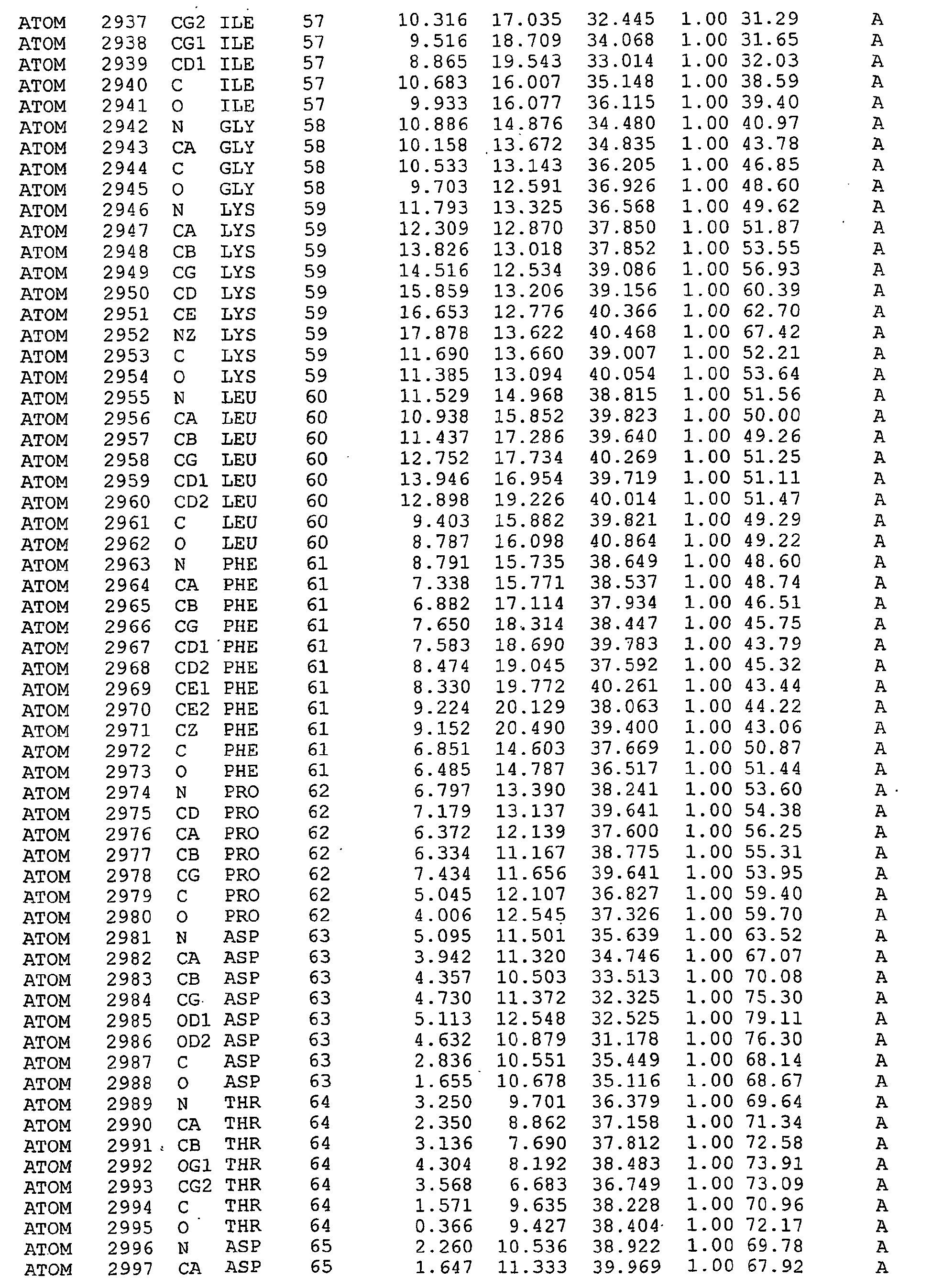

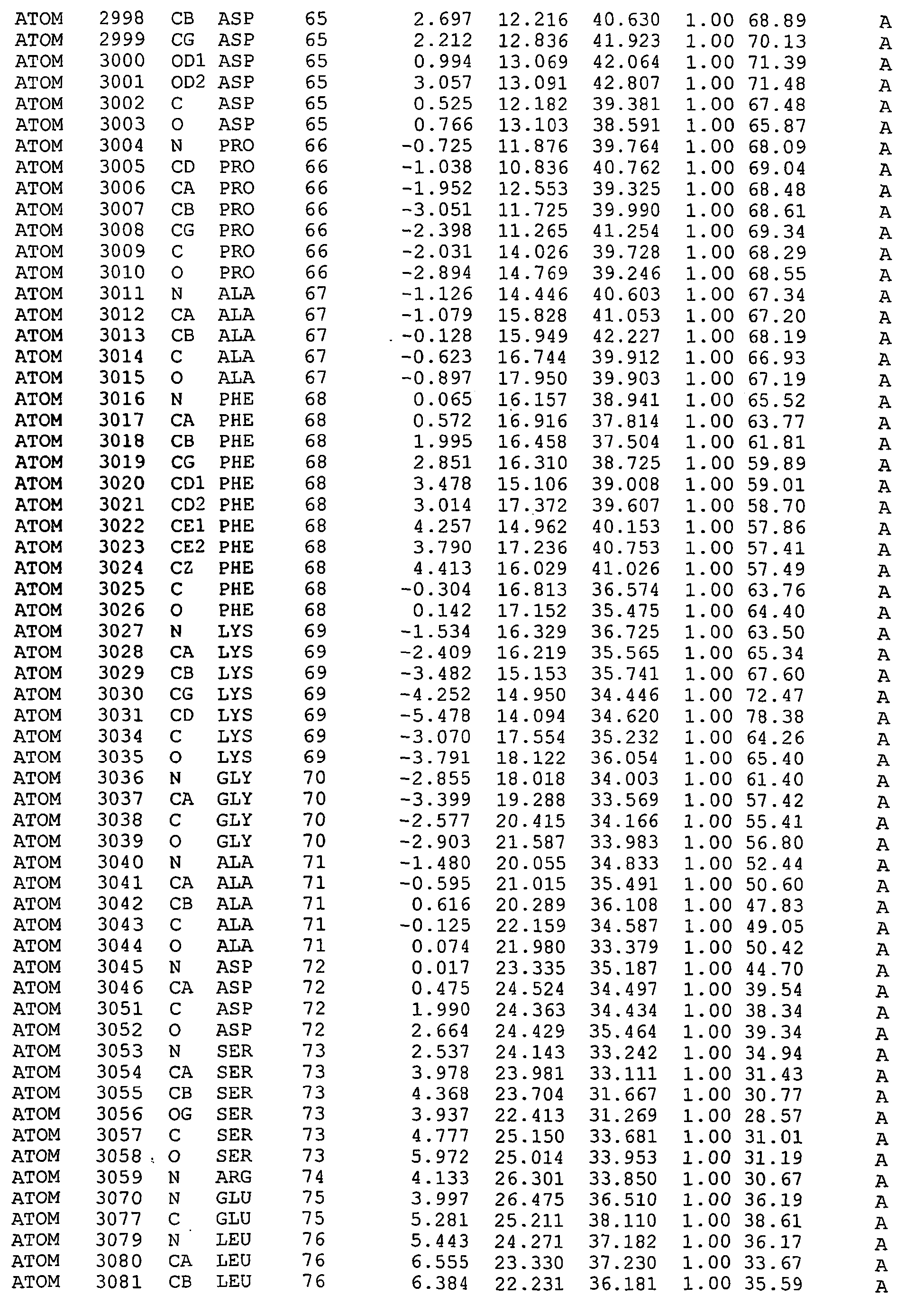

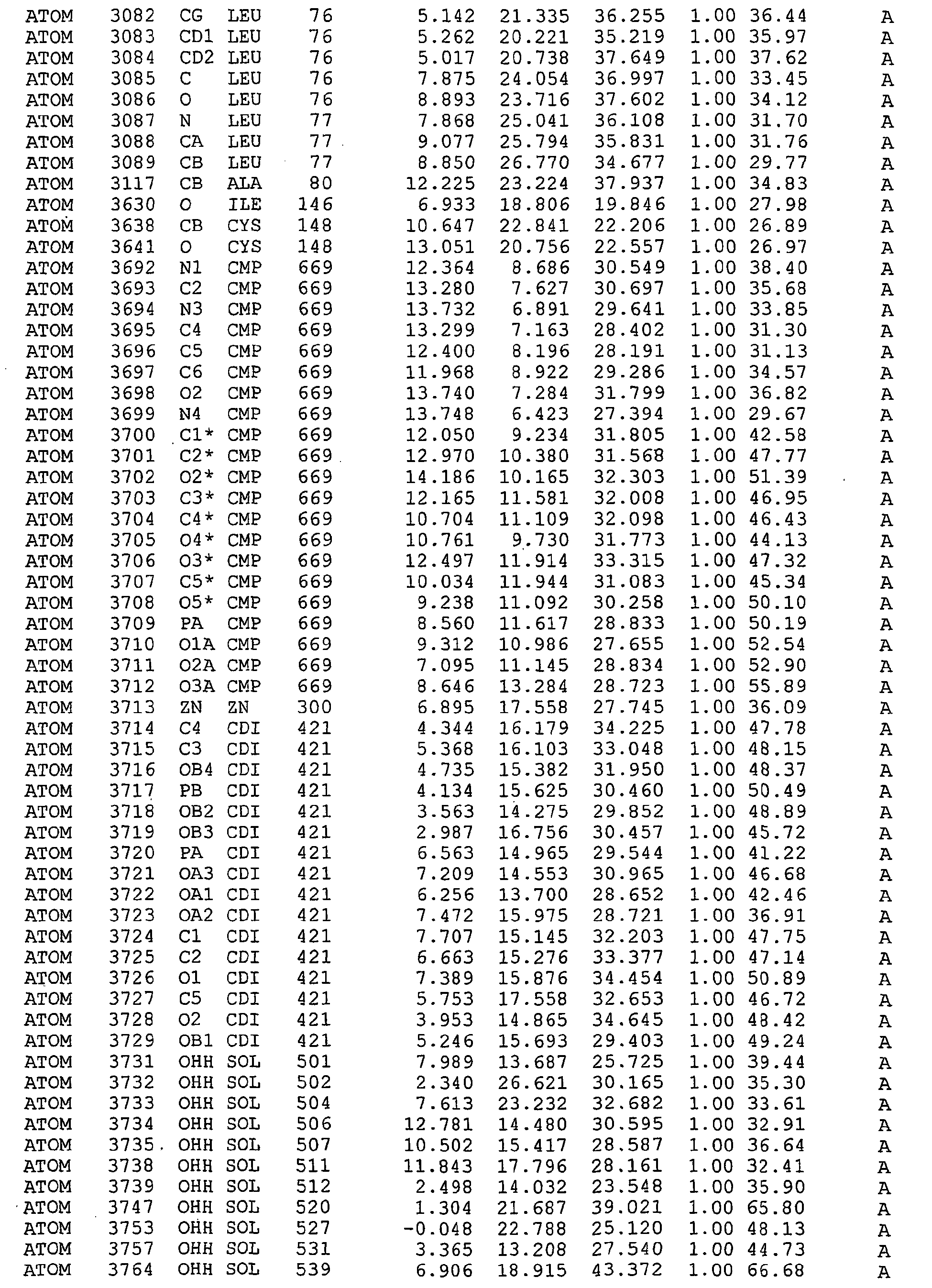

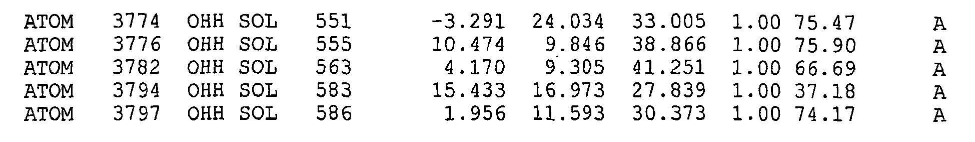

### Annex 1: Coordinates of structure cyt

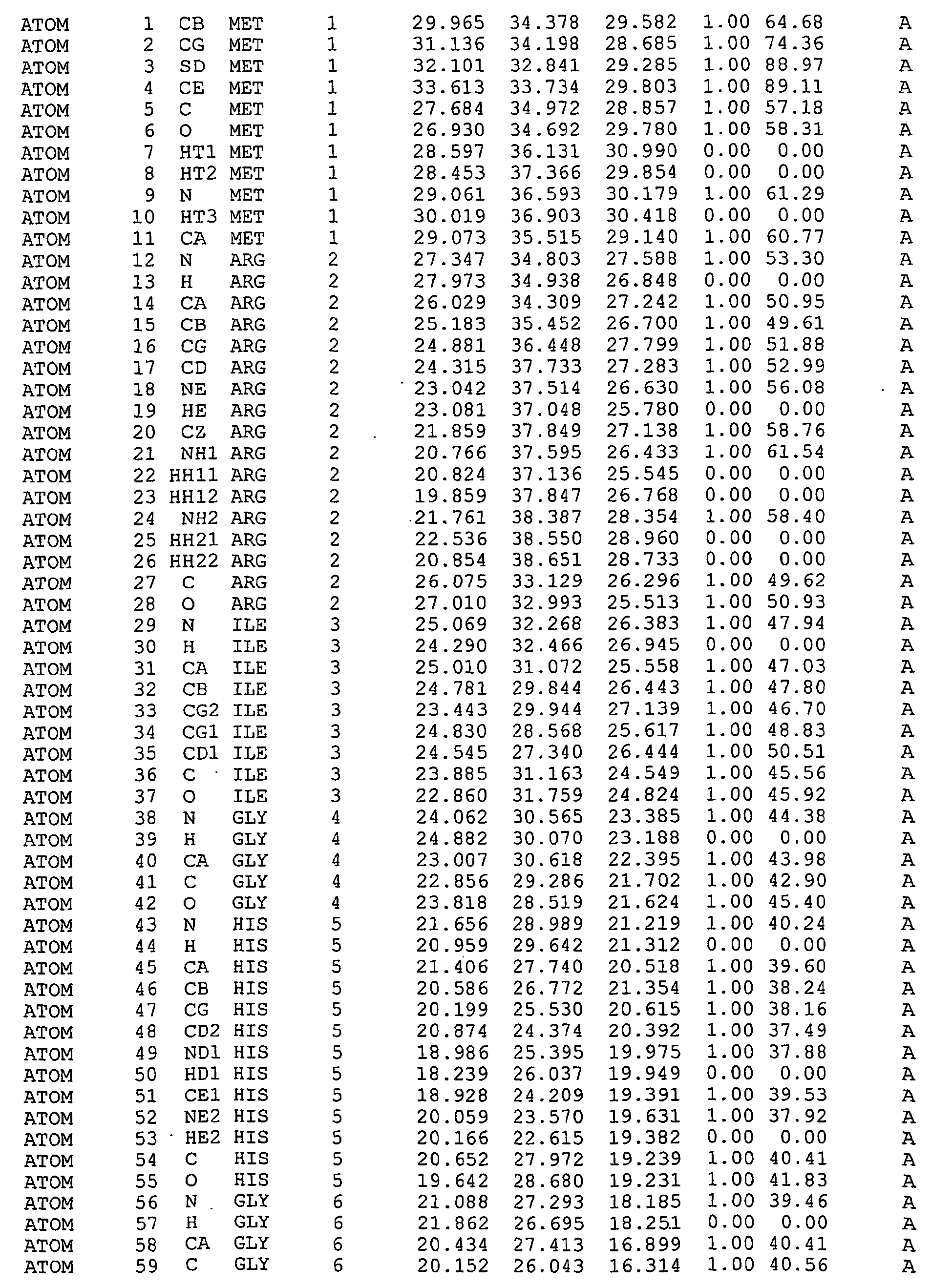

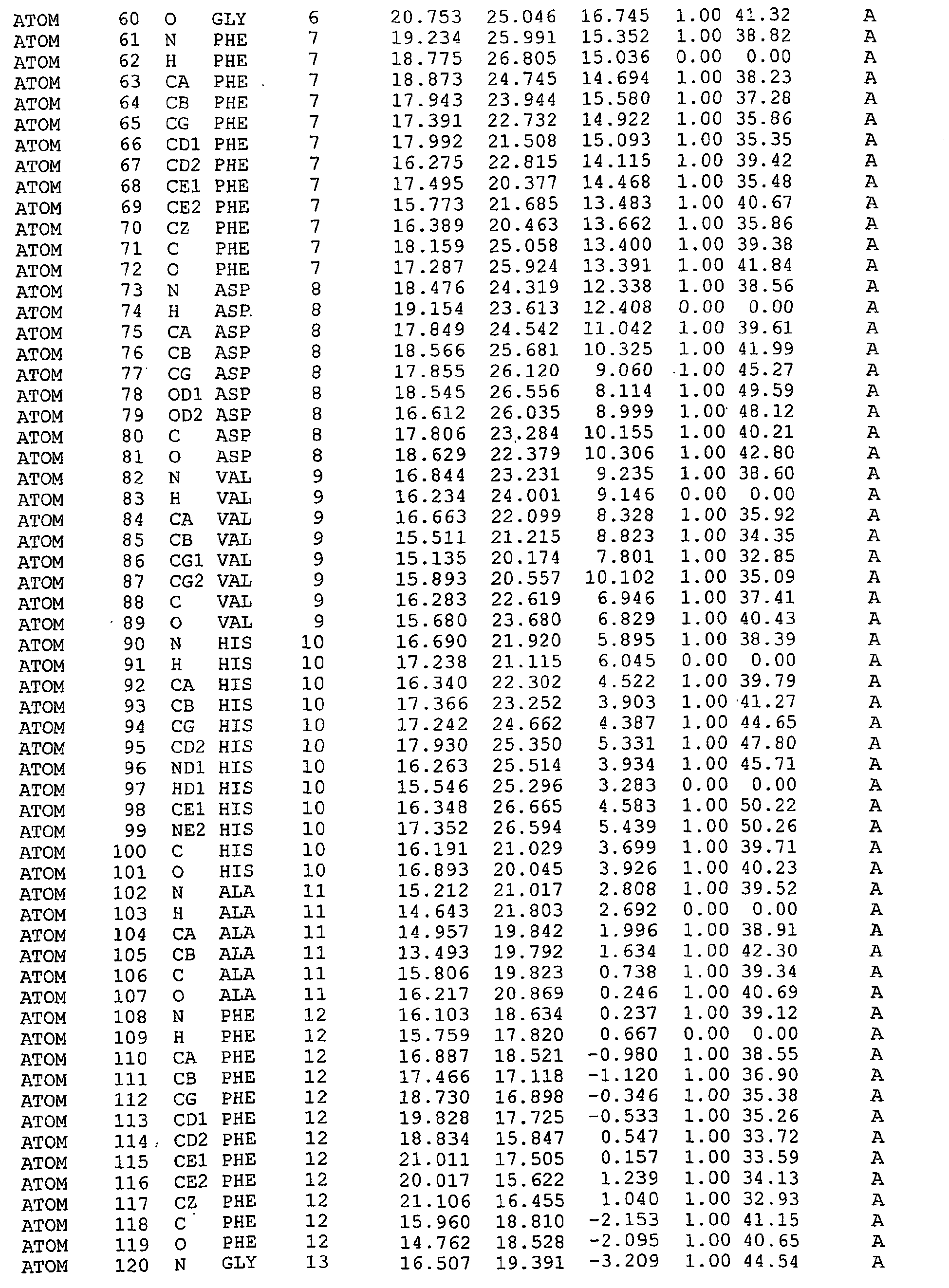

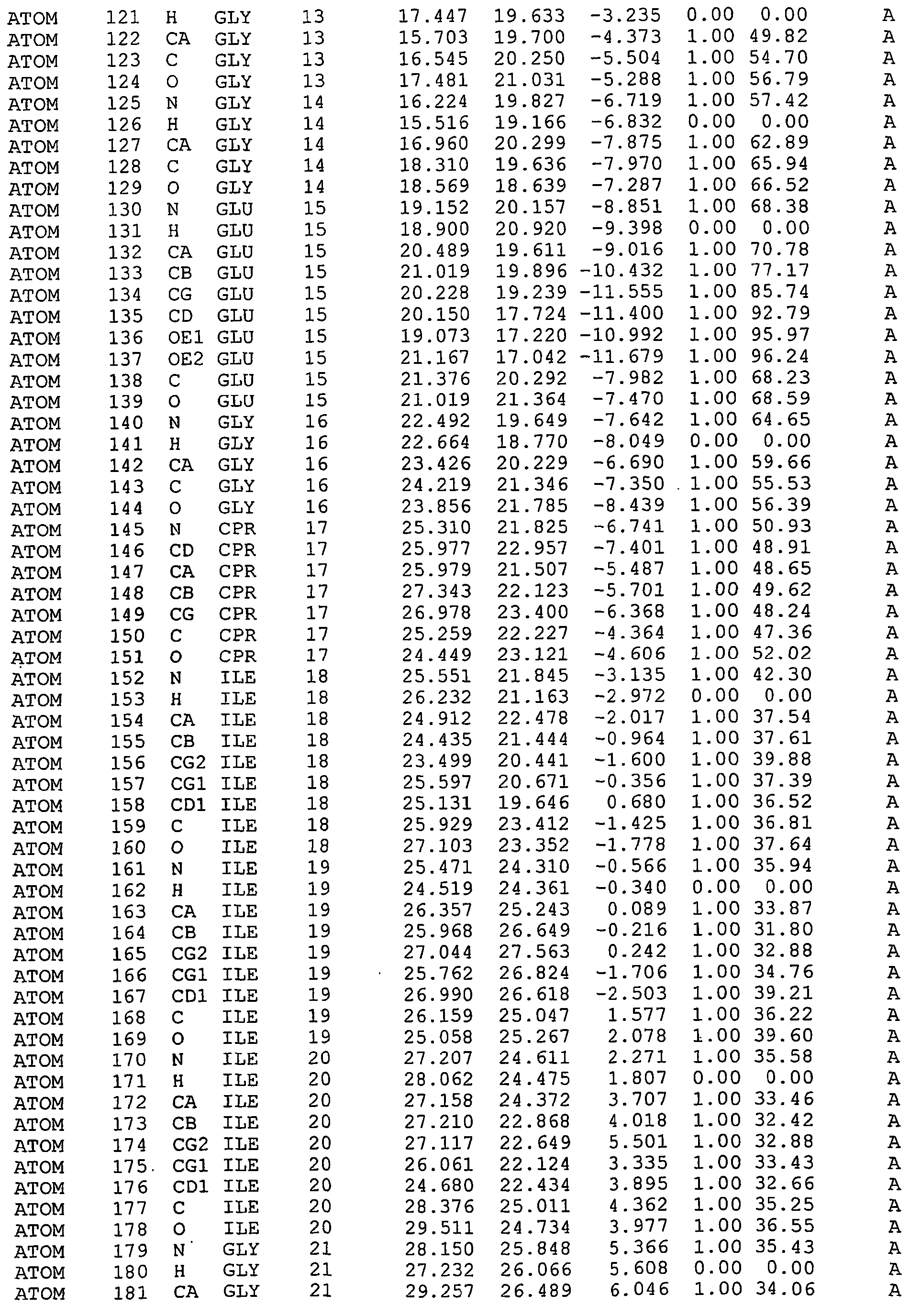

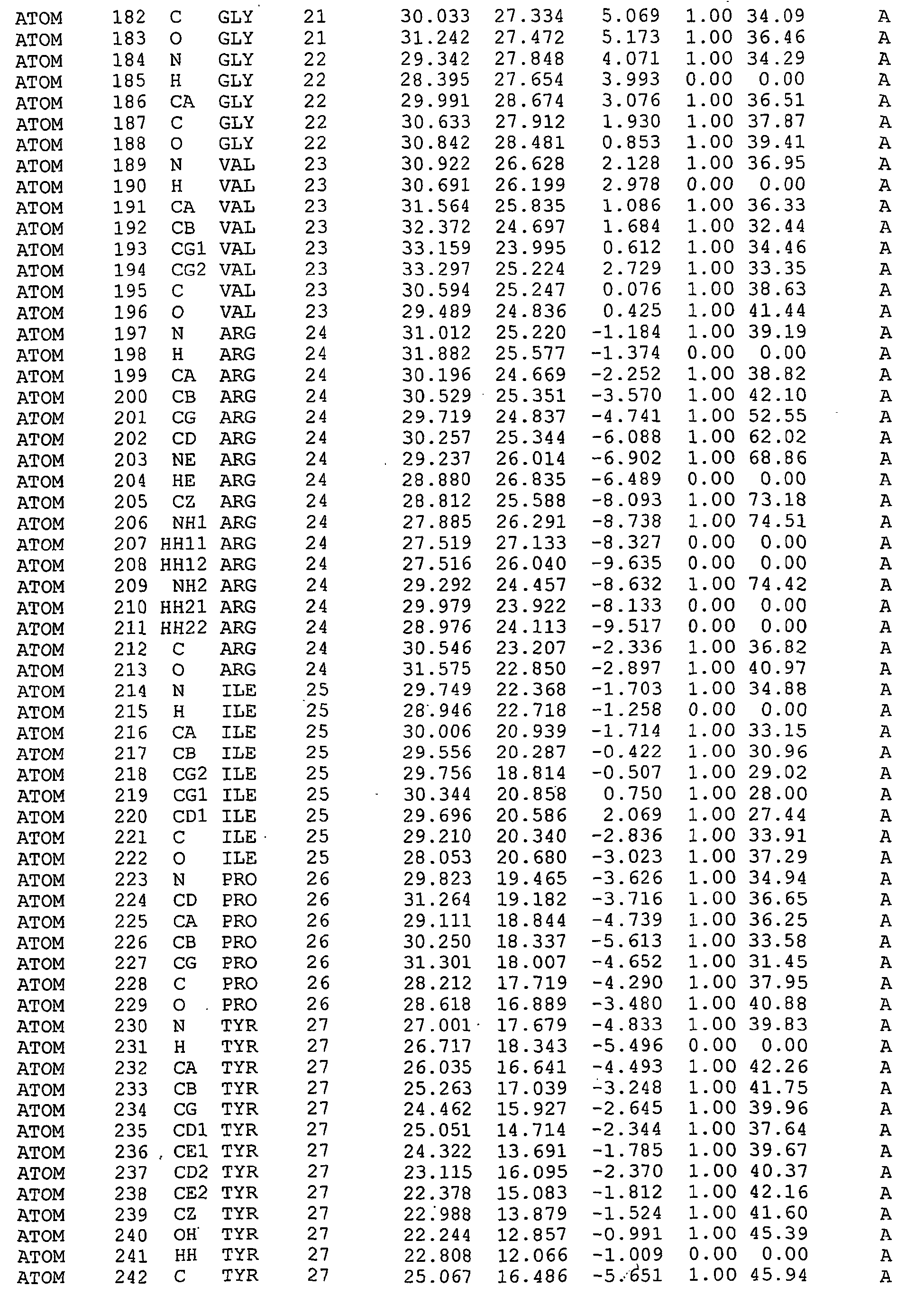

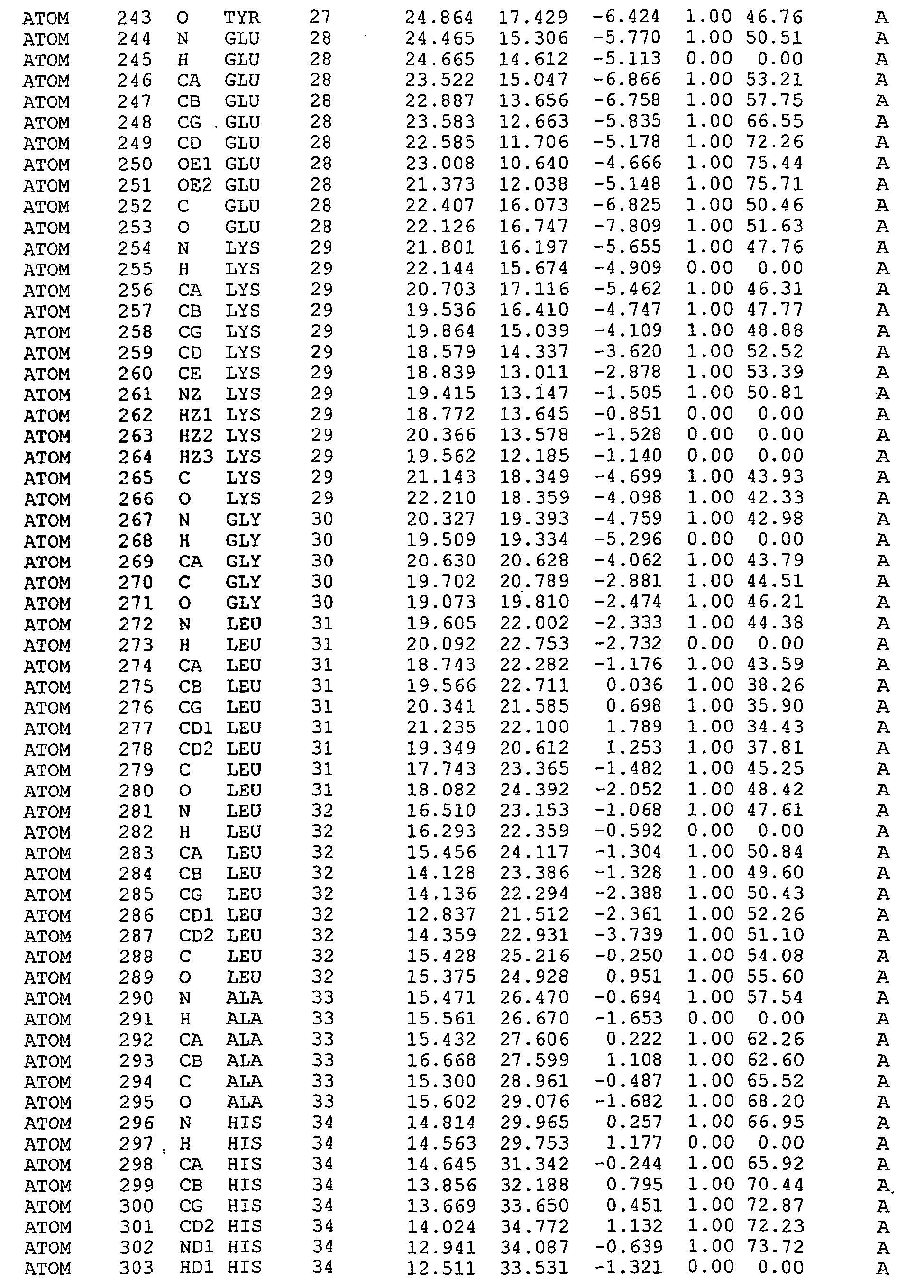

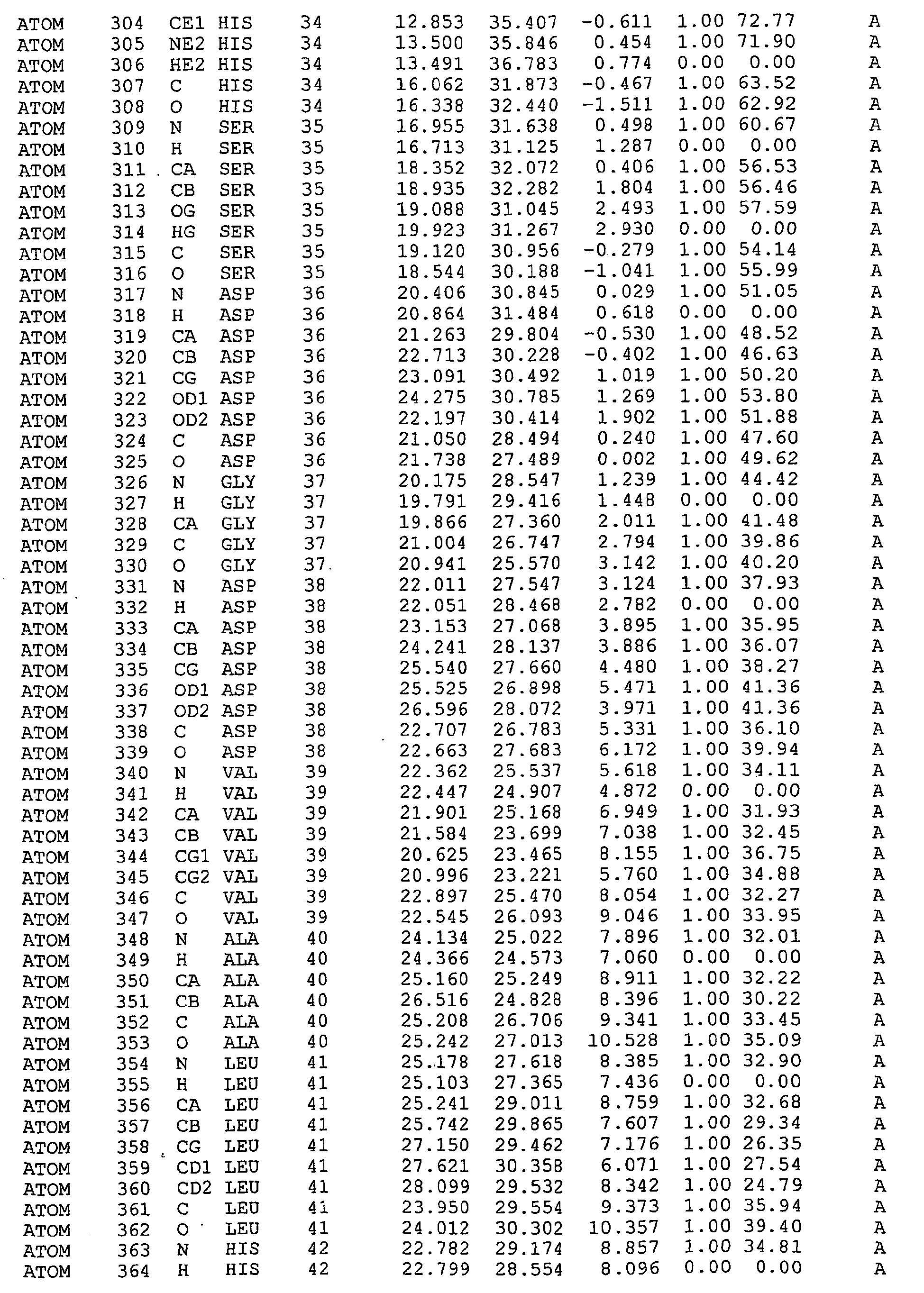

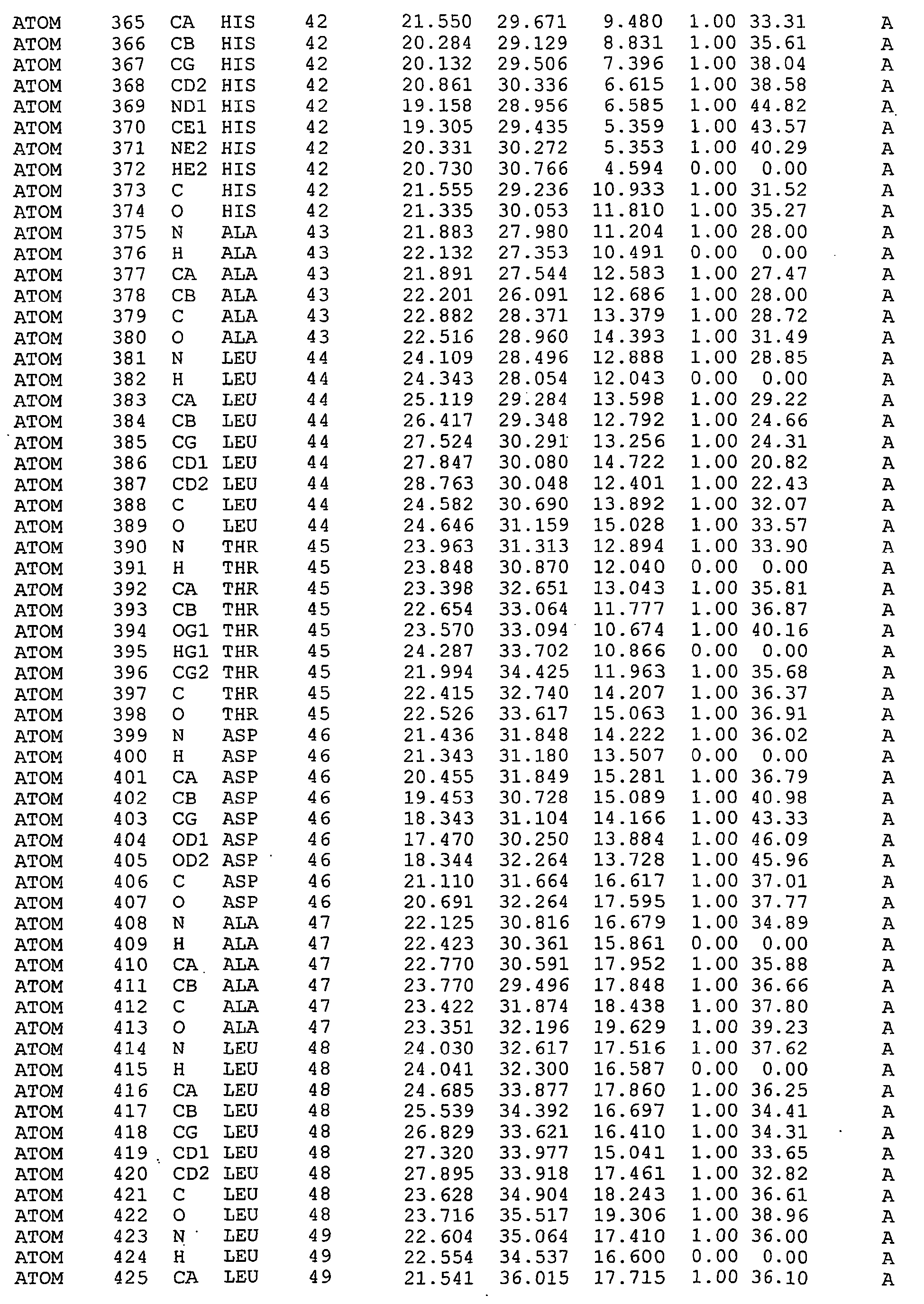

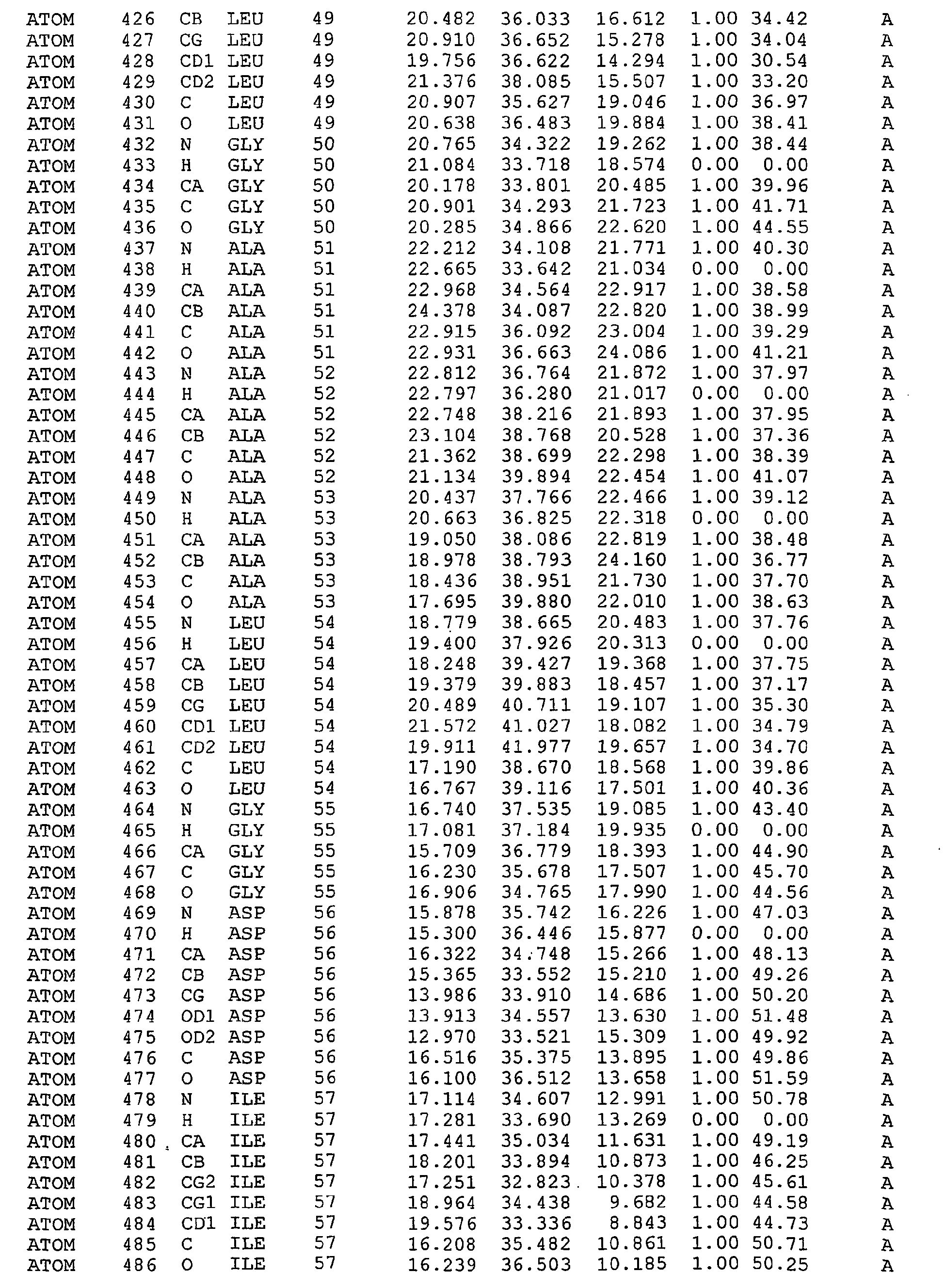

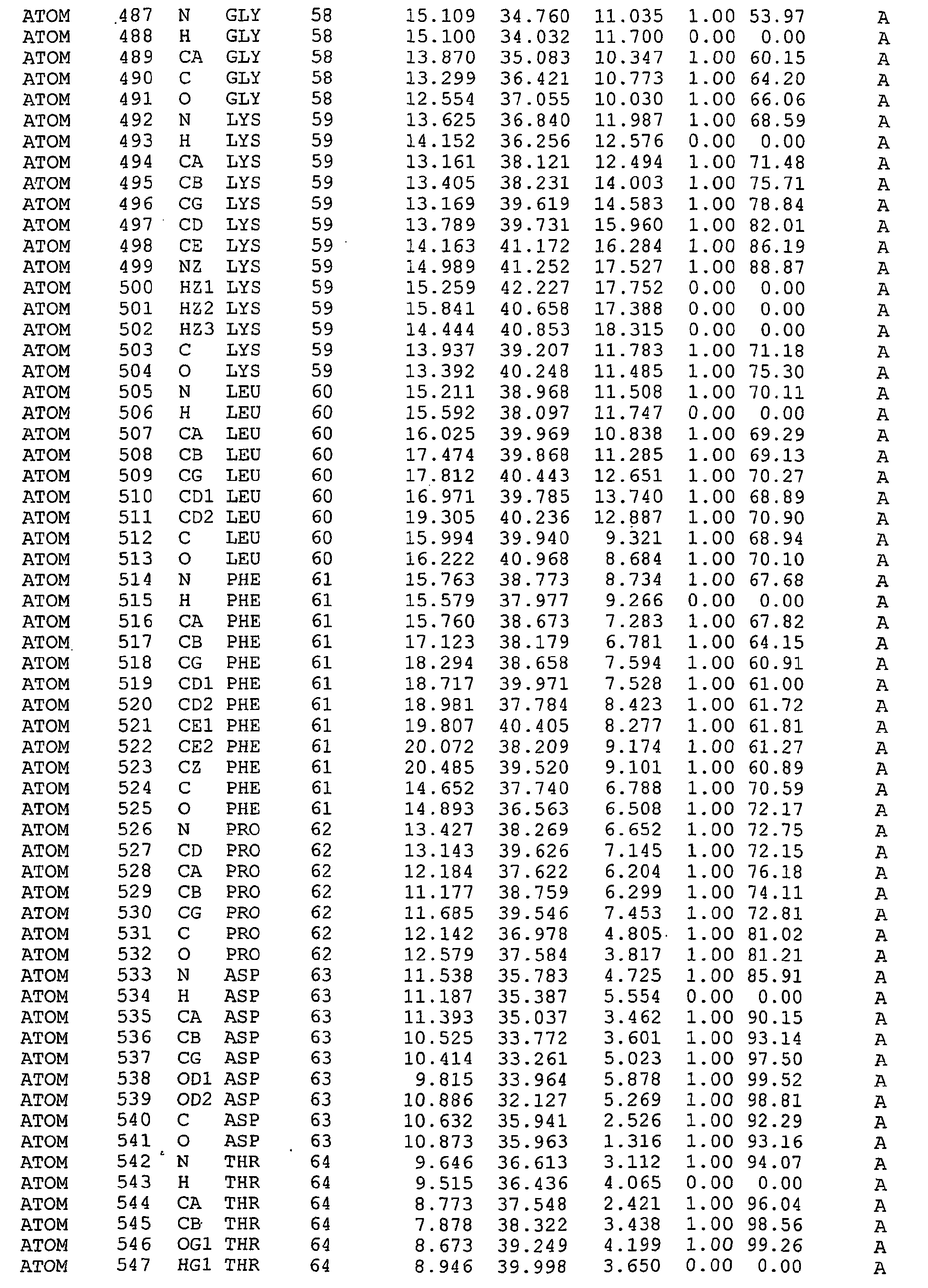

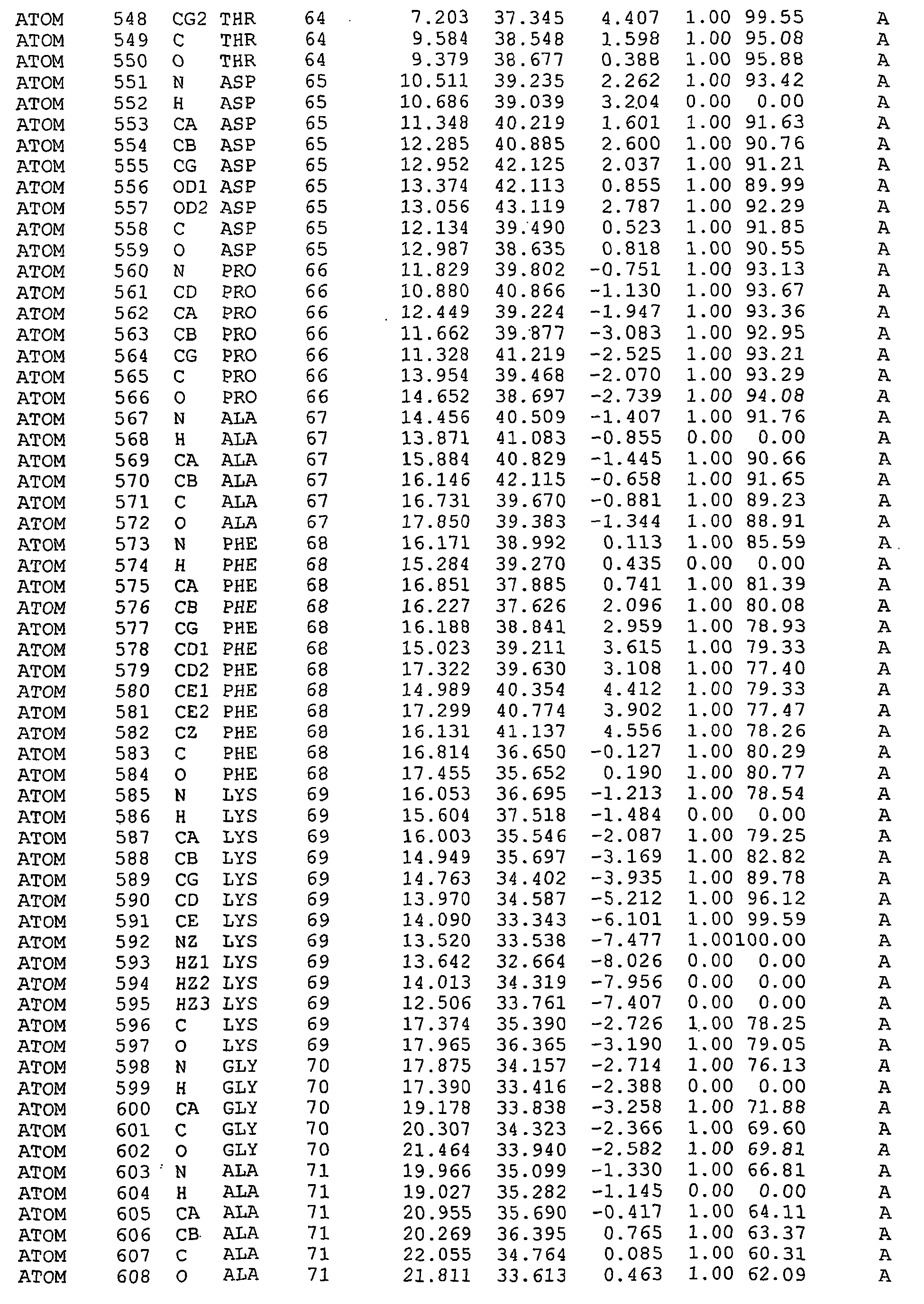

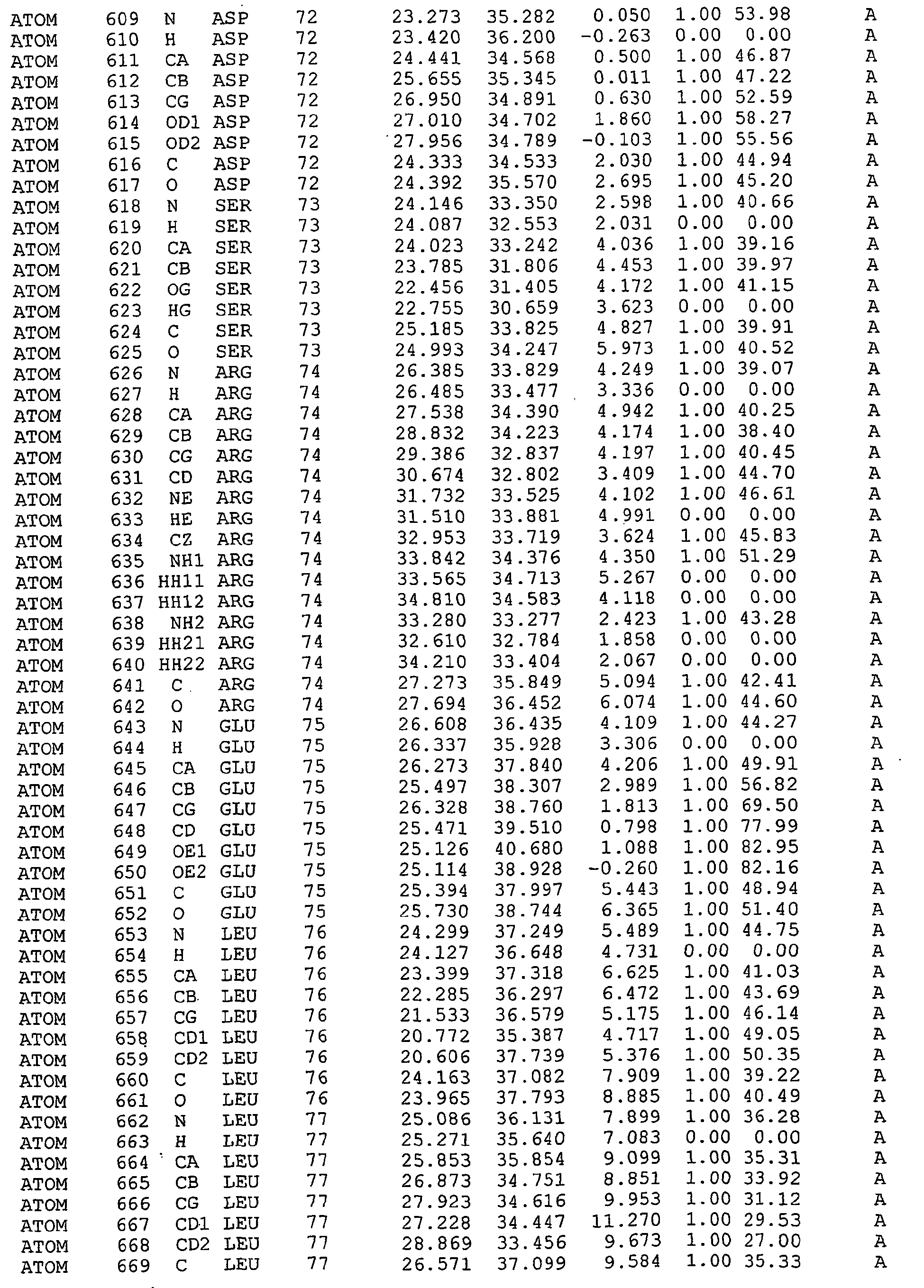

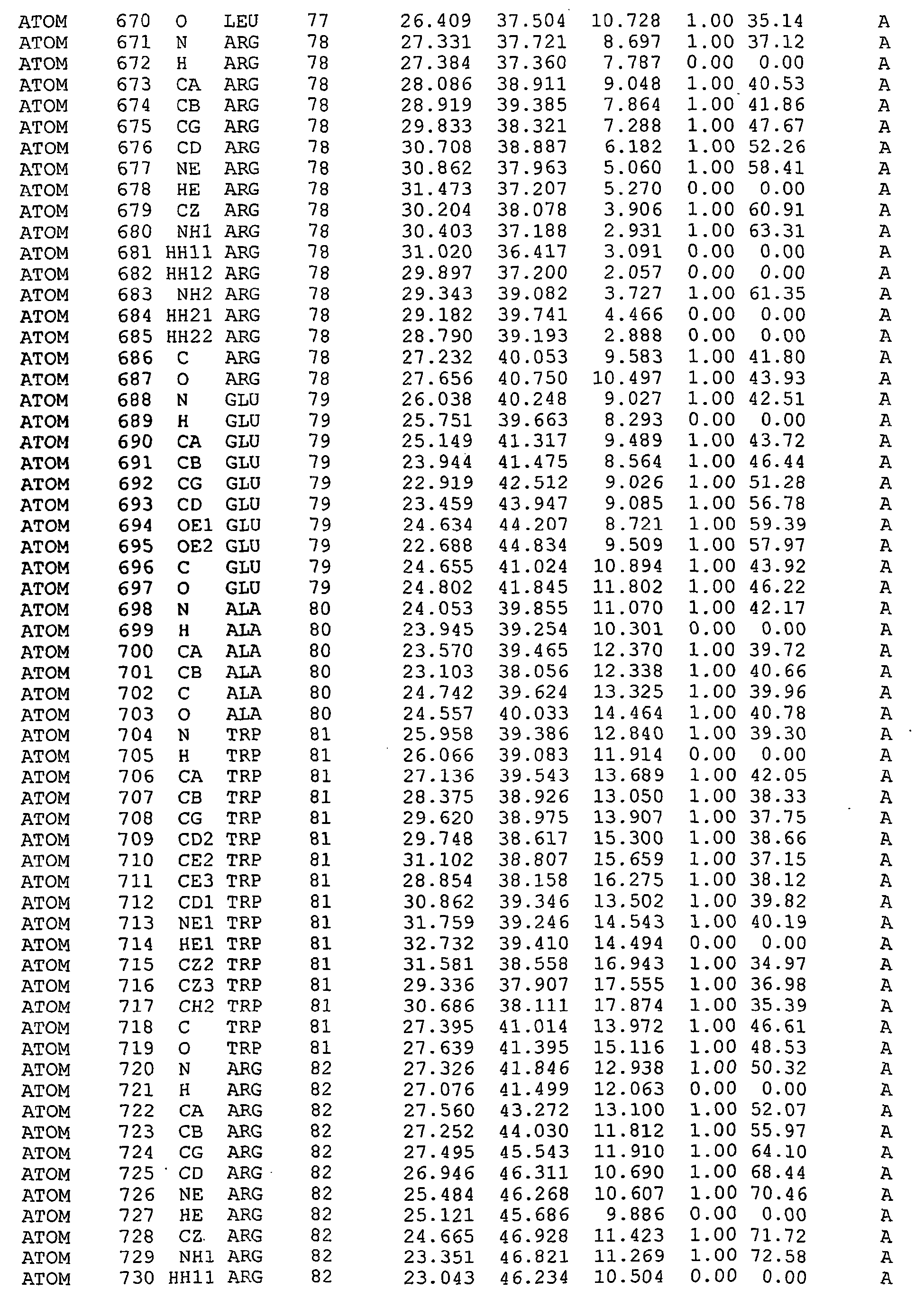

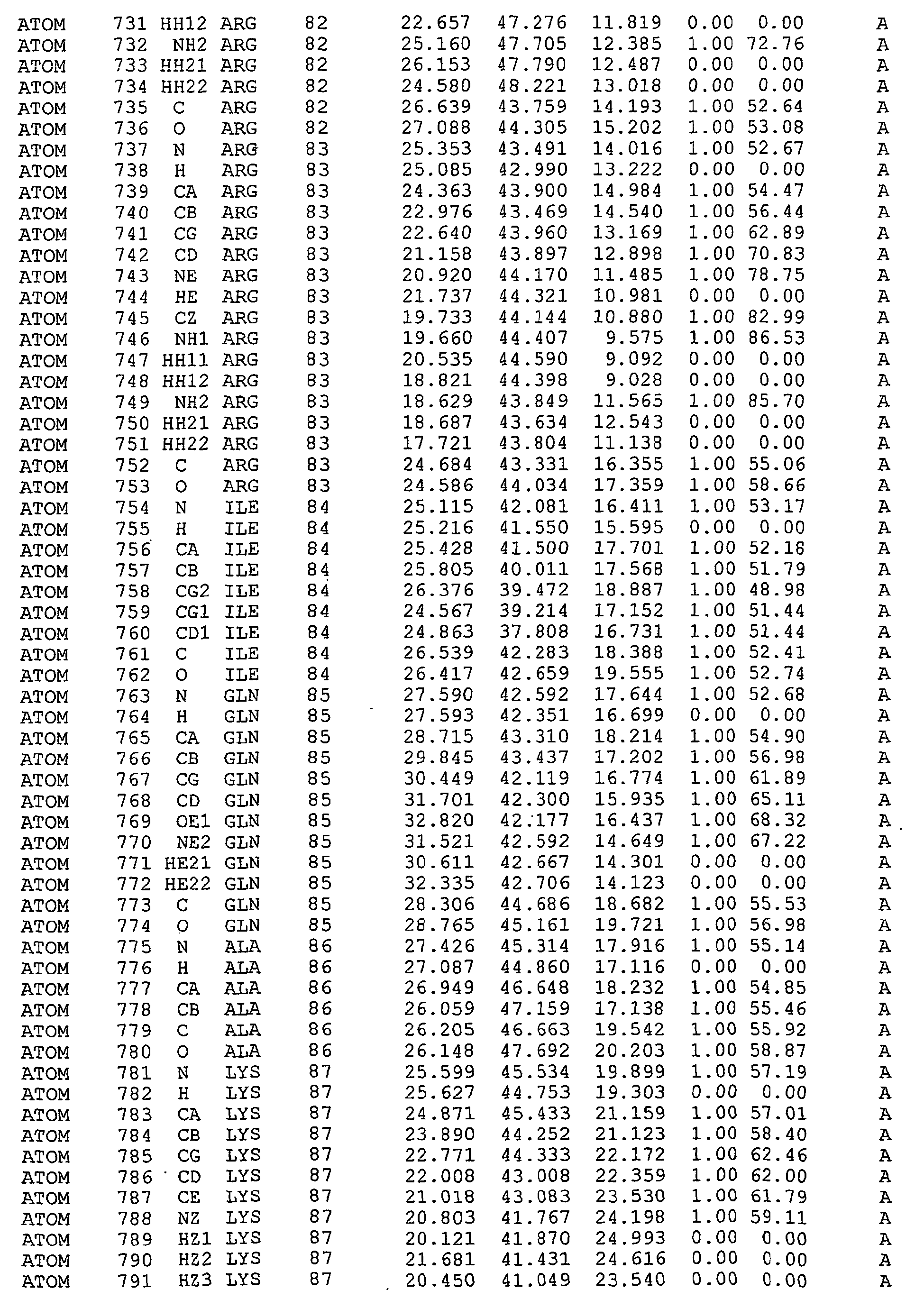

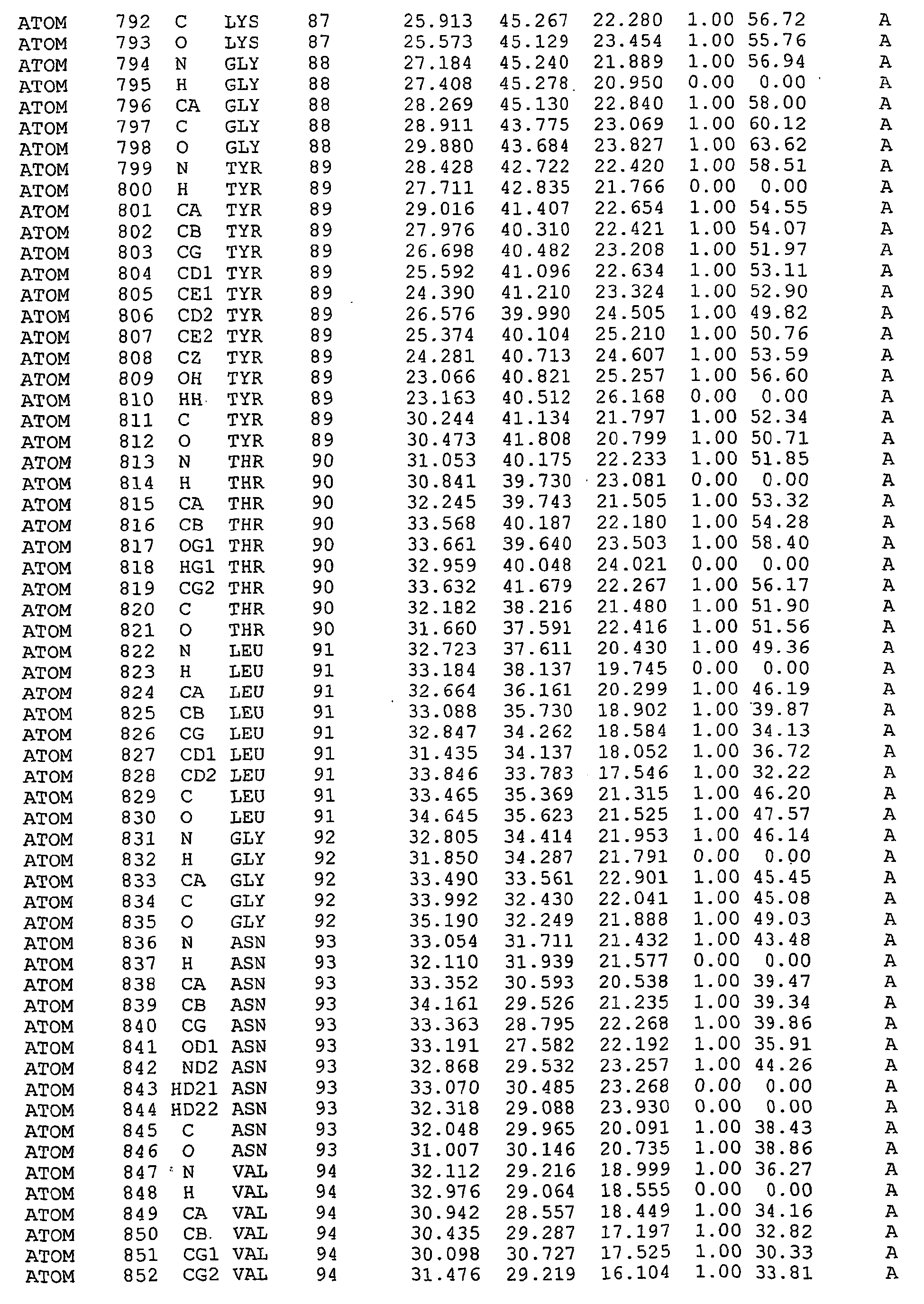

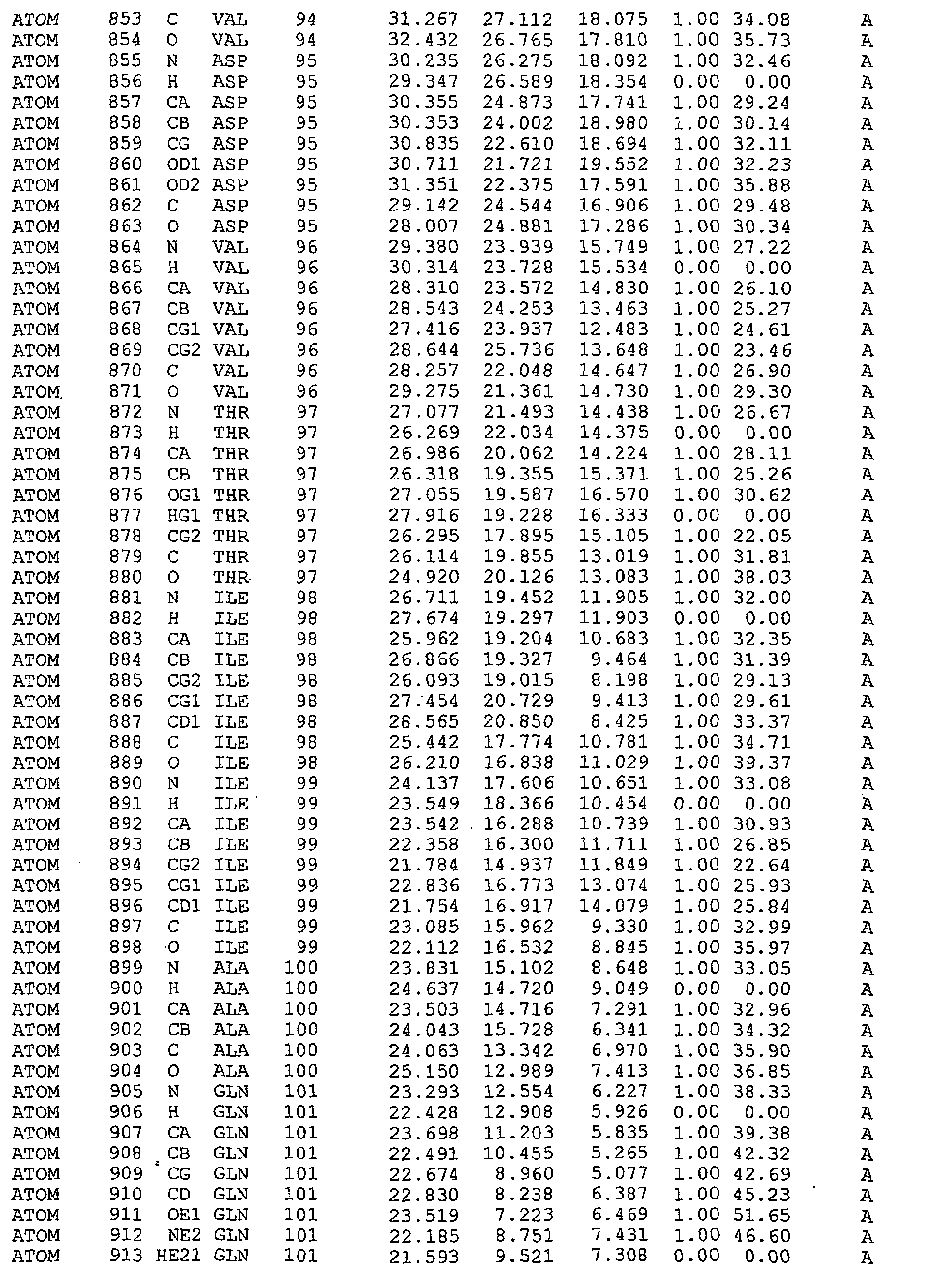

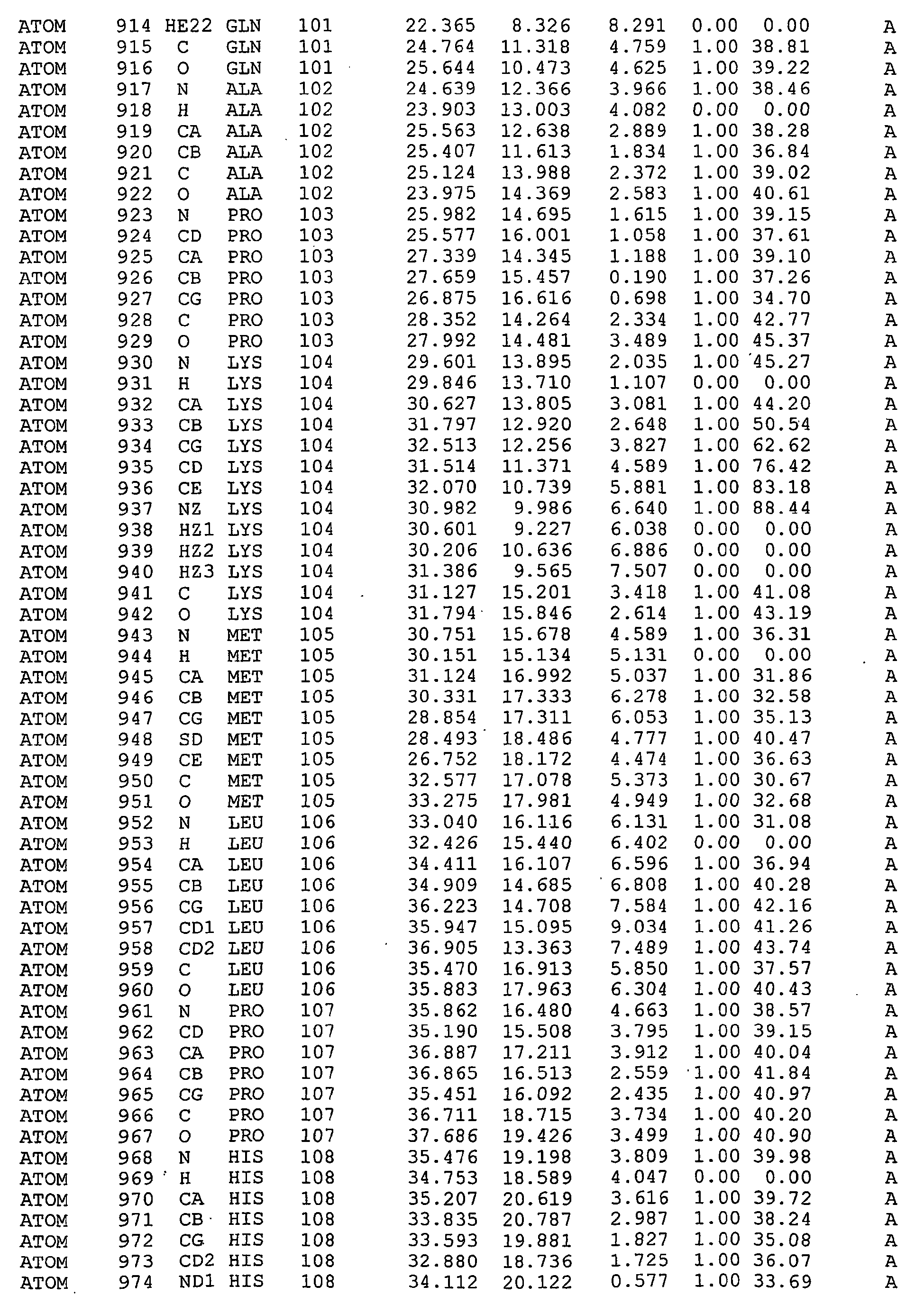

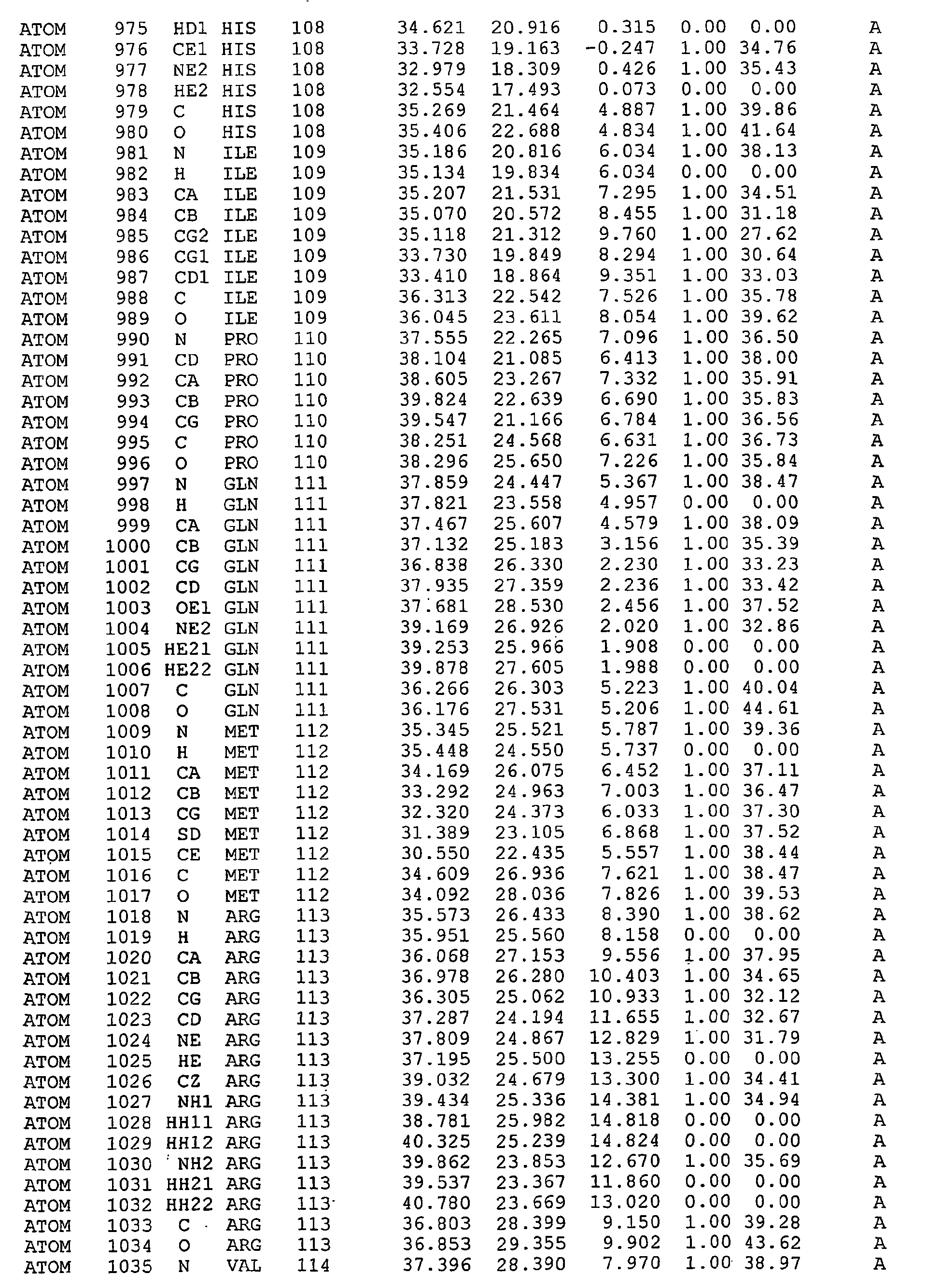

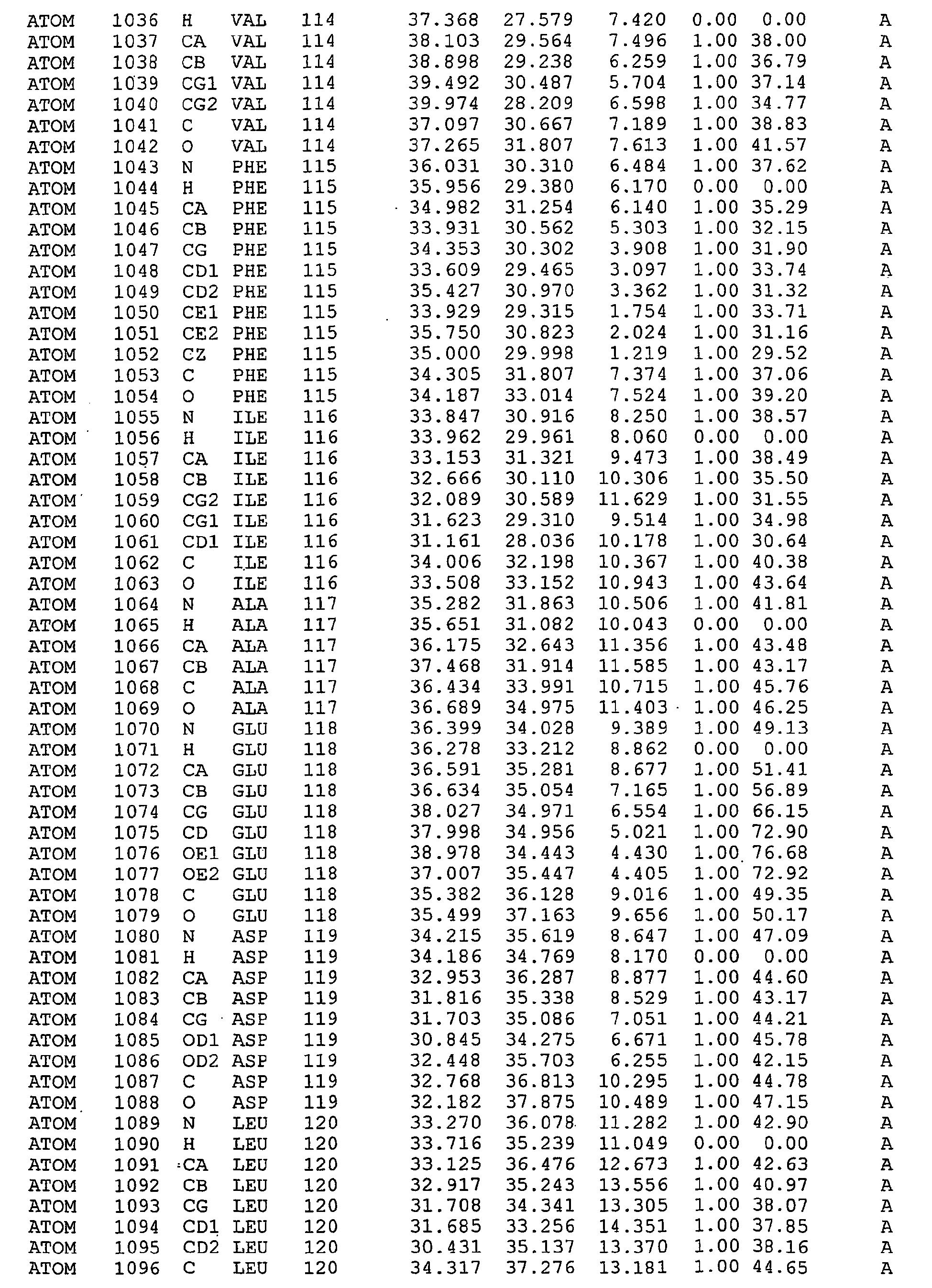

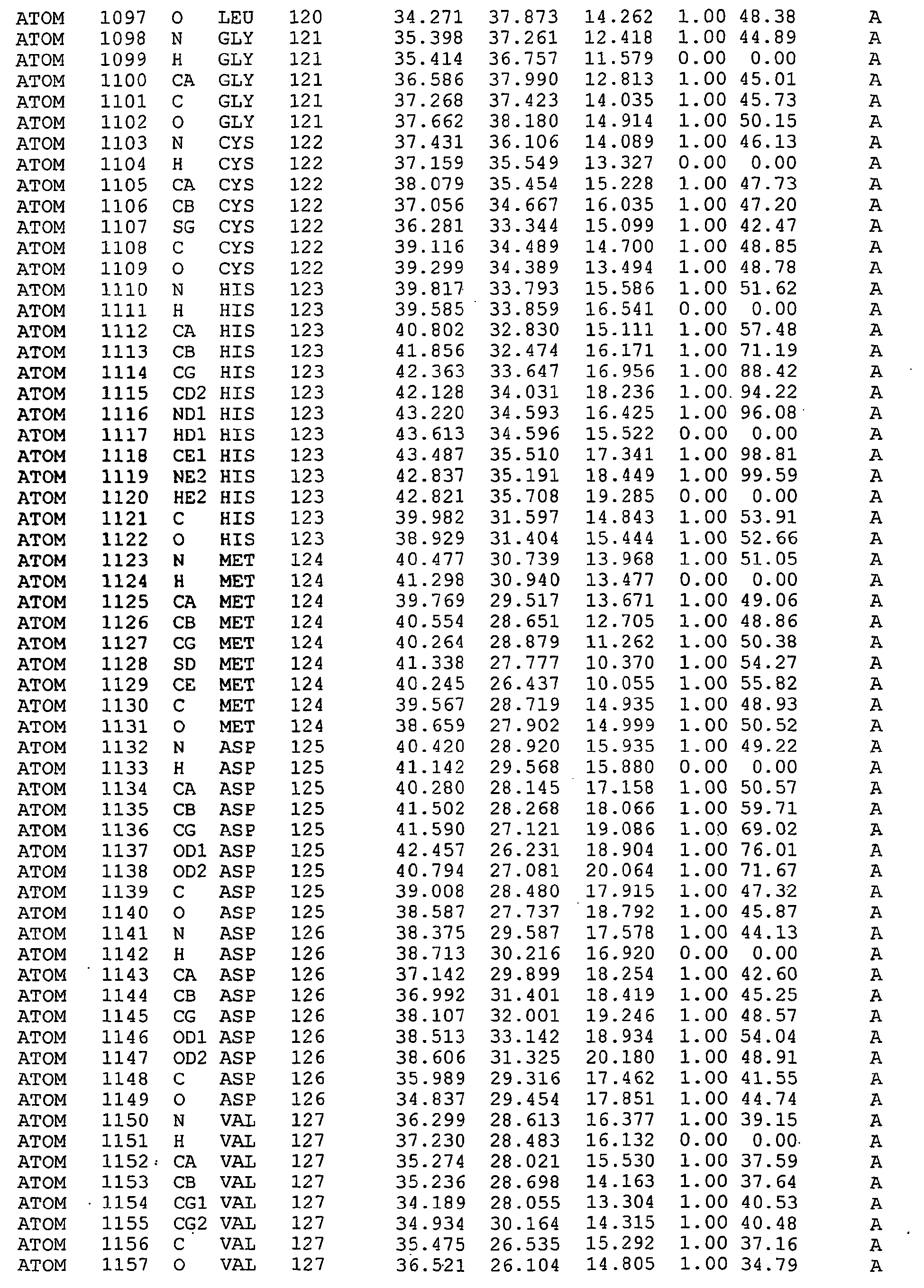

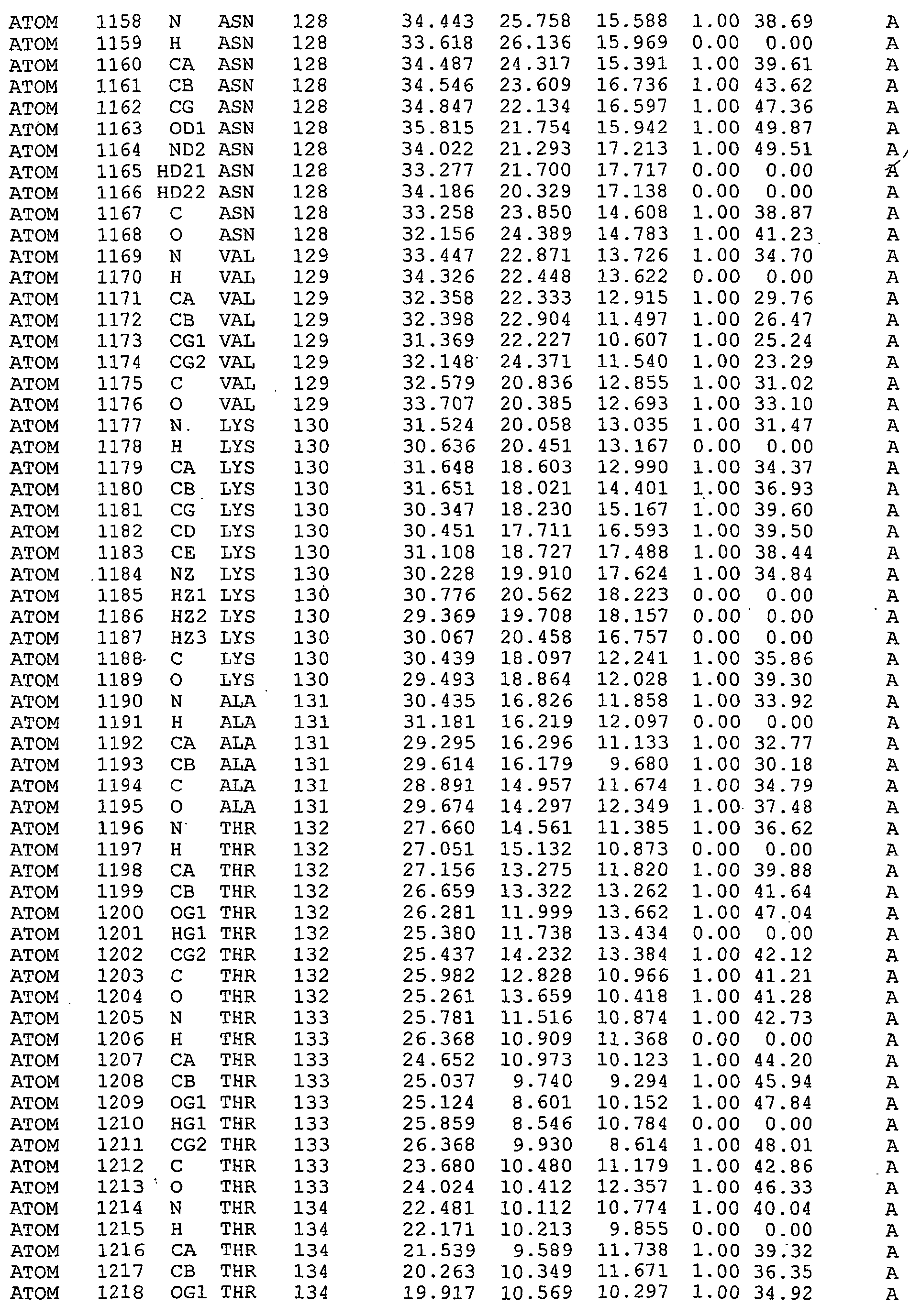

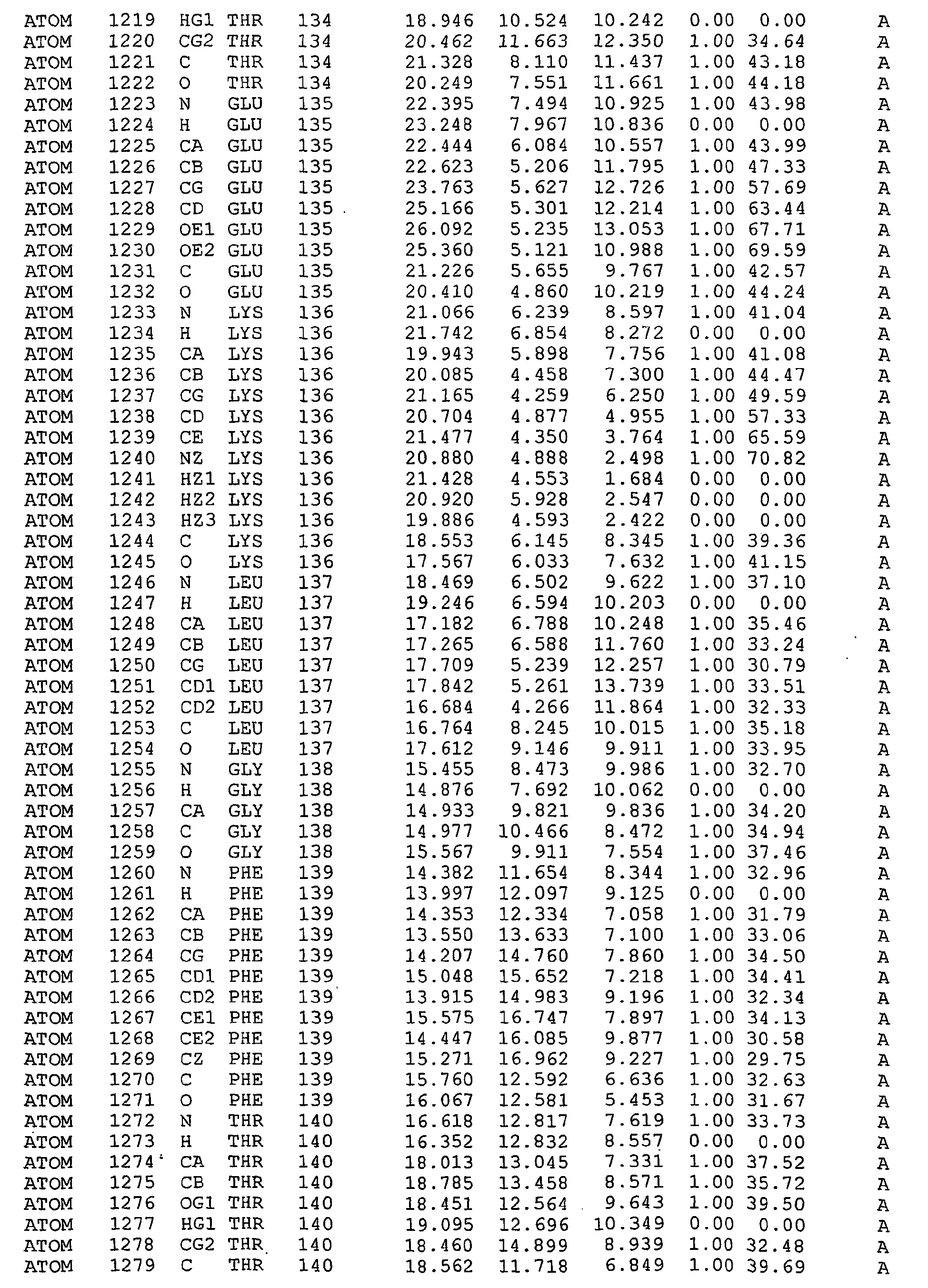

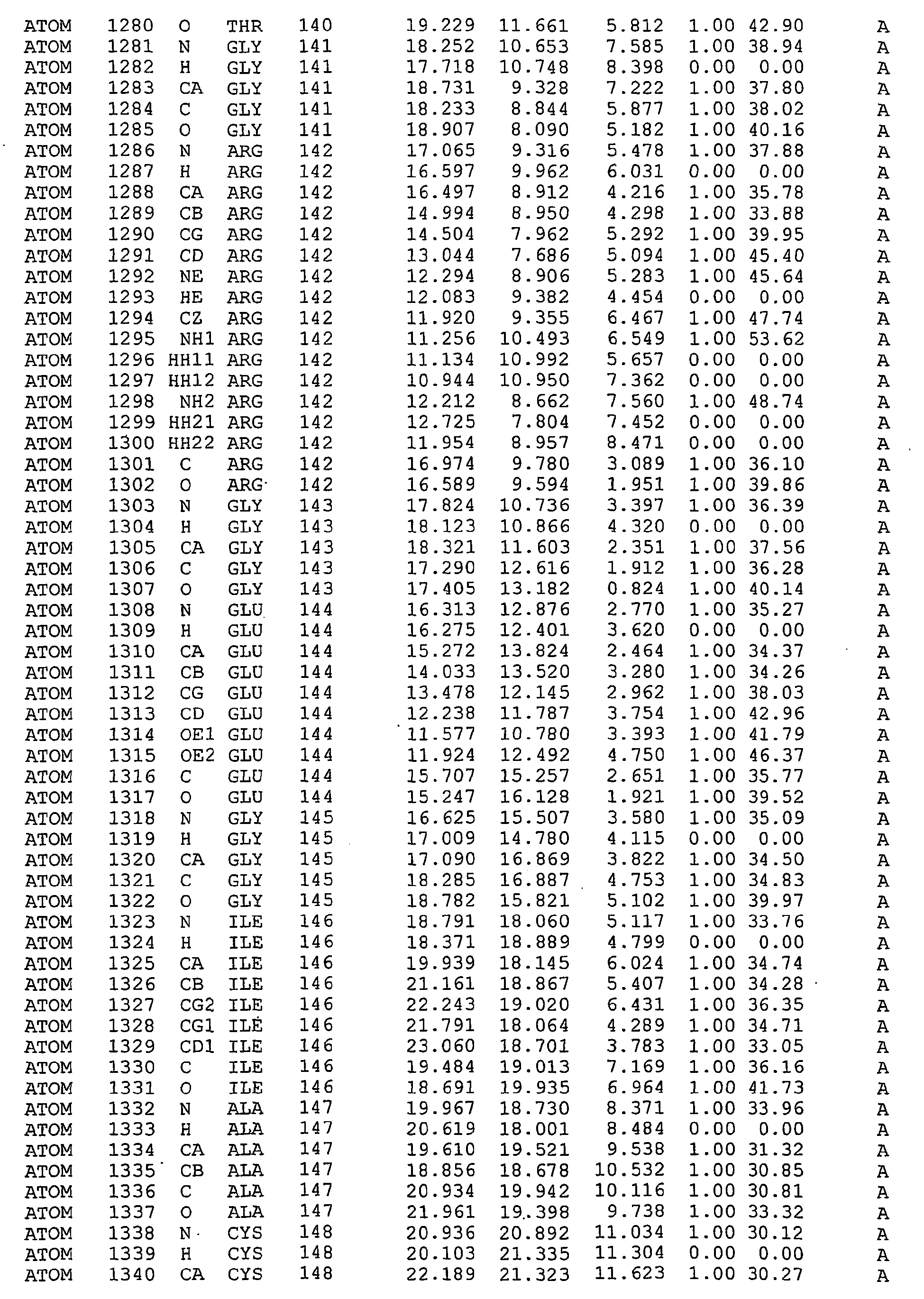

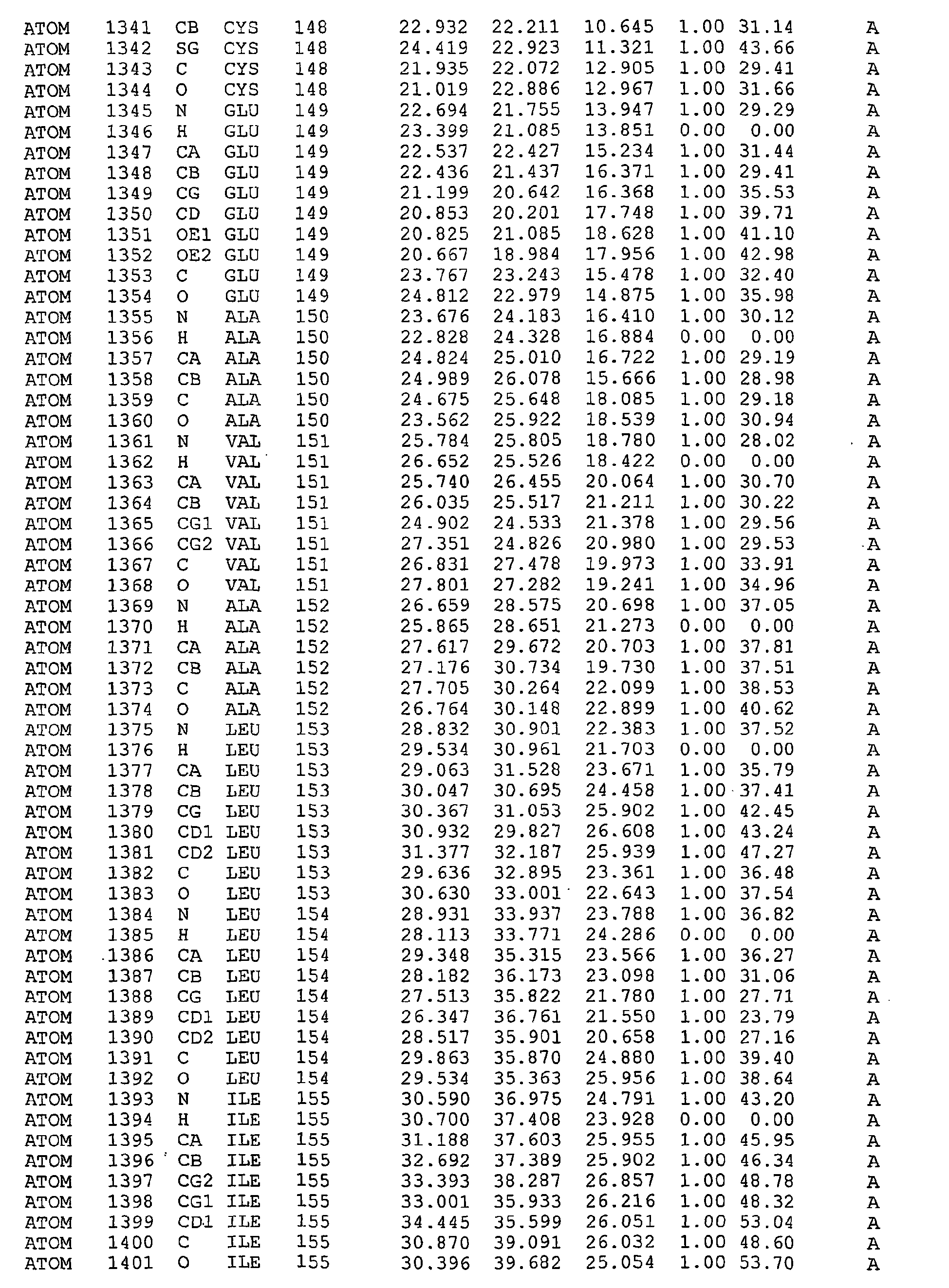

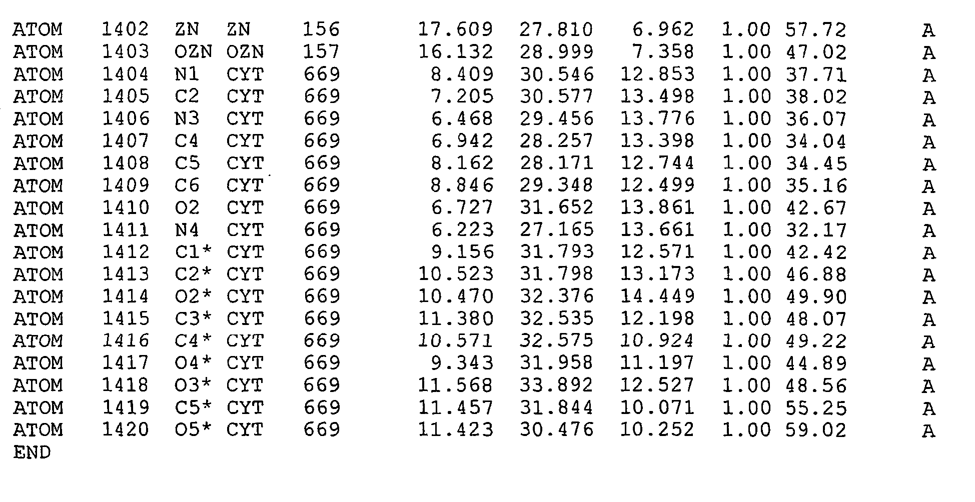

### Annex 2: Coordinates of structure mgcdp2

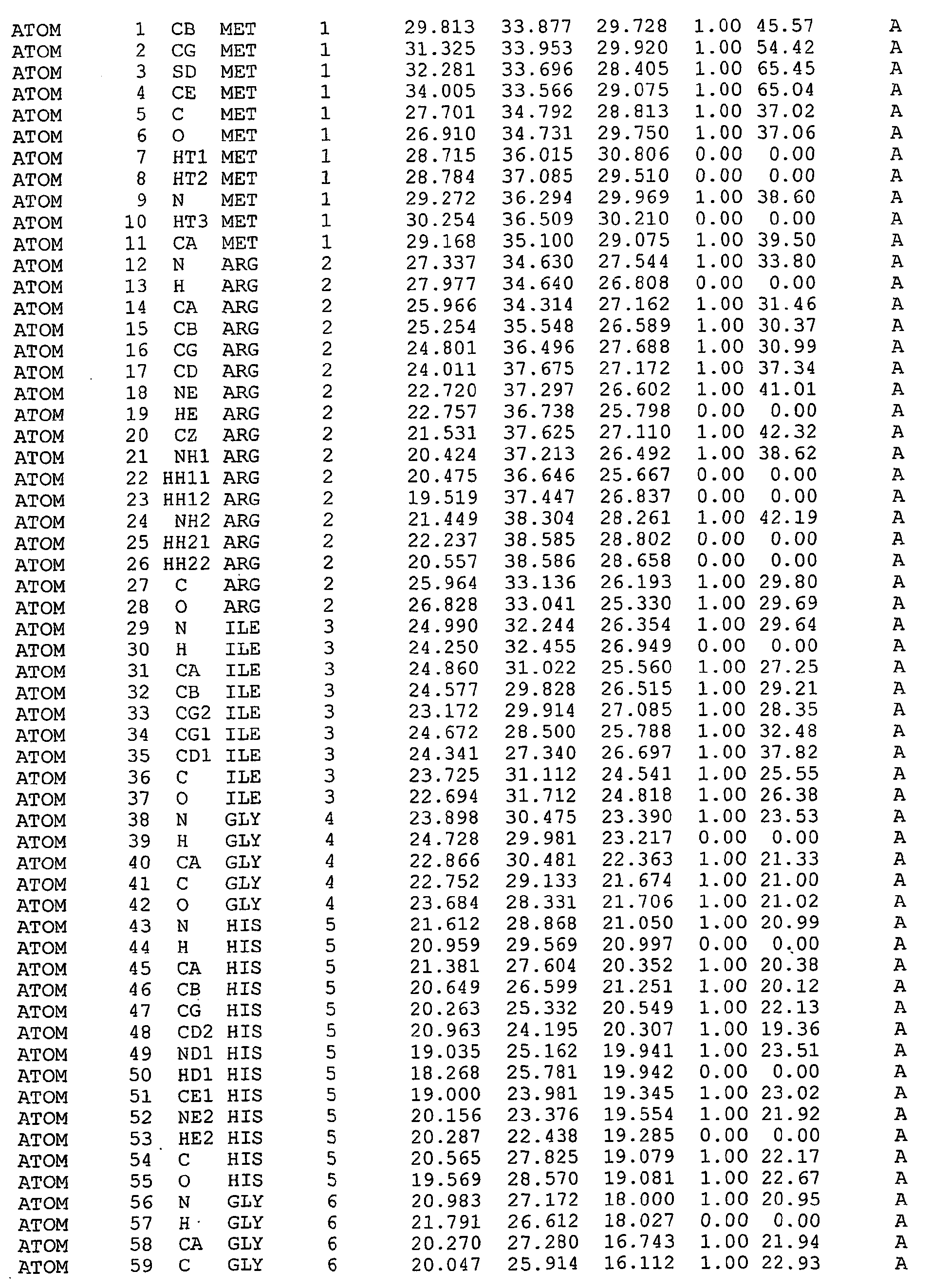

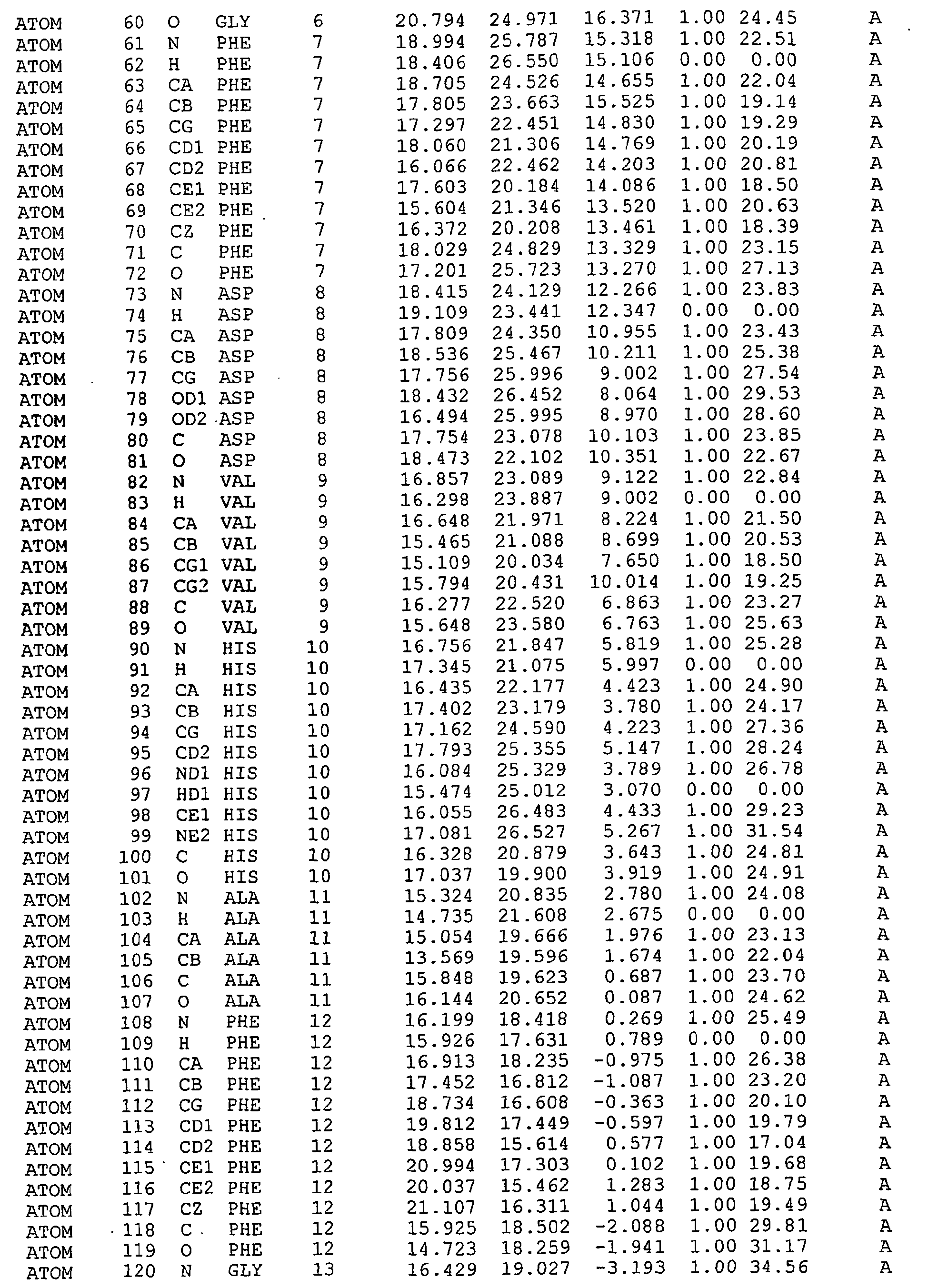

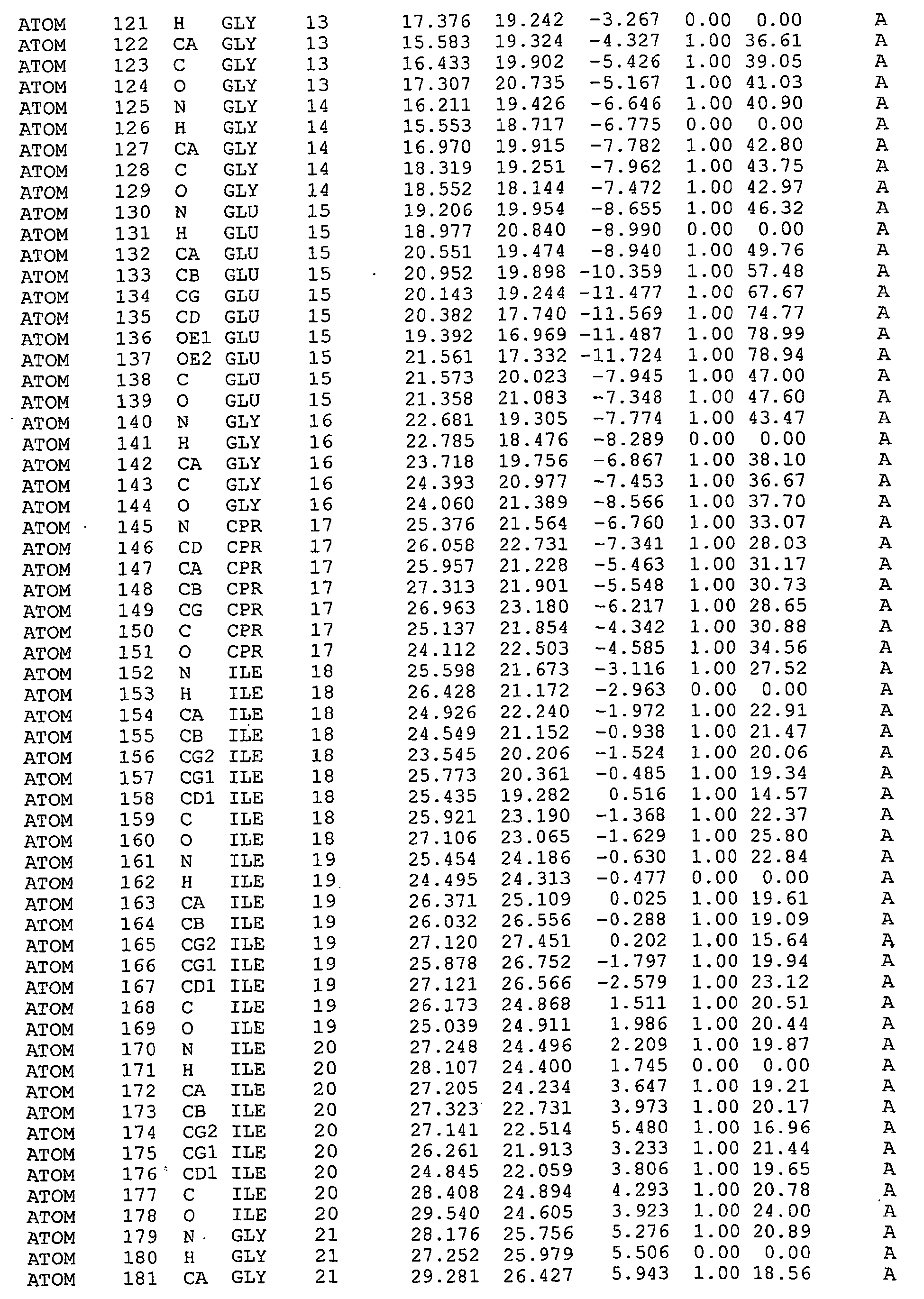

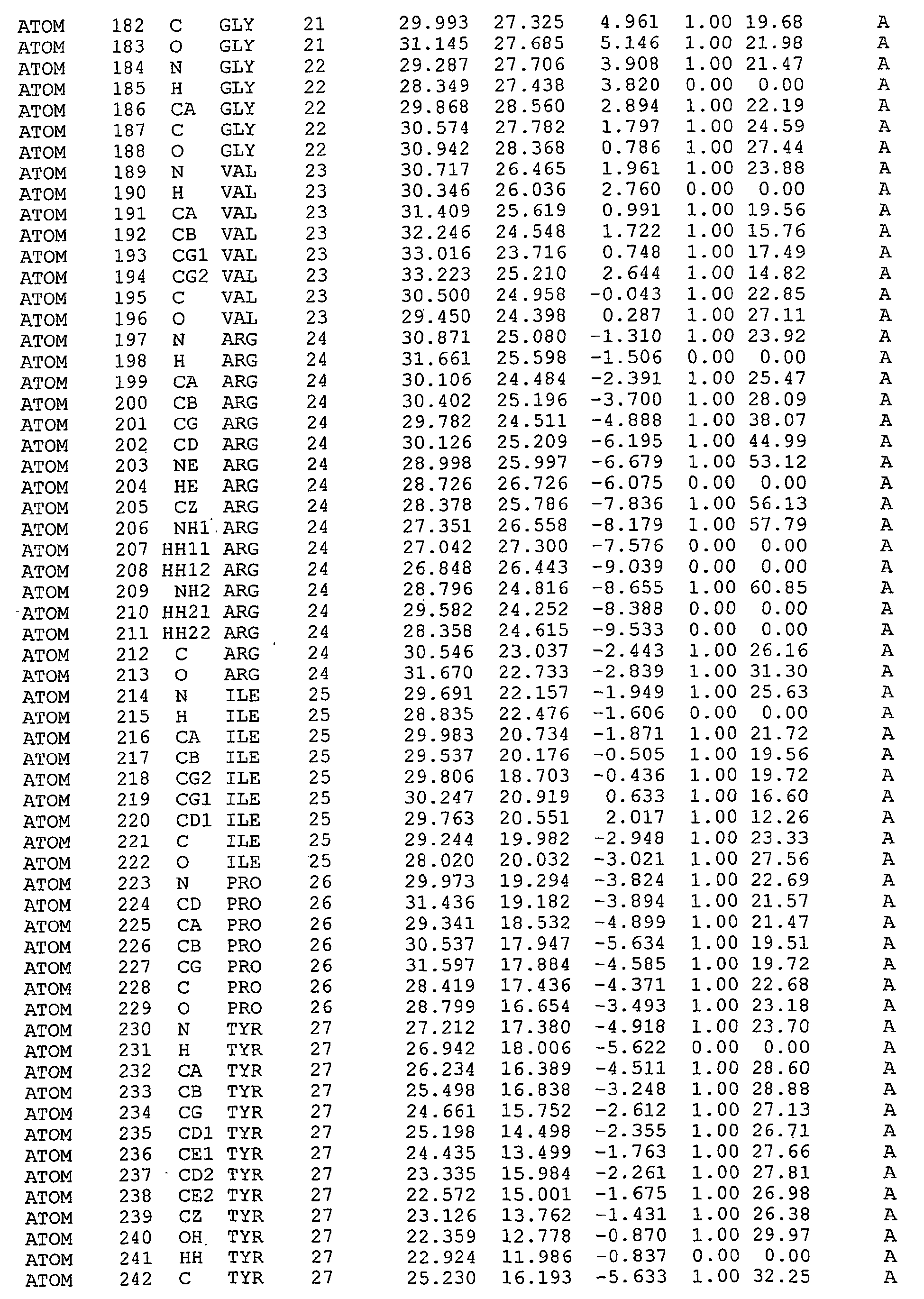

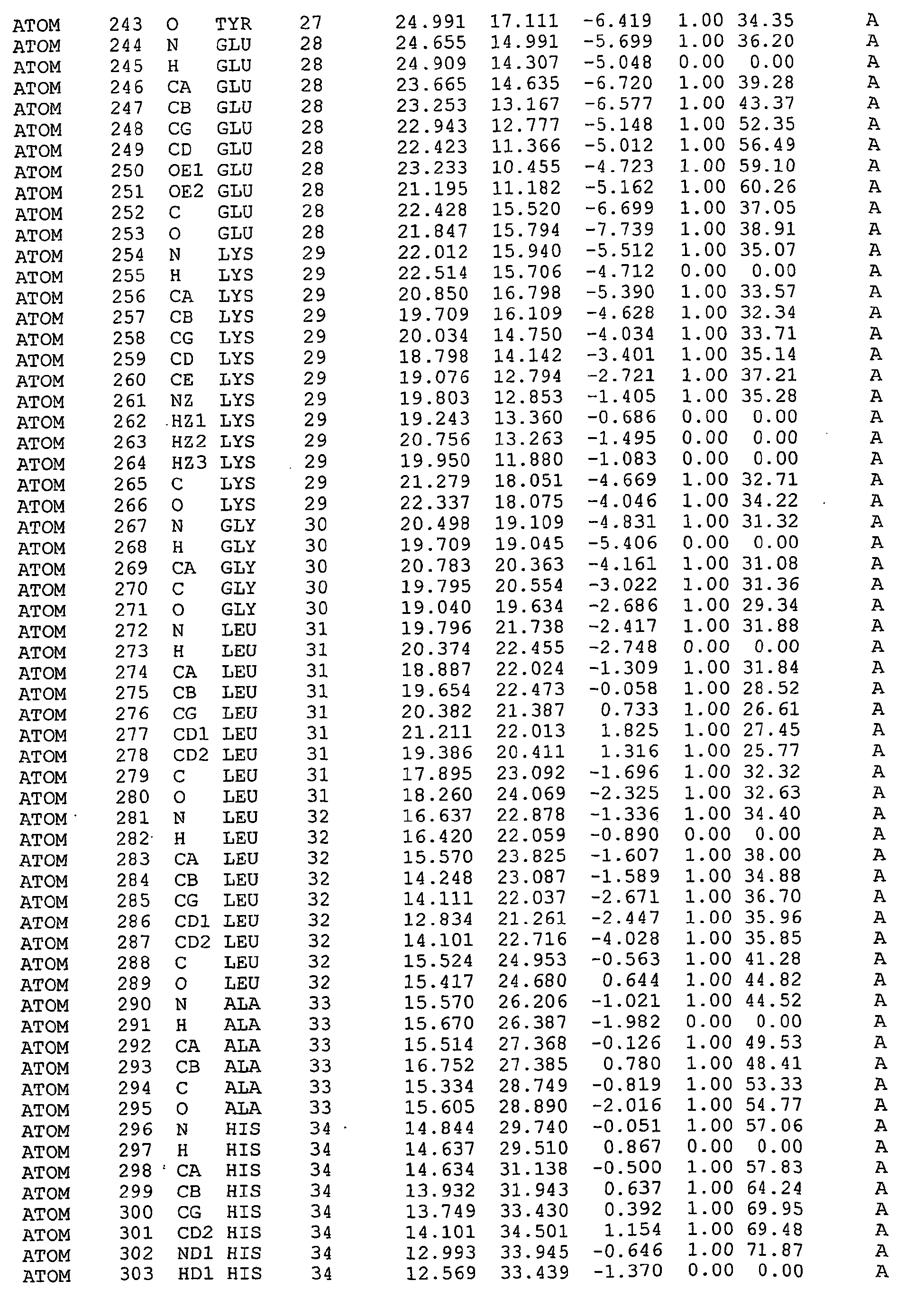

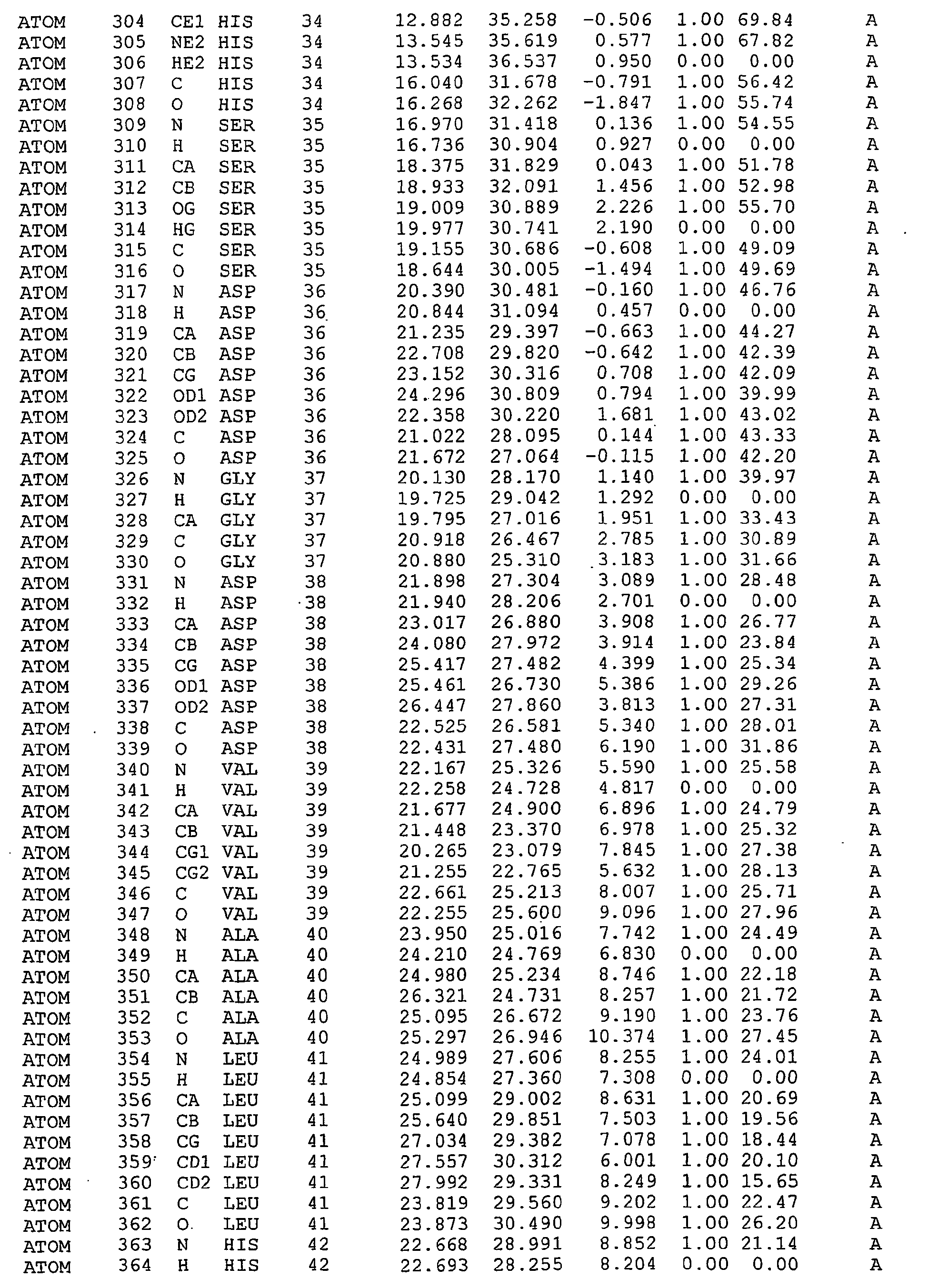

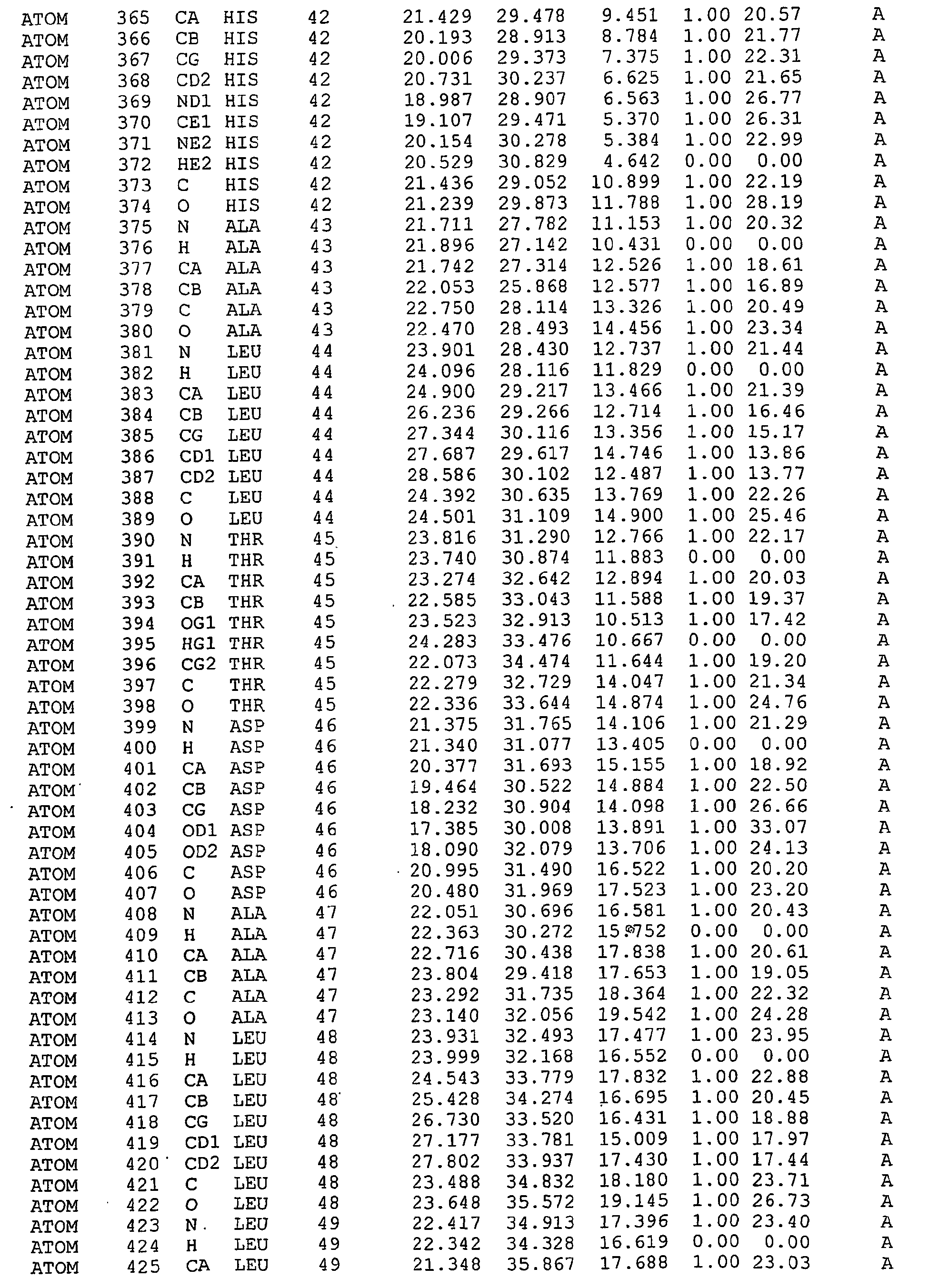

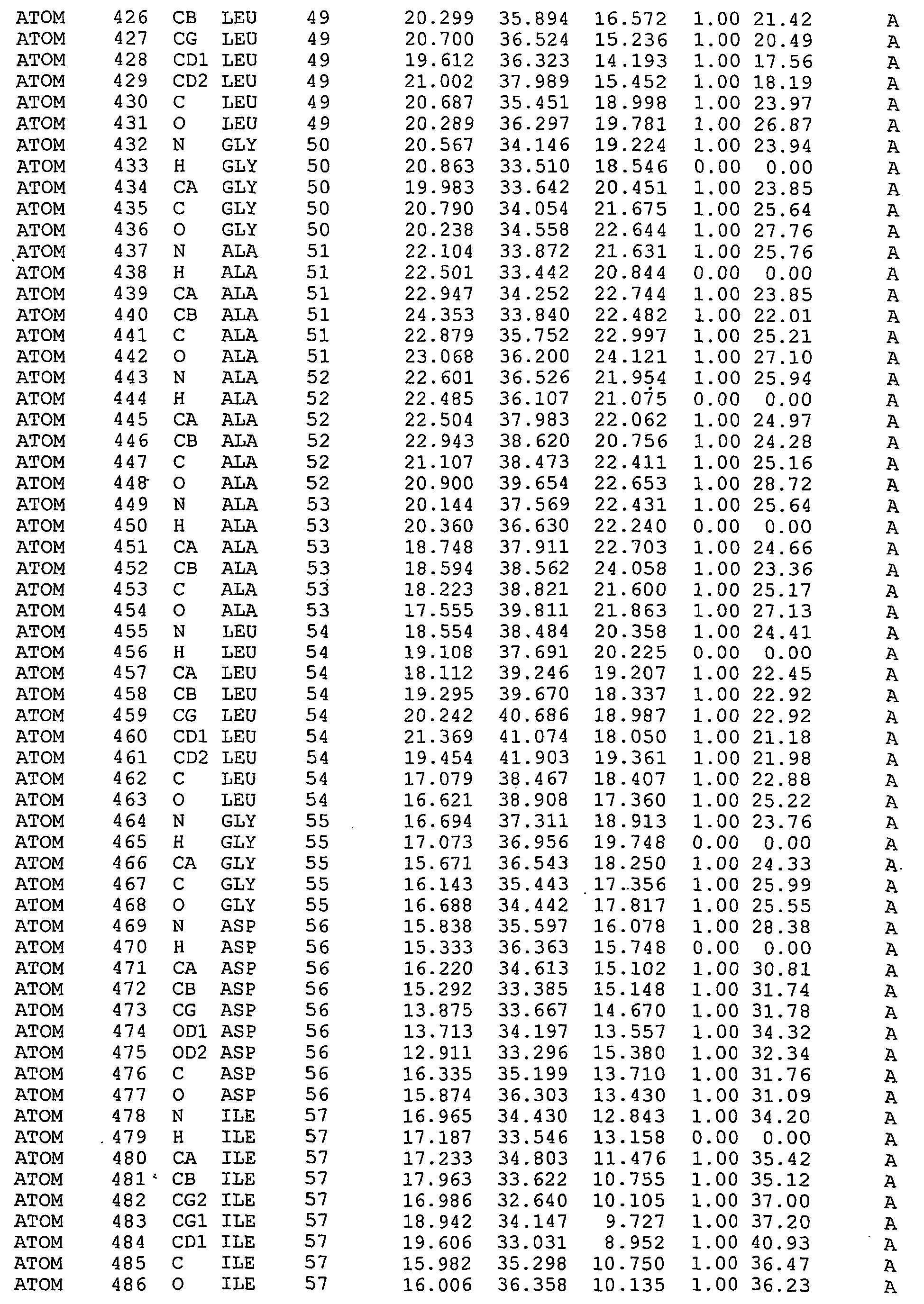

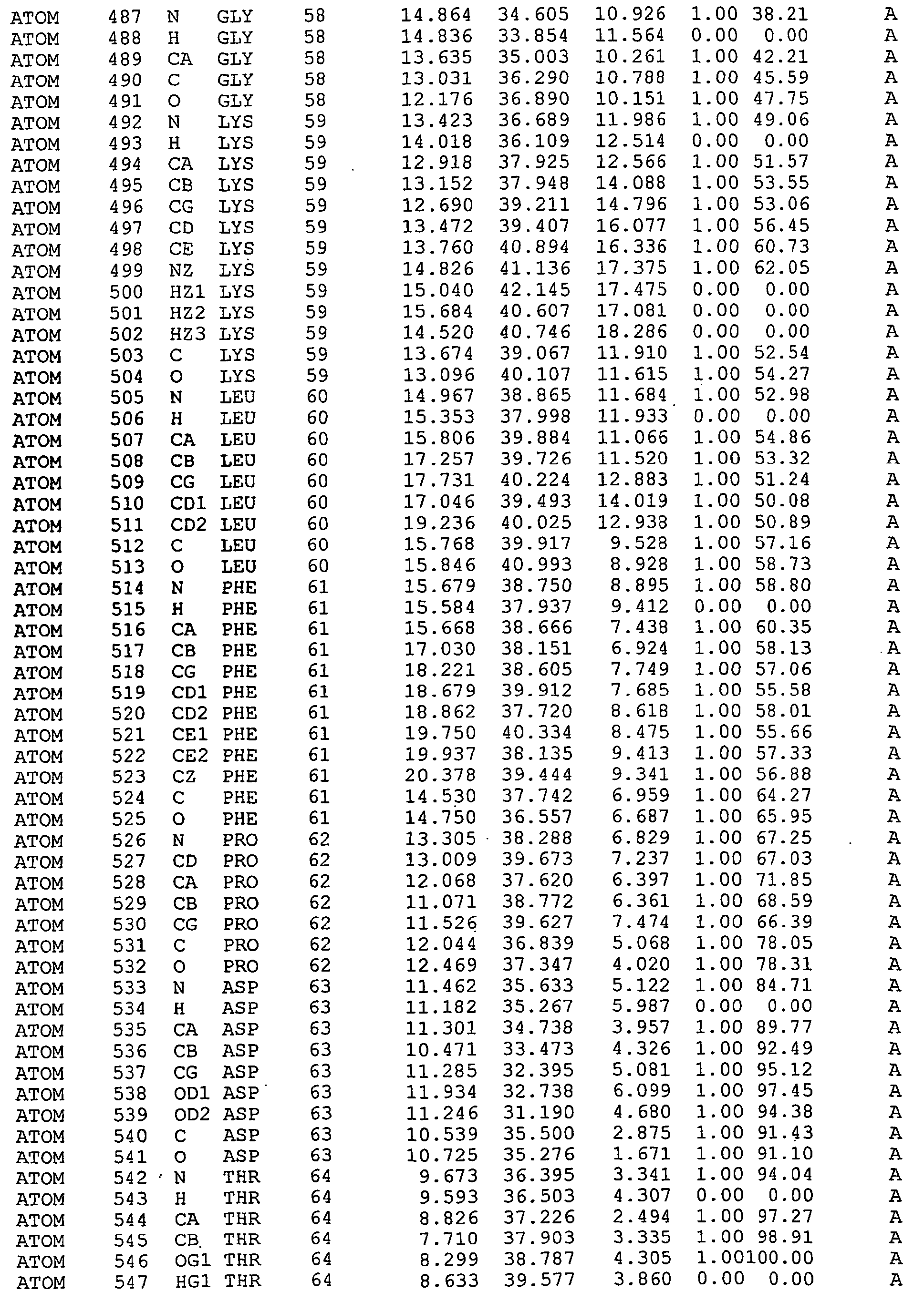

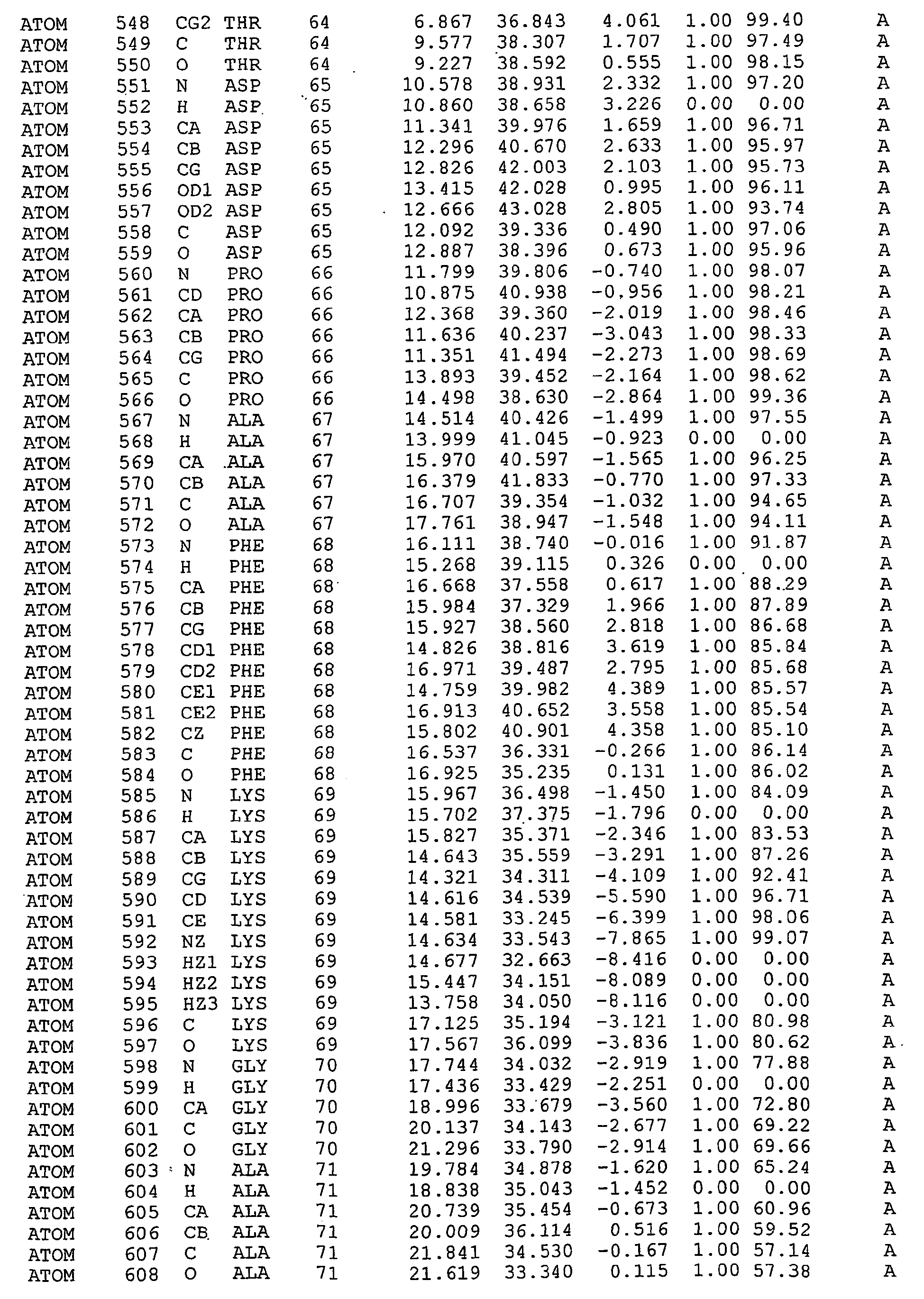

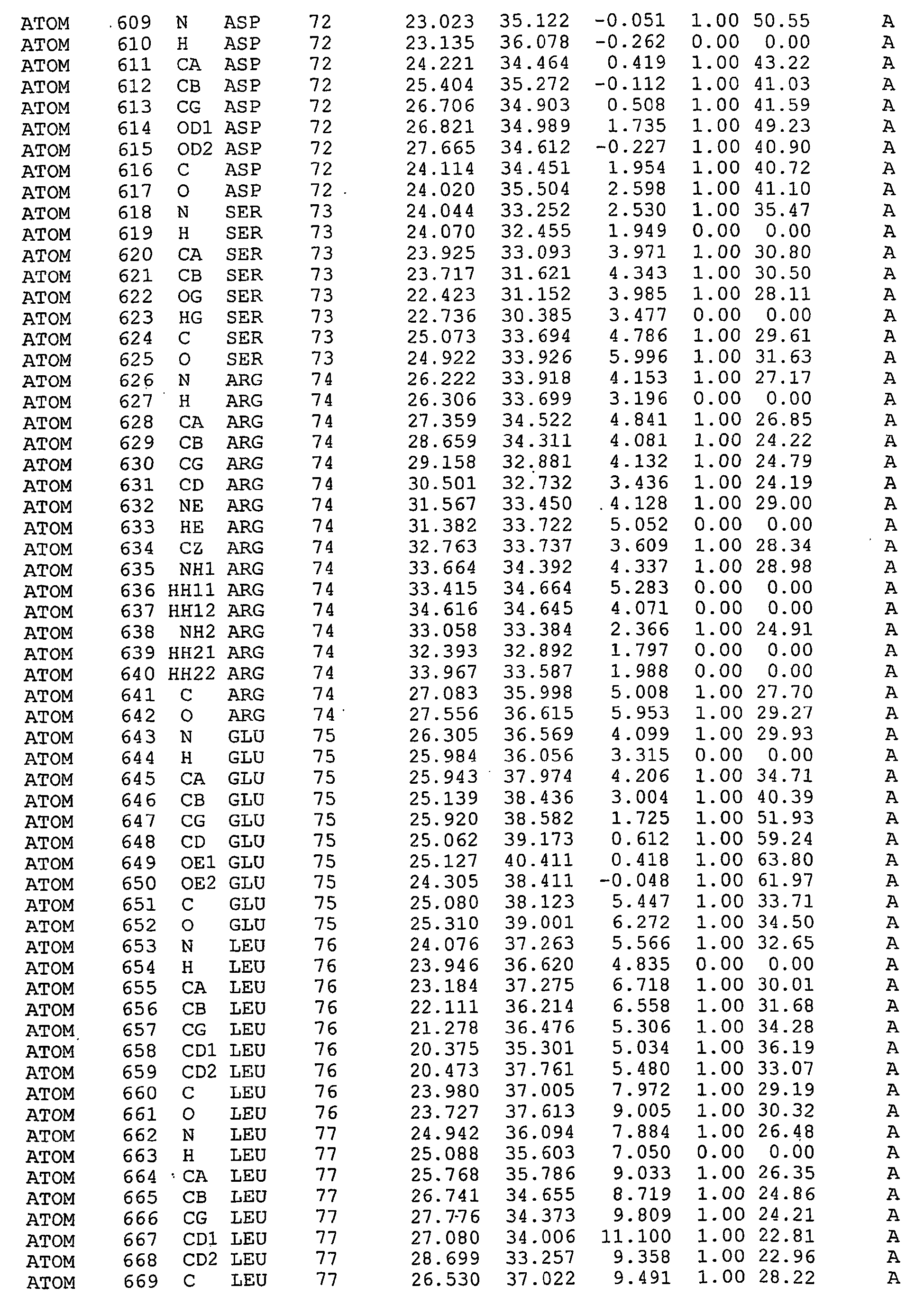

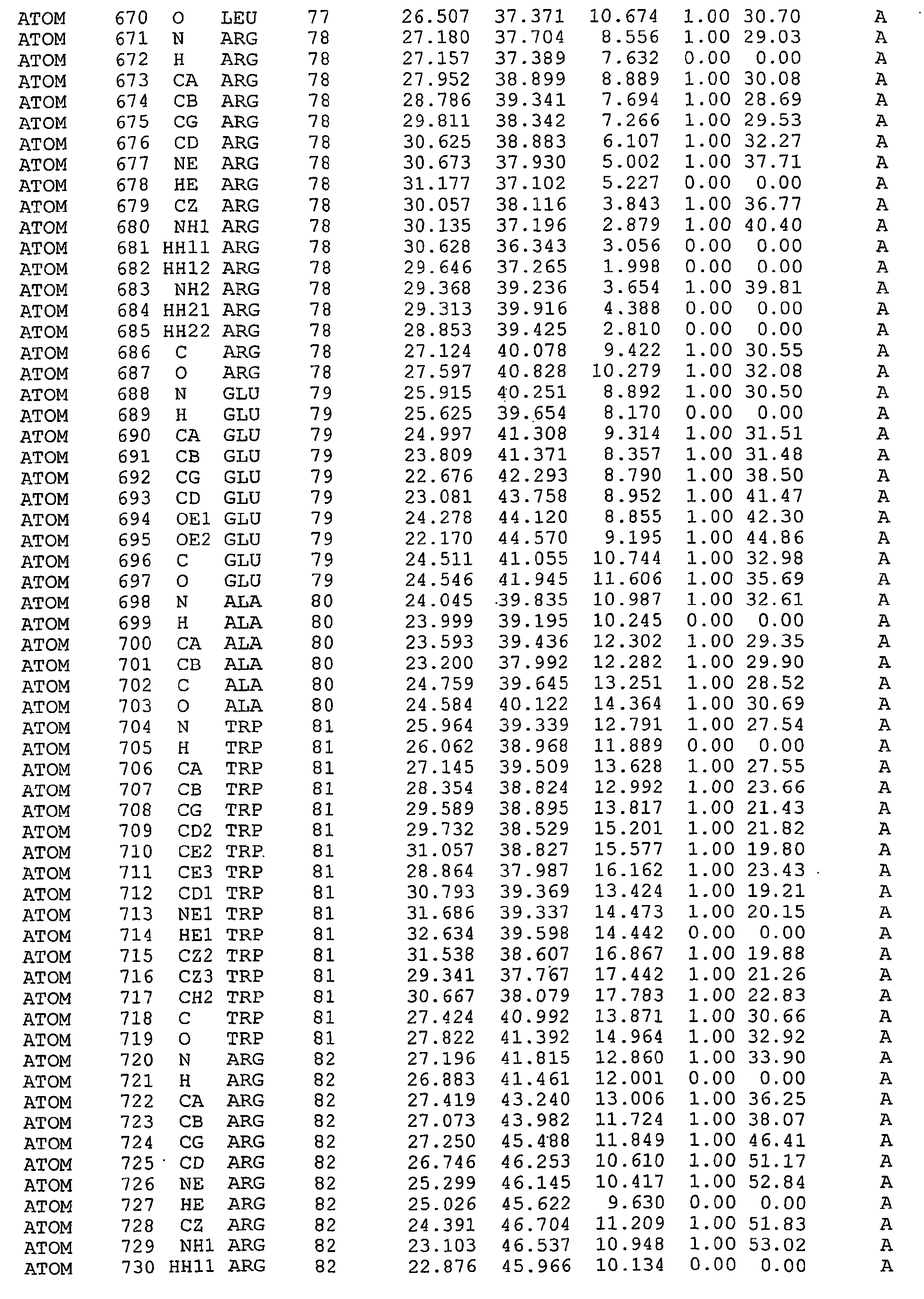

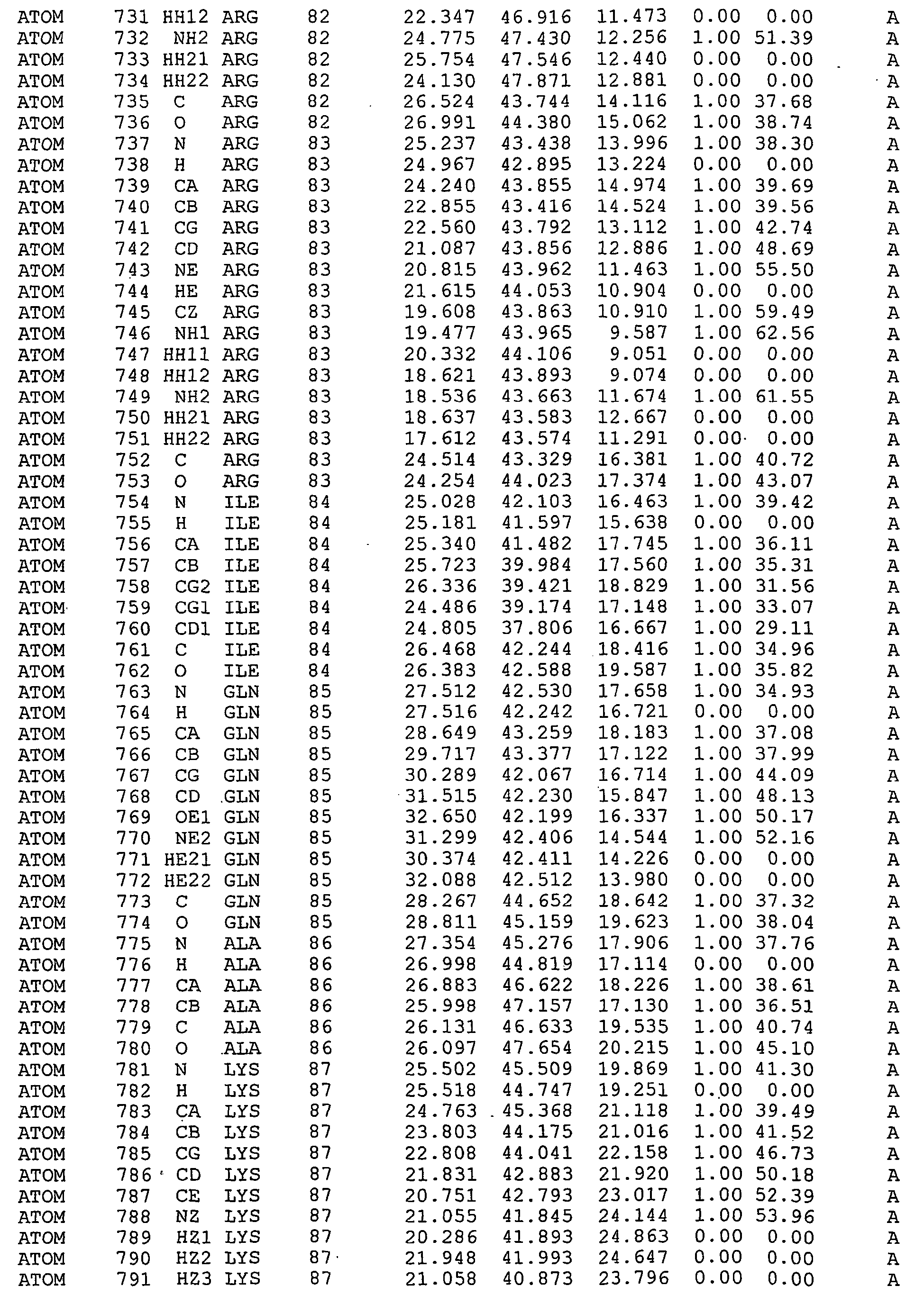

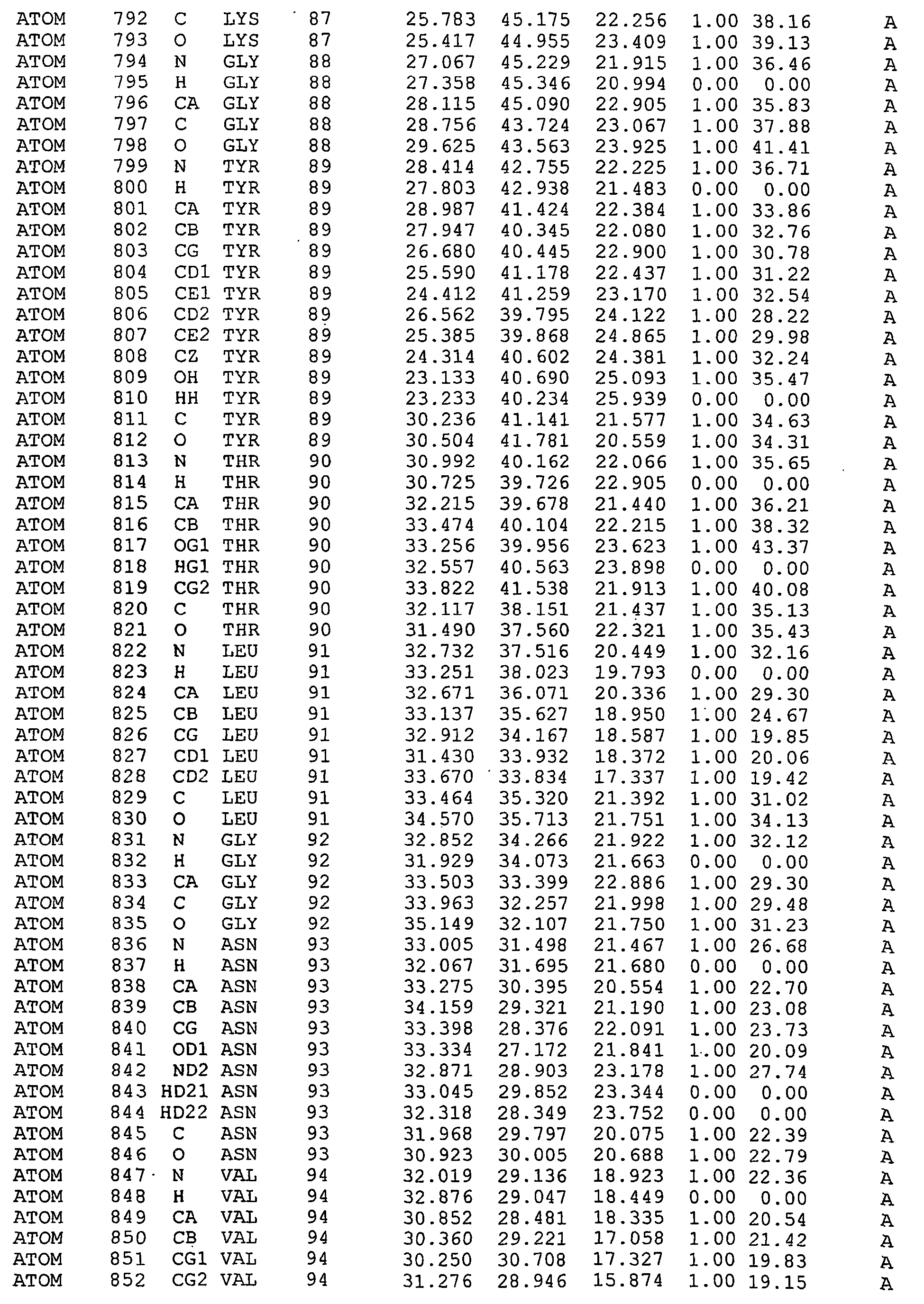

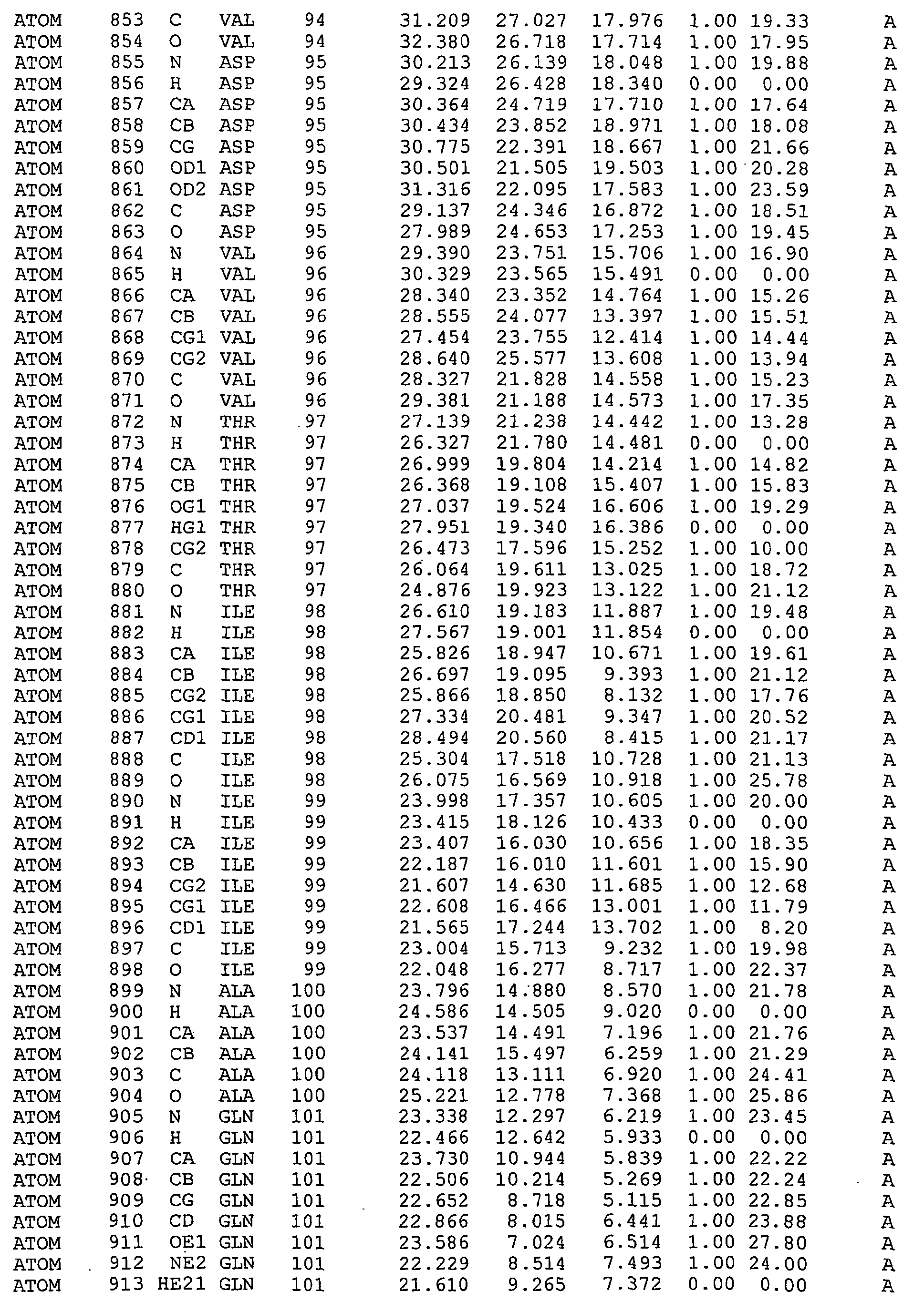

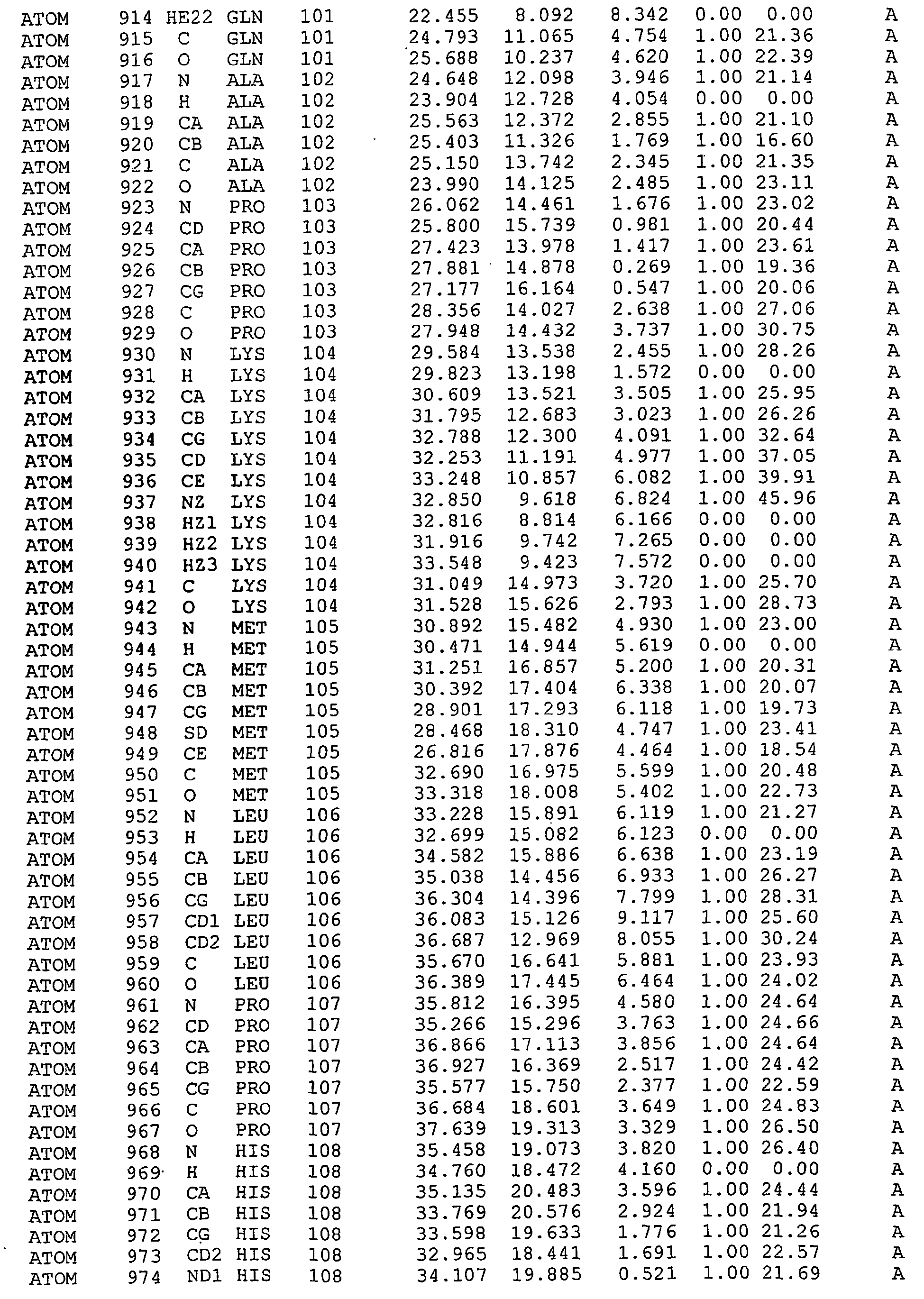

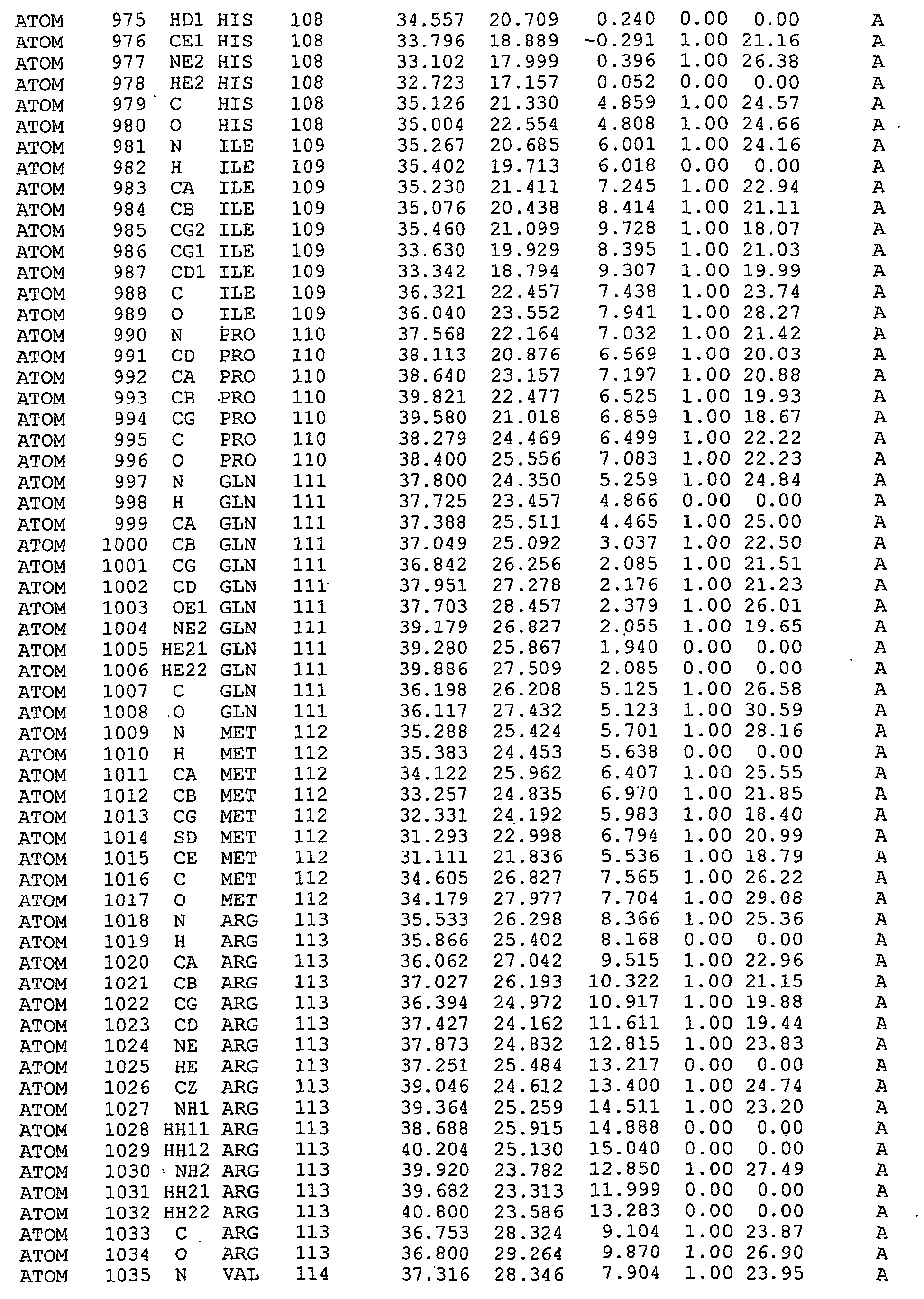

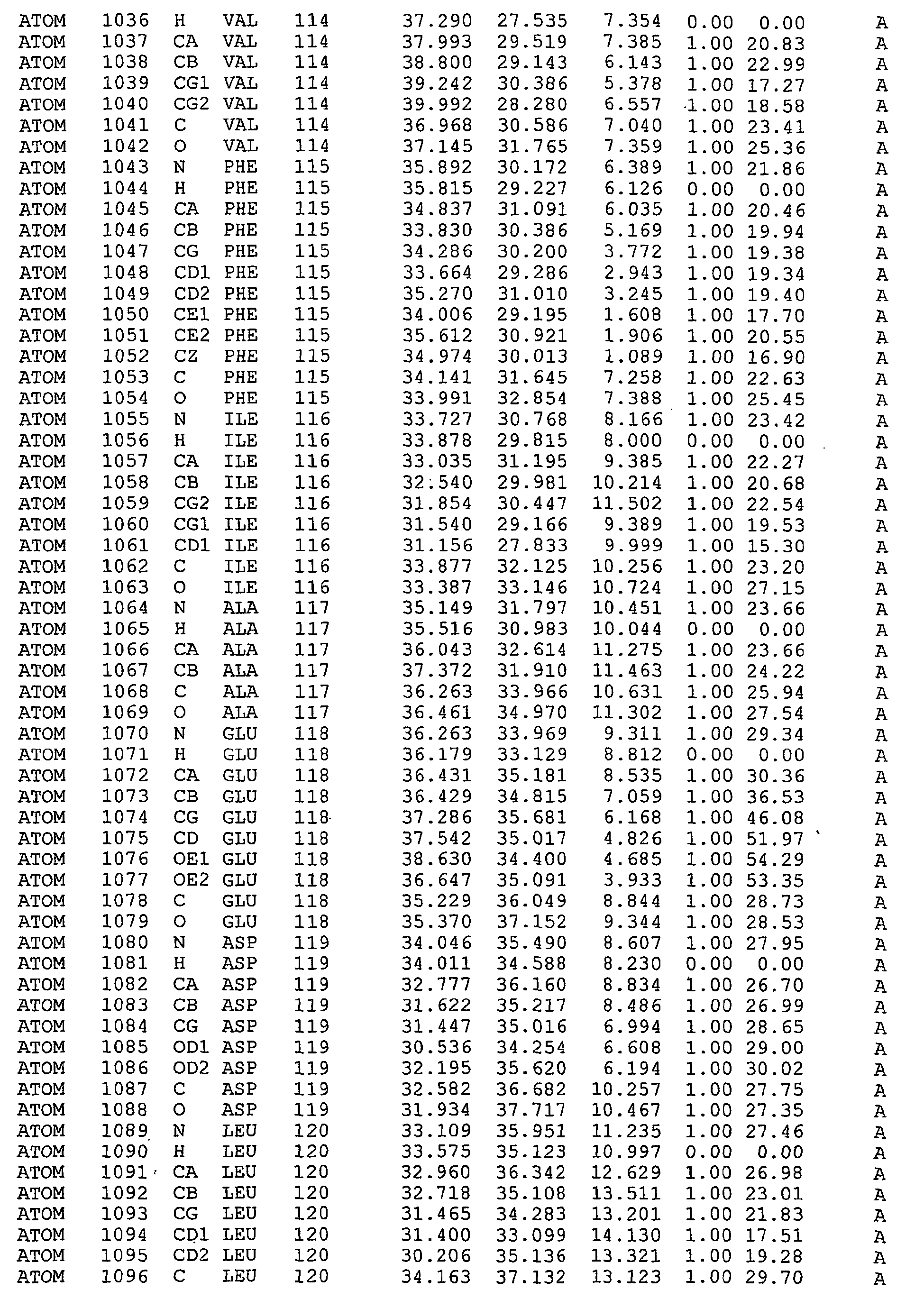

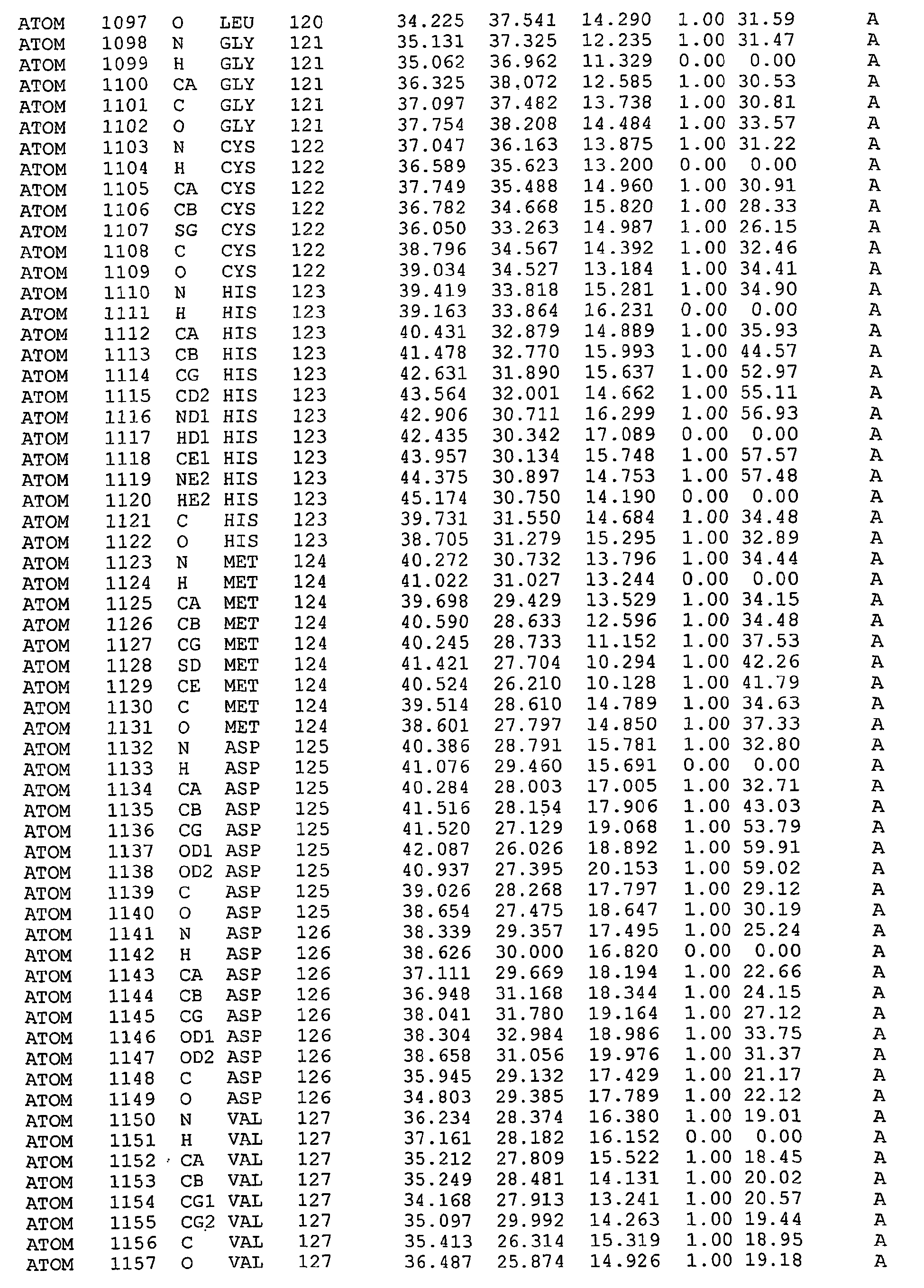

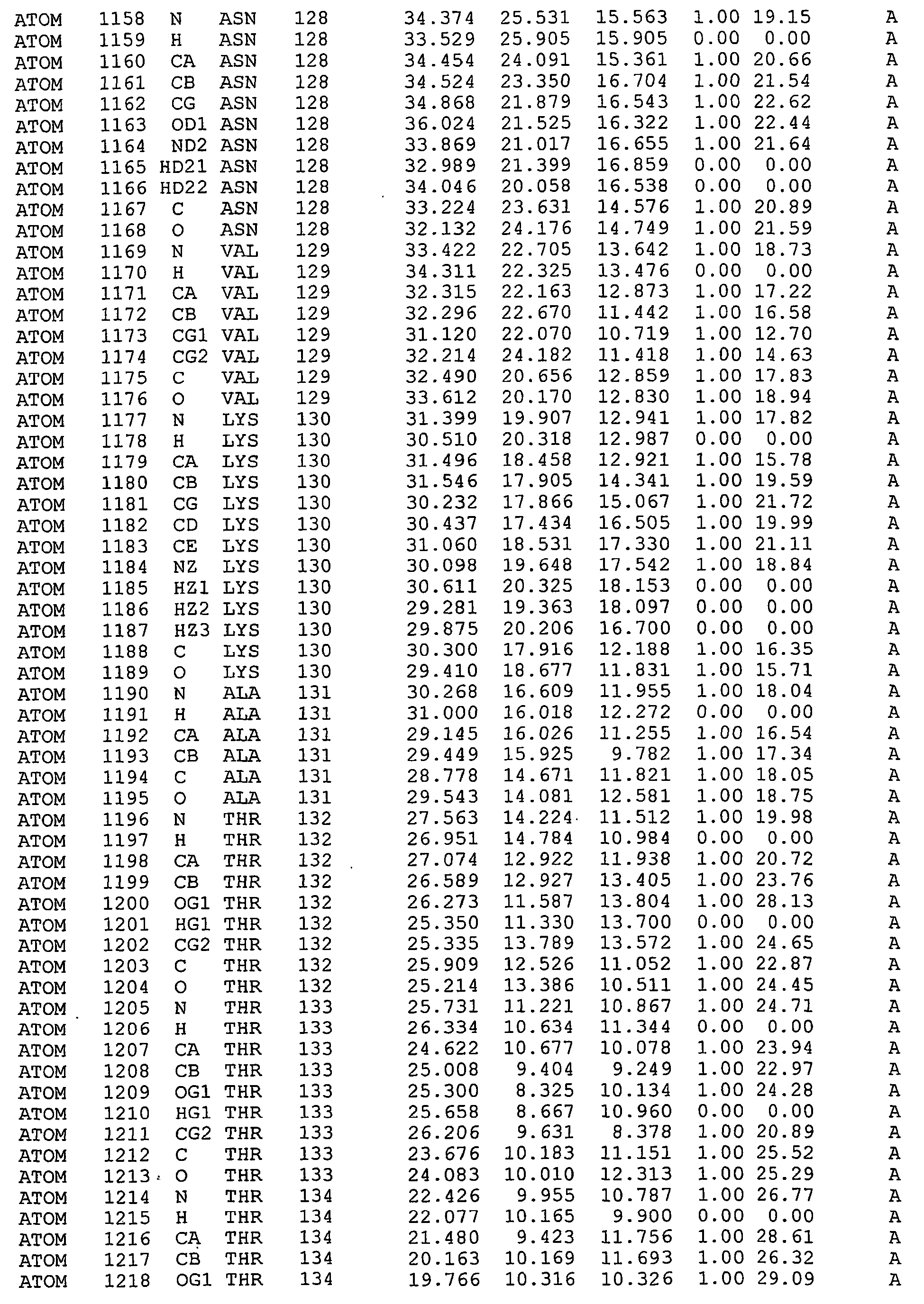

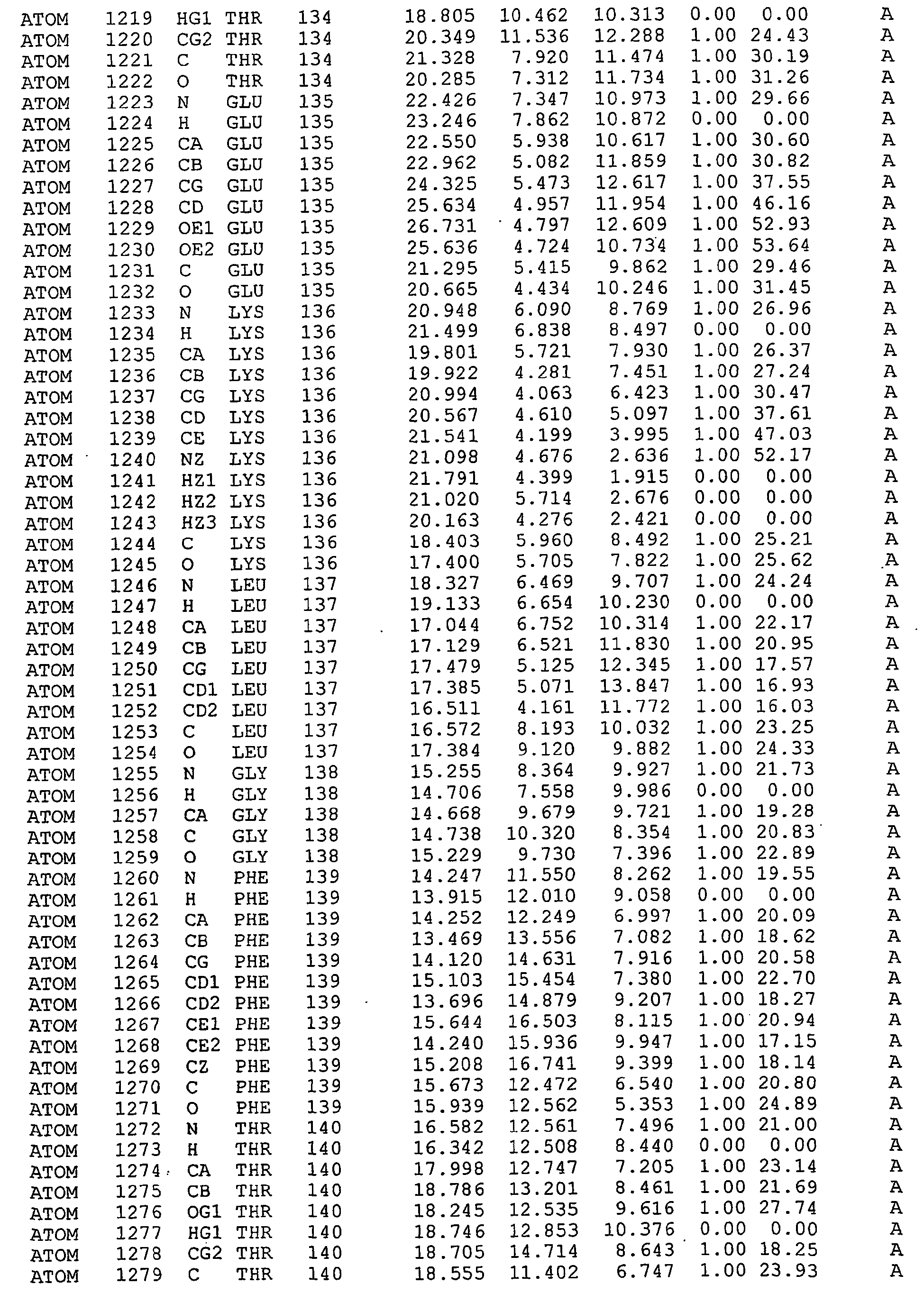

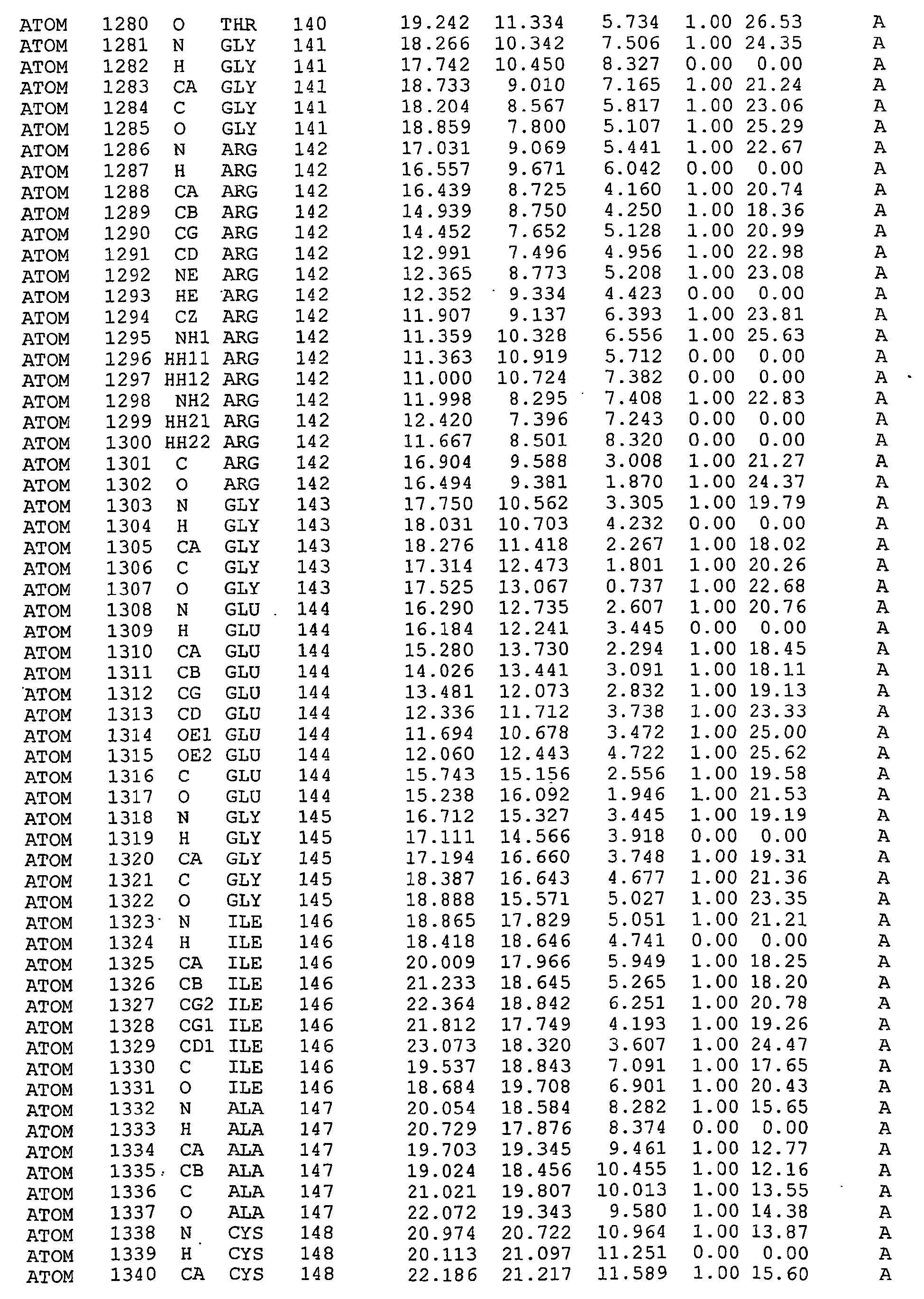

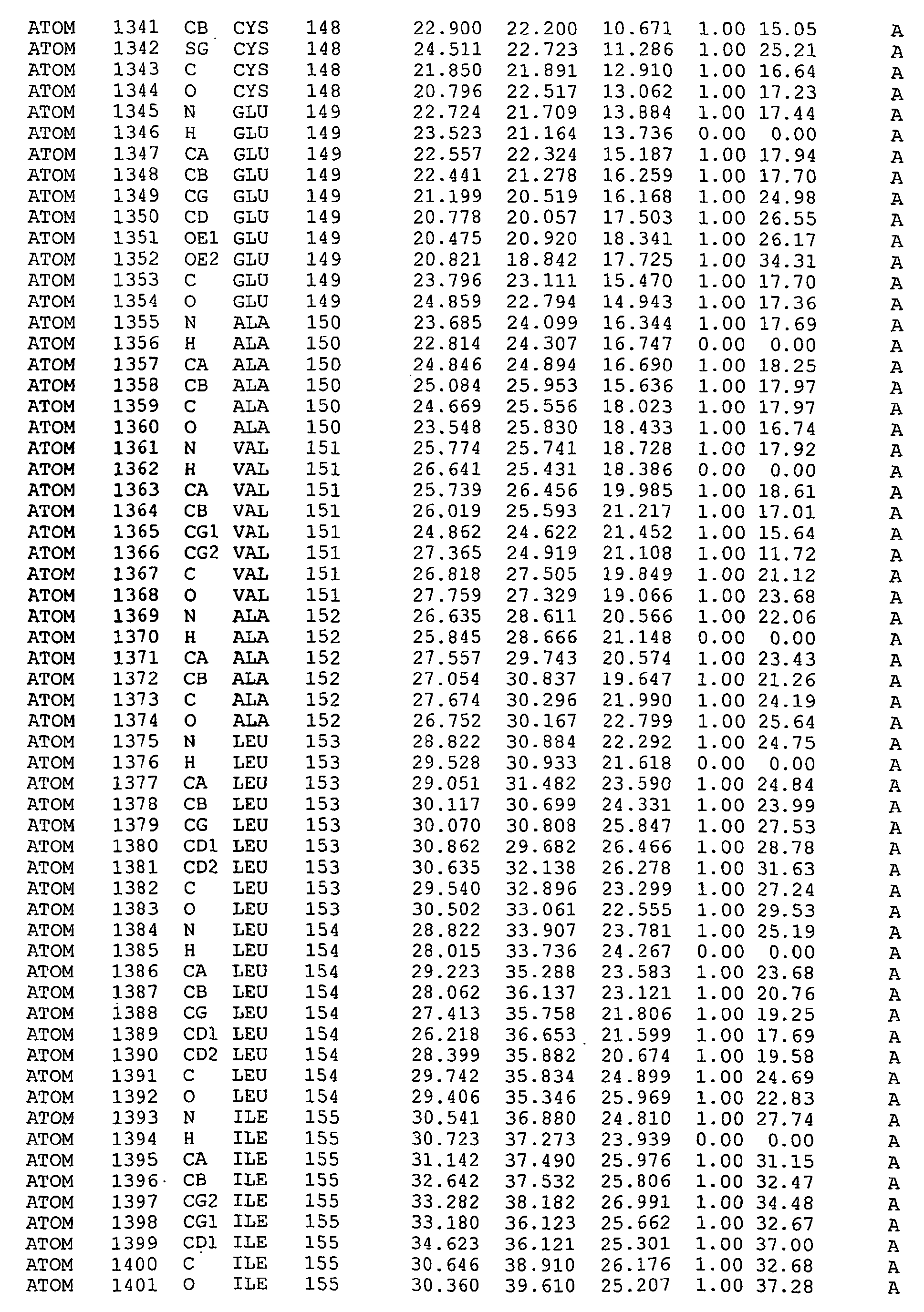

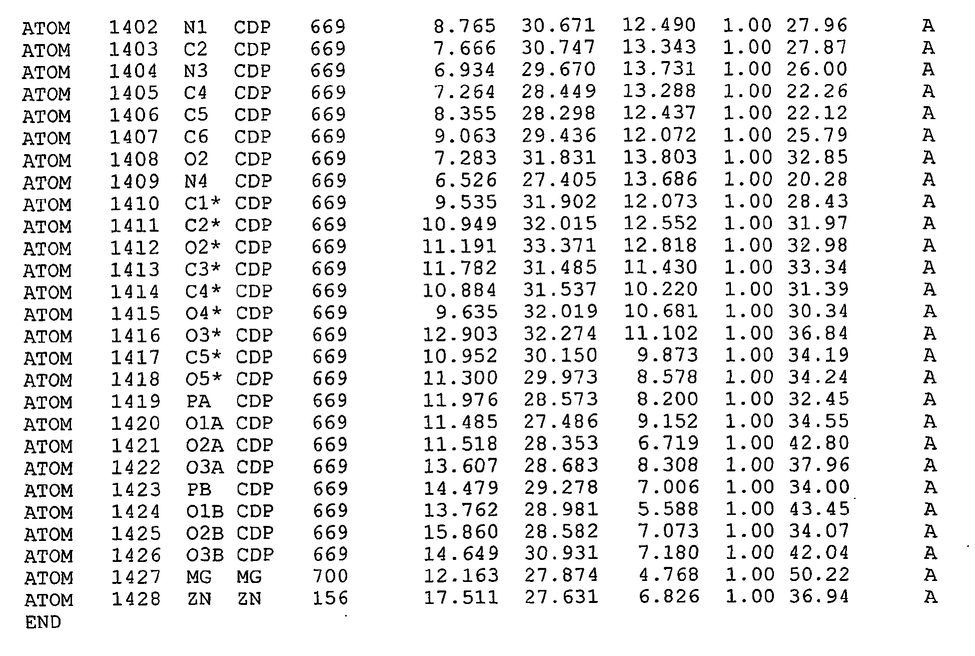

### Annex 3: Coordinates of structure nat2

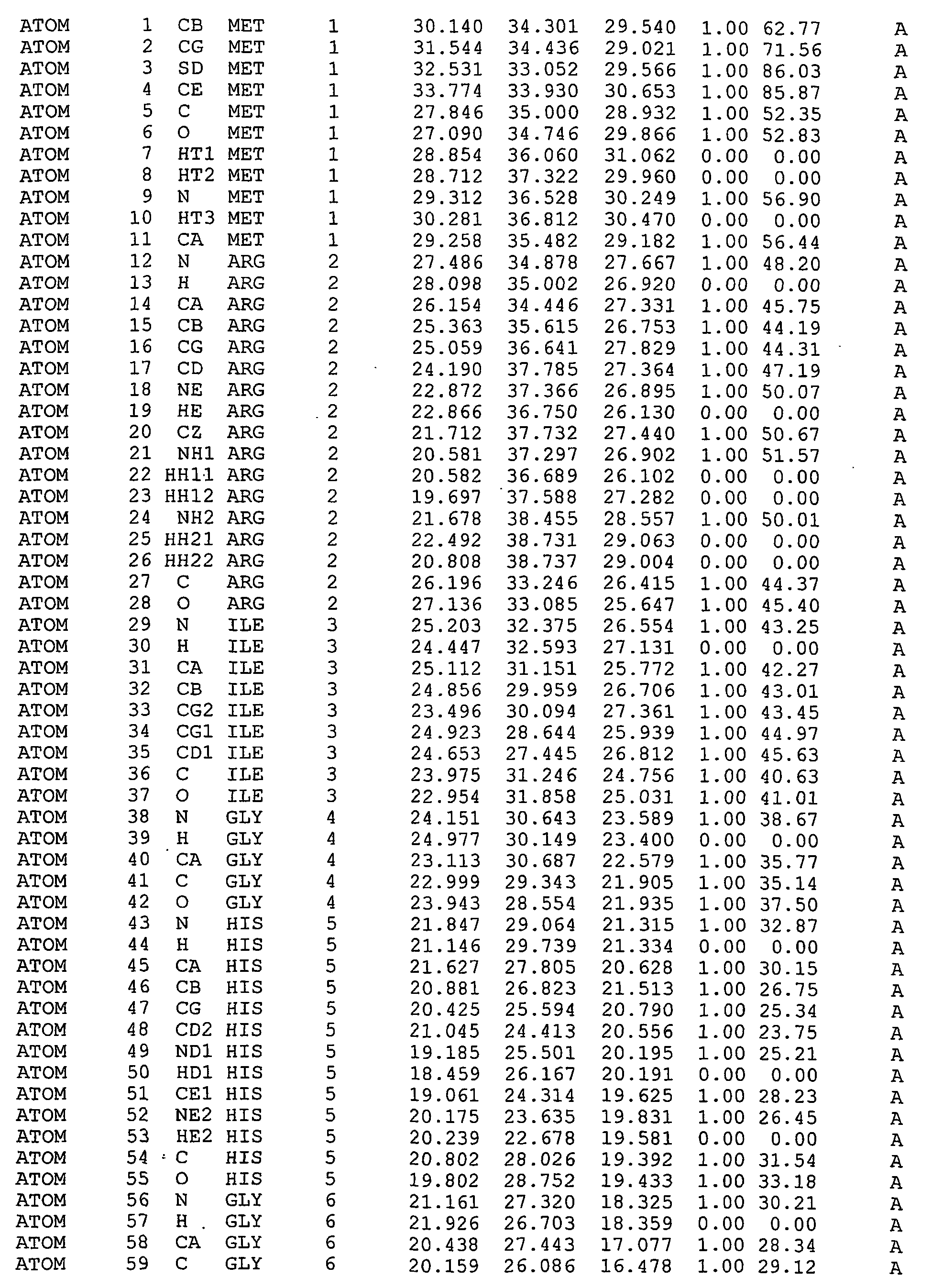

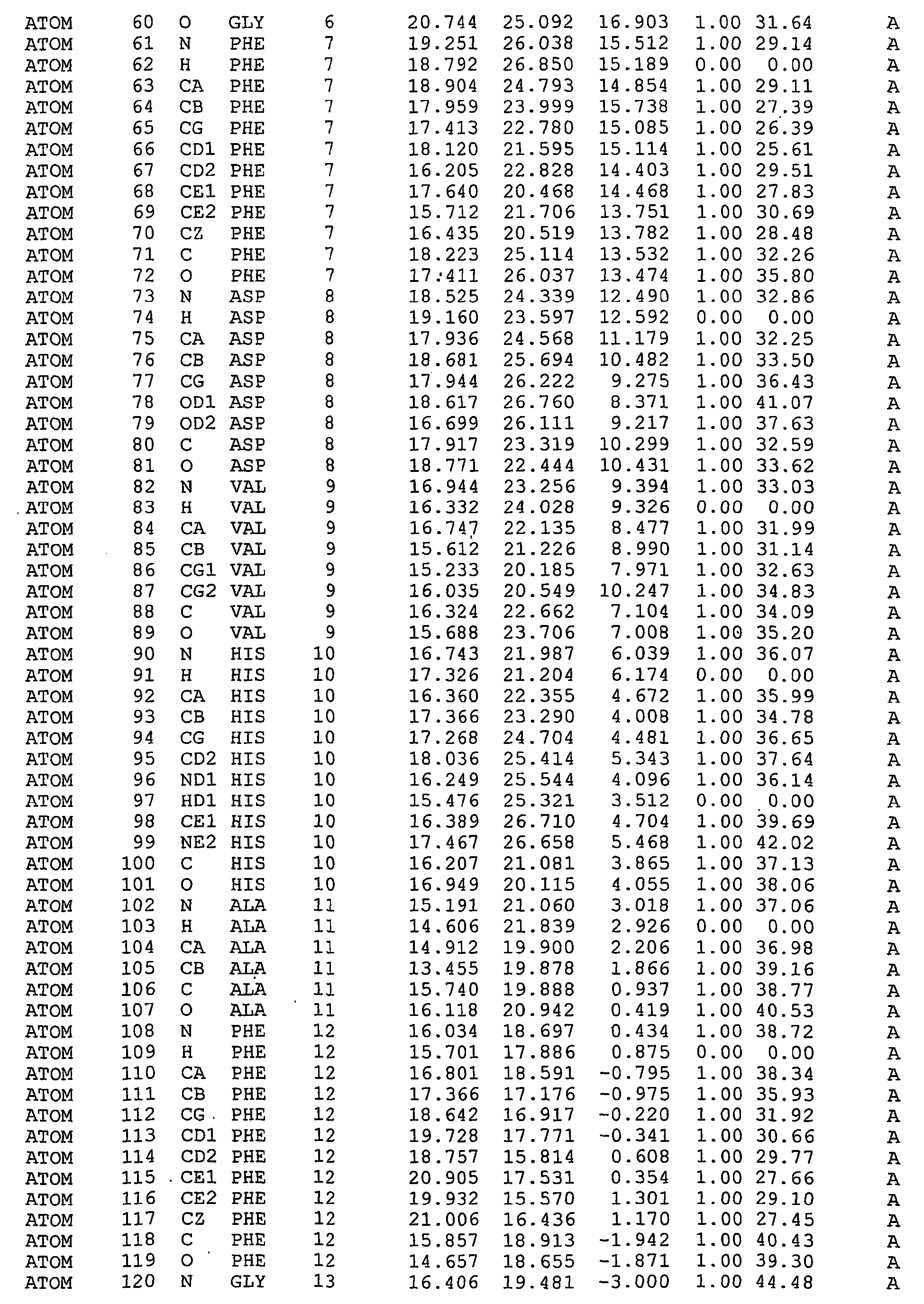

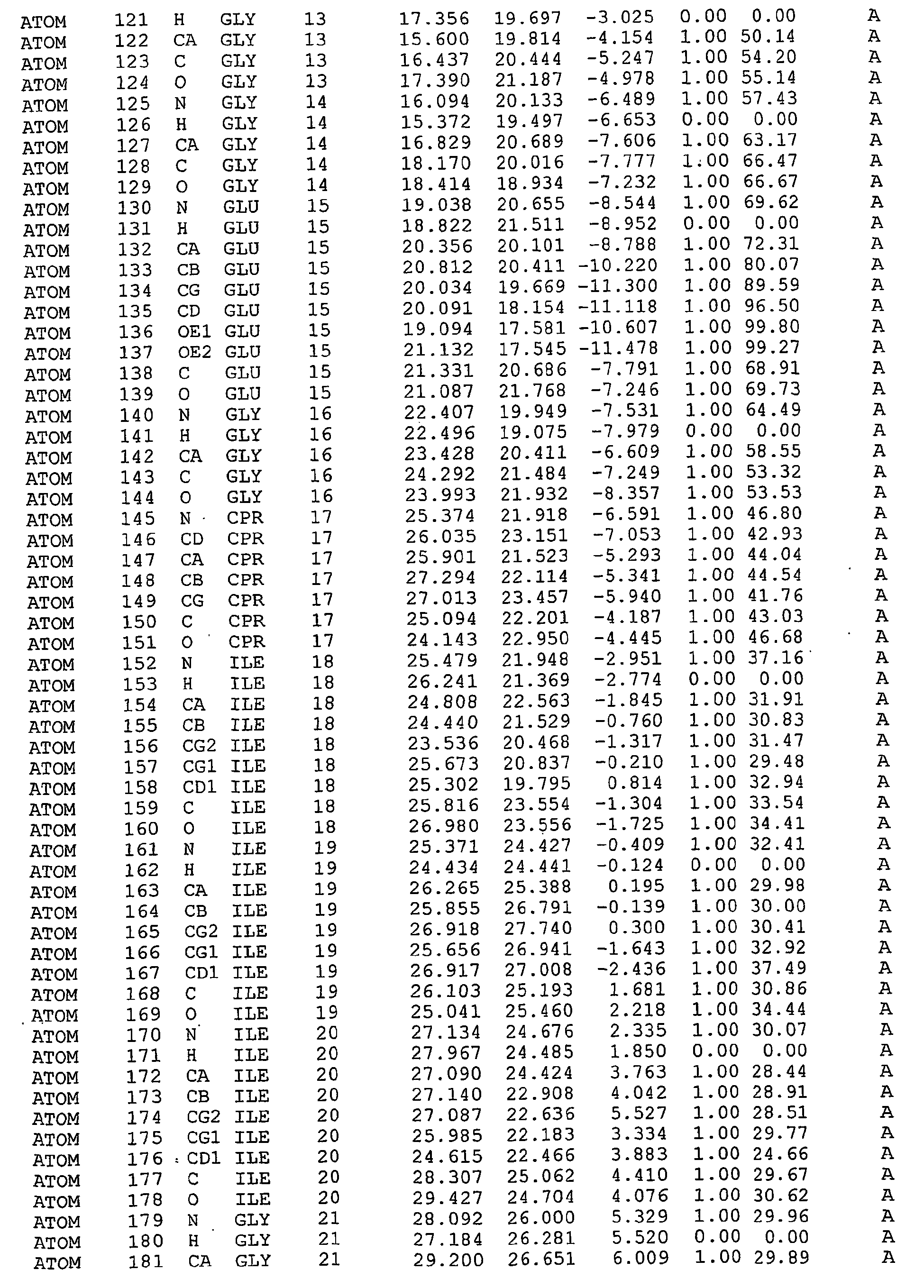

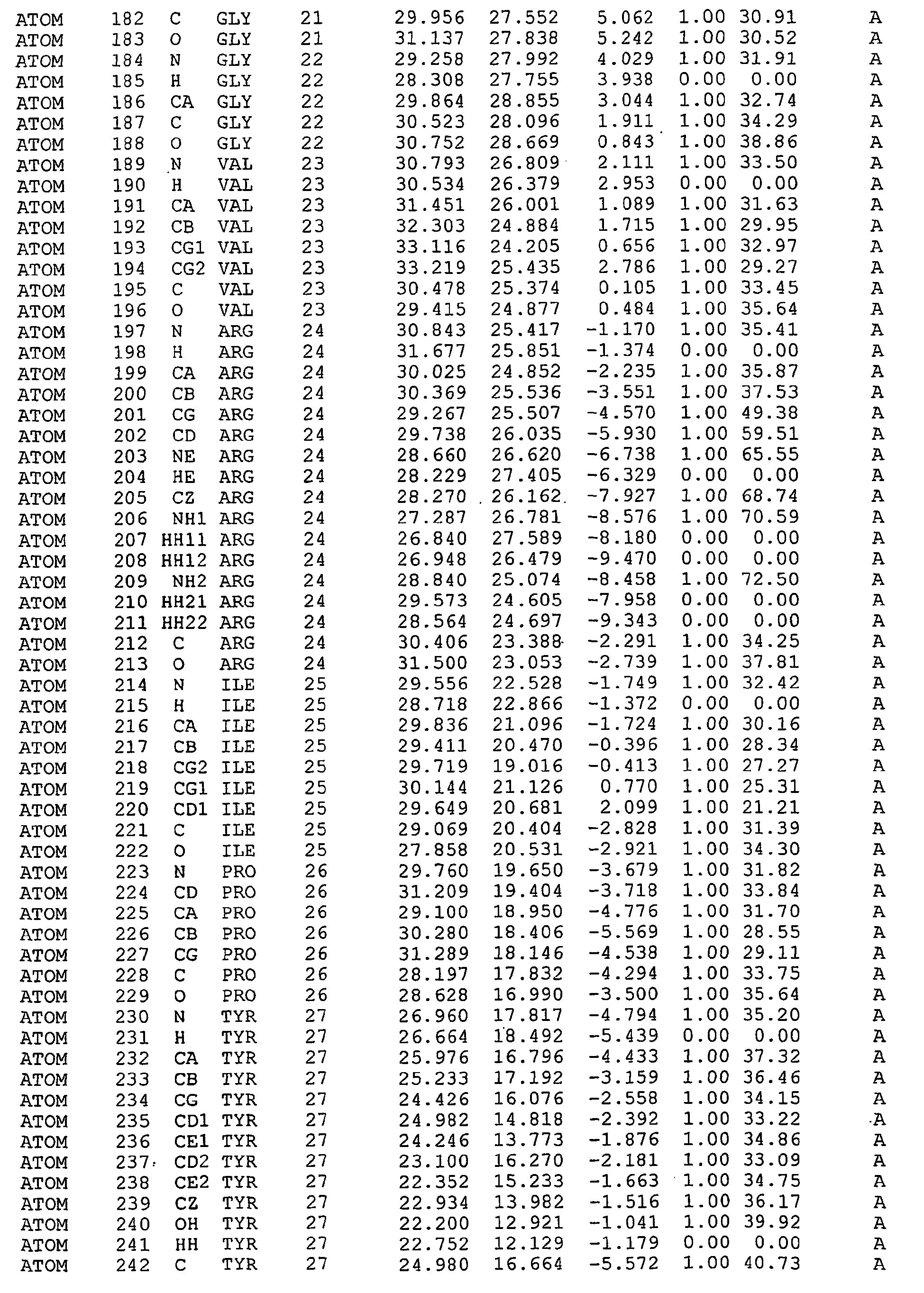

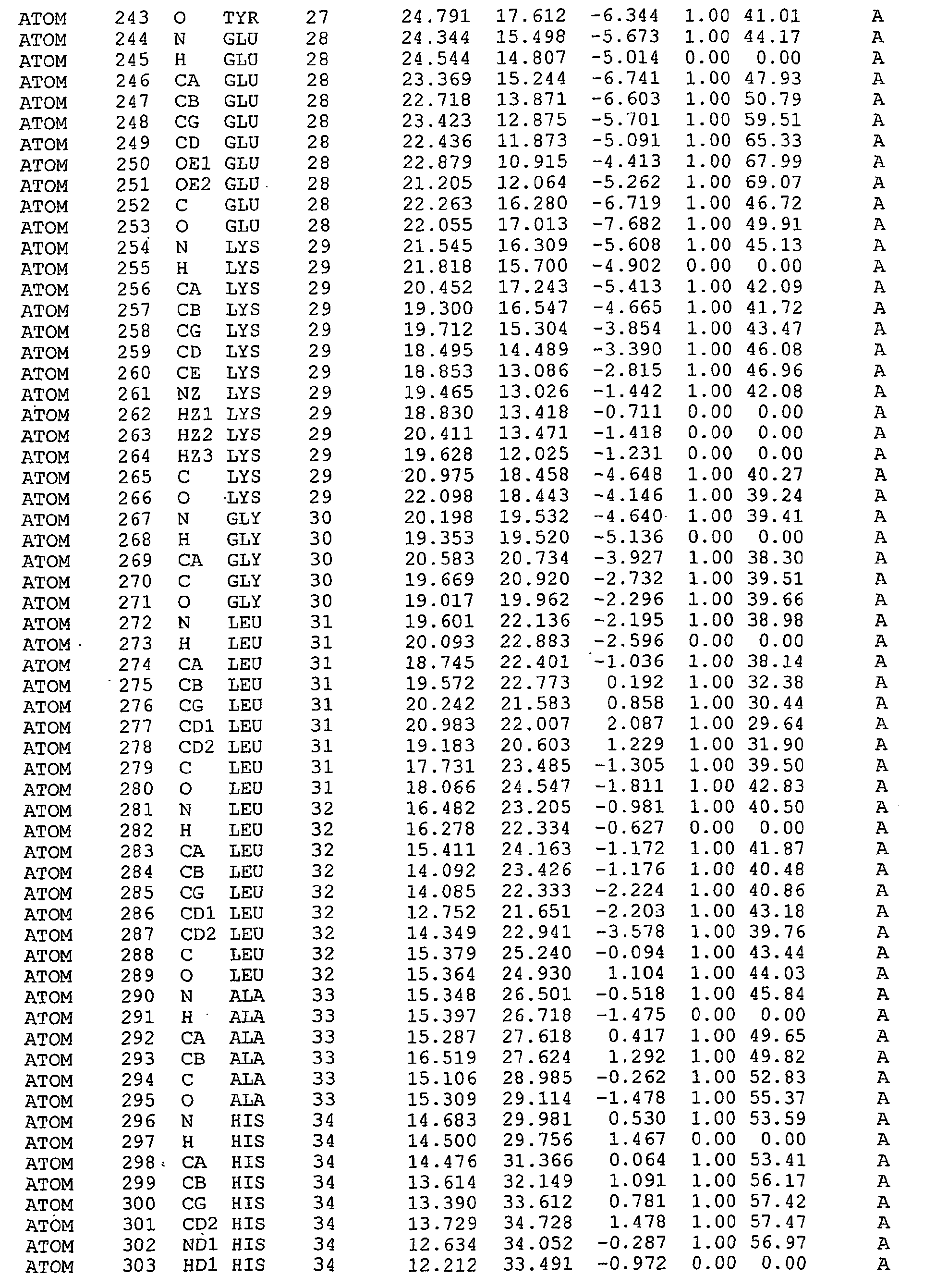

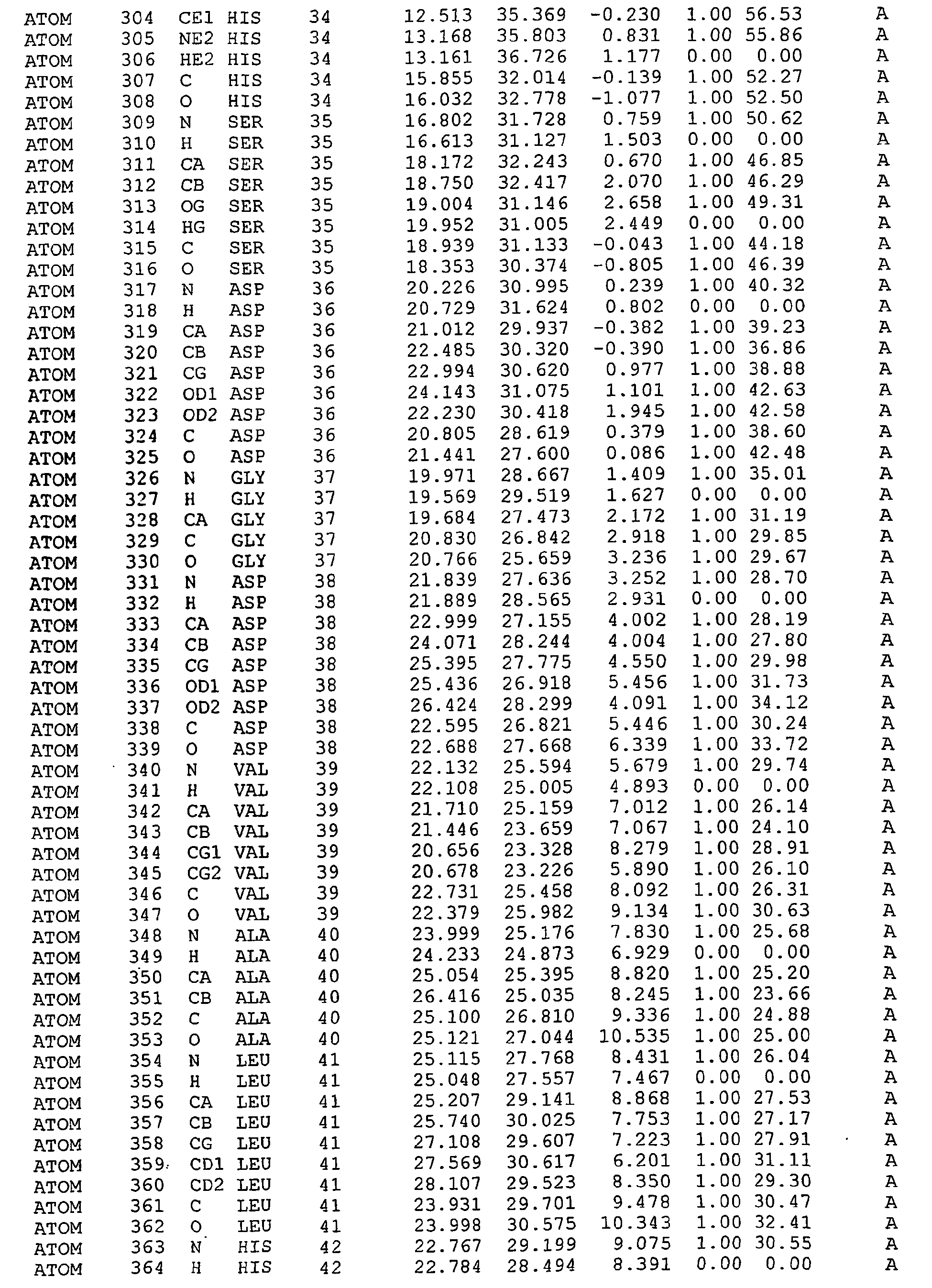

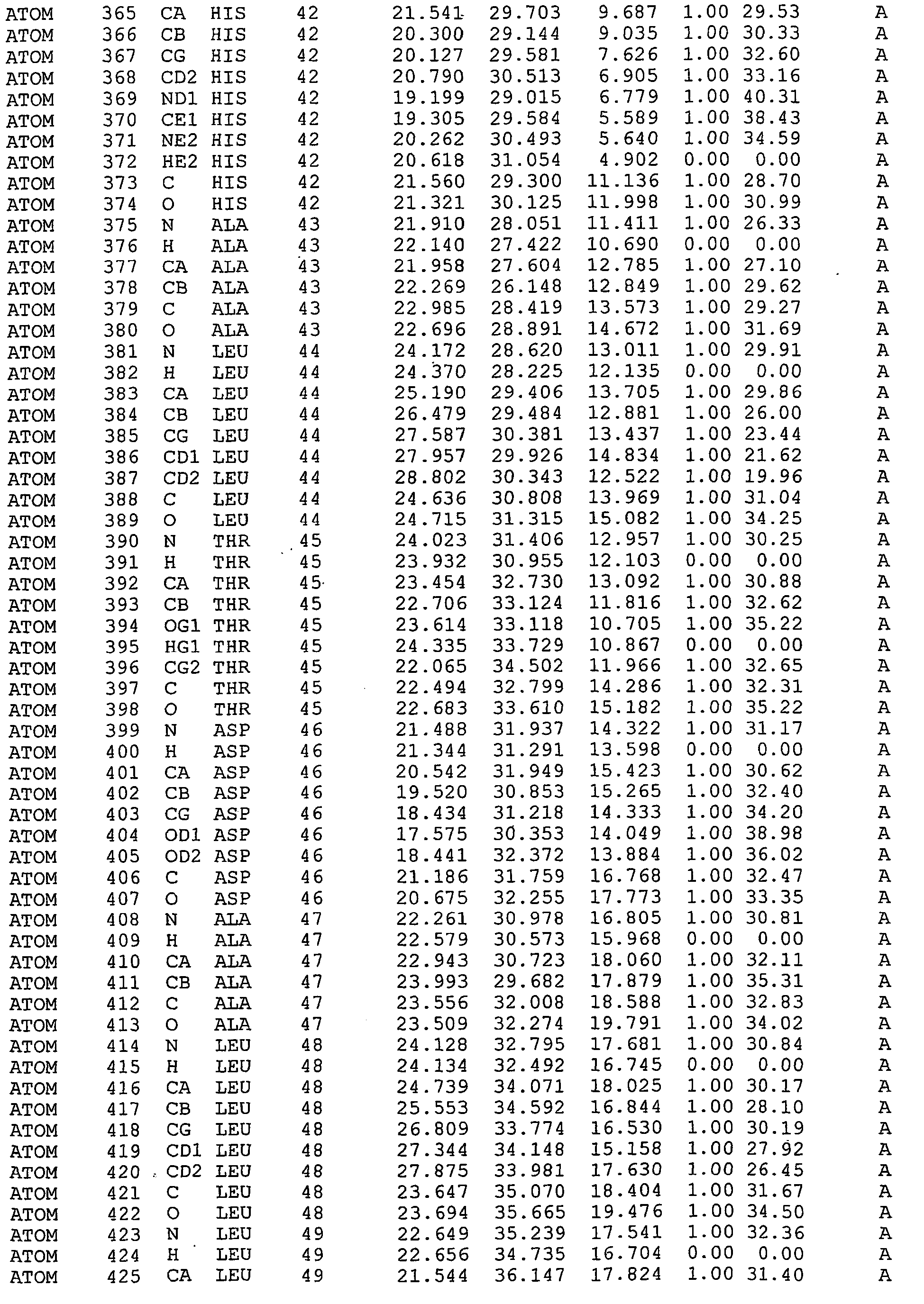

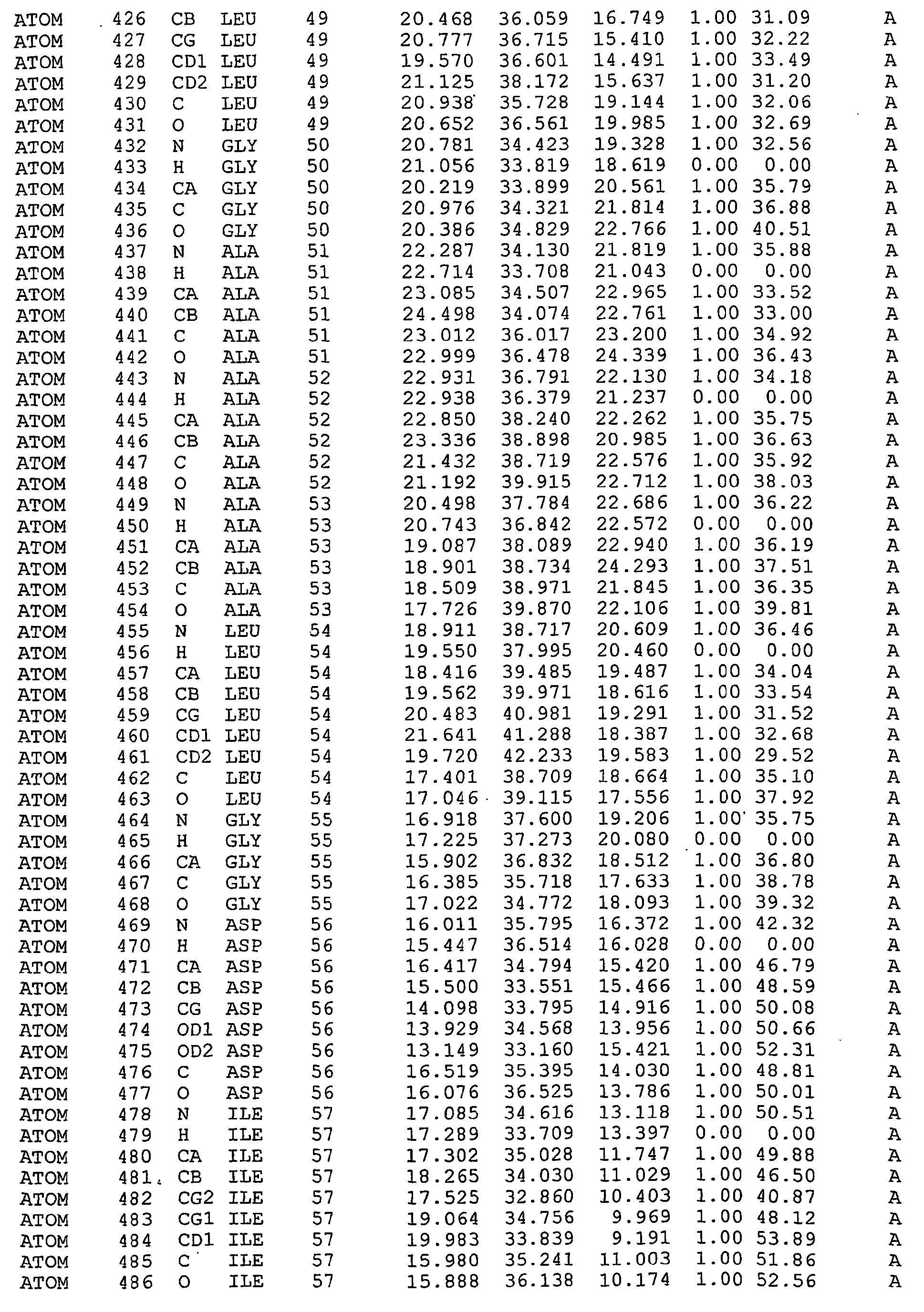

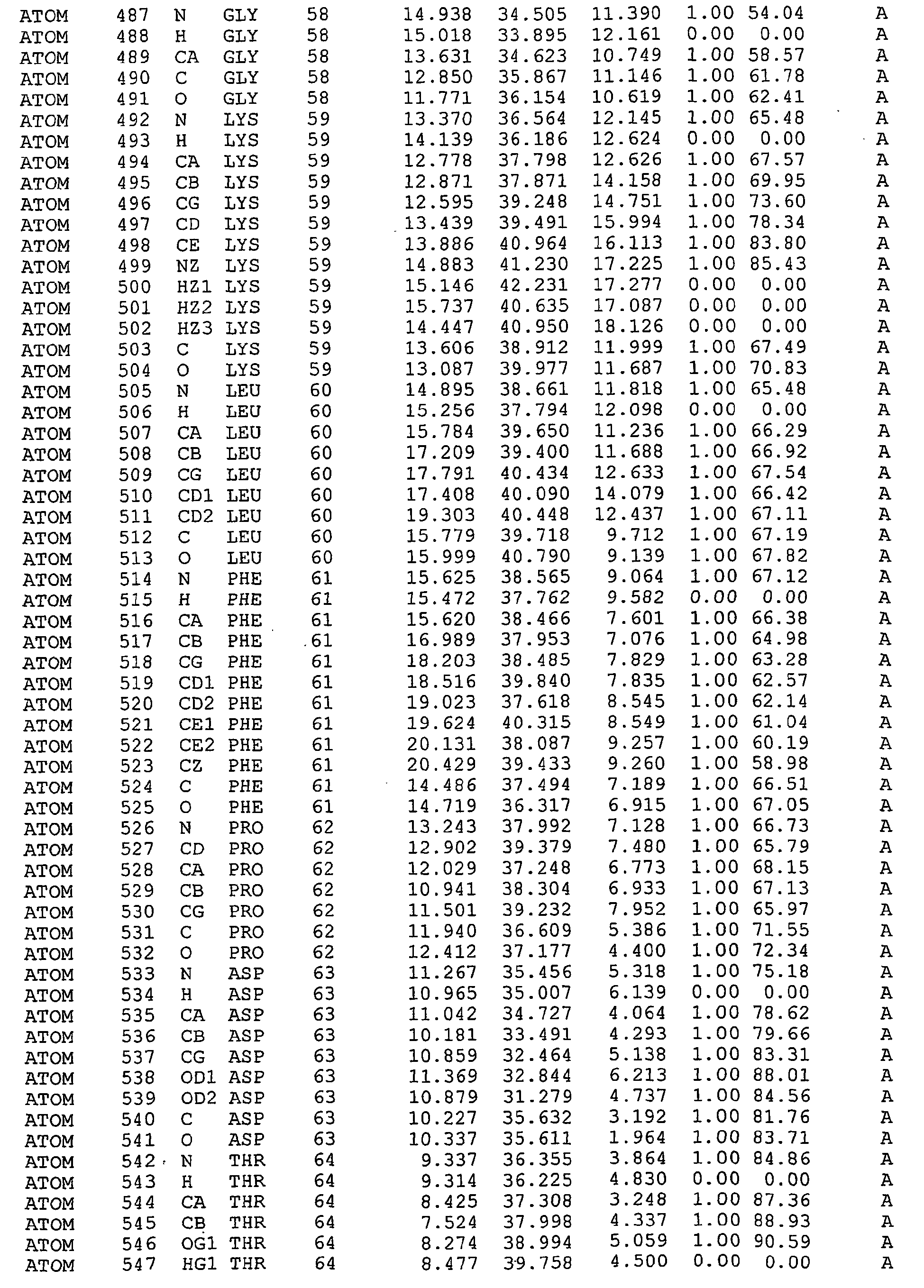

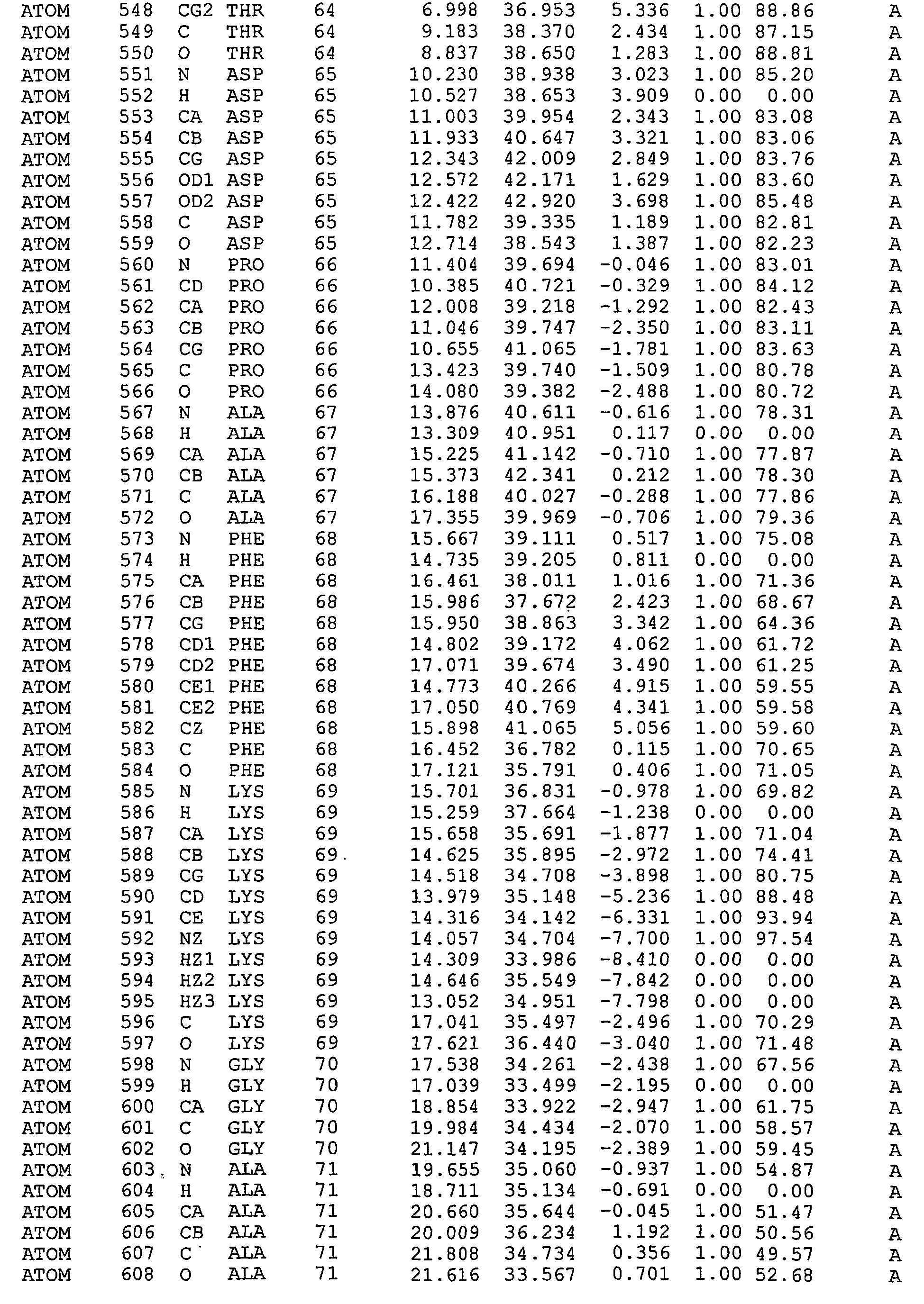

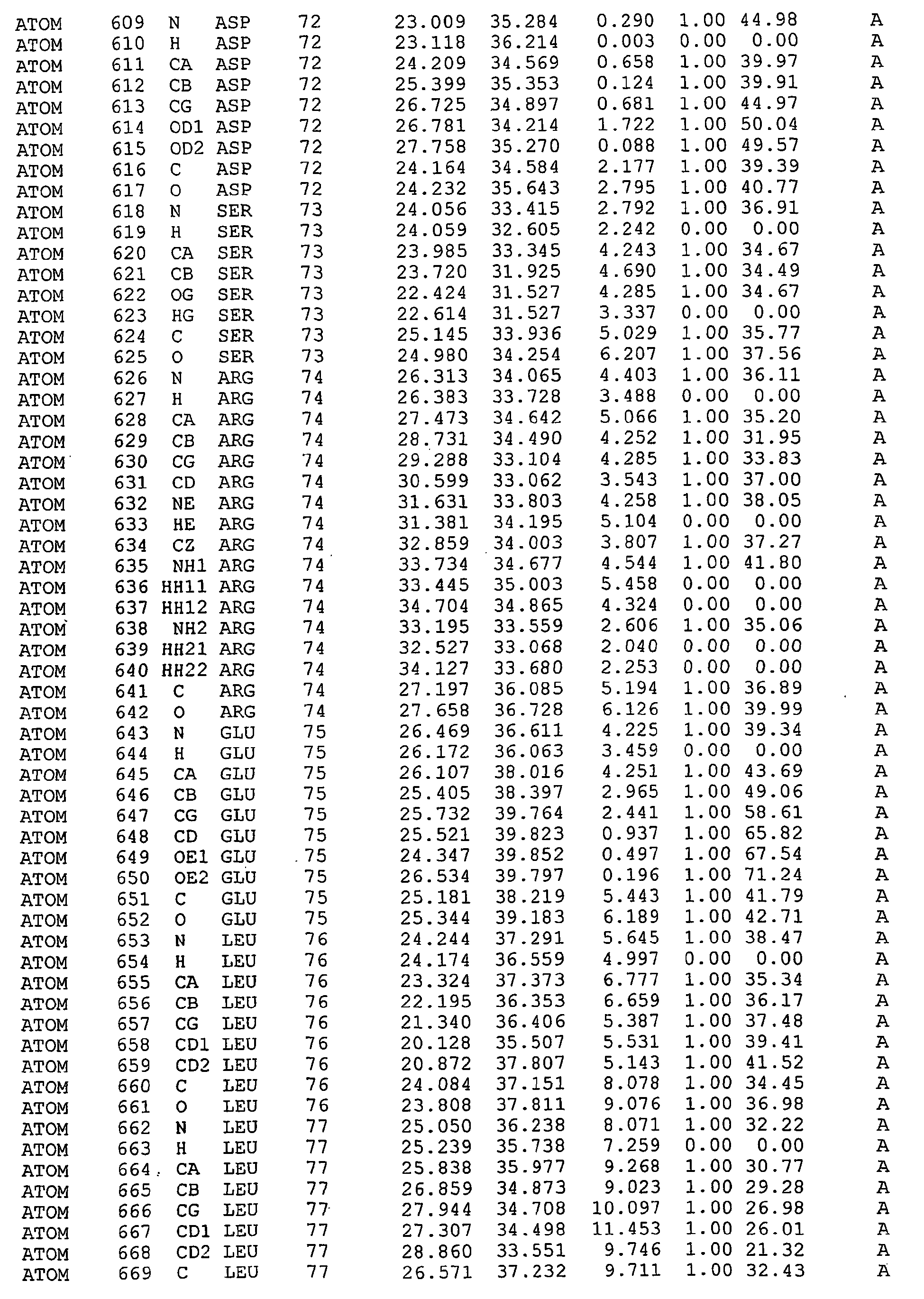

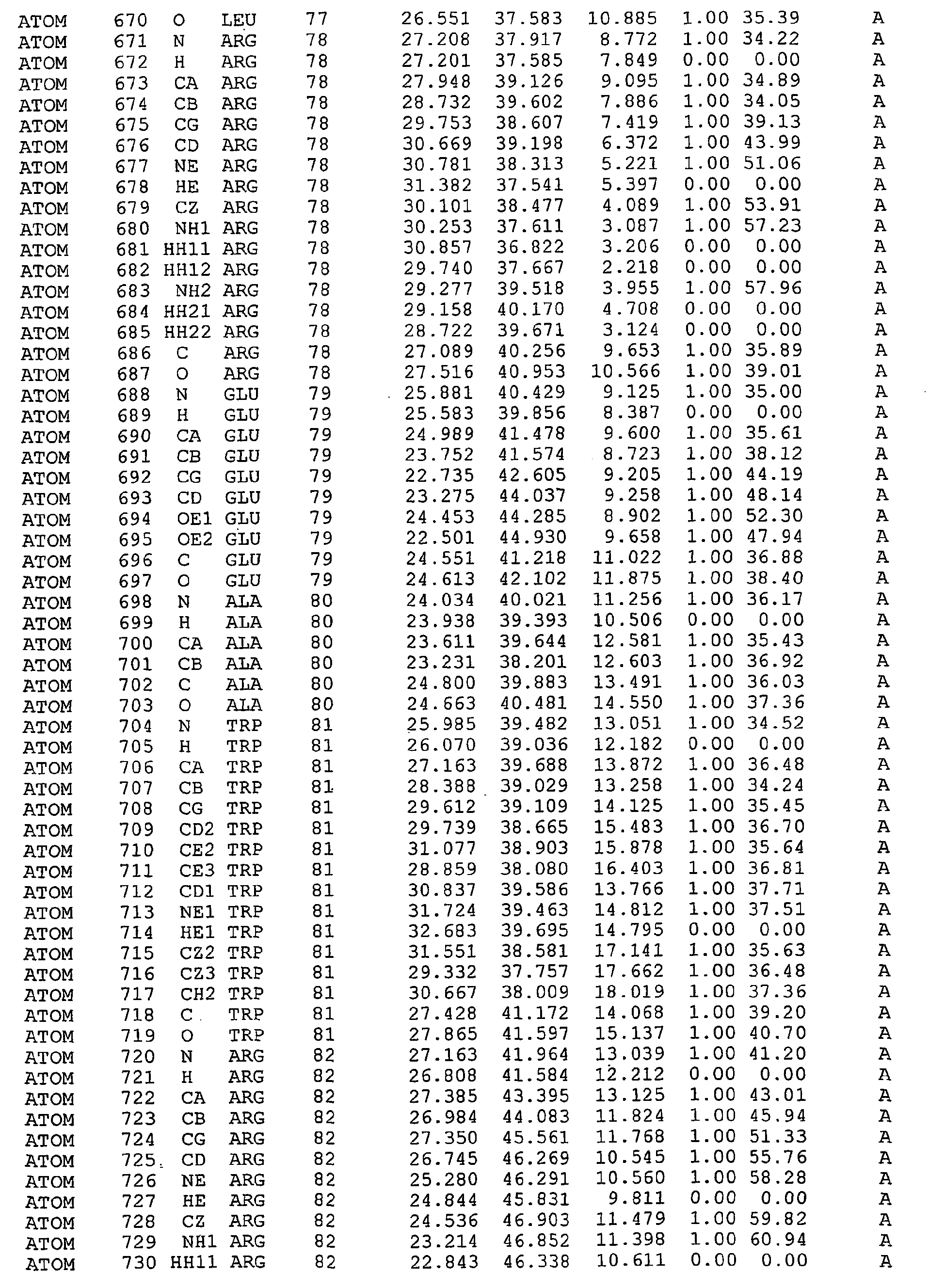

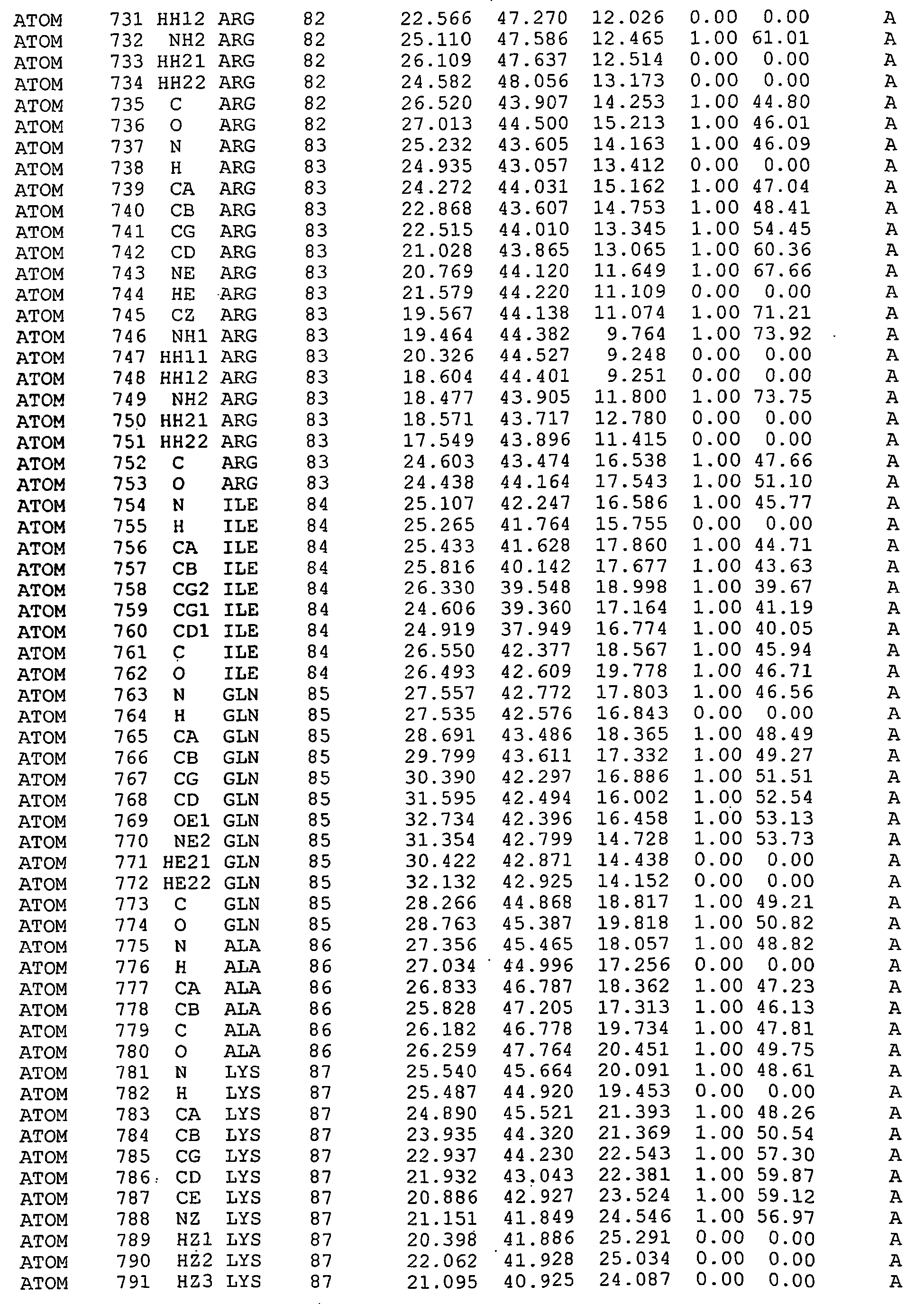

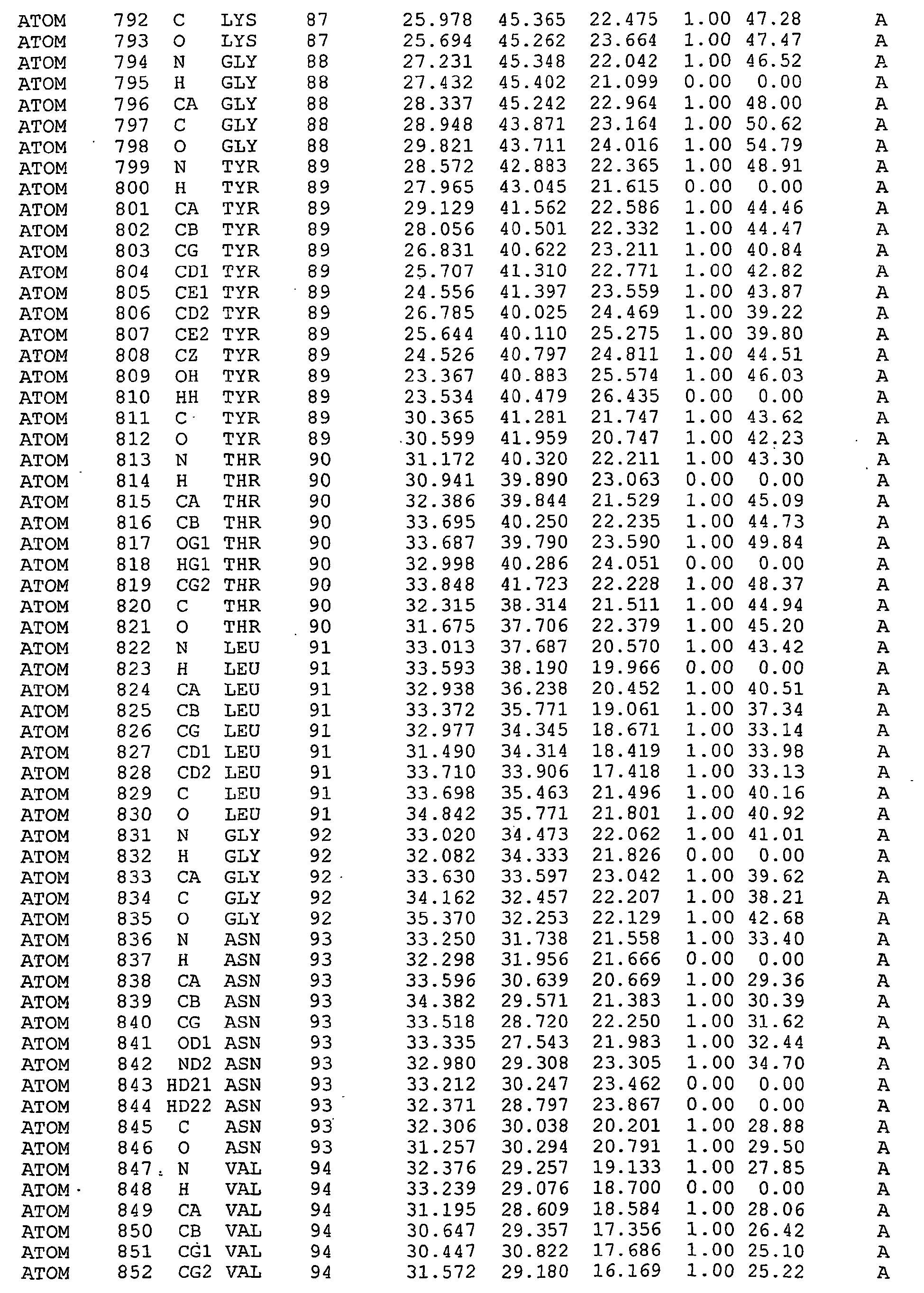

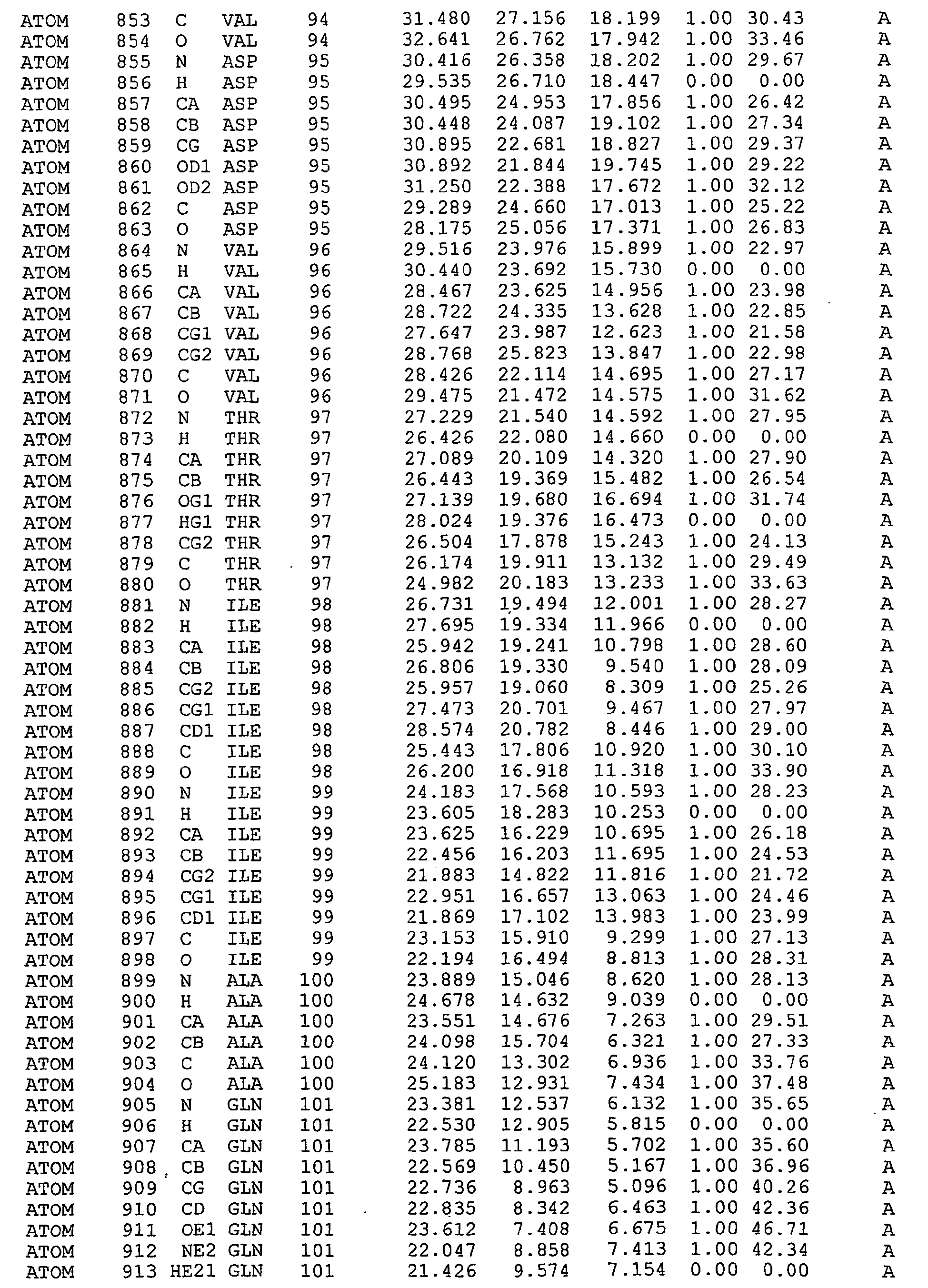

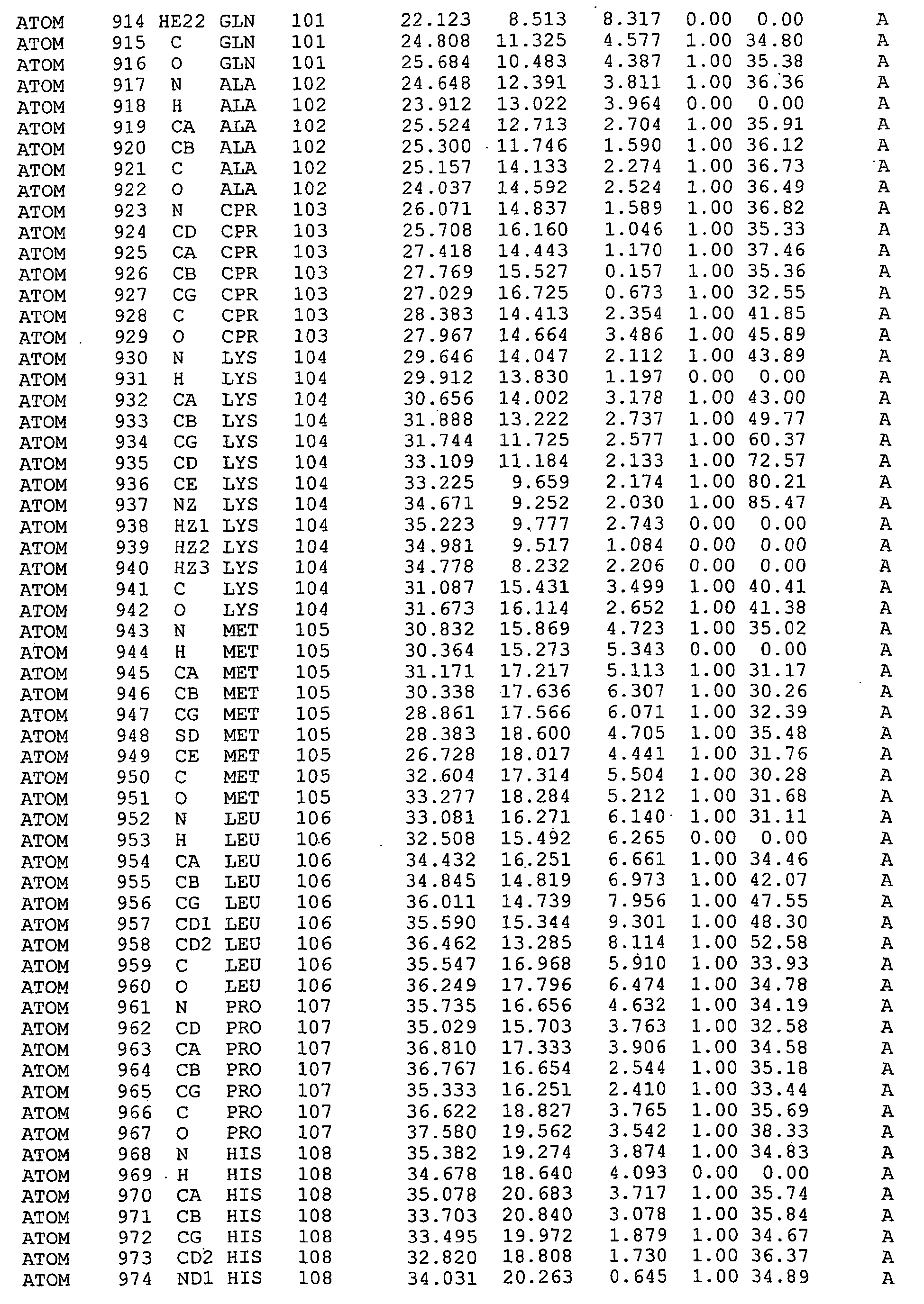

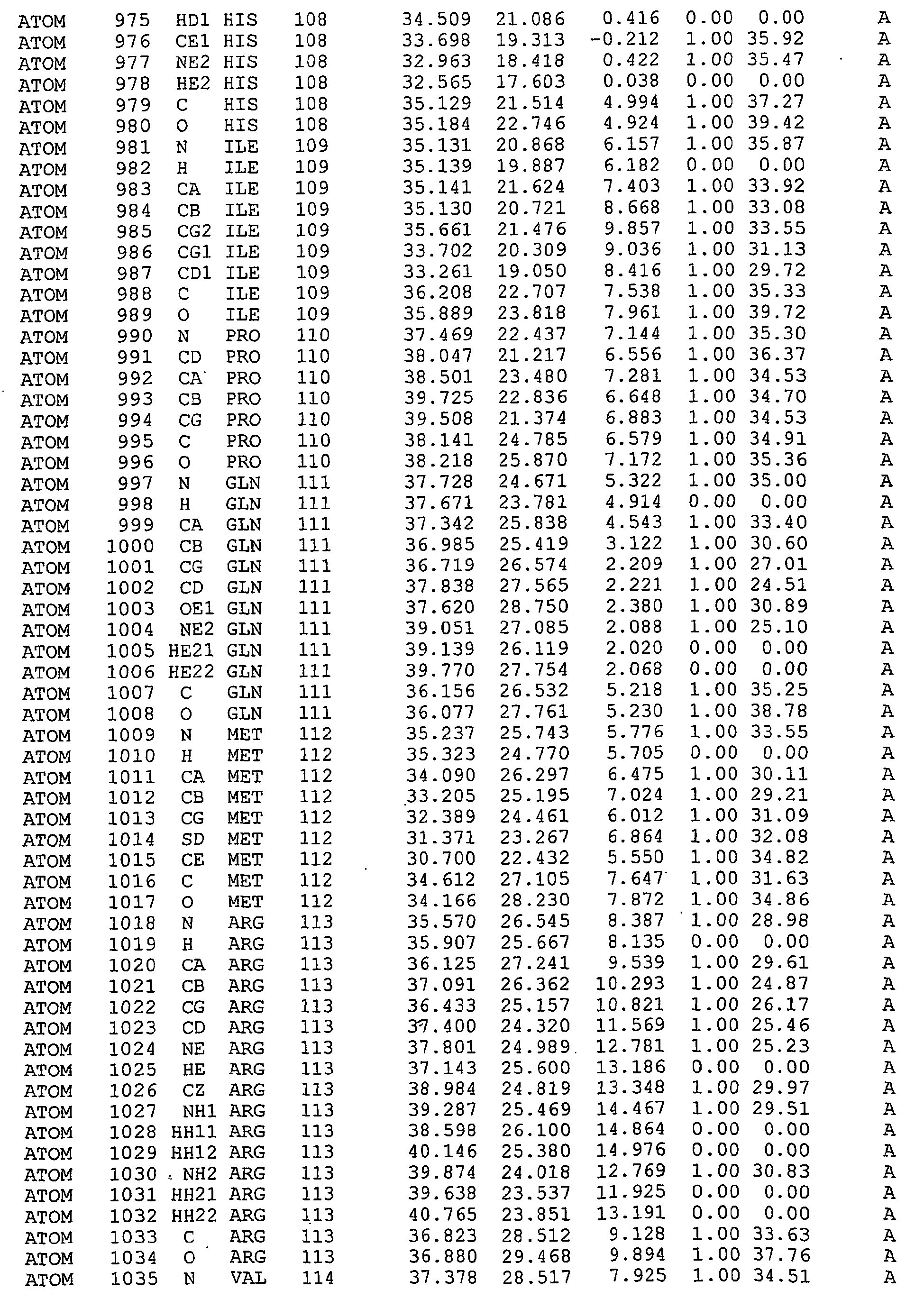

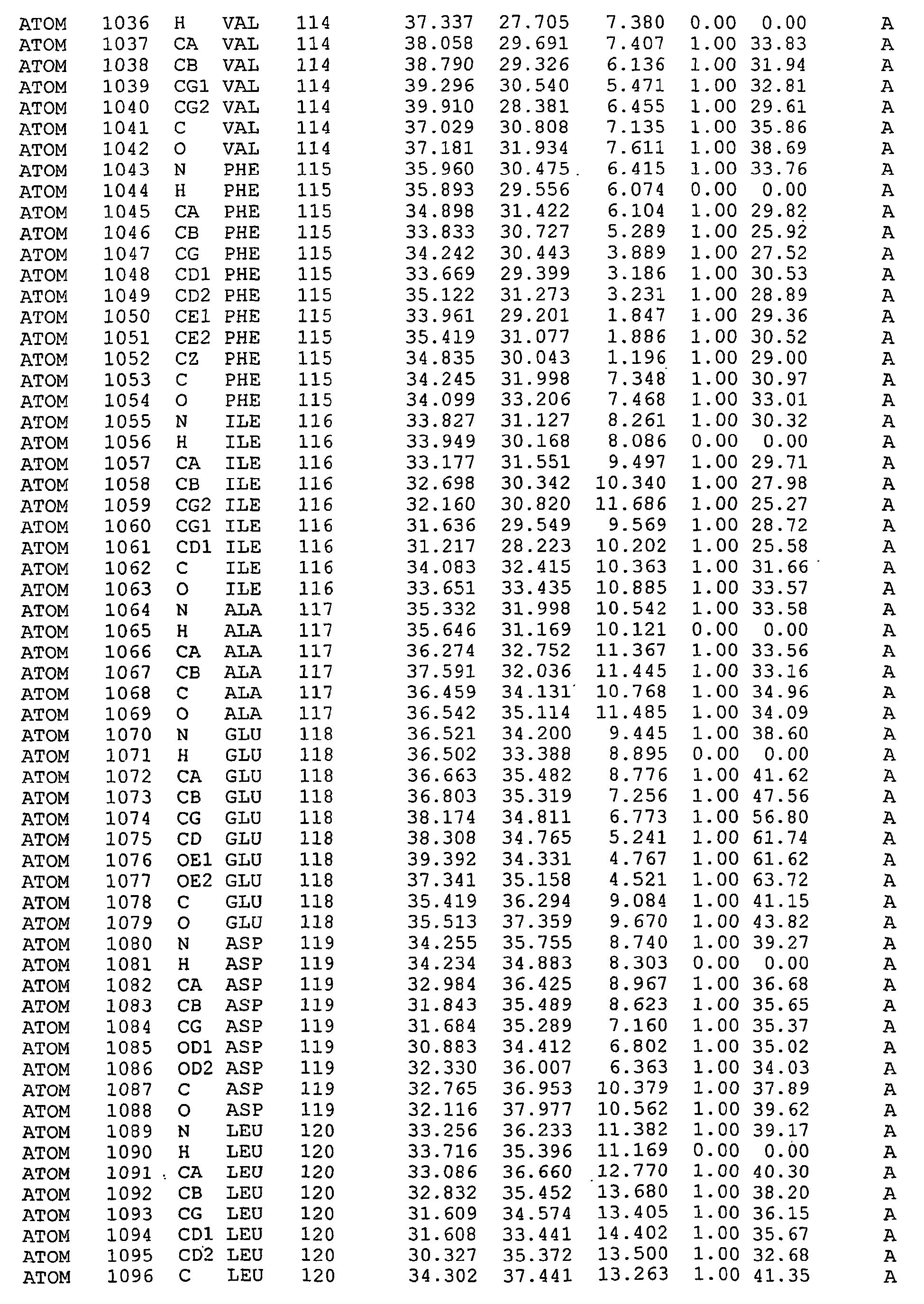

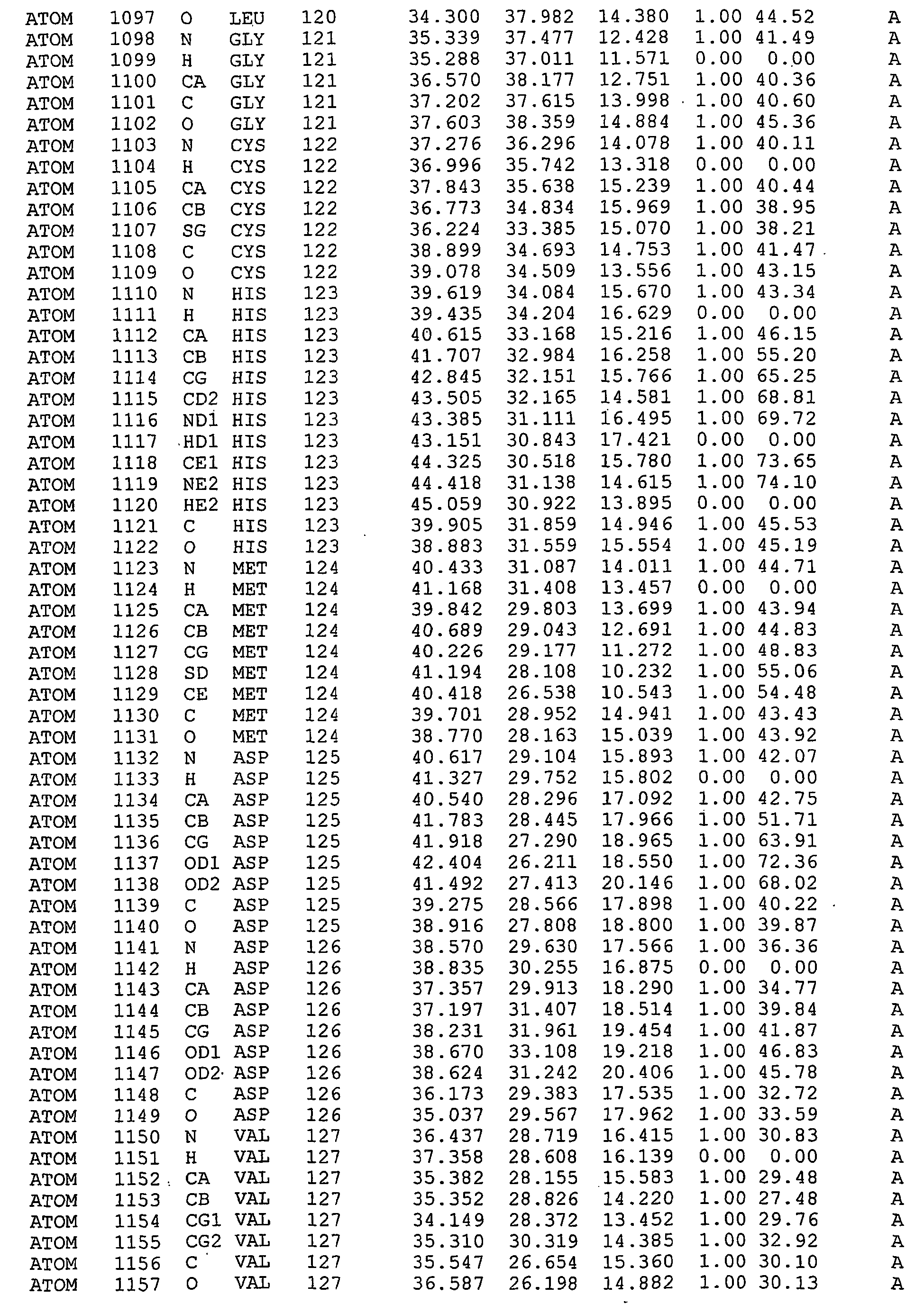

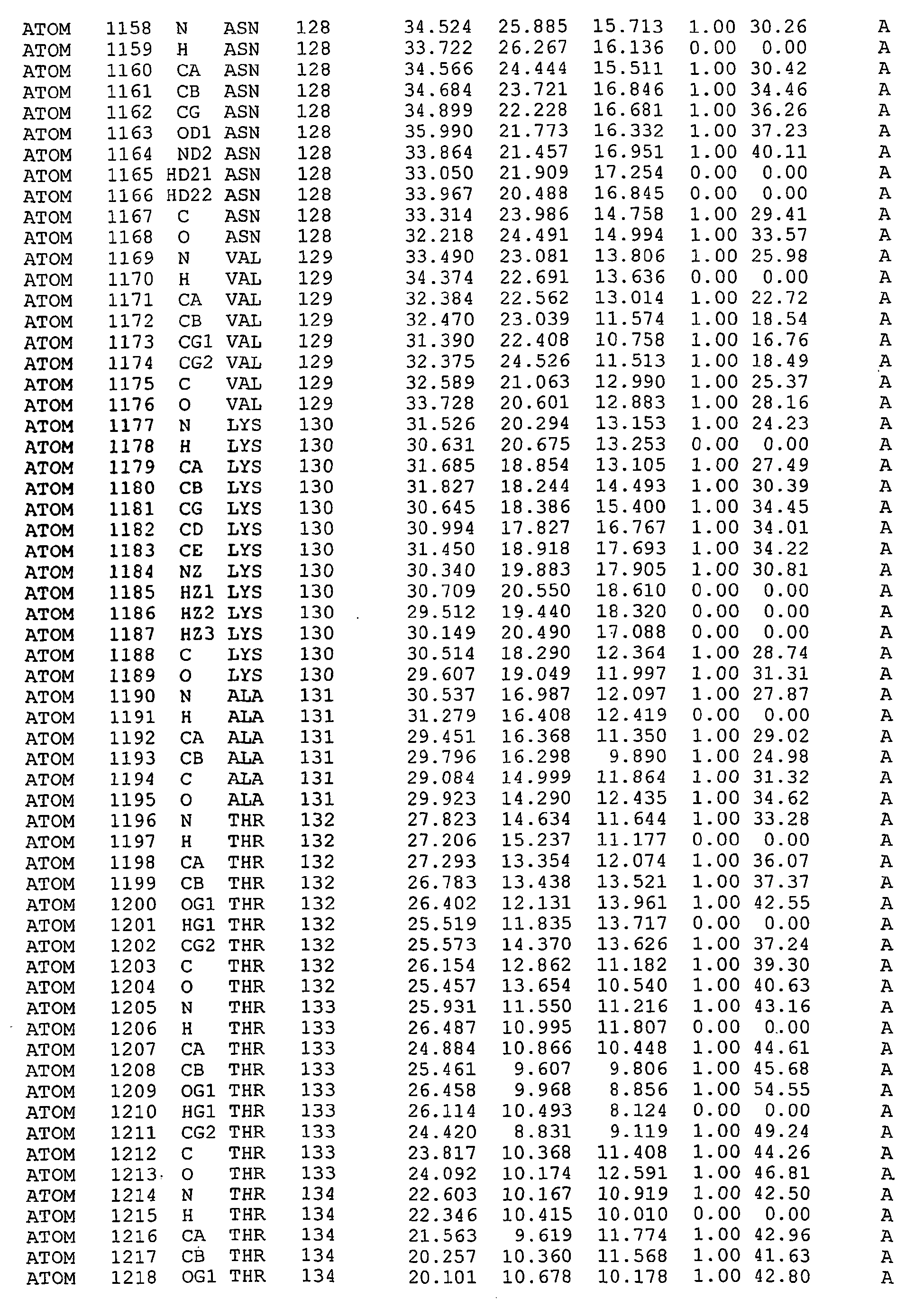

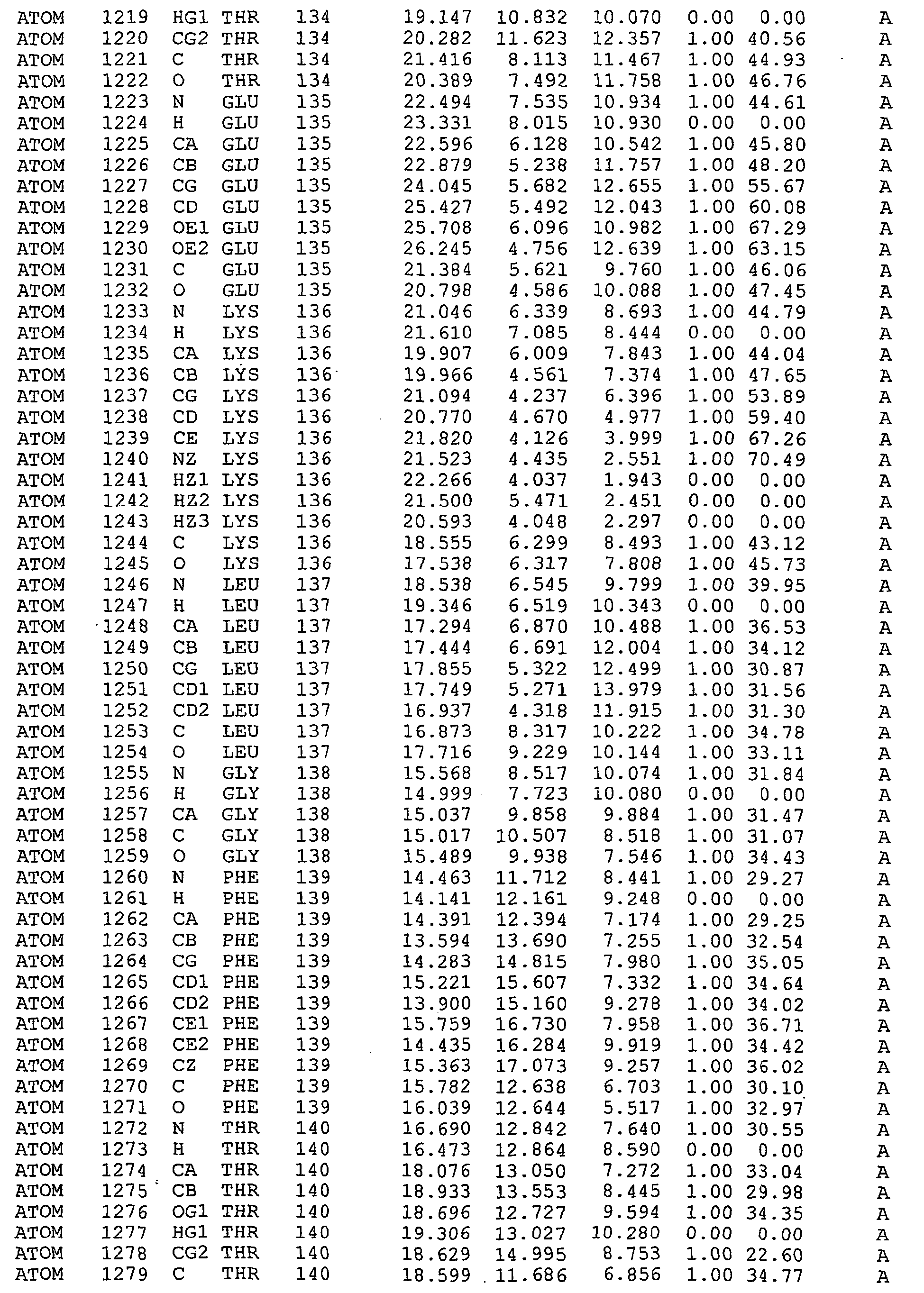

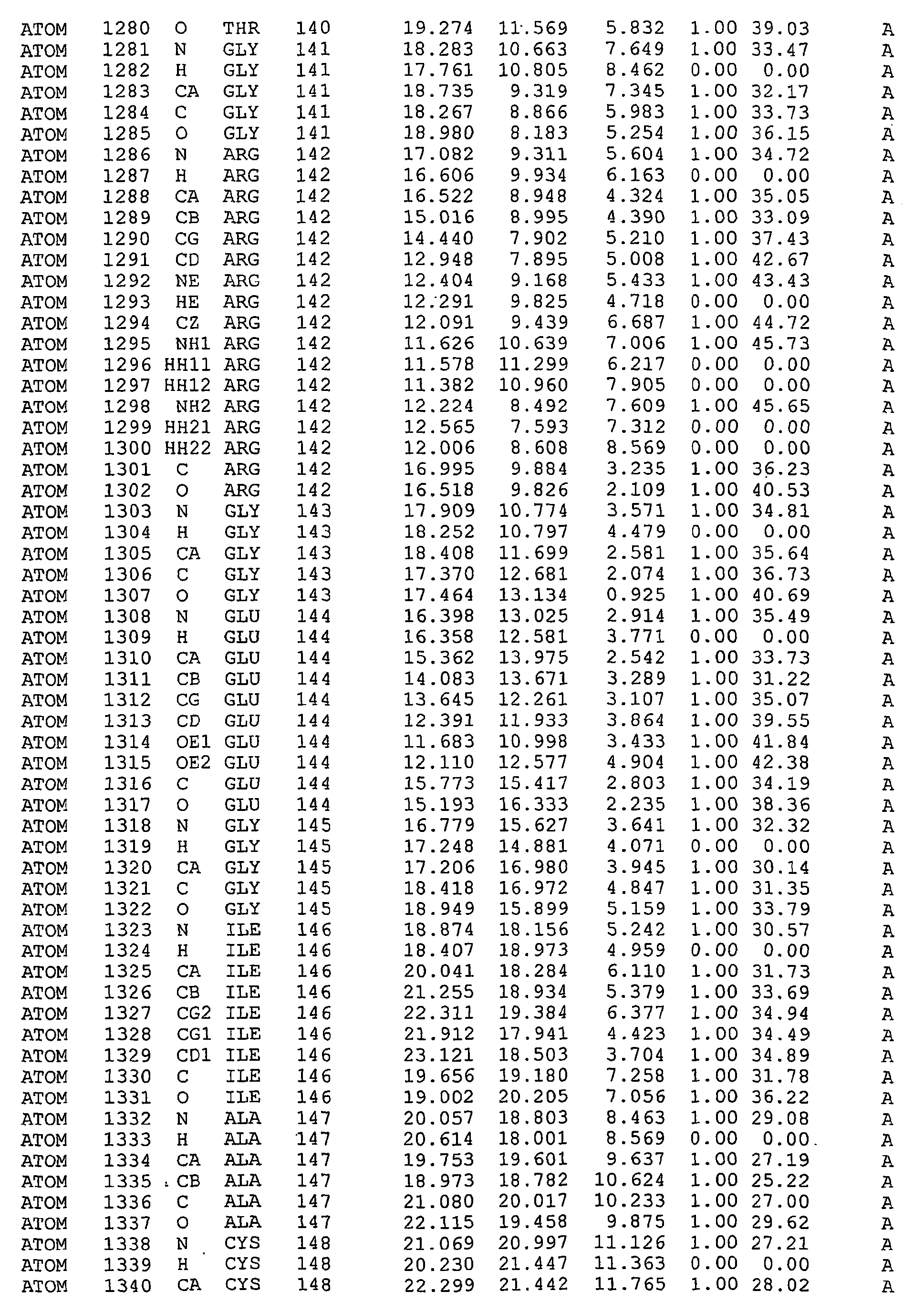

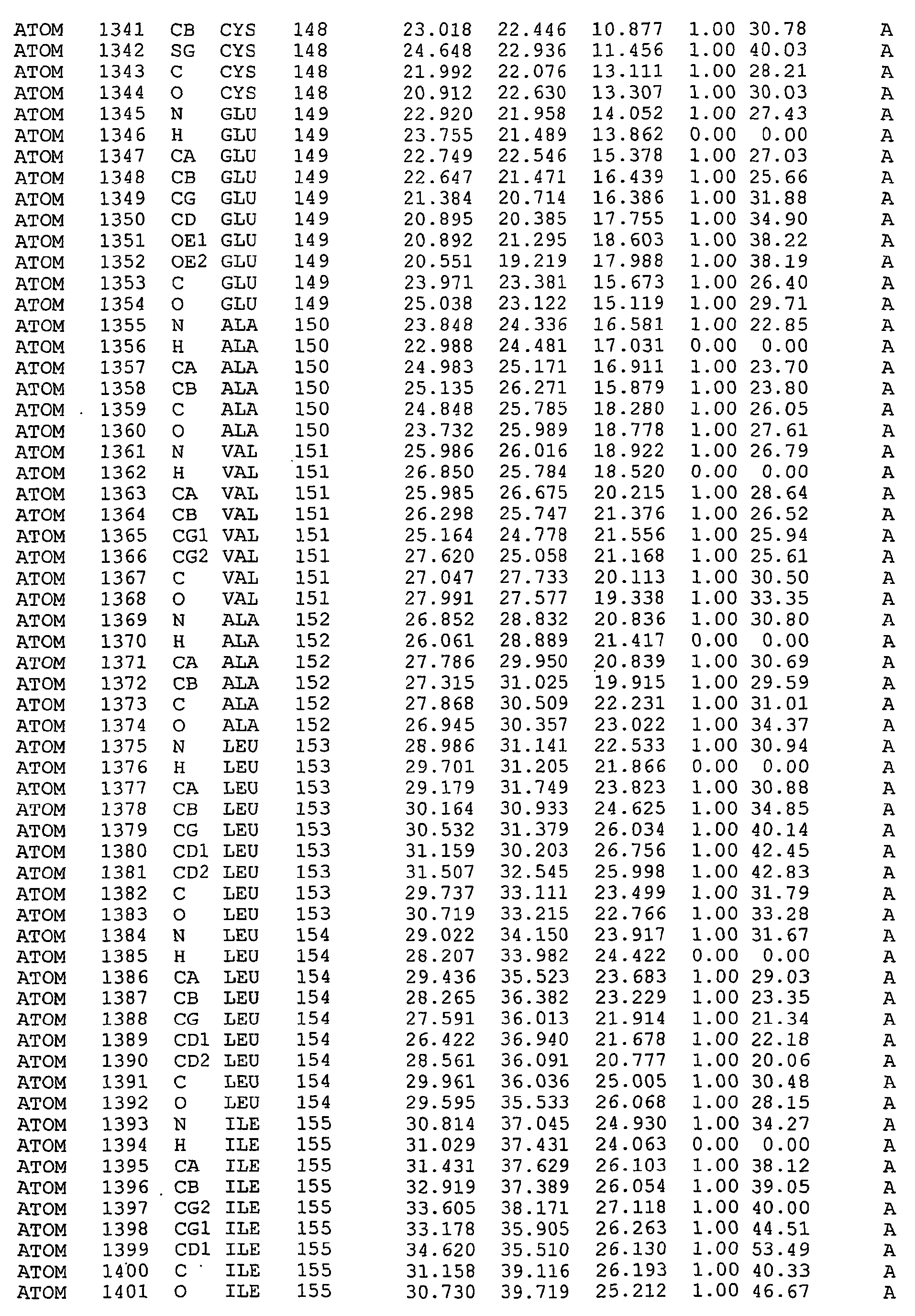

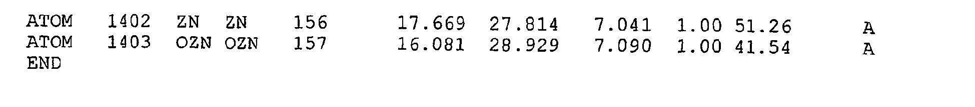

### Annex 4: Coordinates of structure sub2

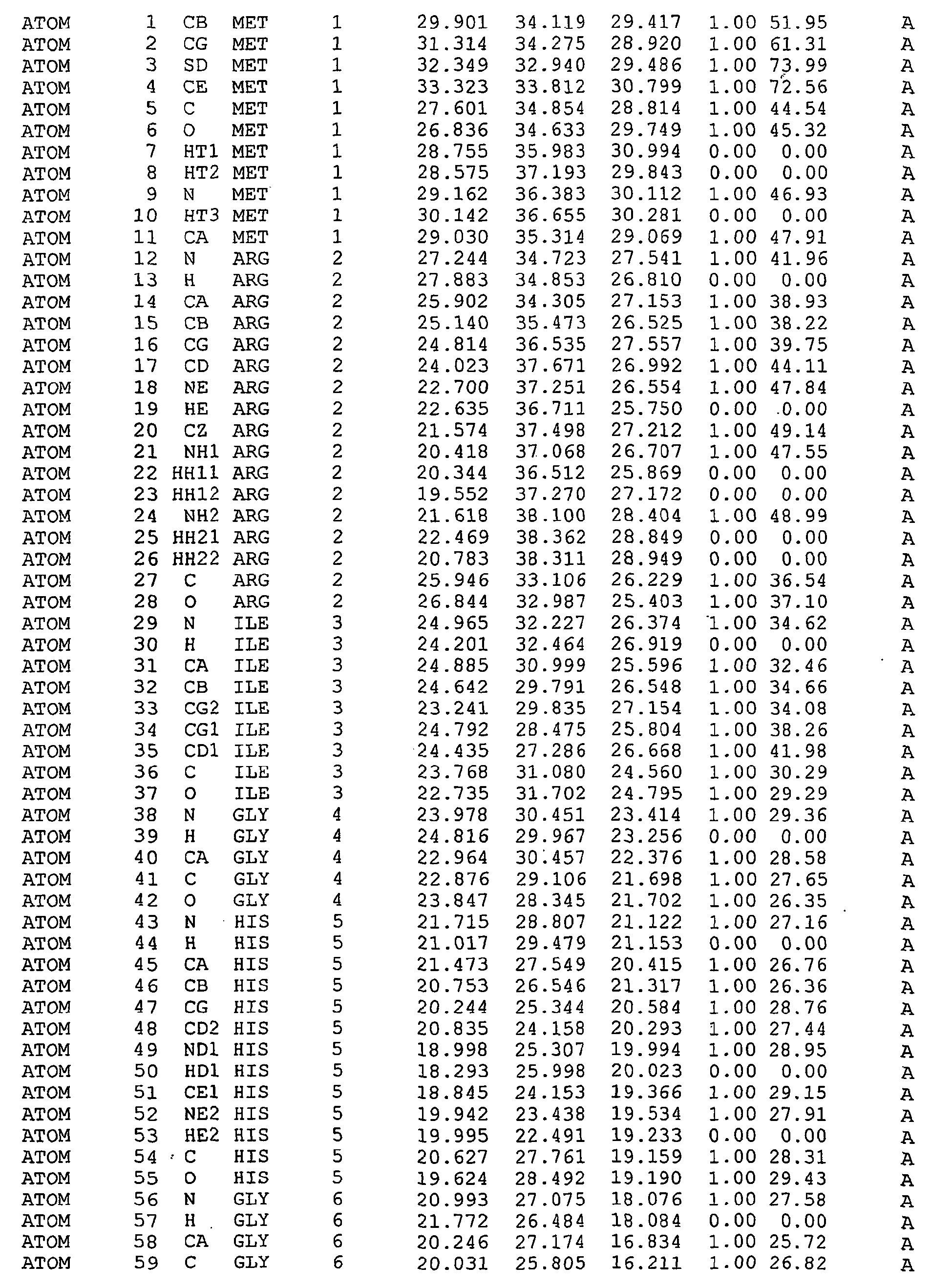

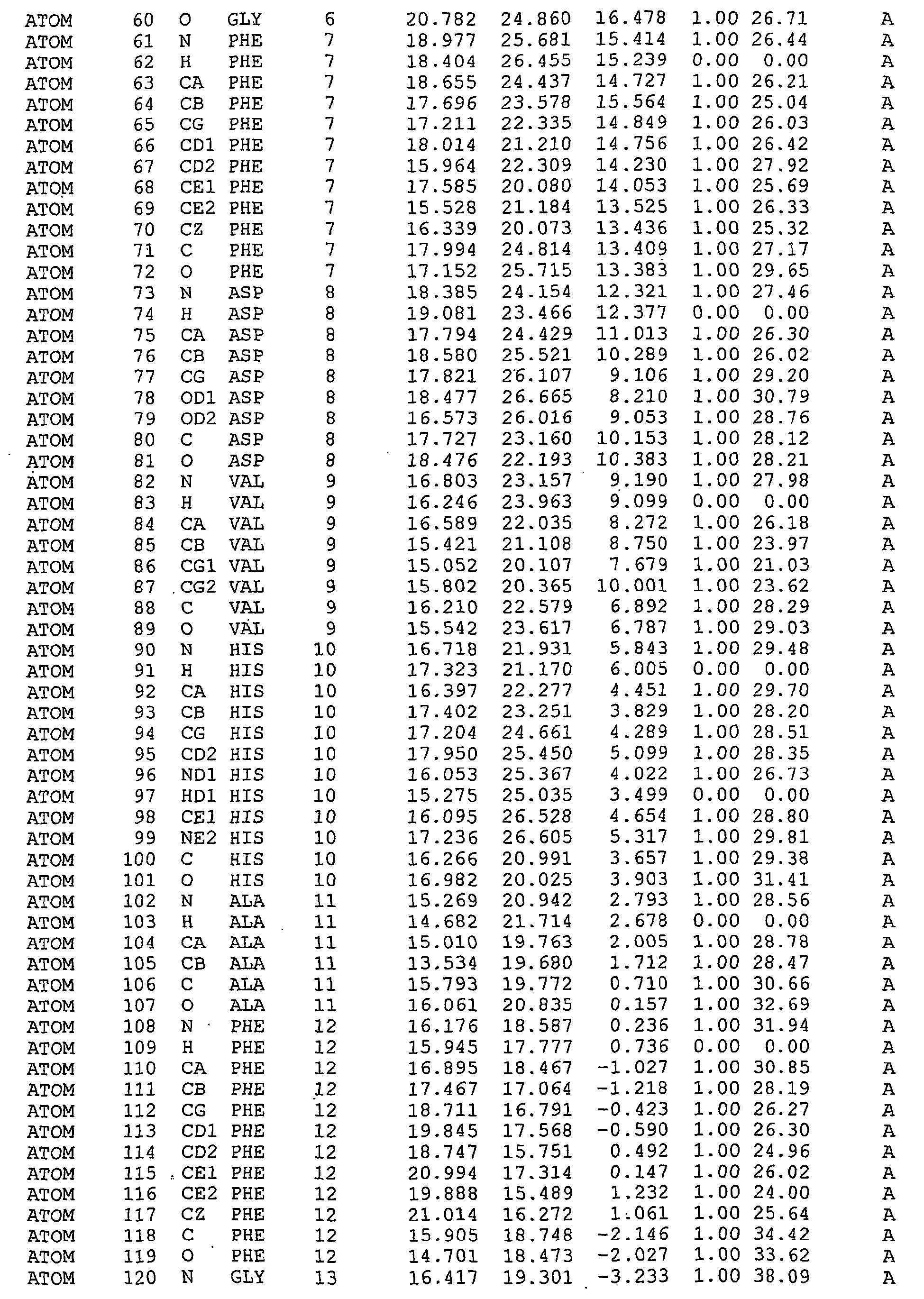

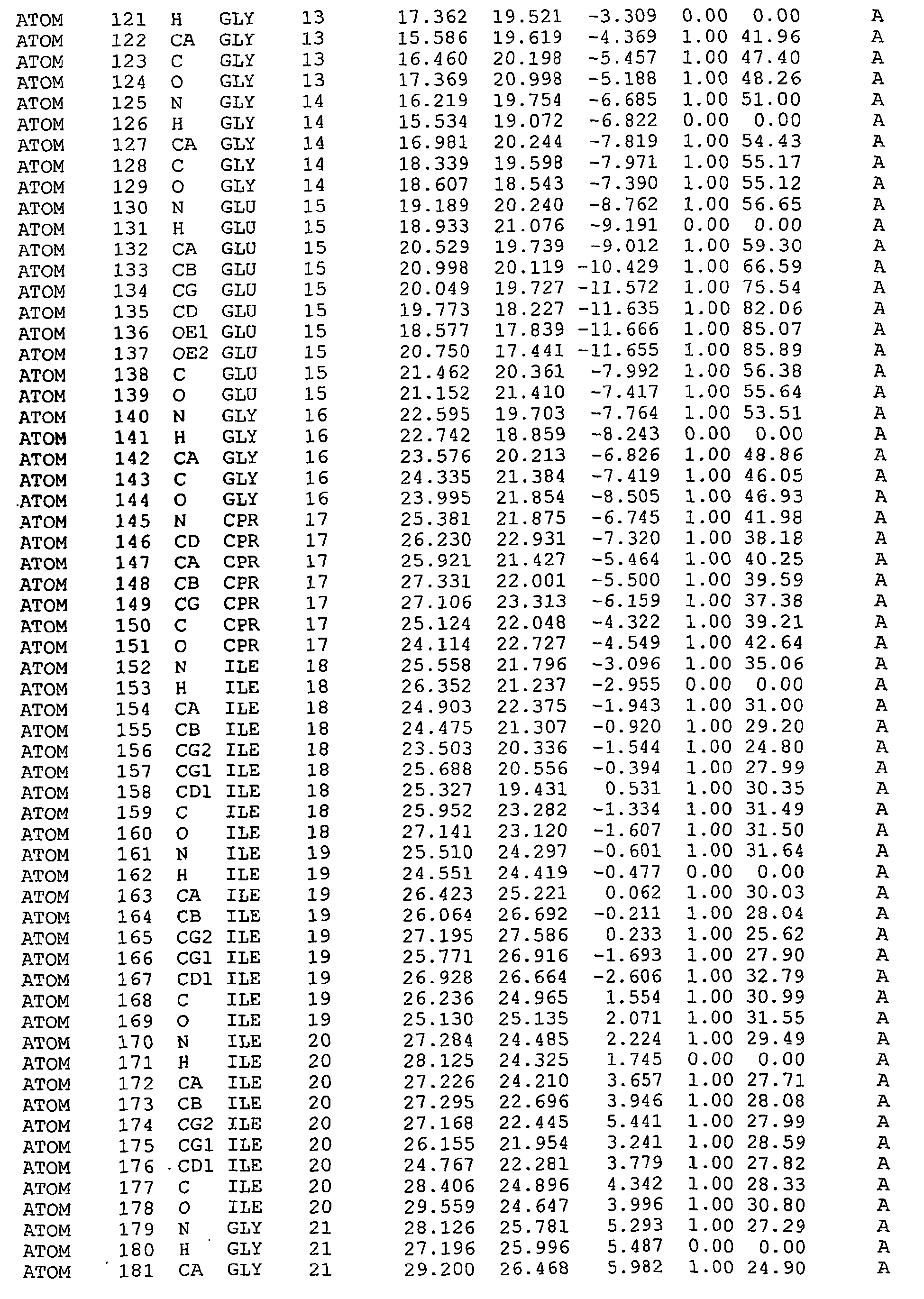

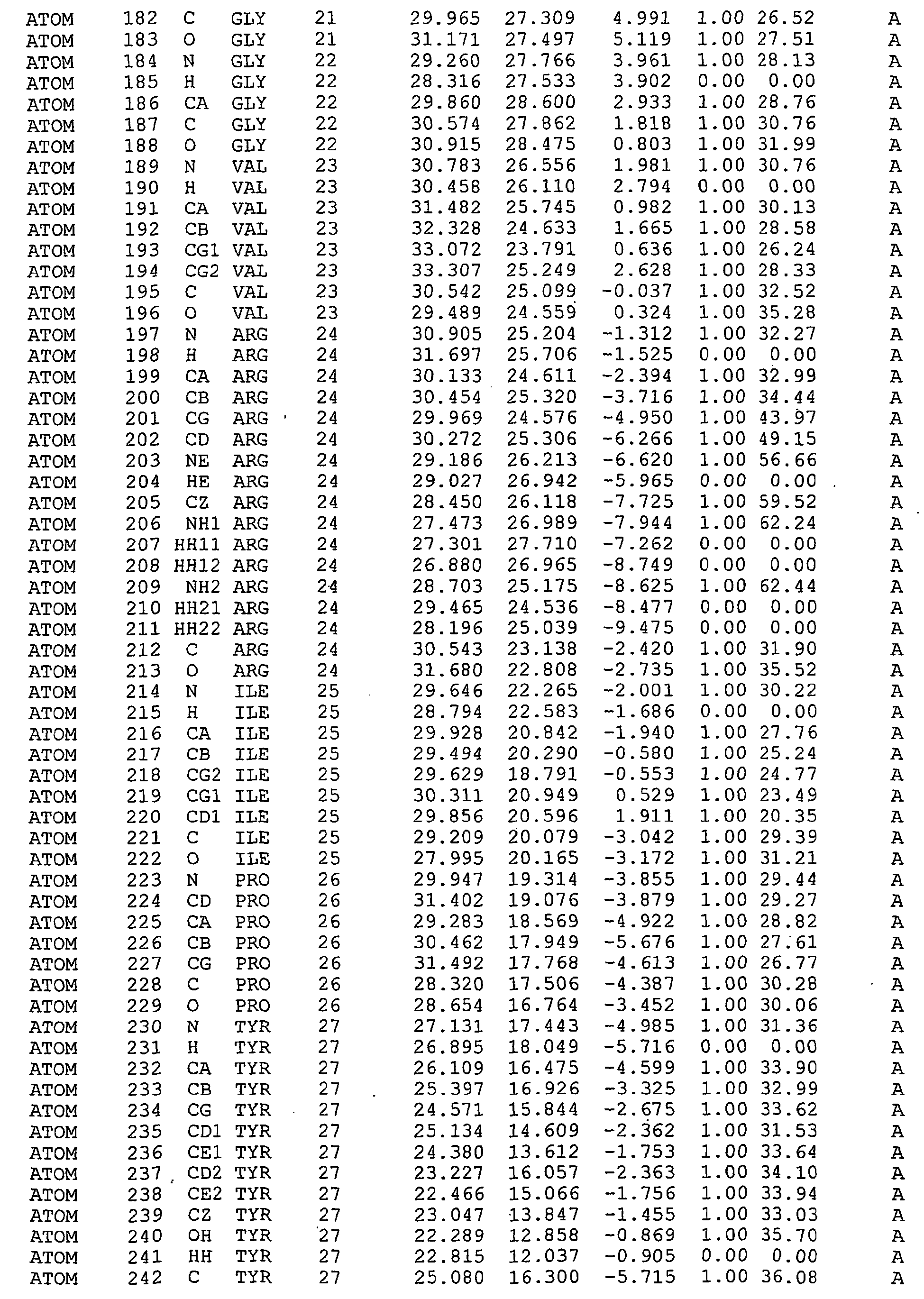

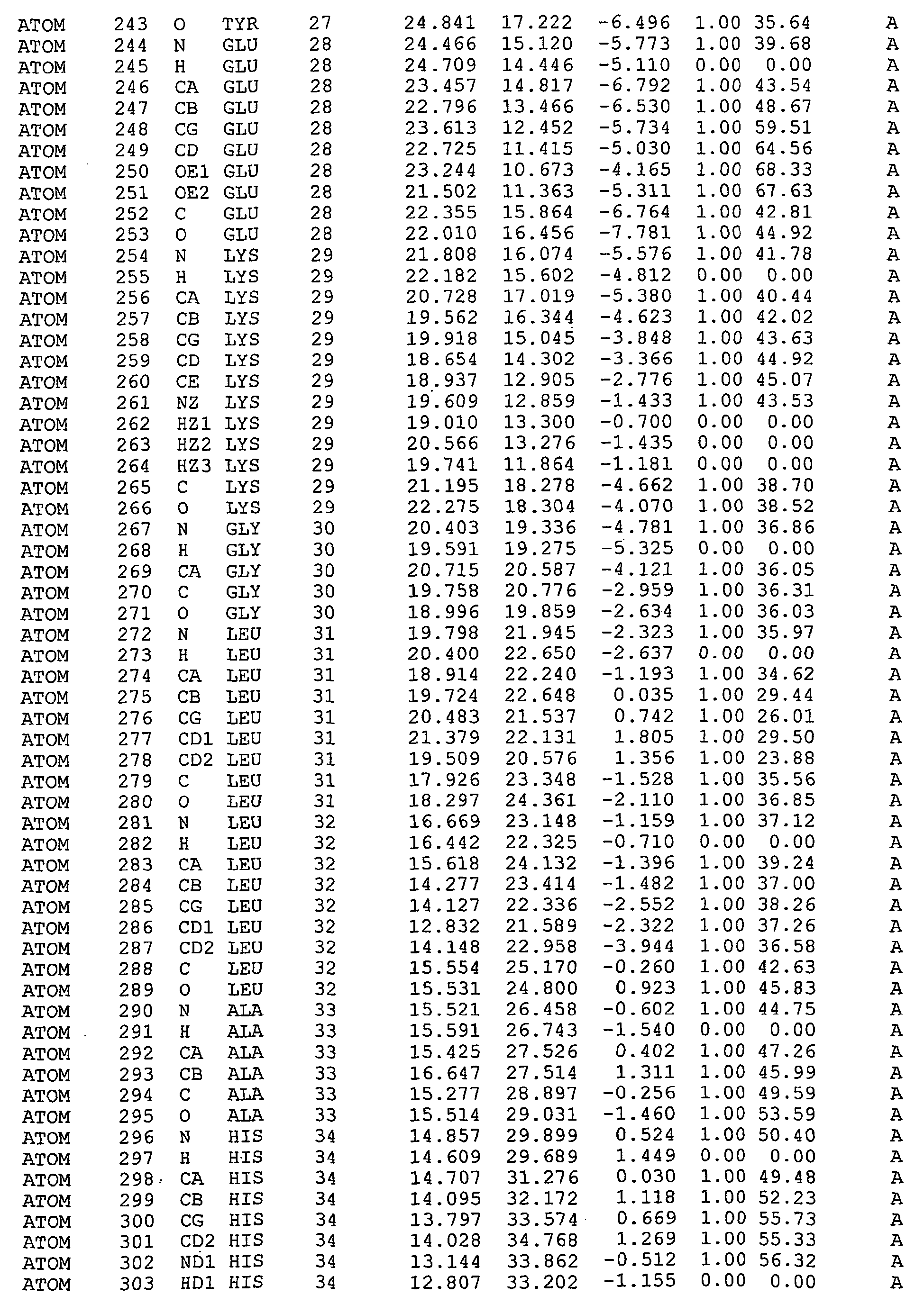

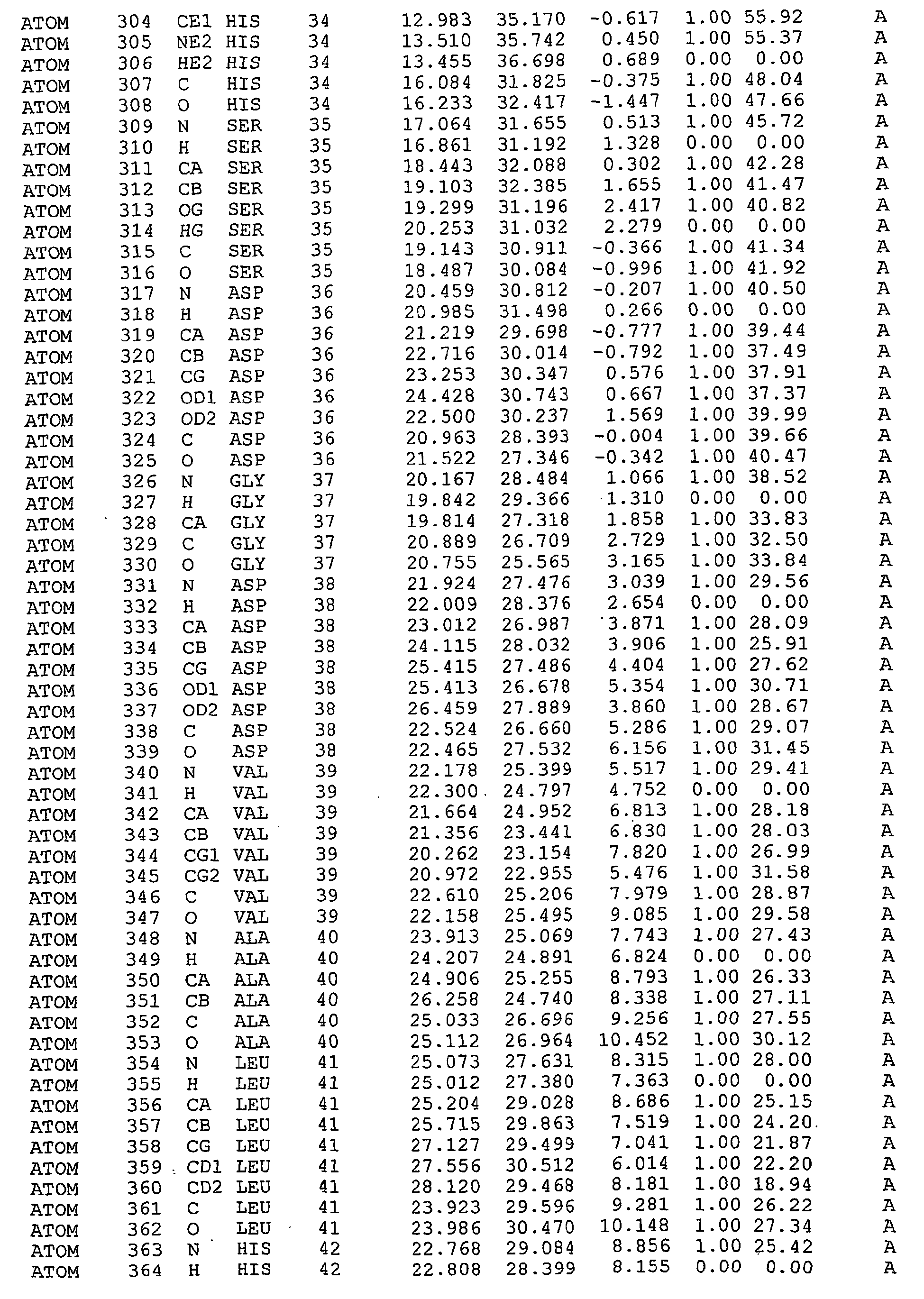

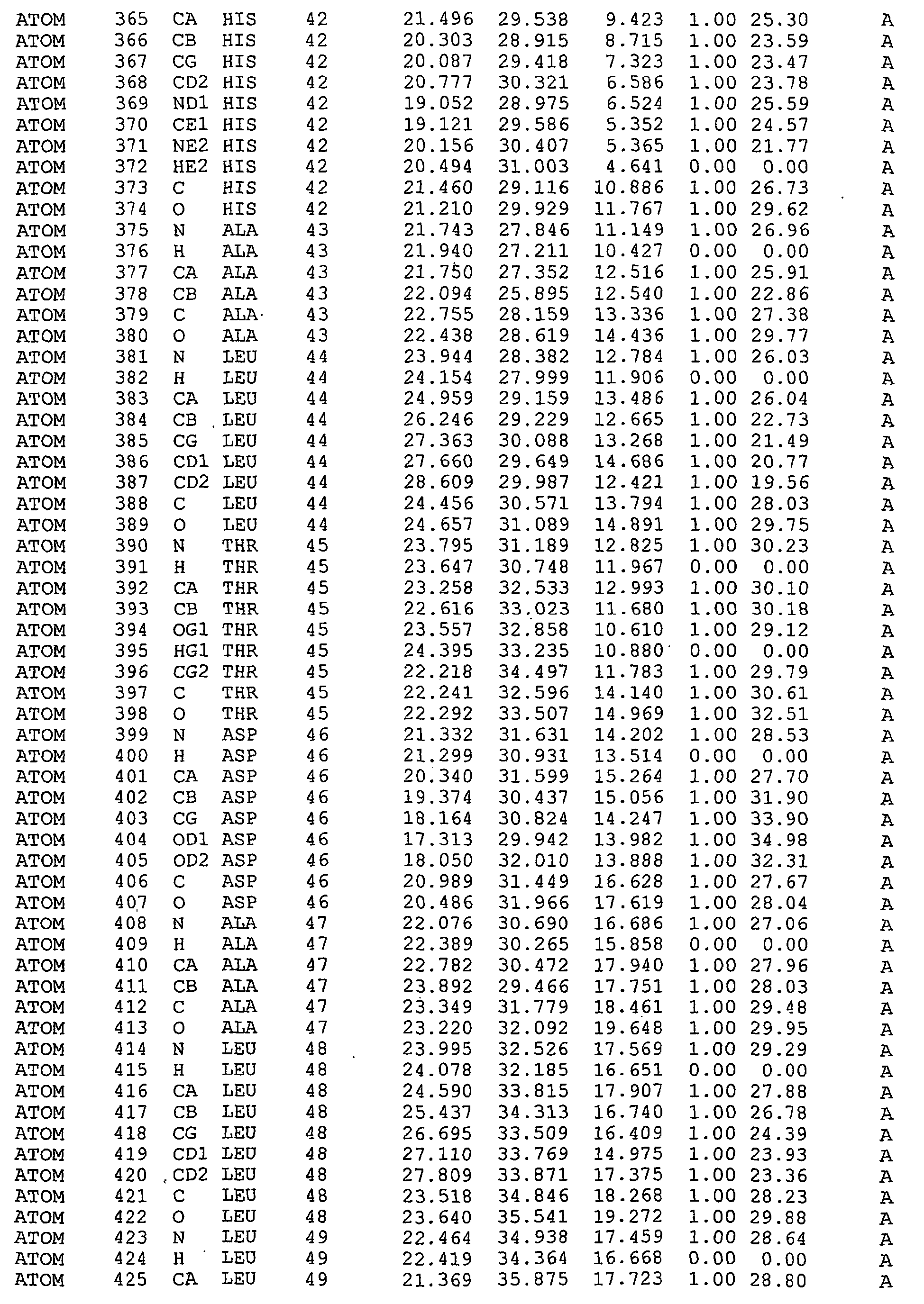

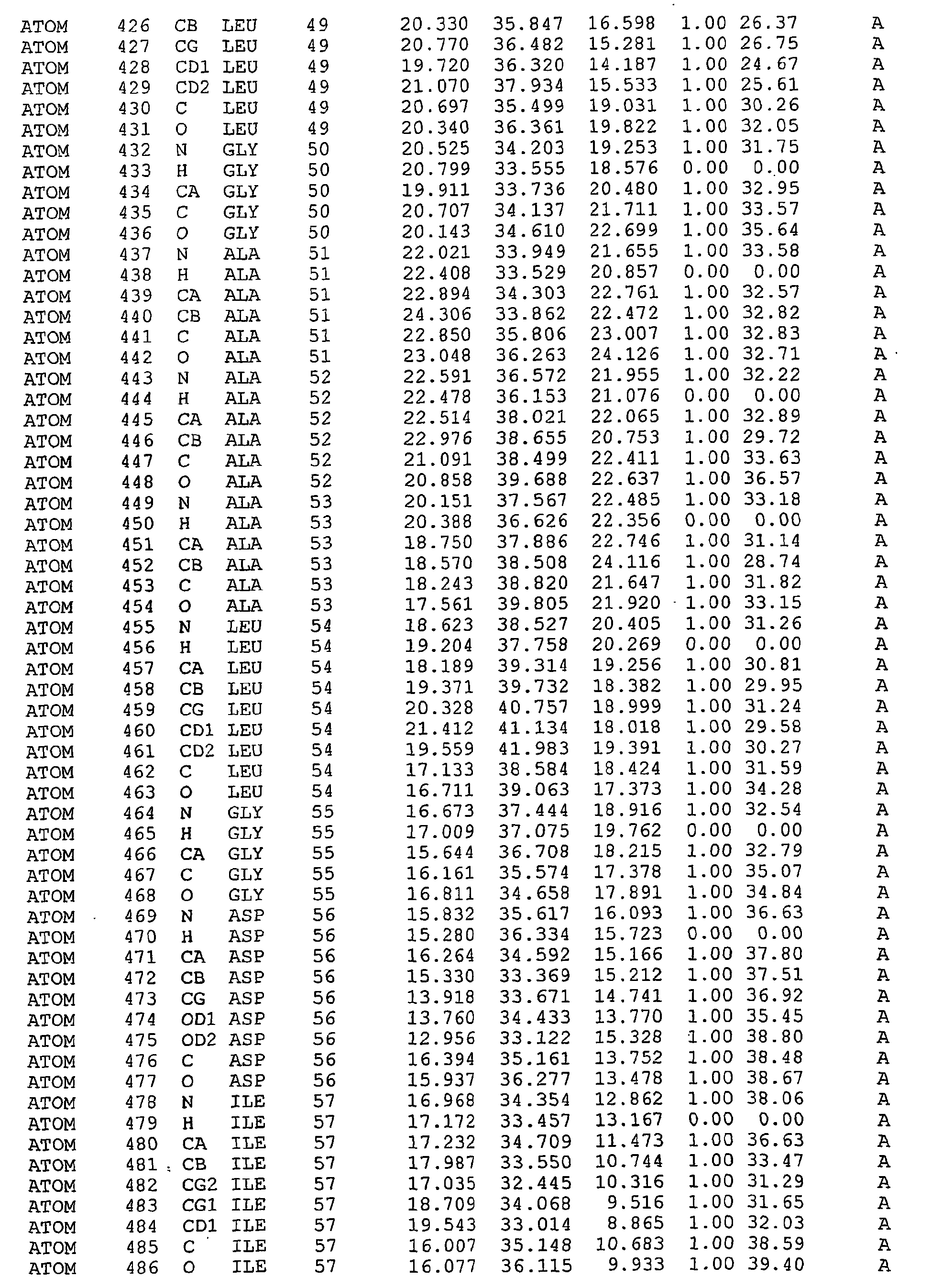

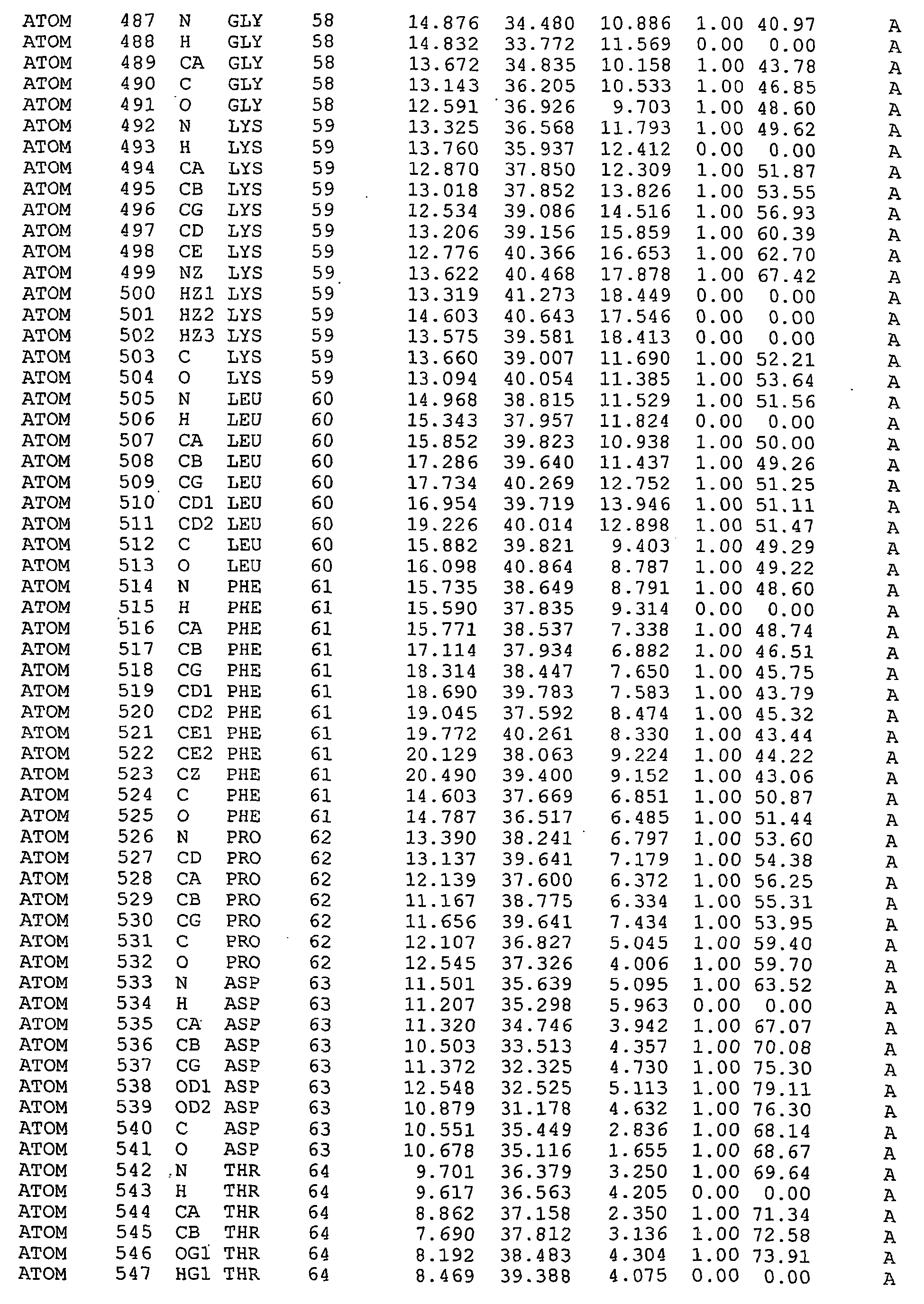

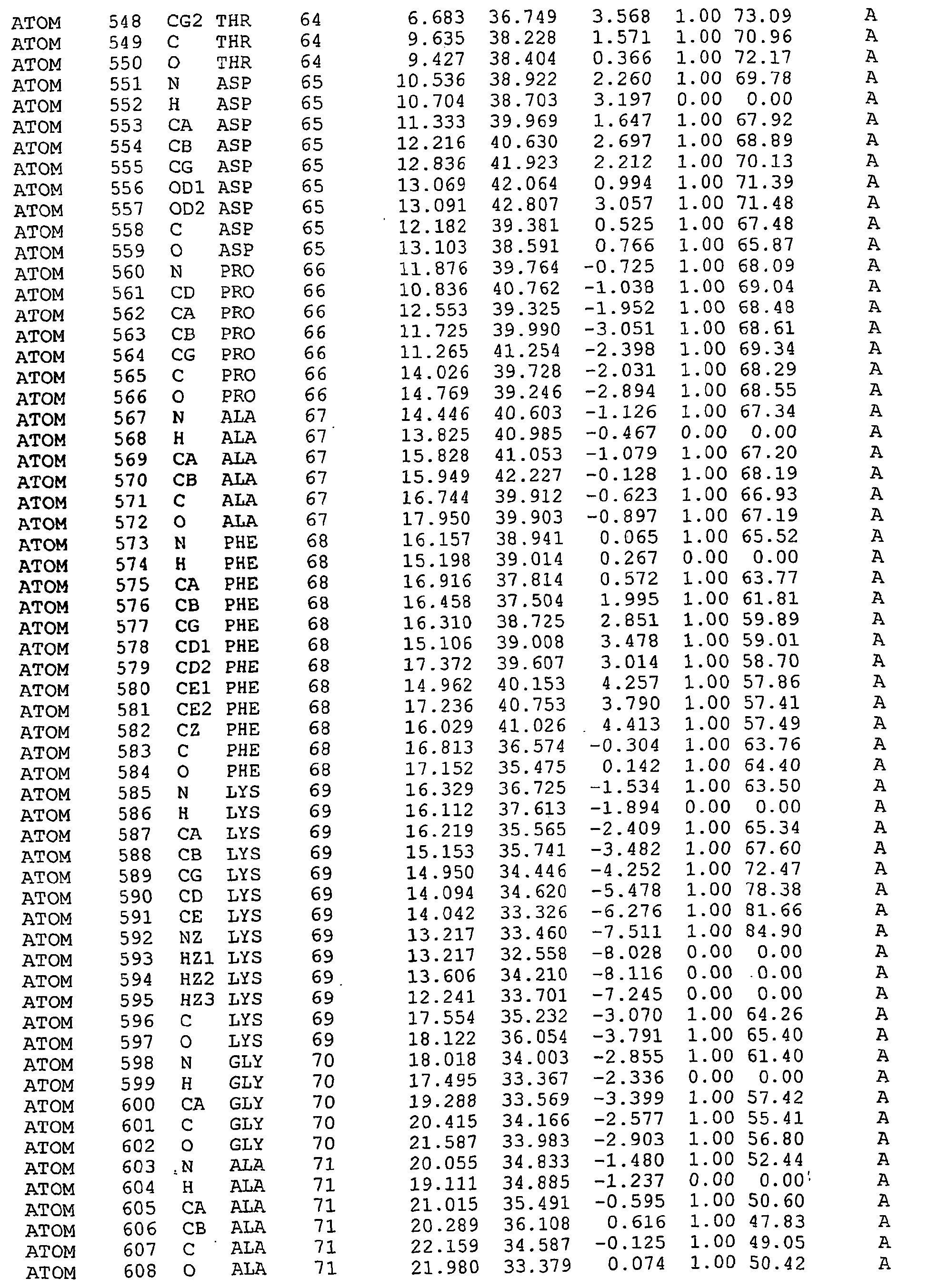

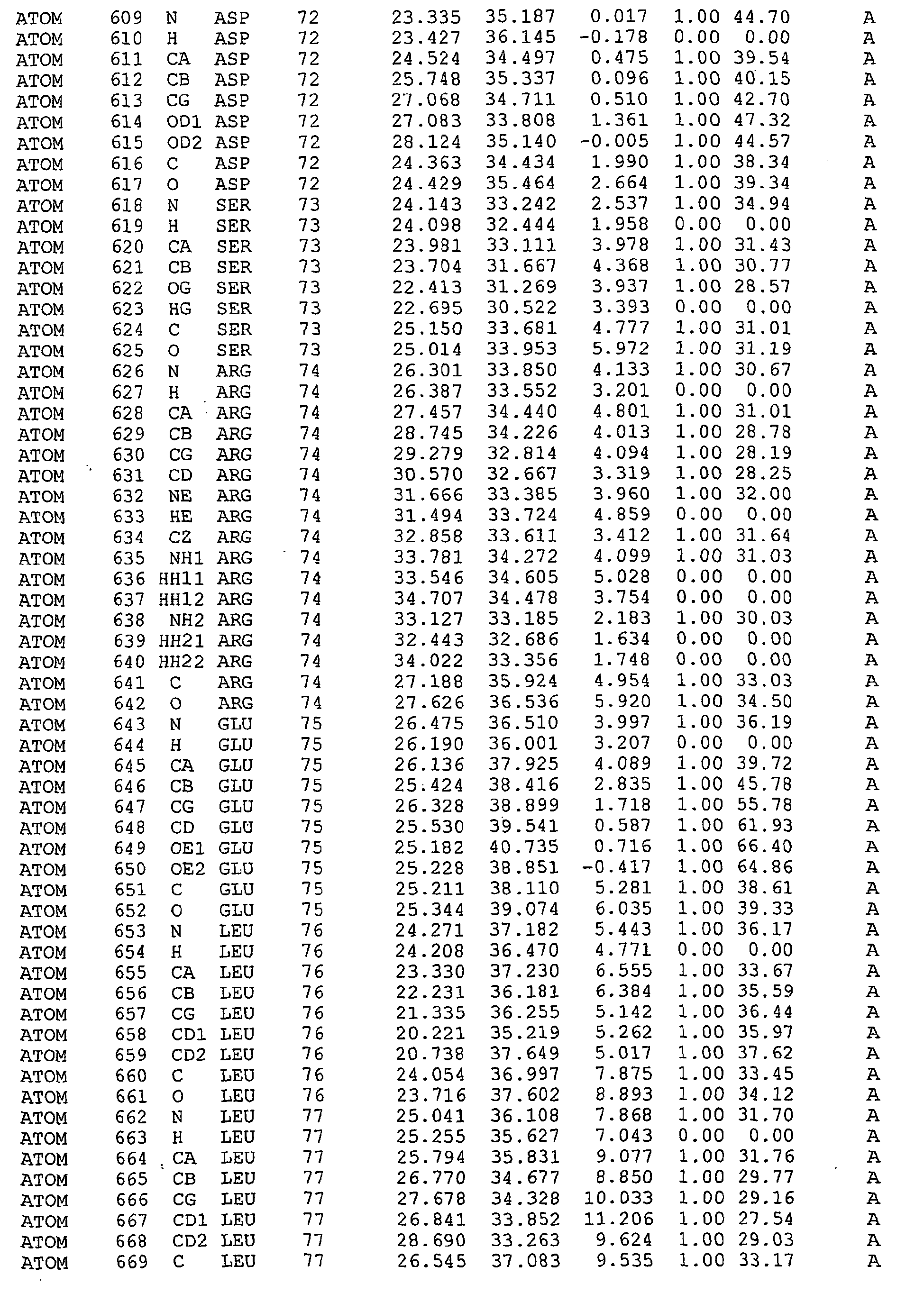

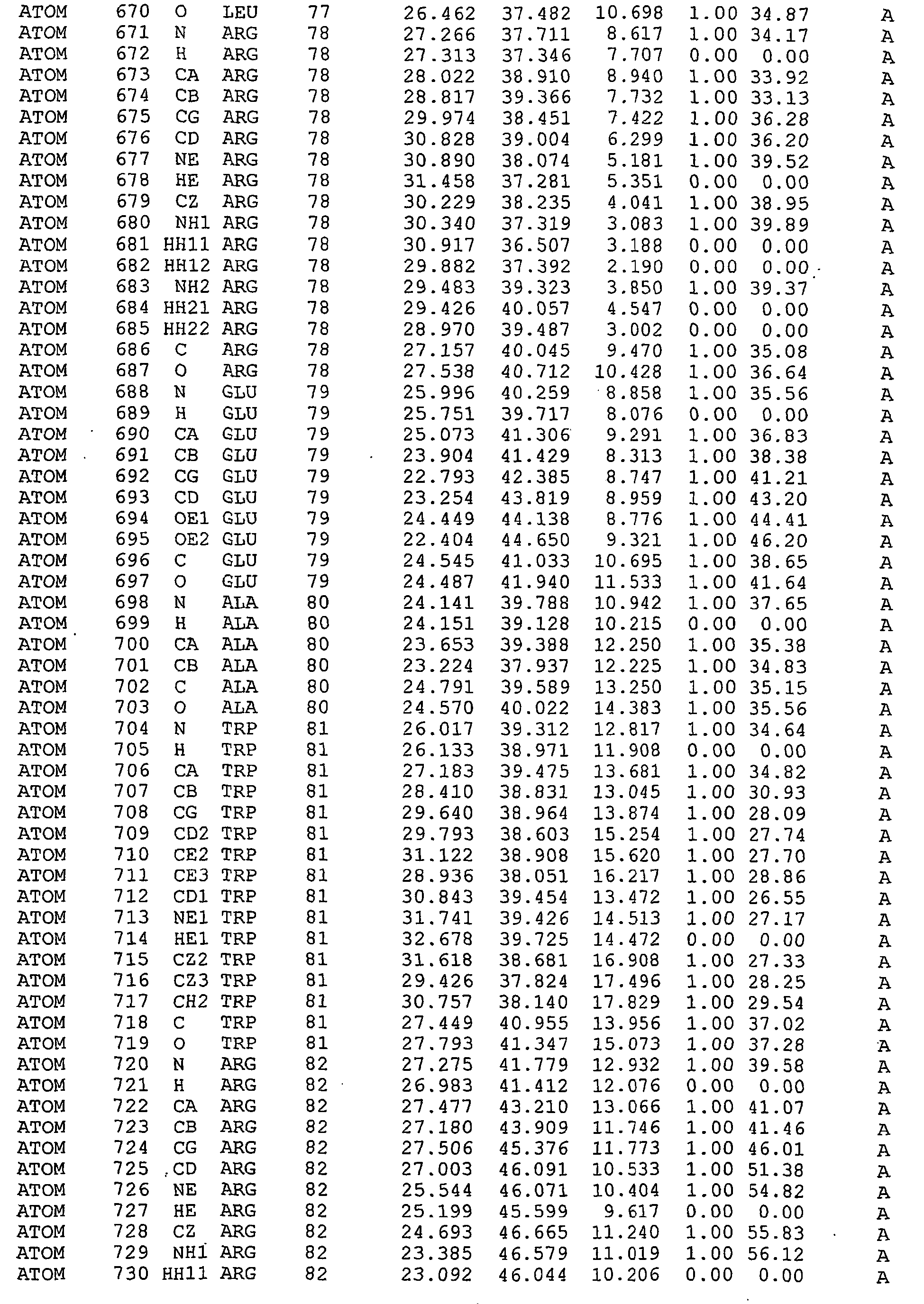

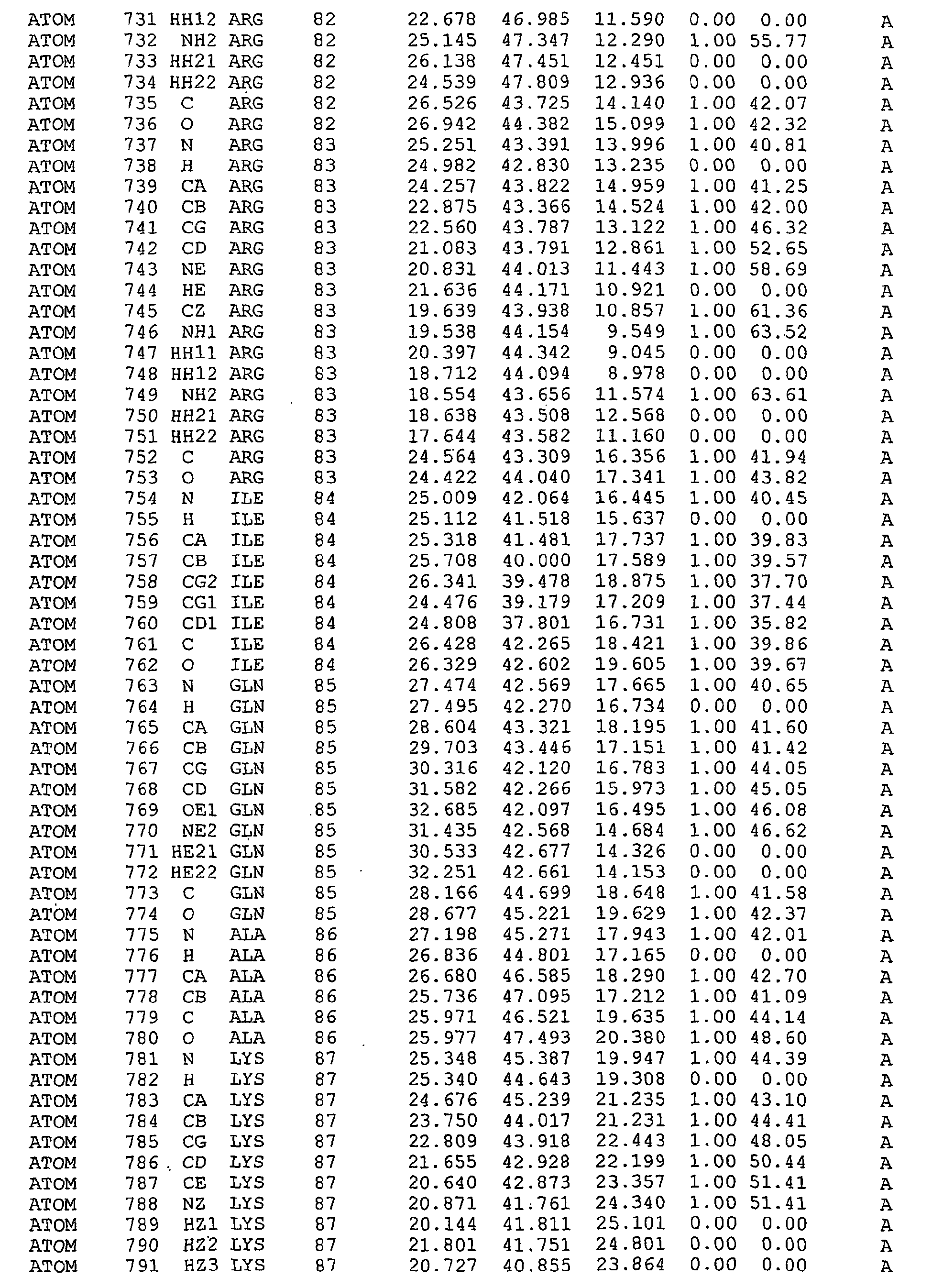

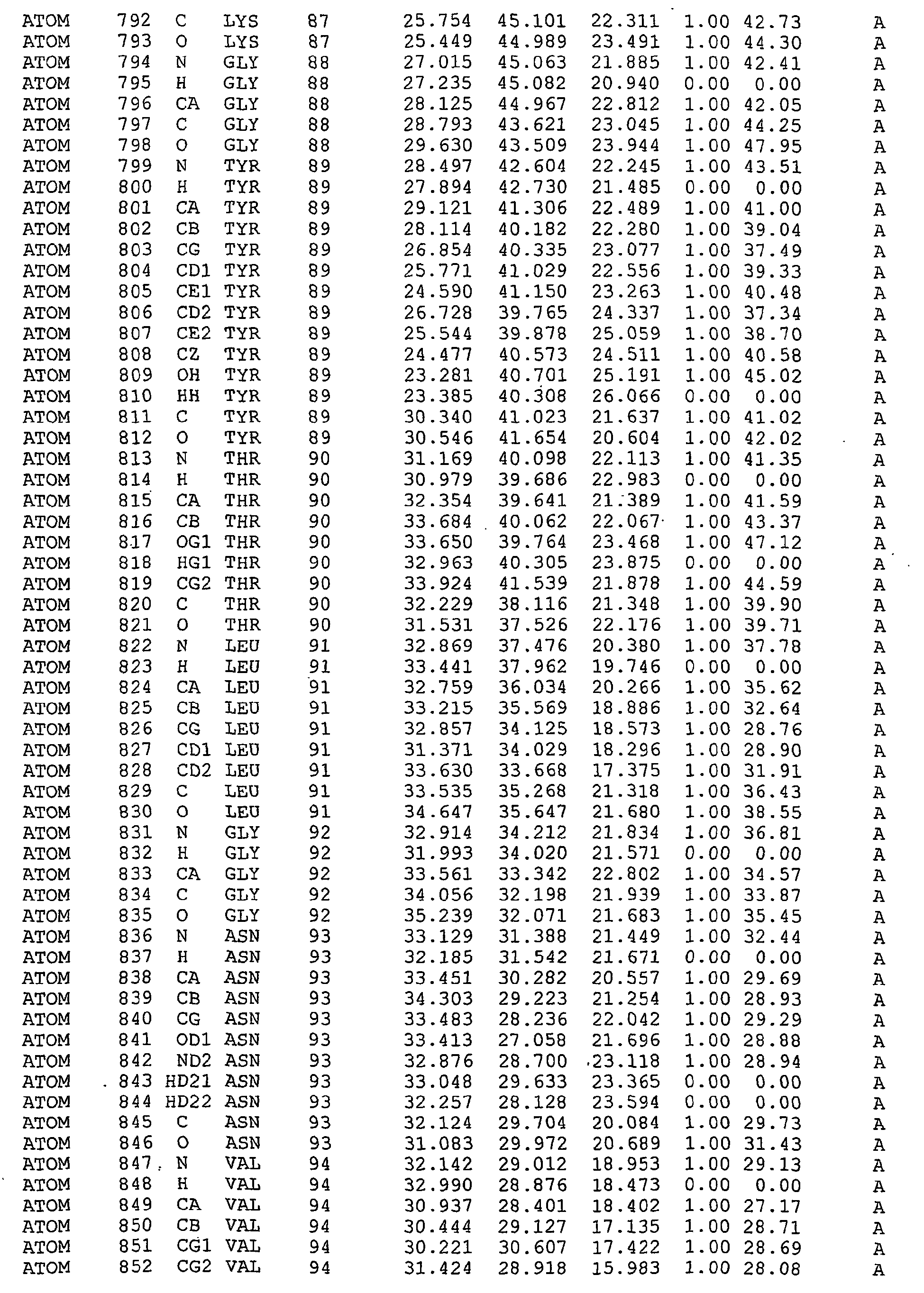

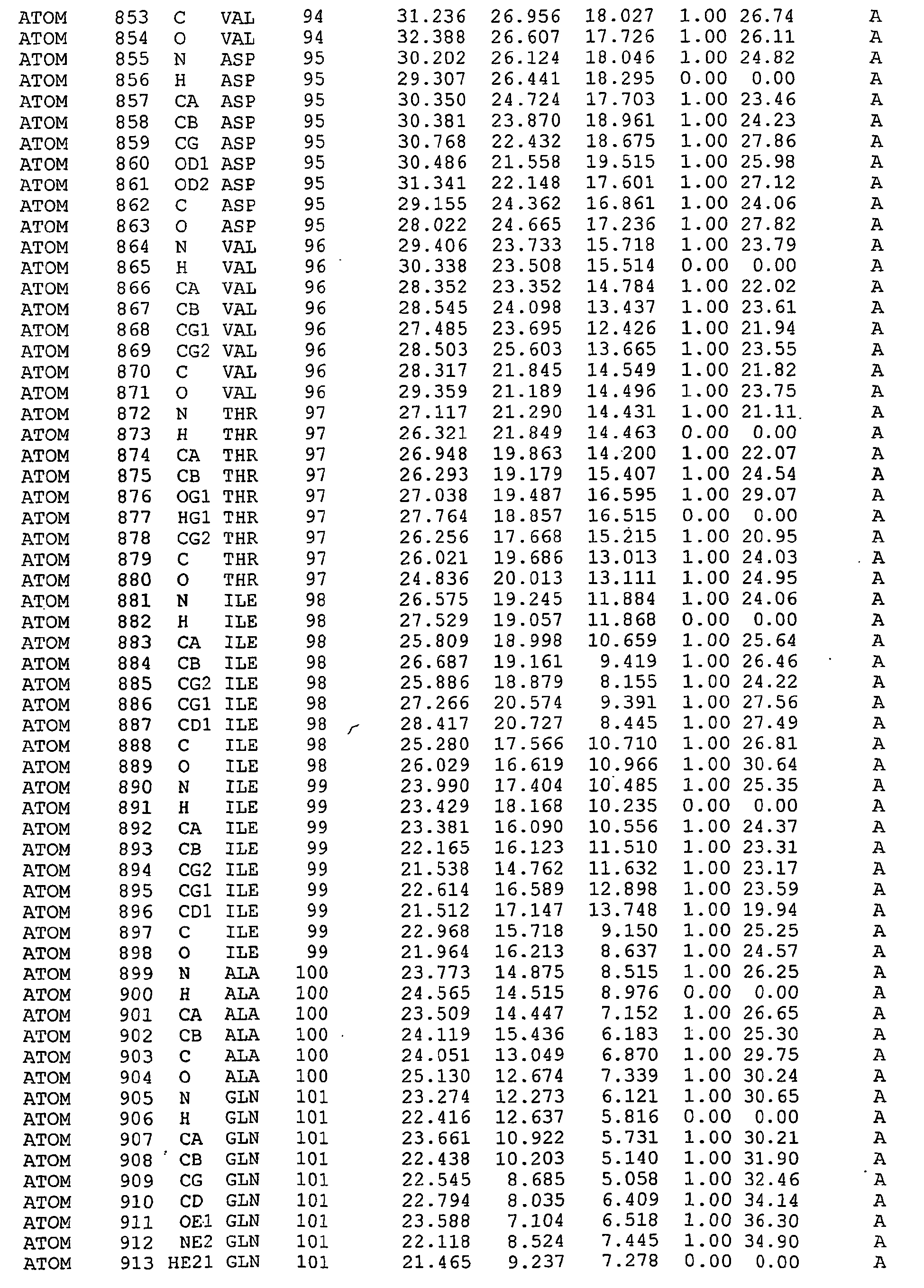

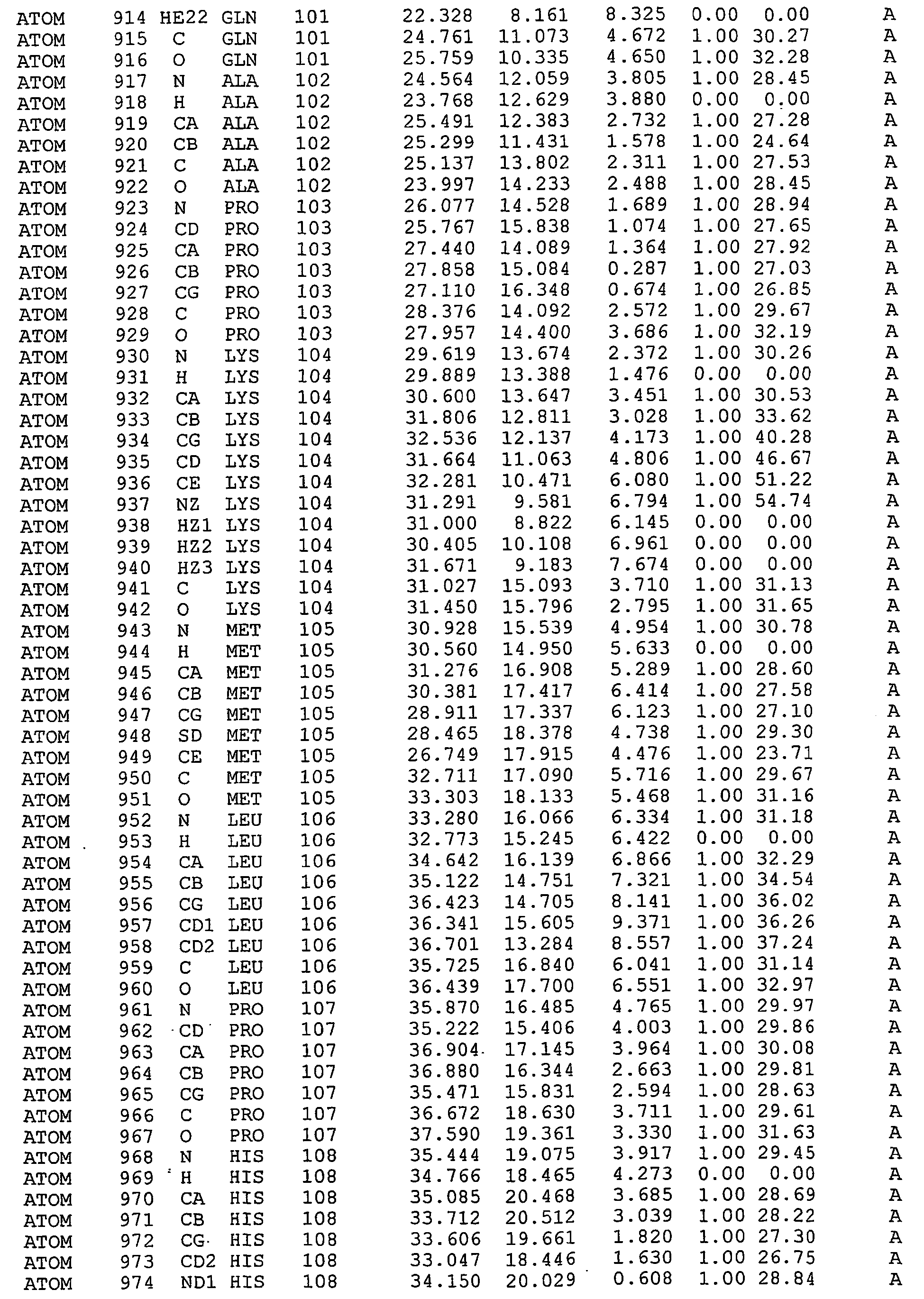

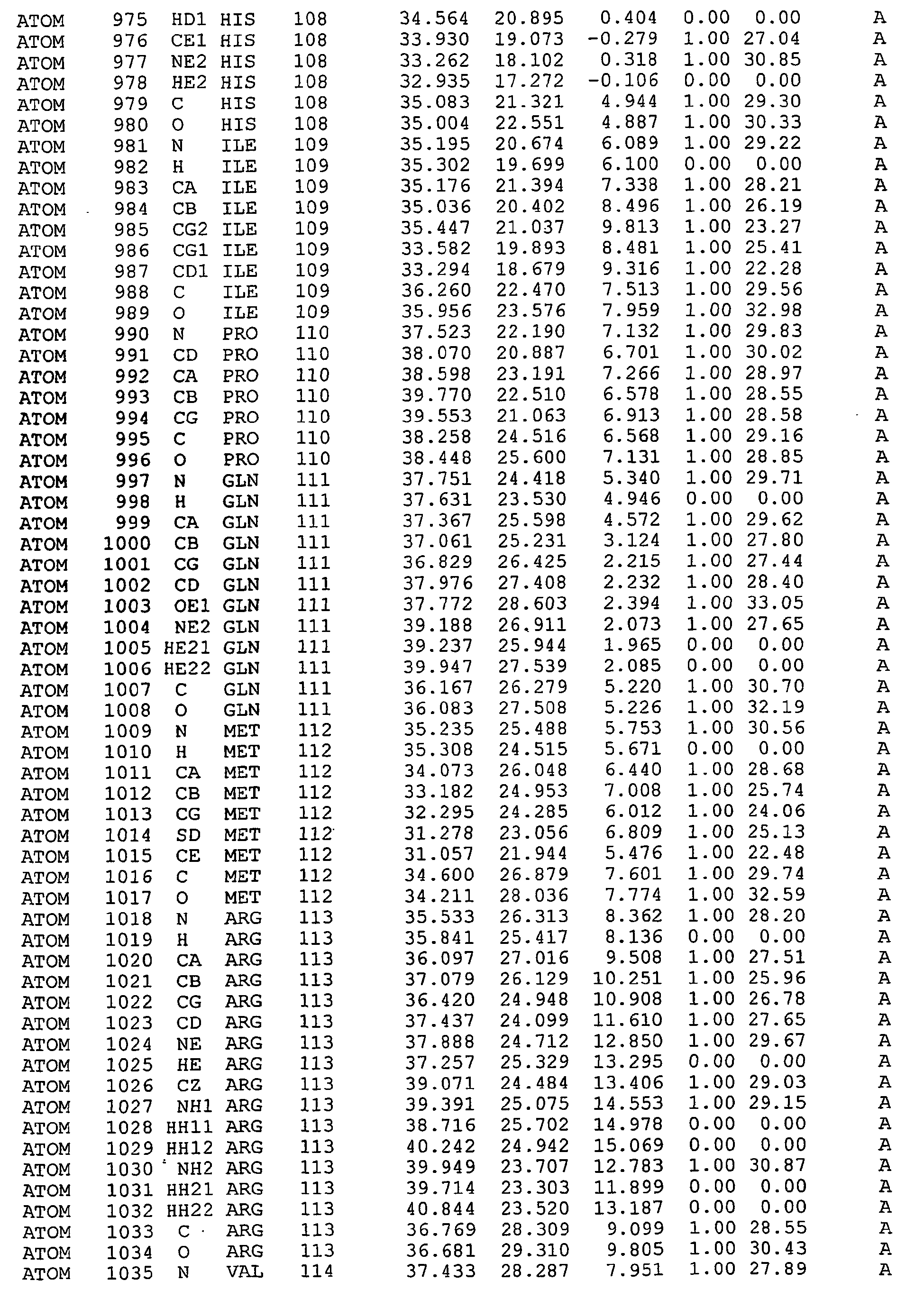

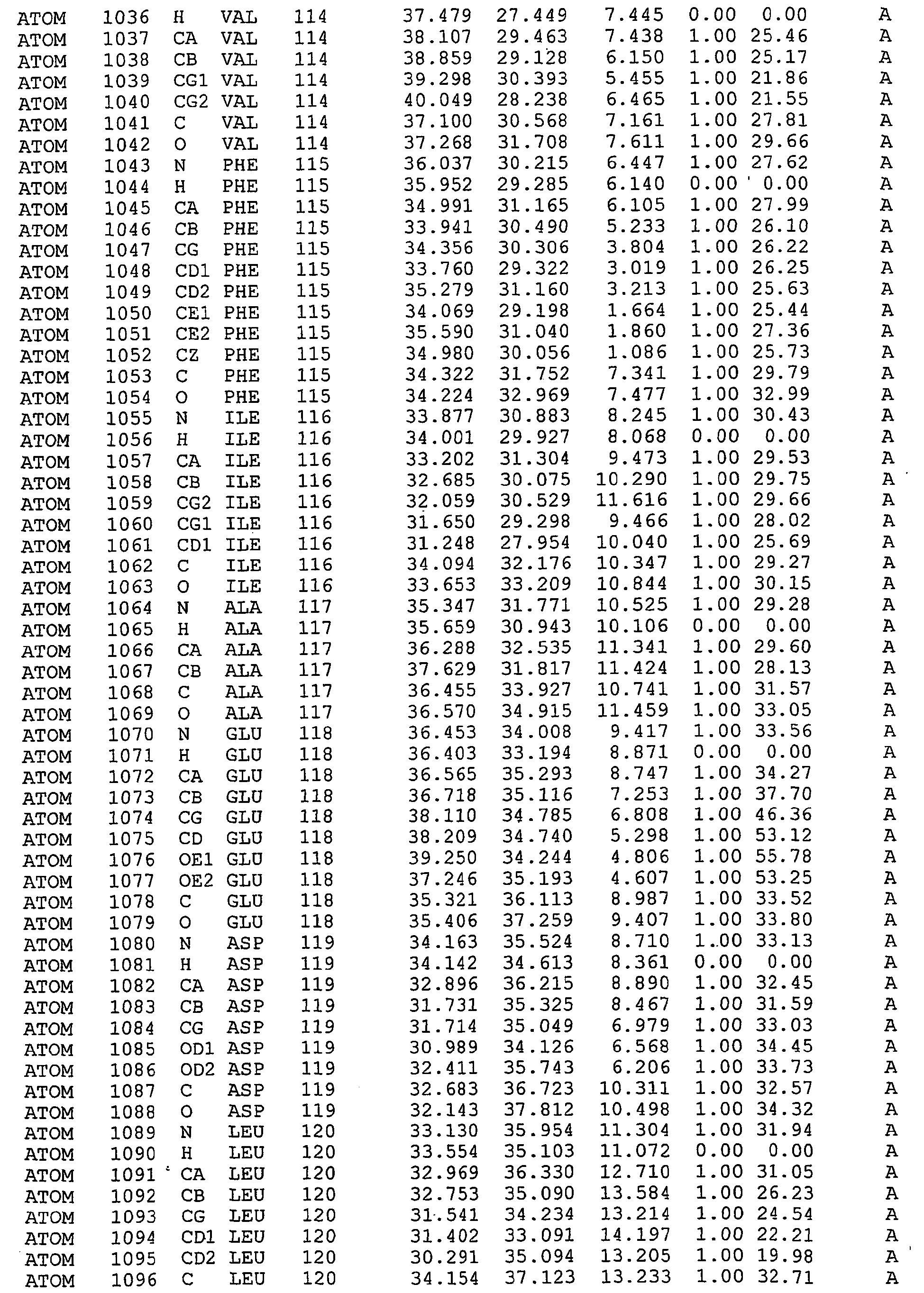

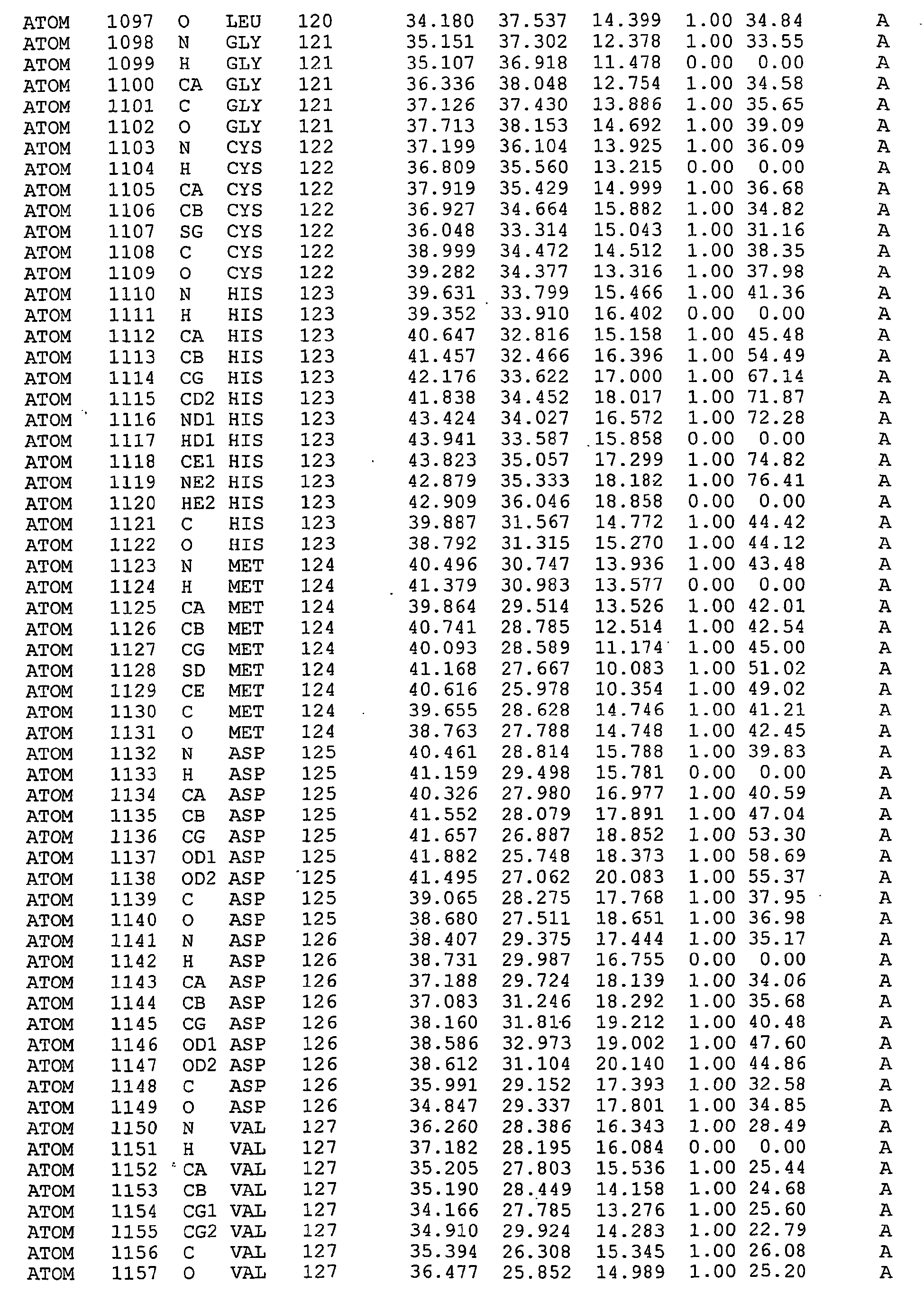

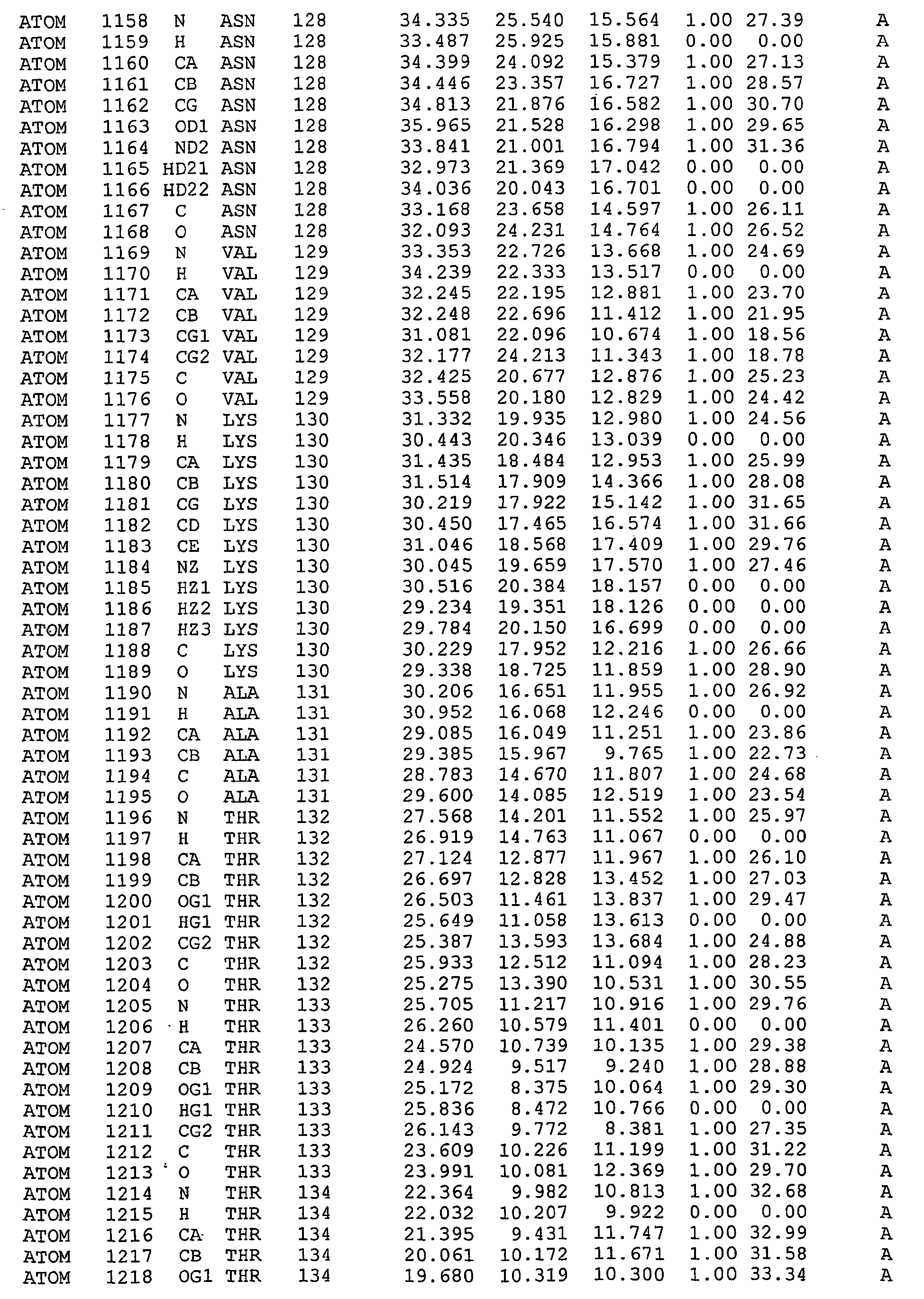

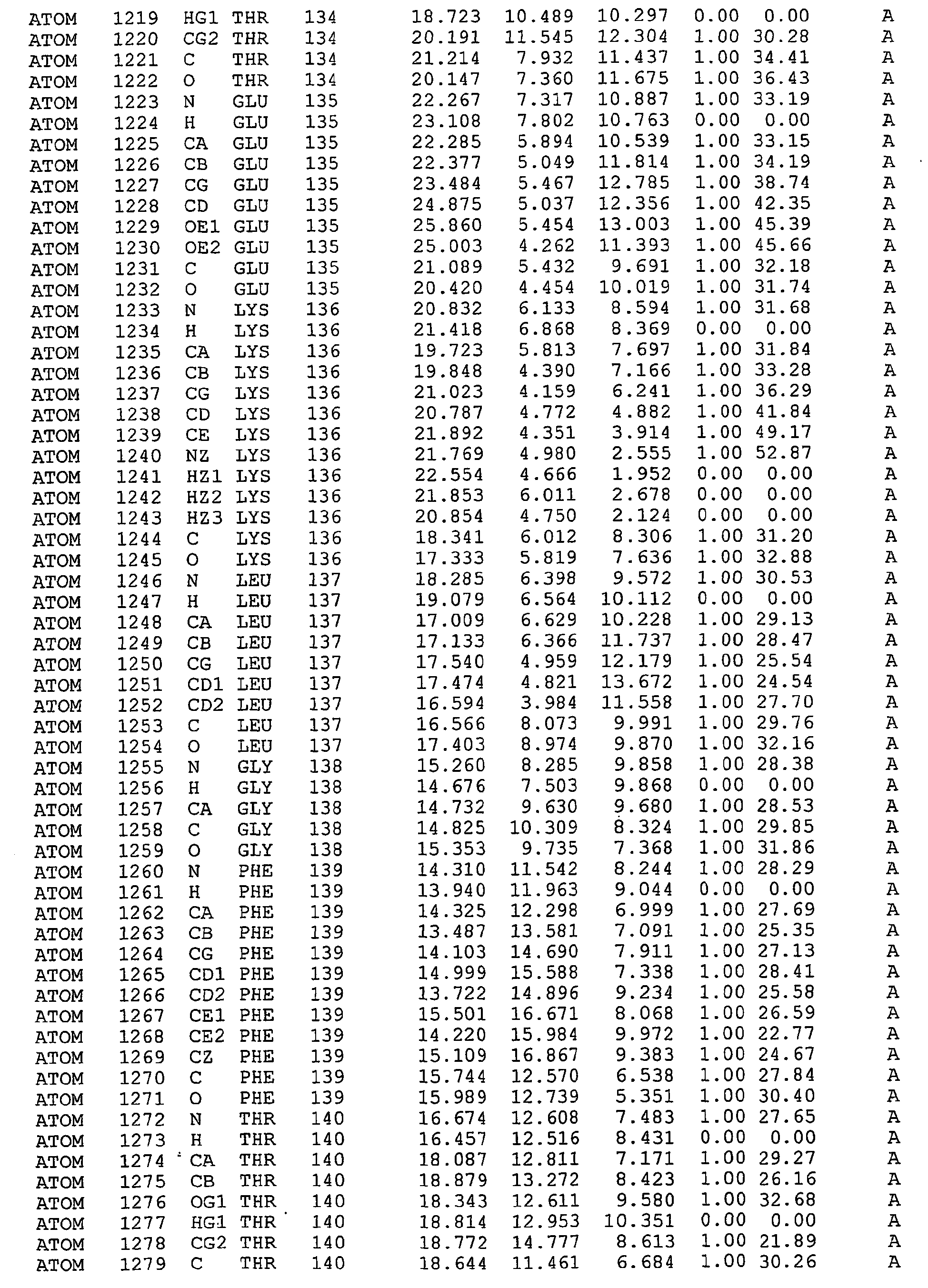

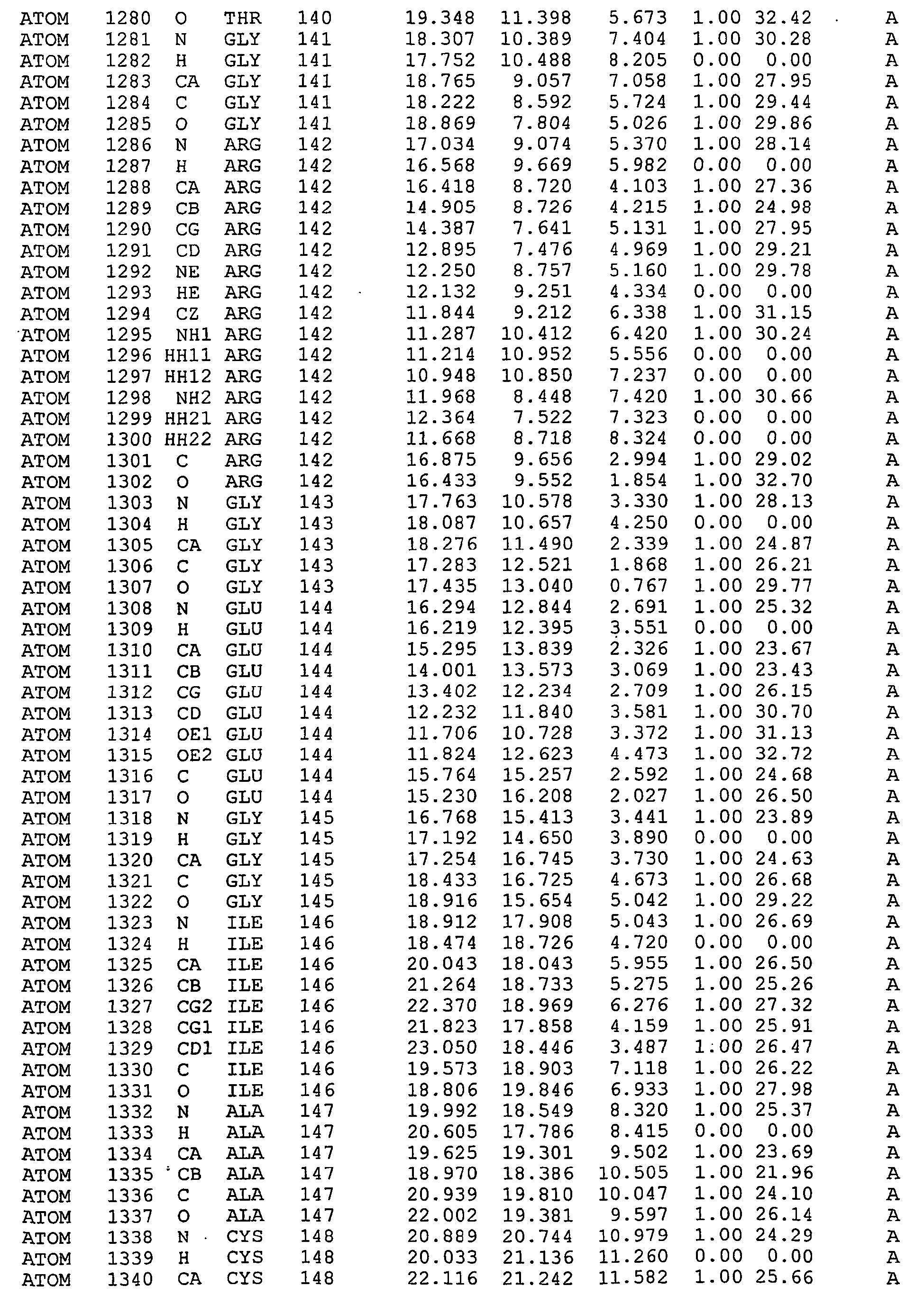

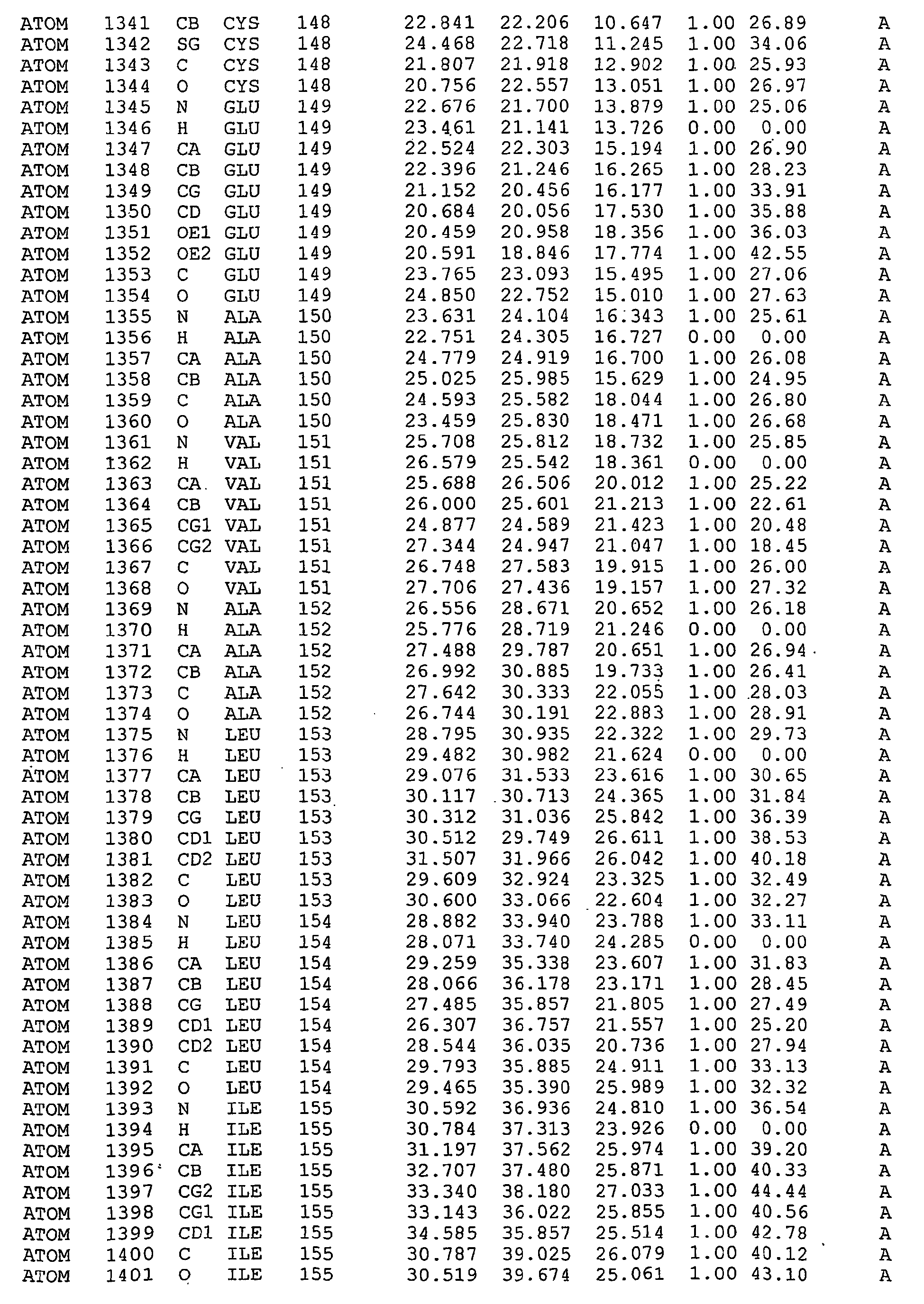

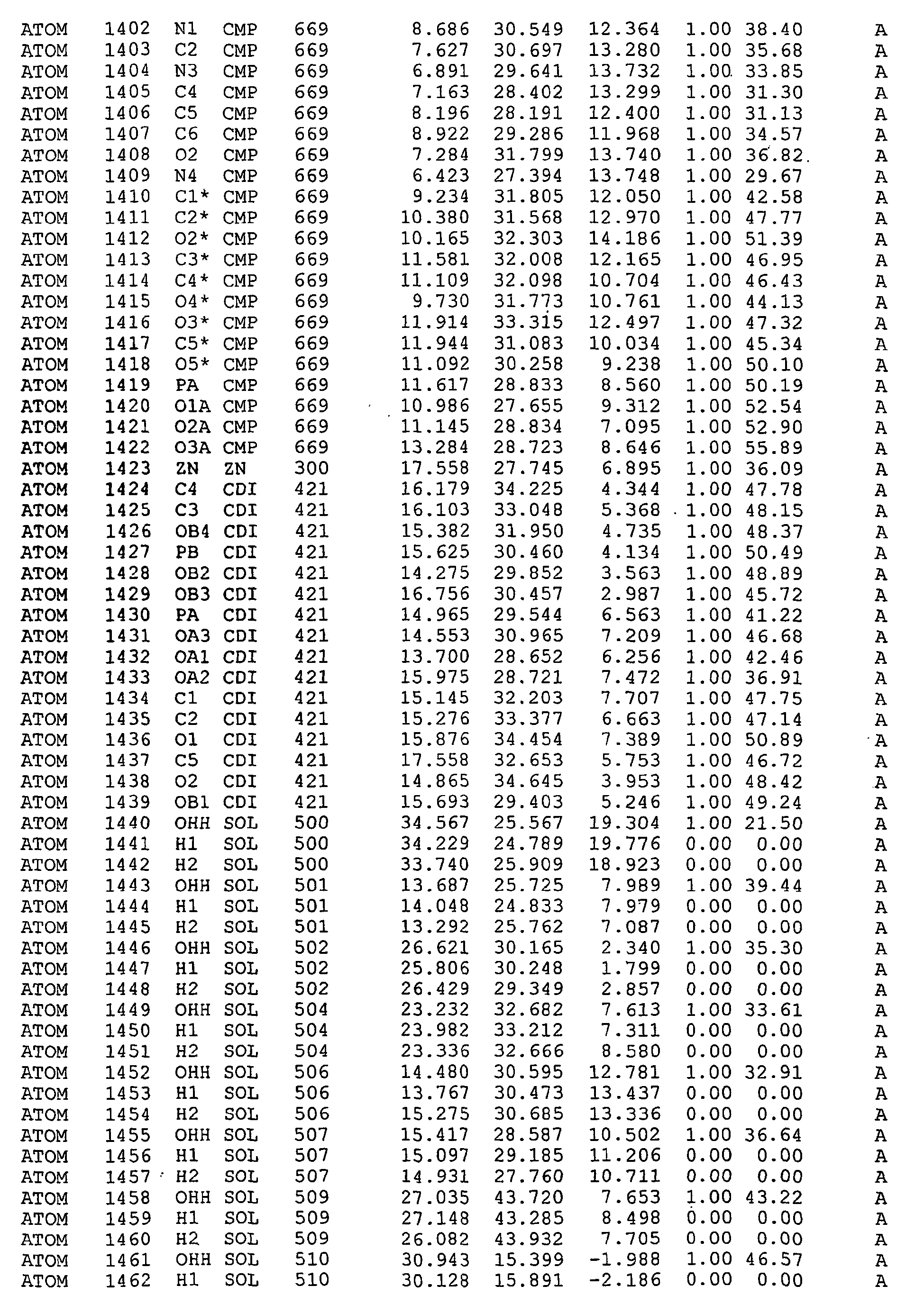

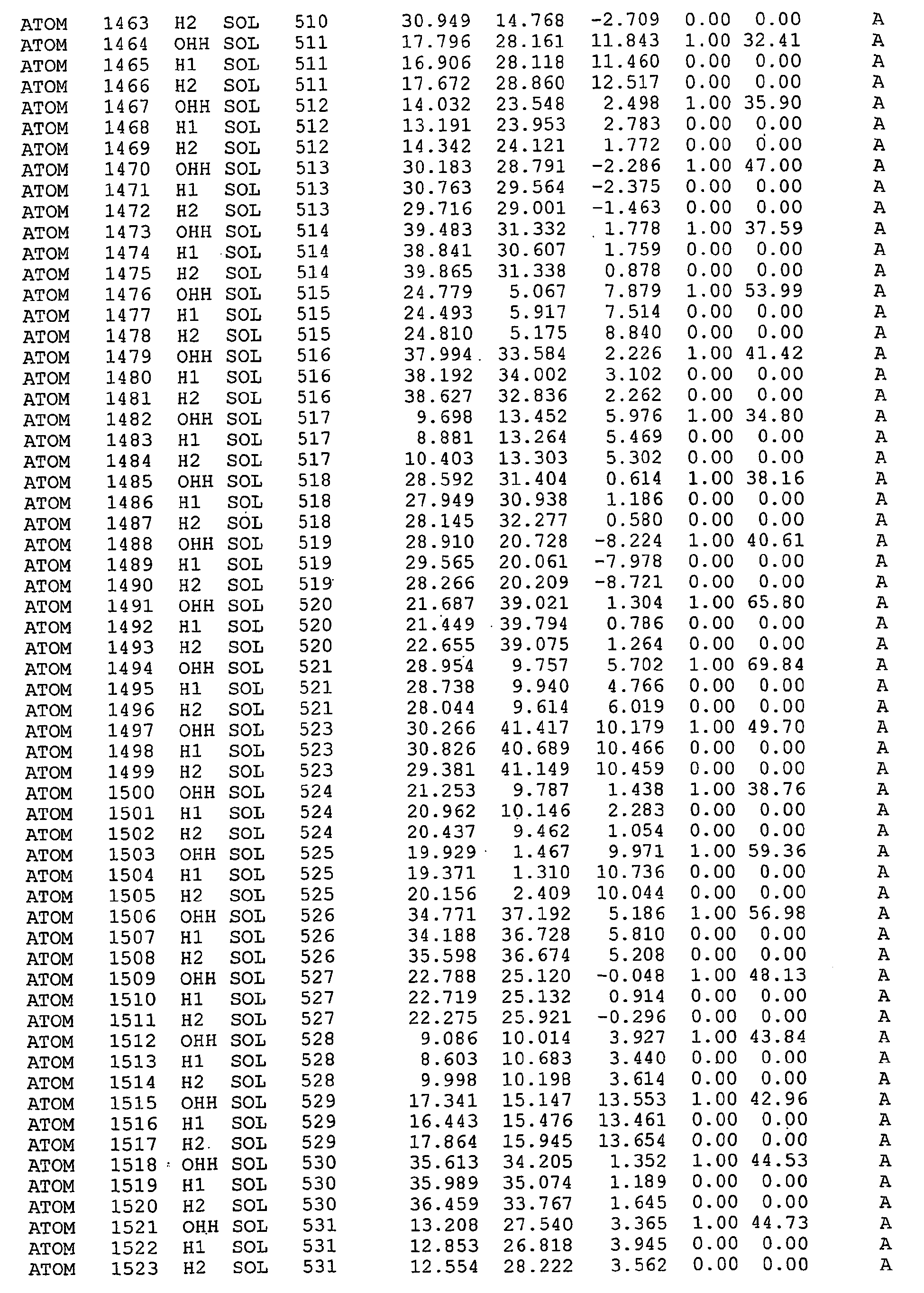

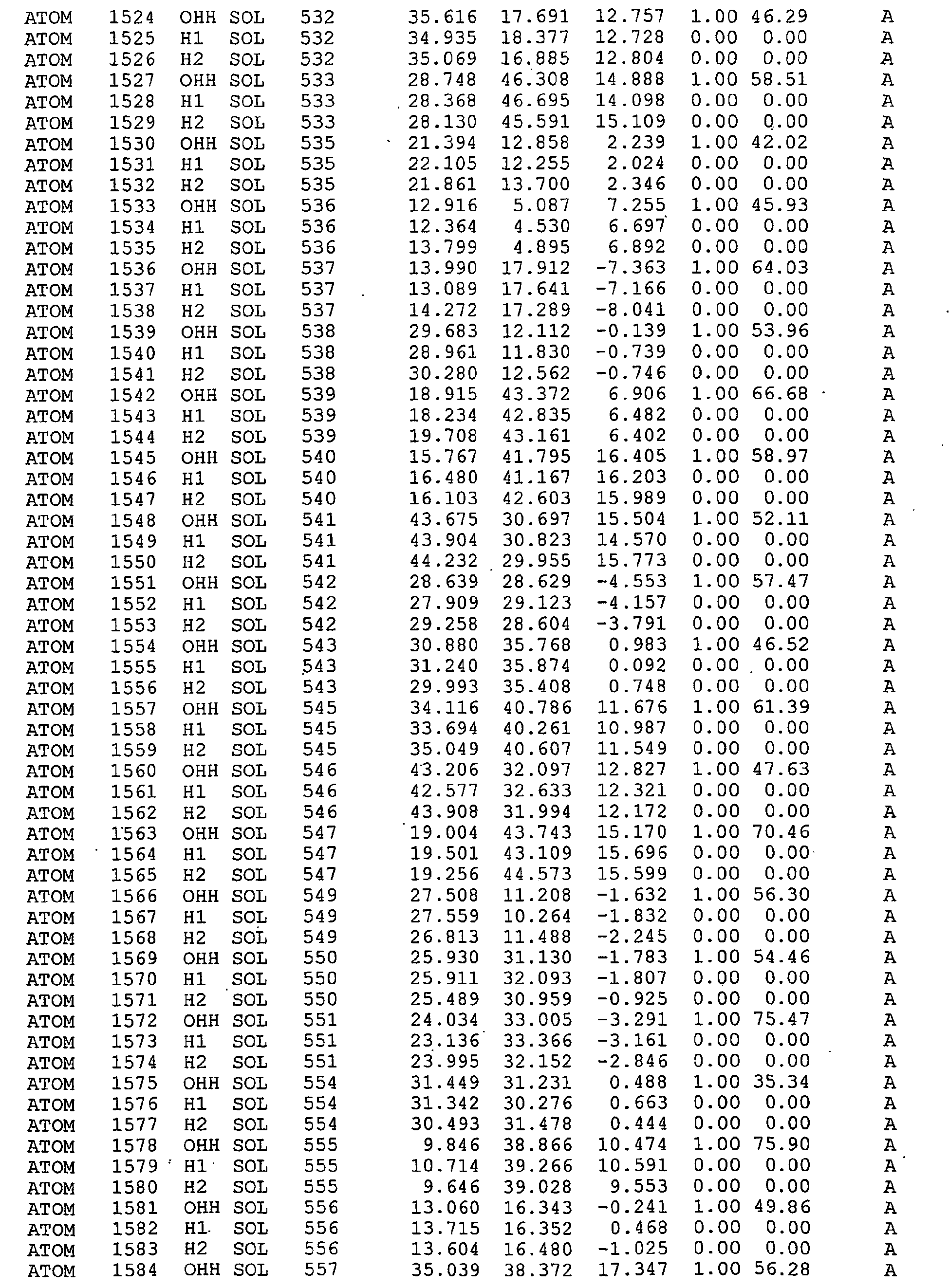

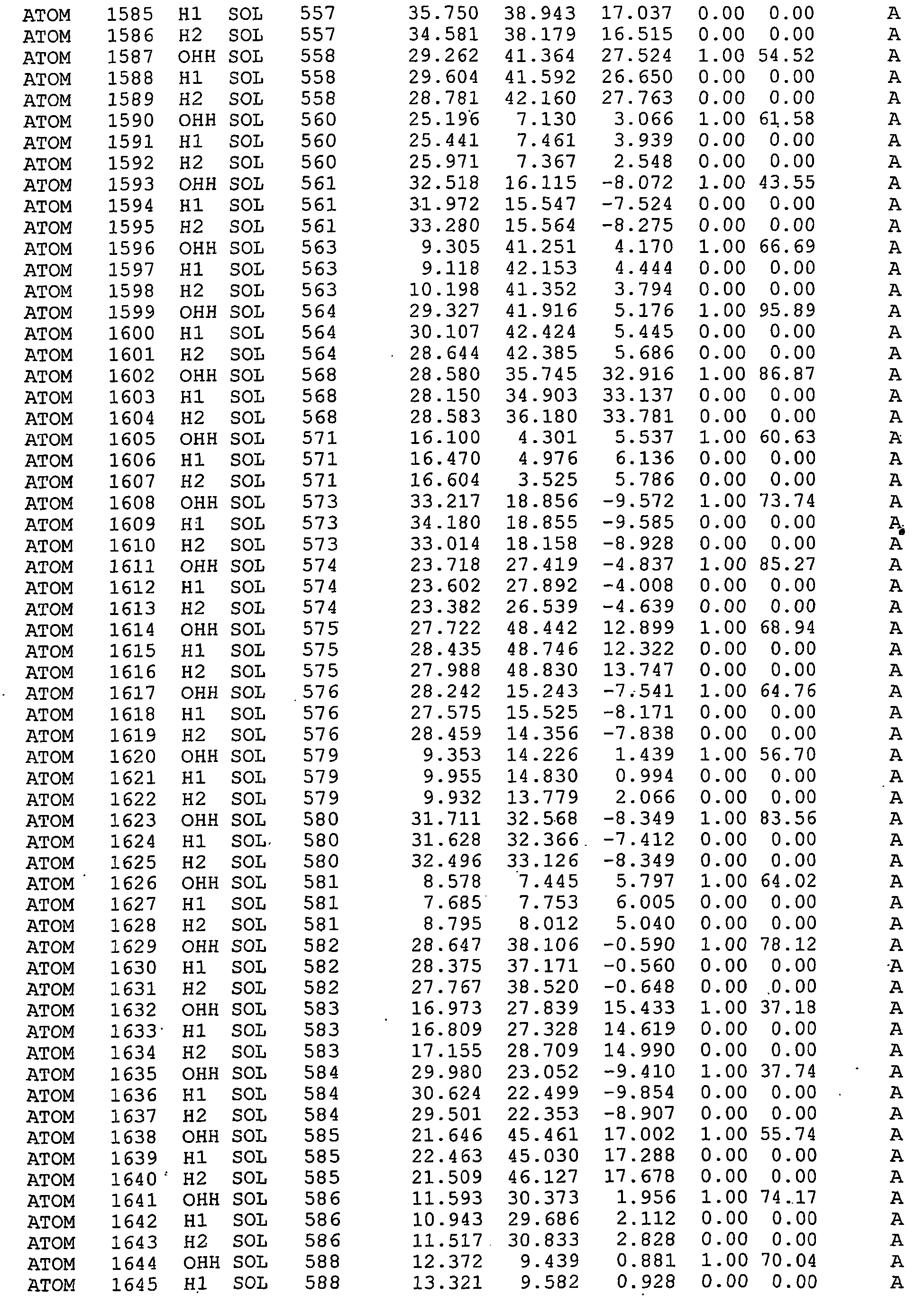

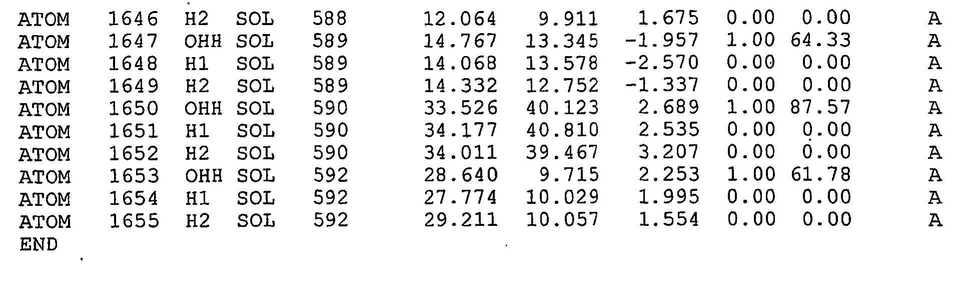

### Annex 5: Coordinates of structure subop

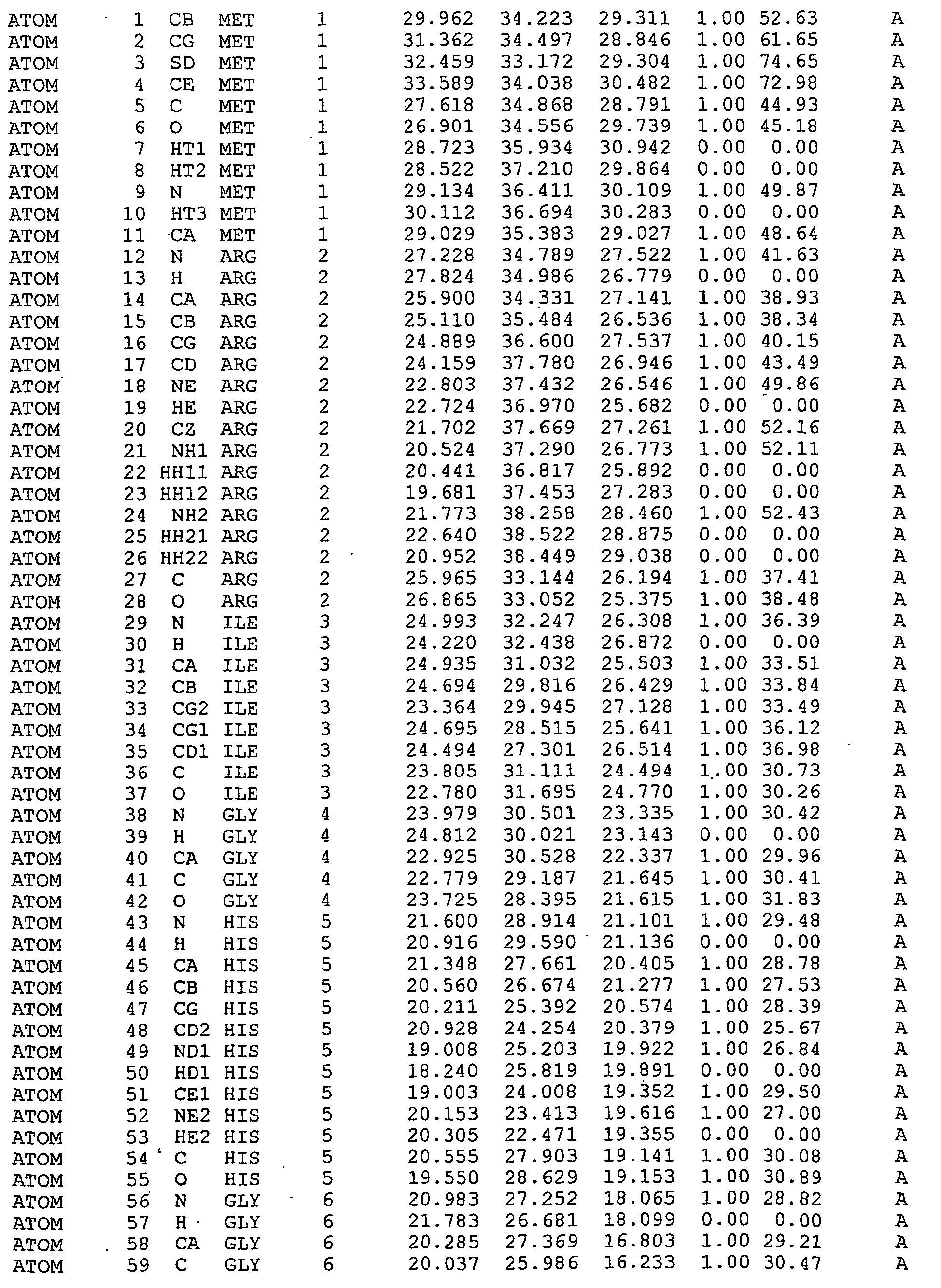

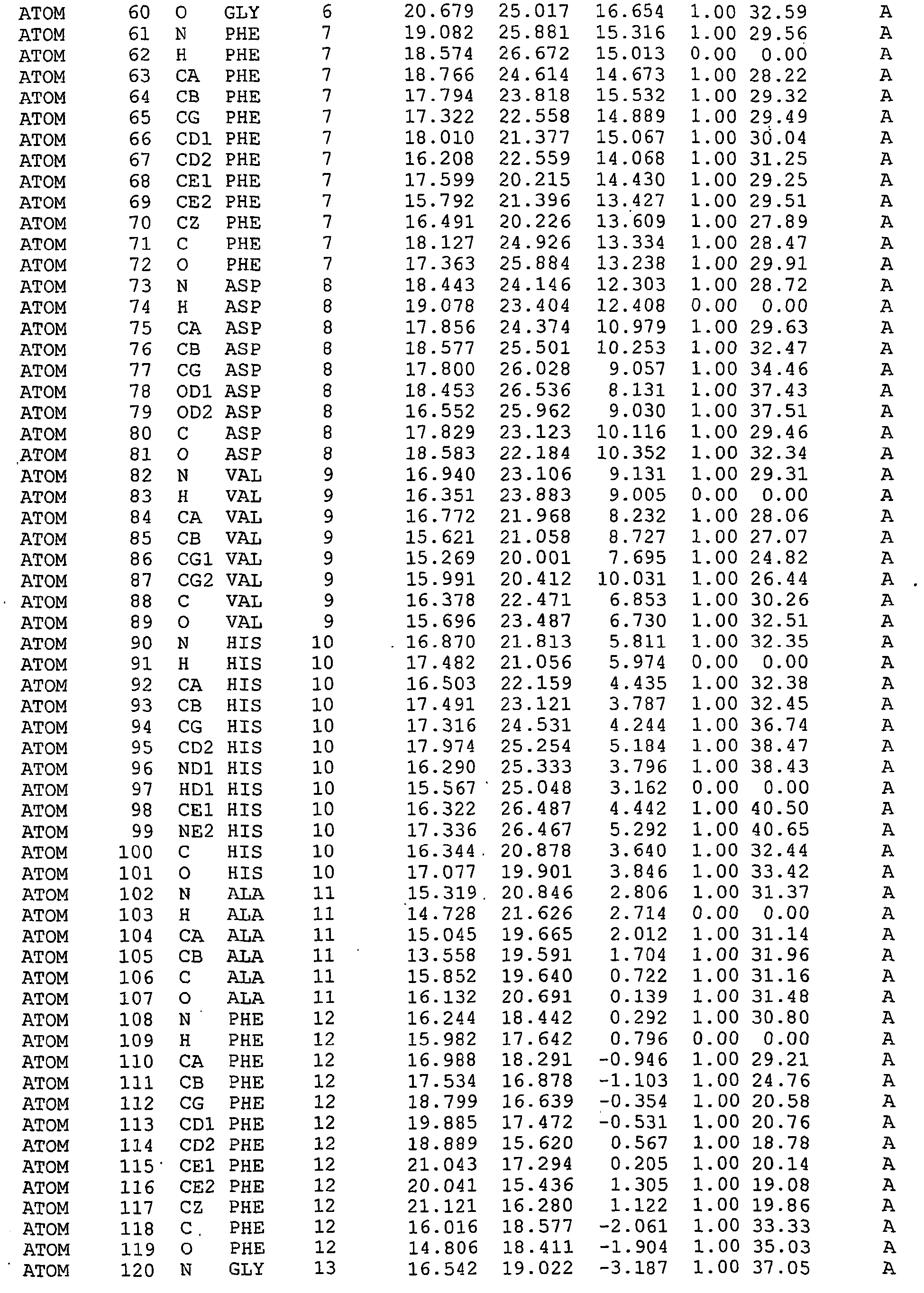

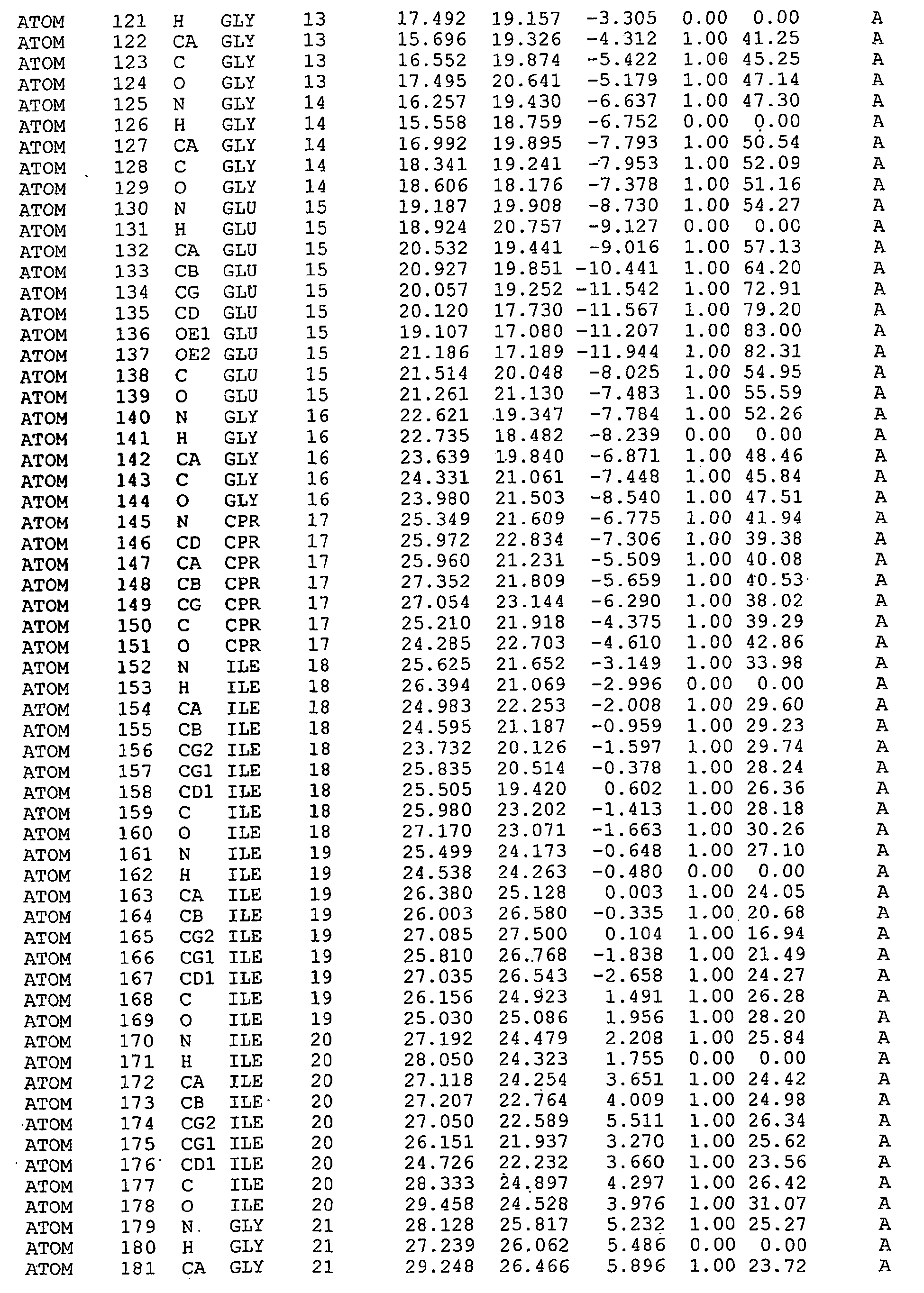

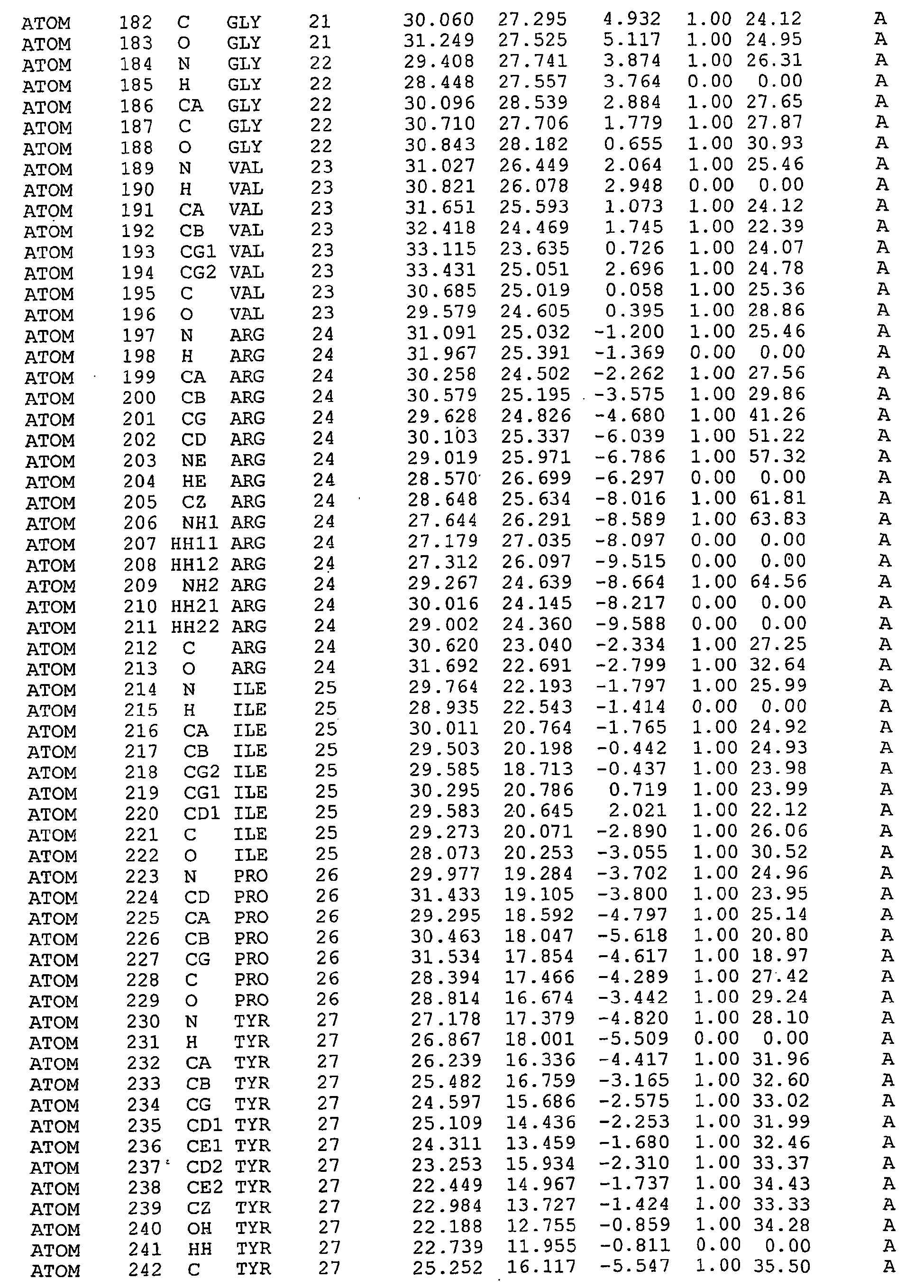

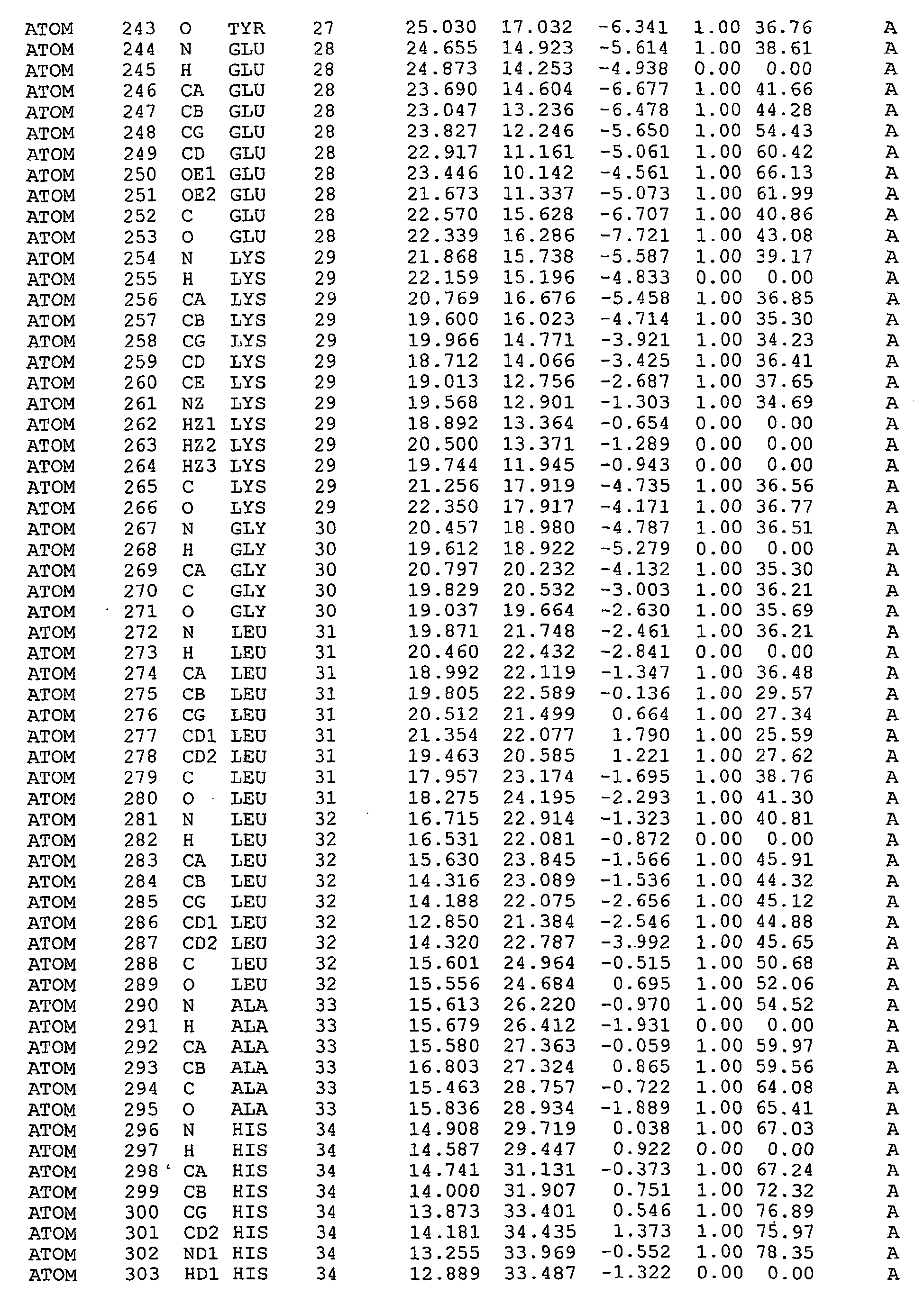

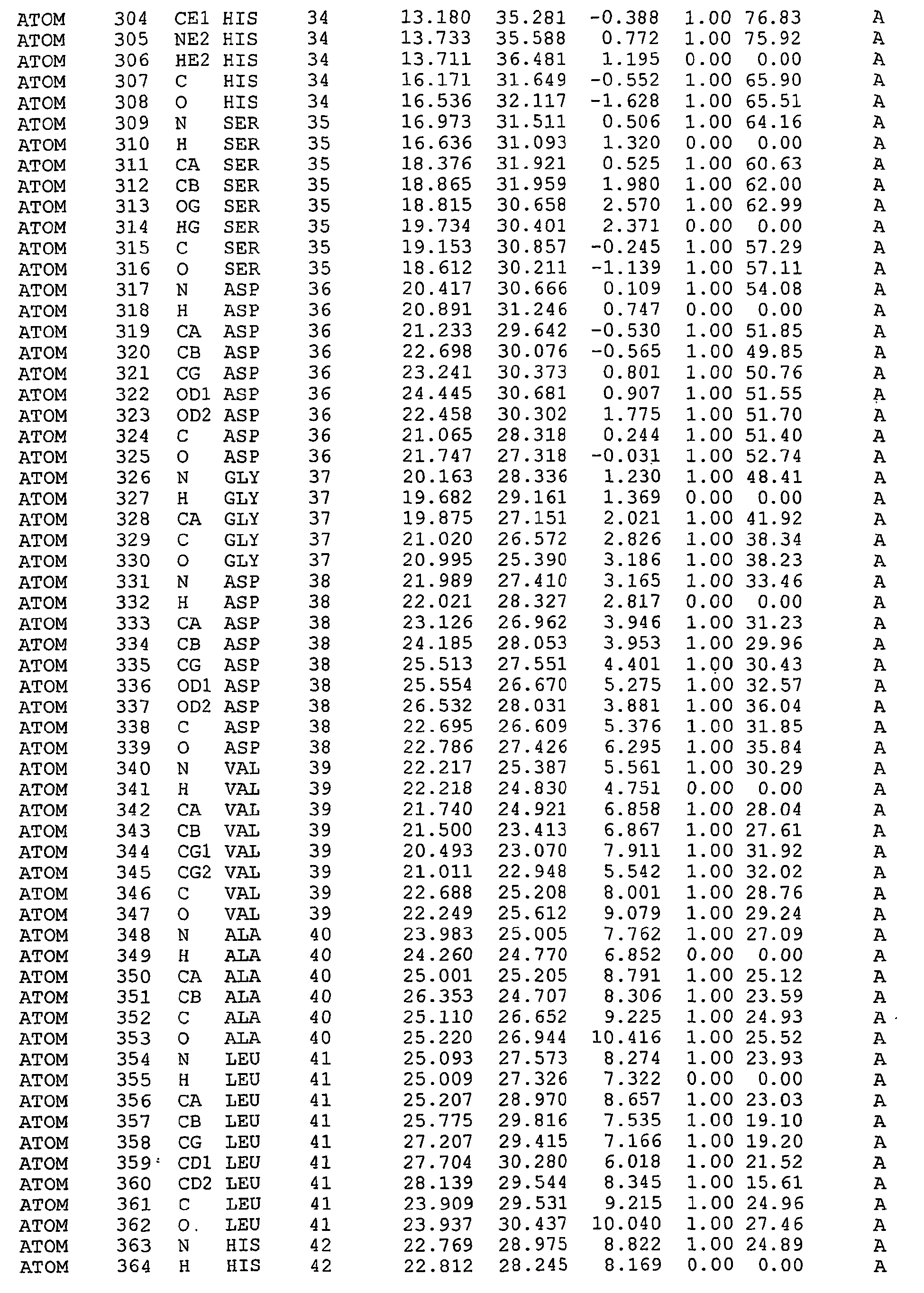

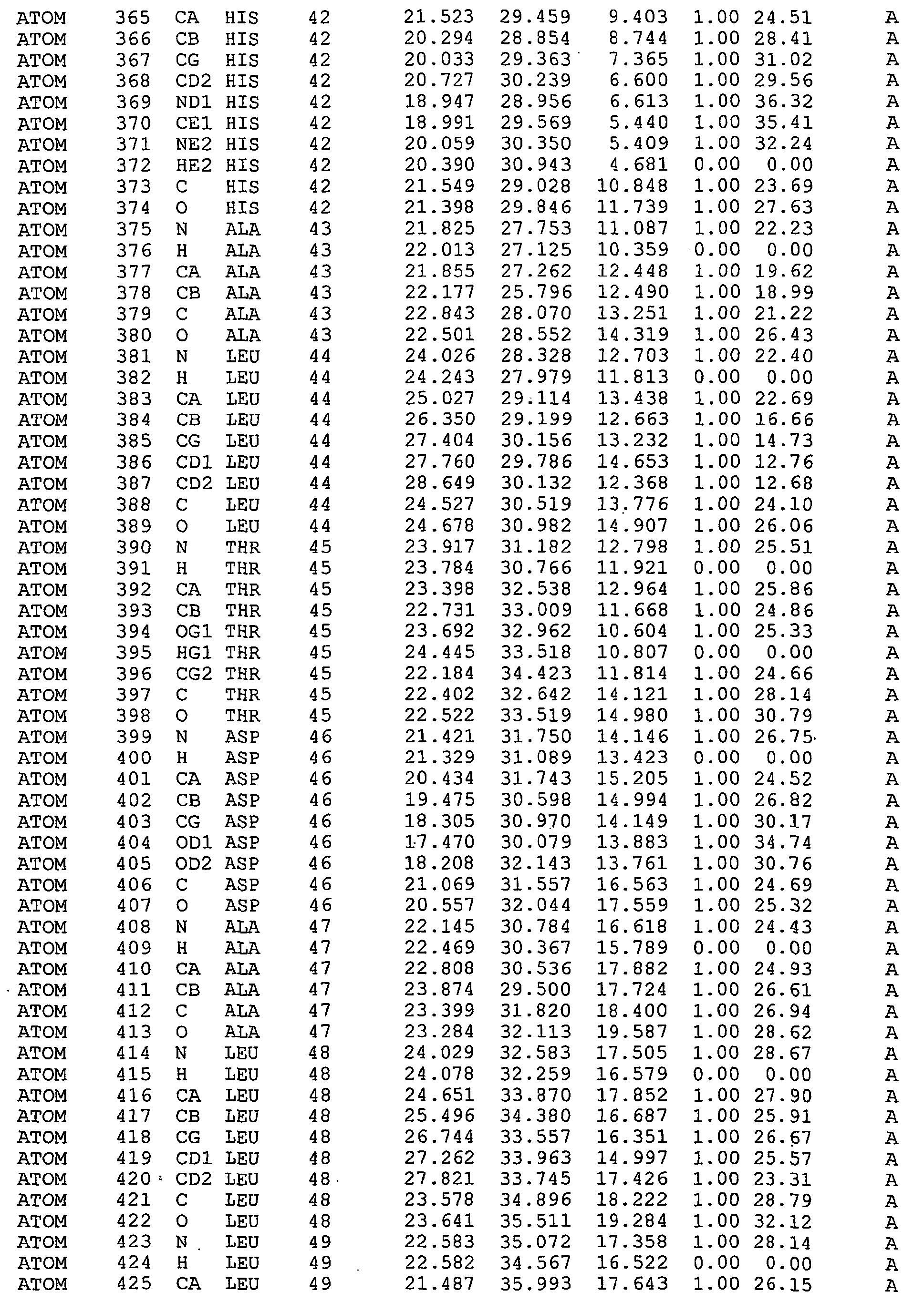

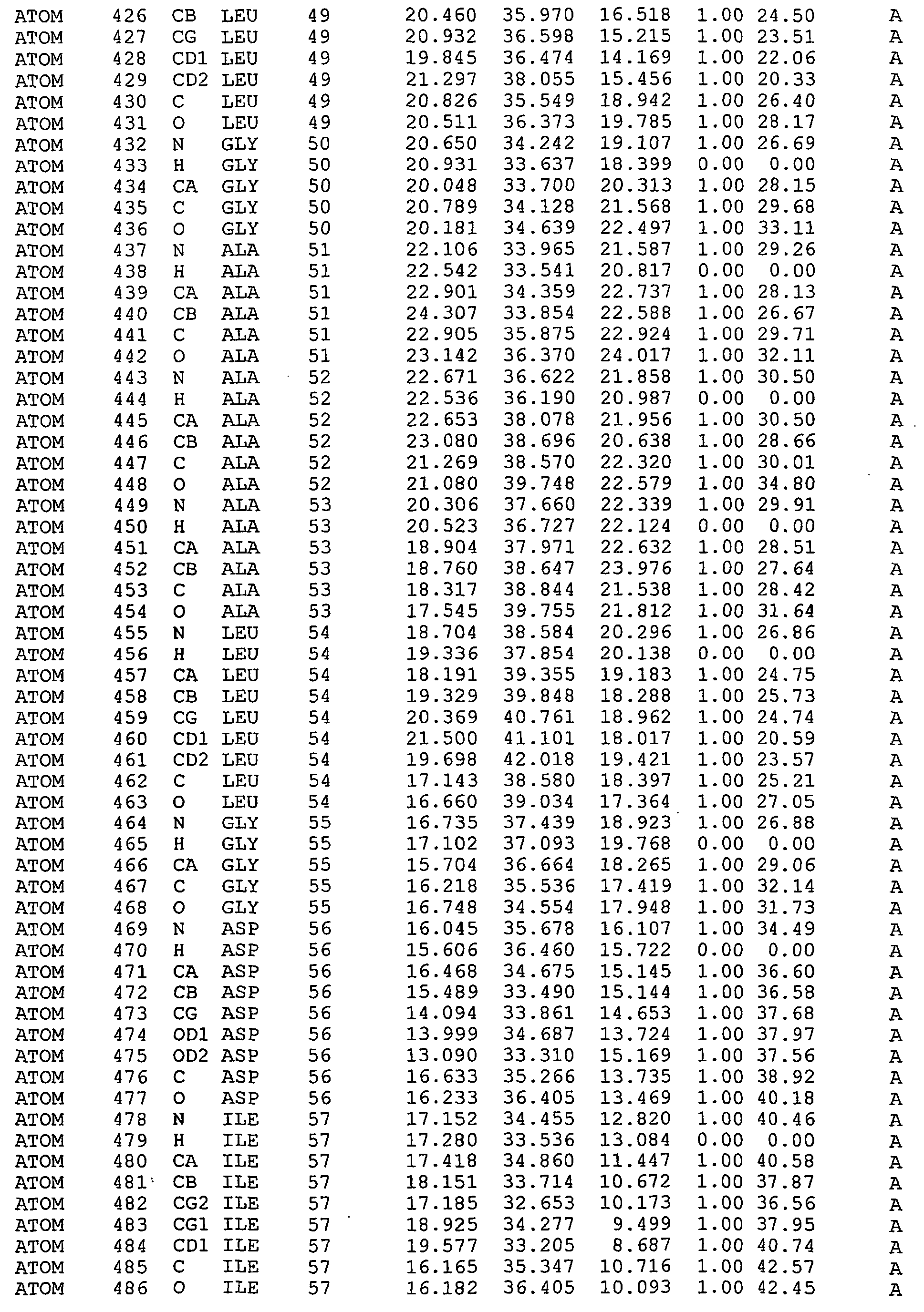

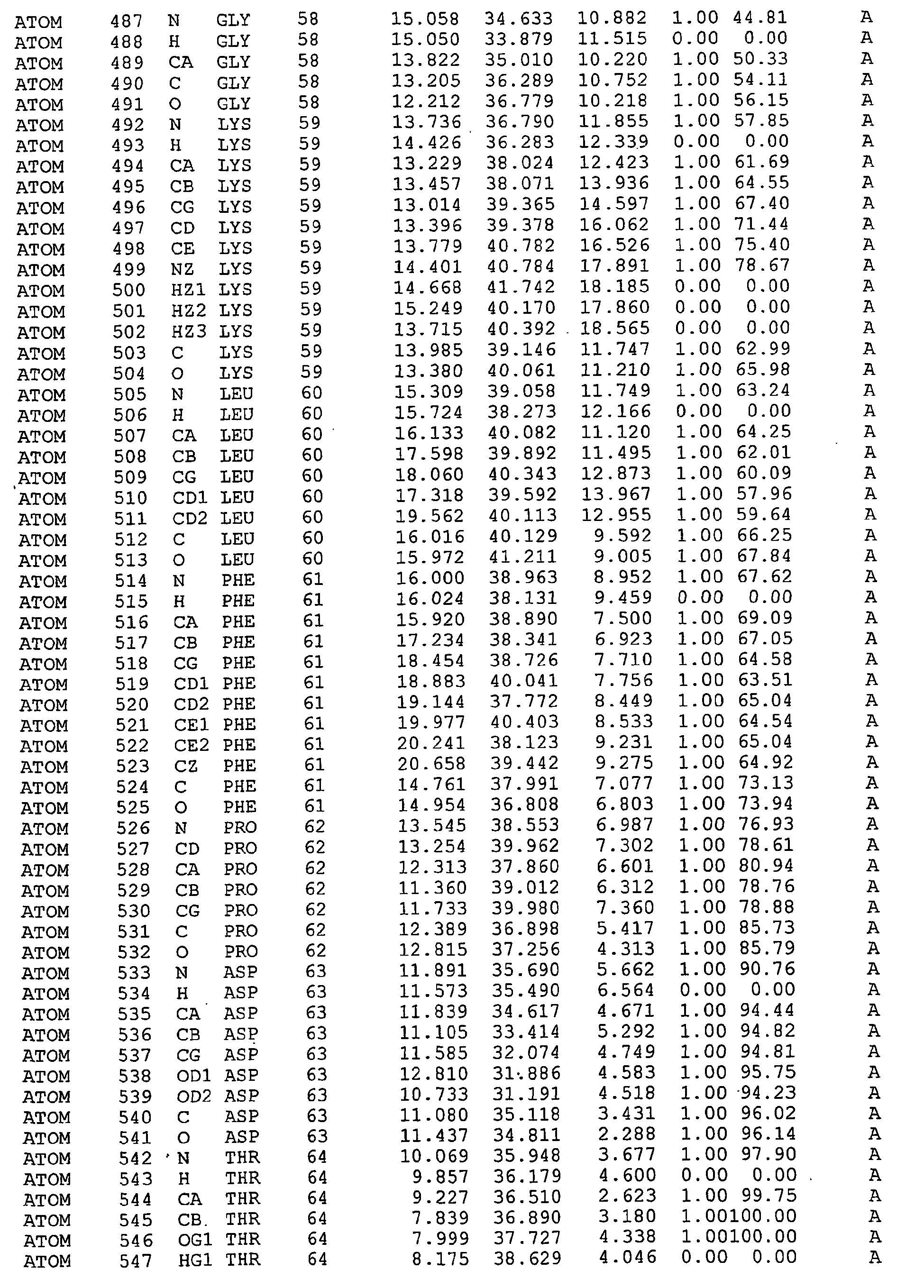

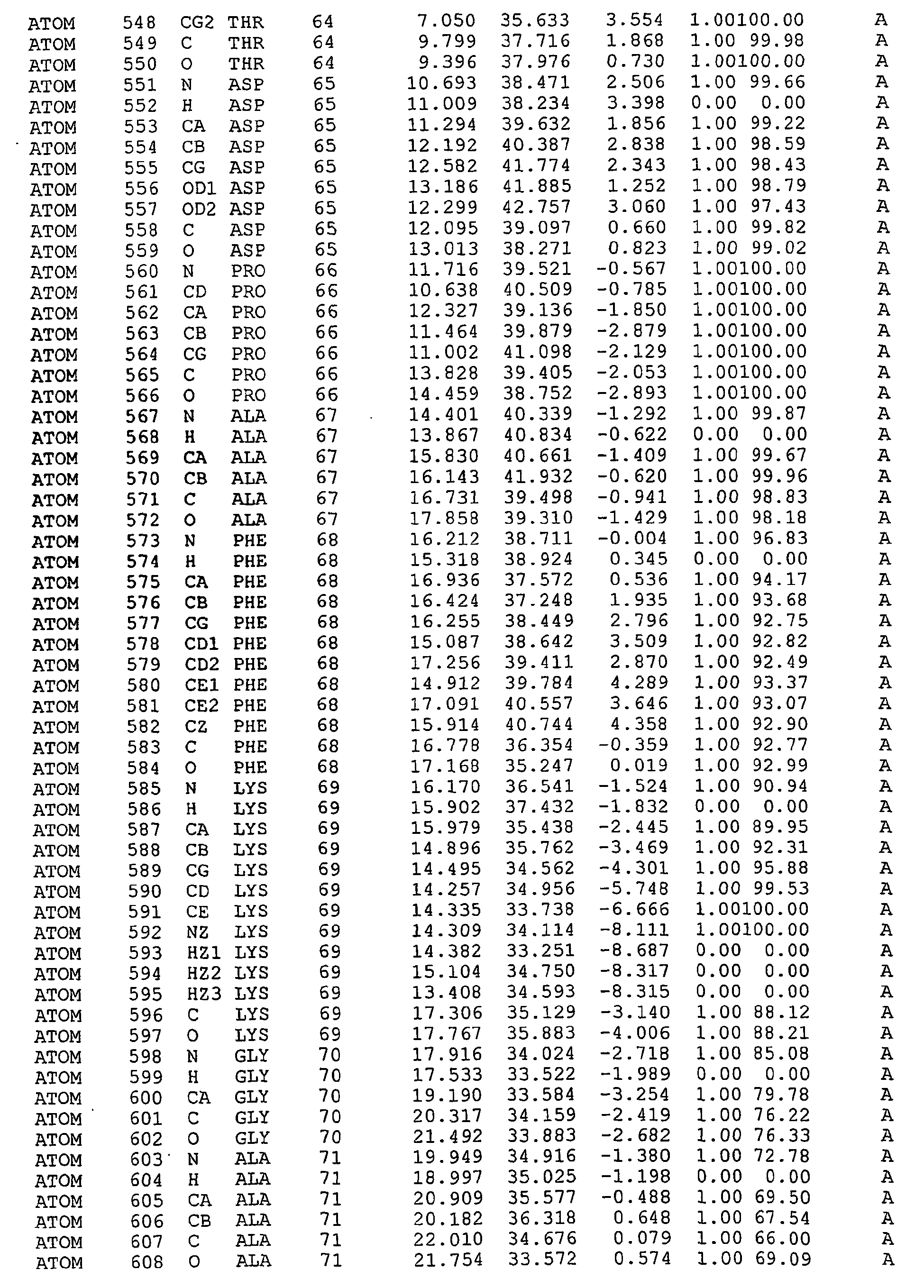

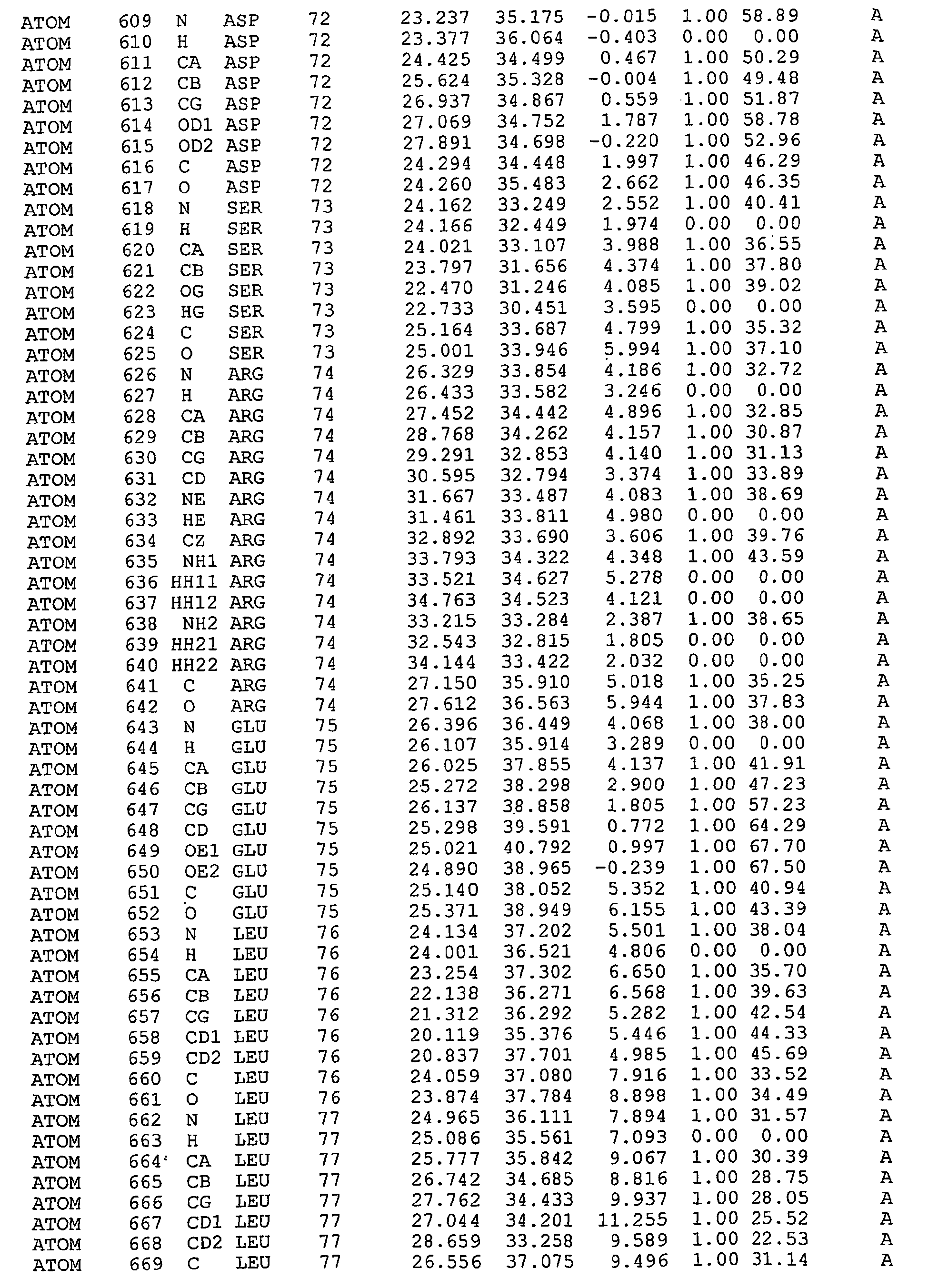

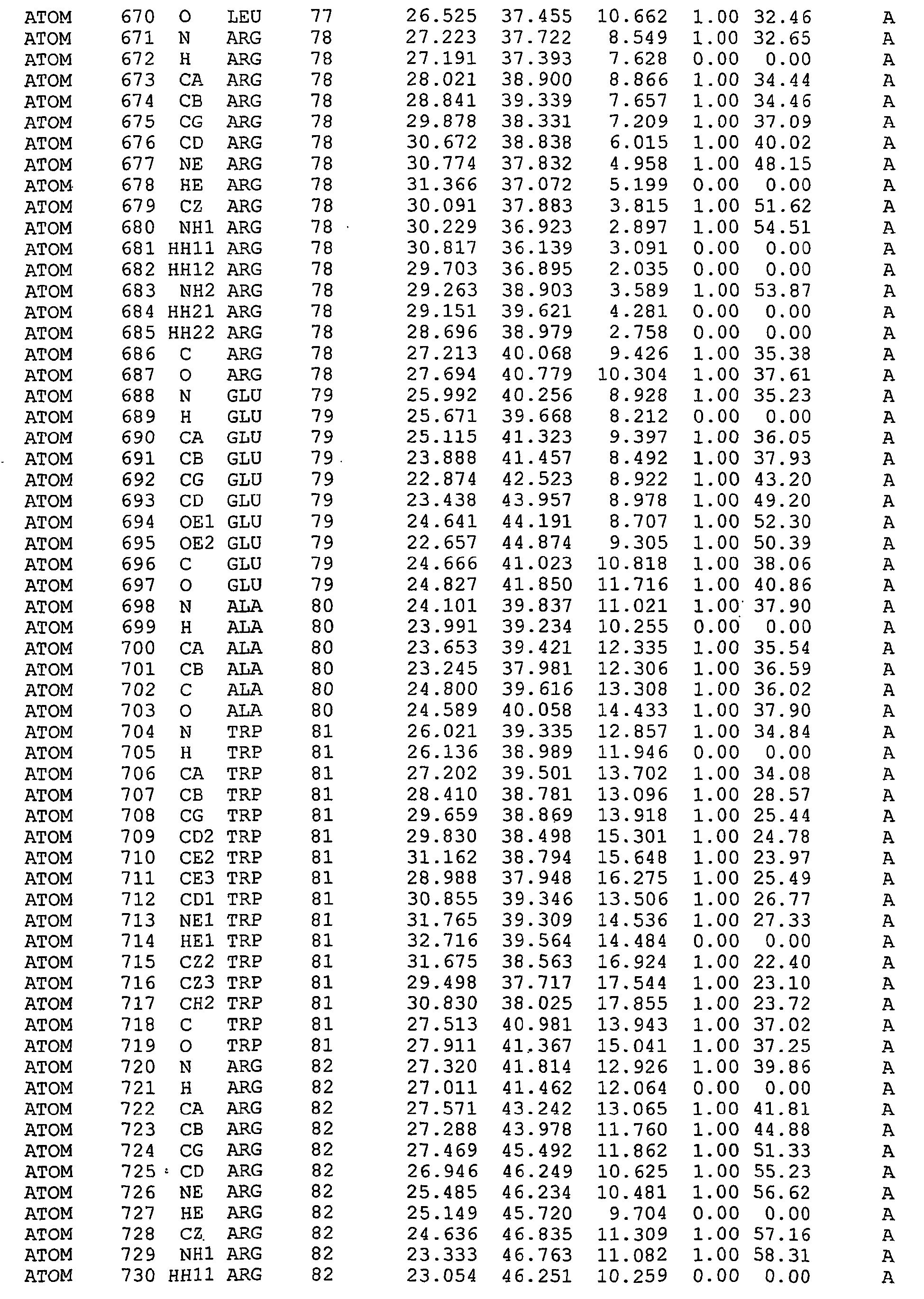

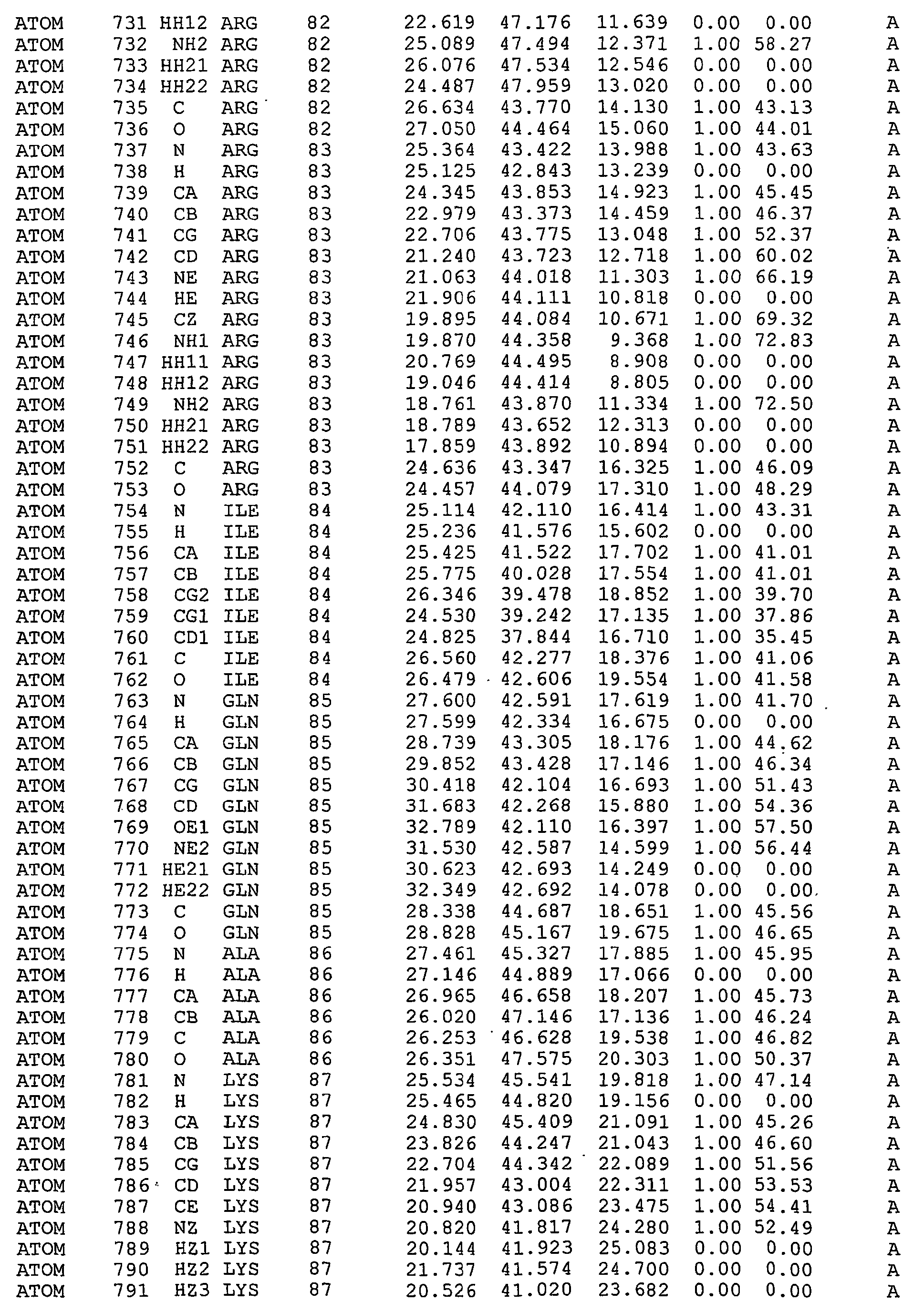

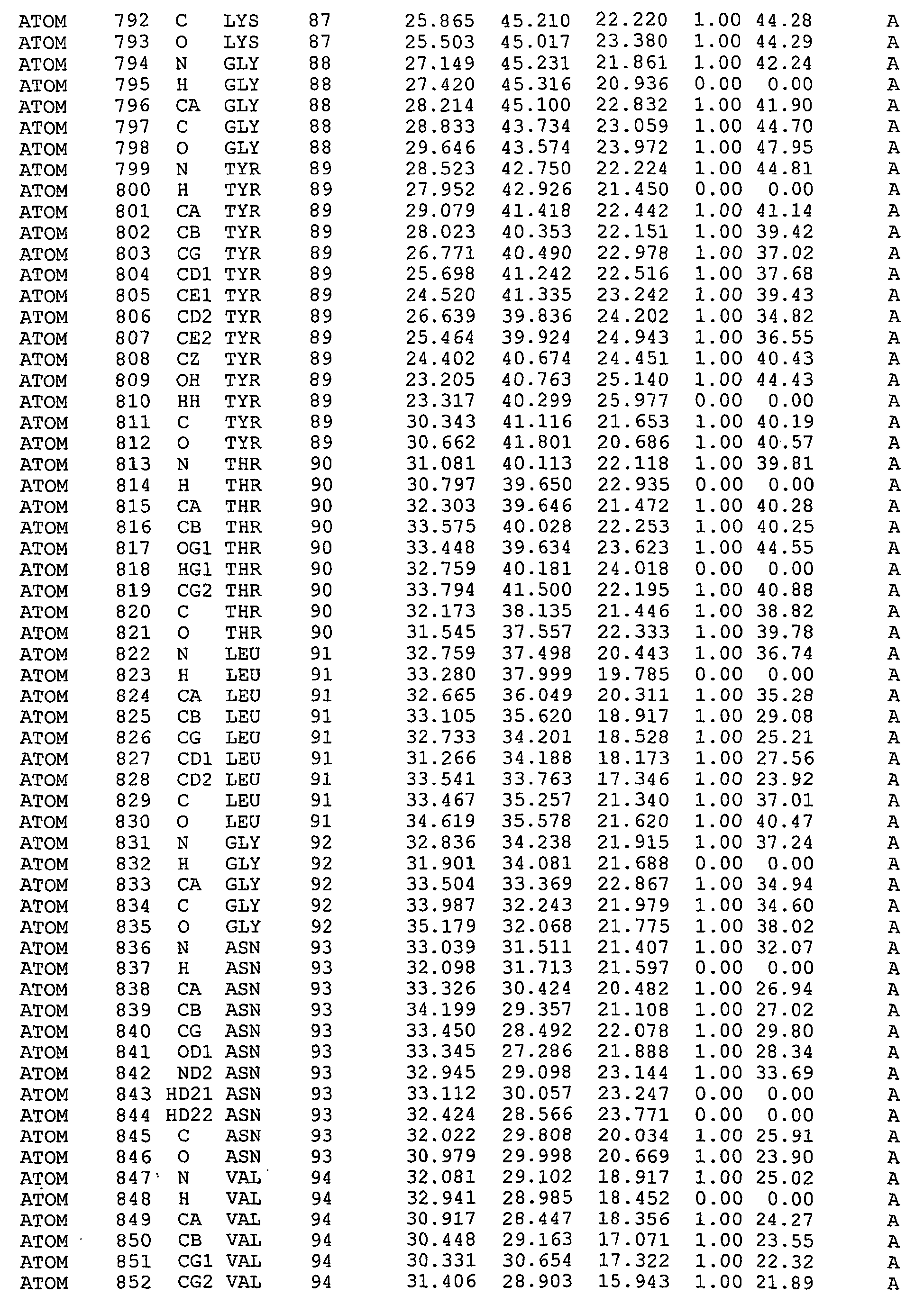

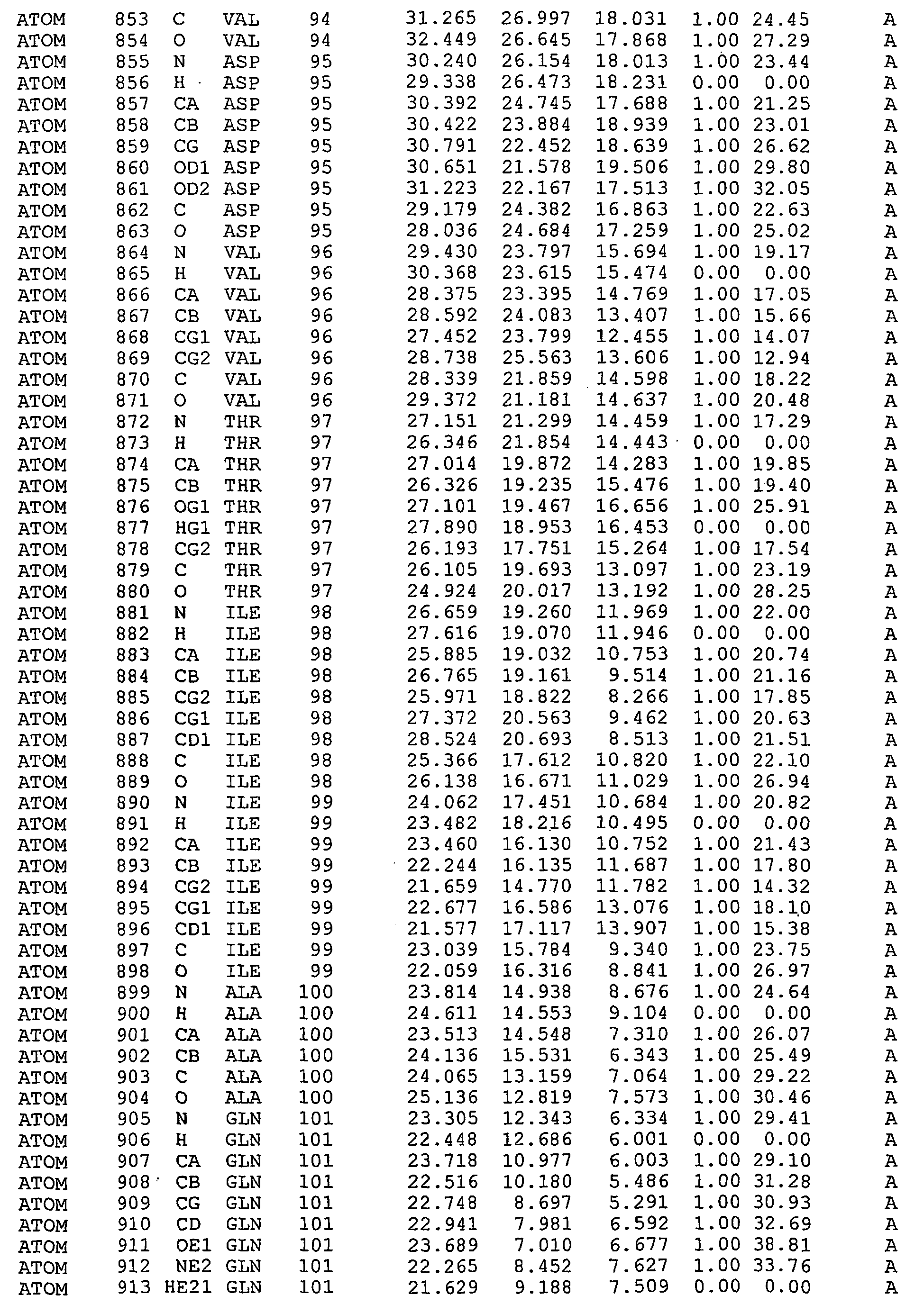

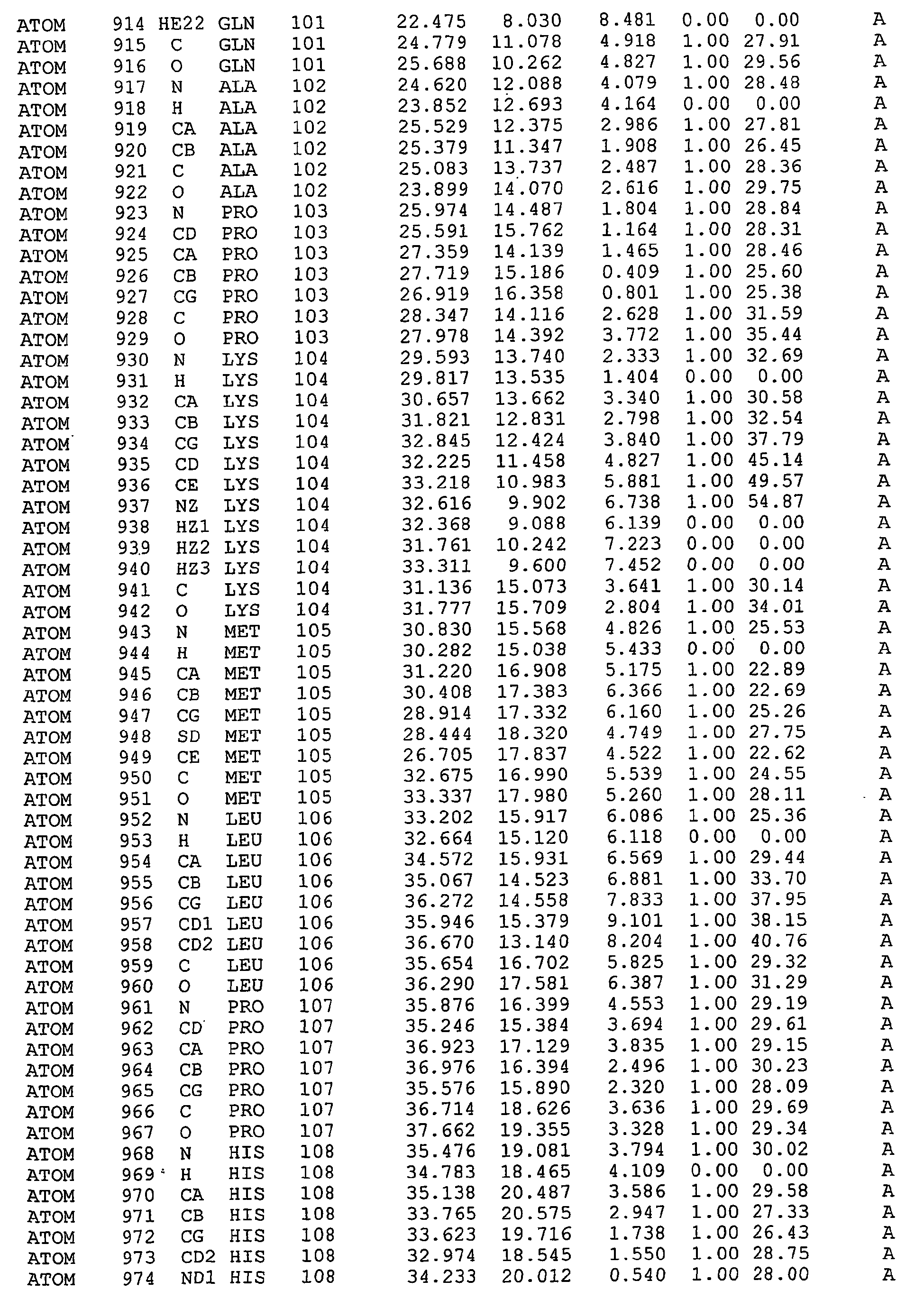

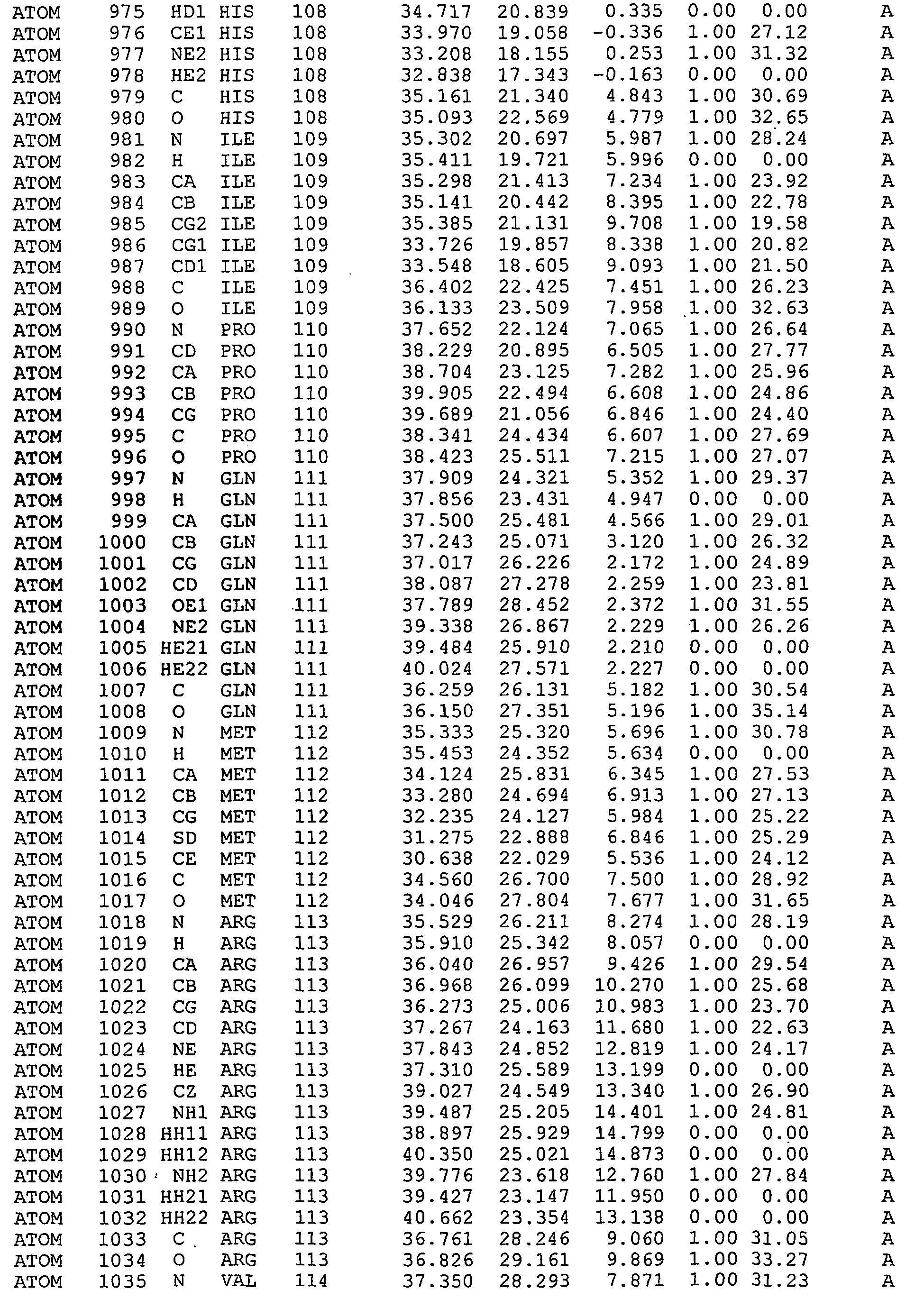

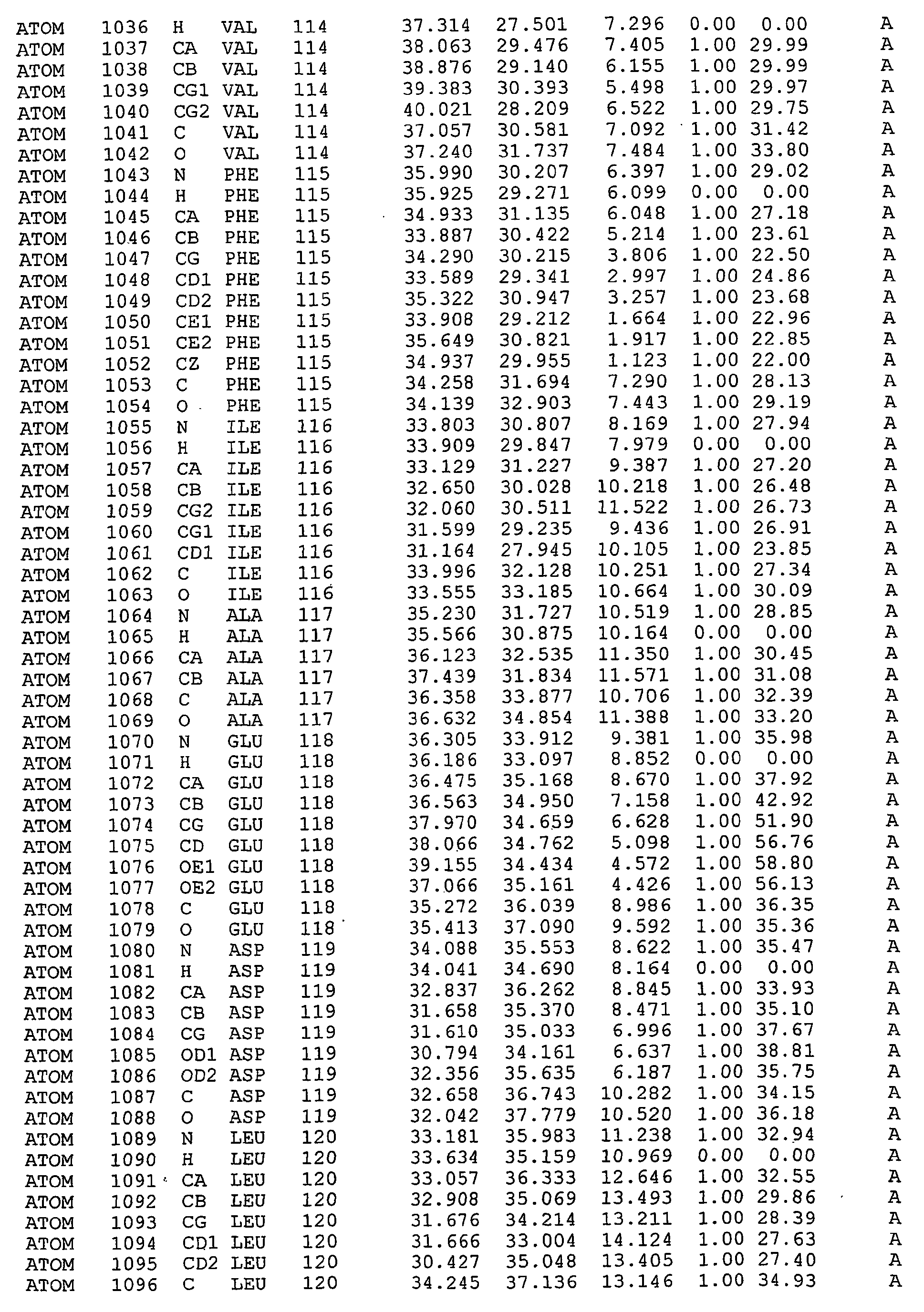

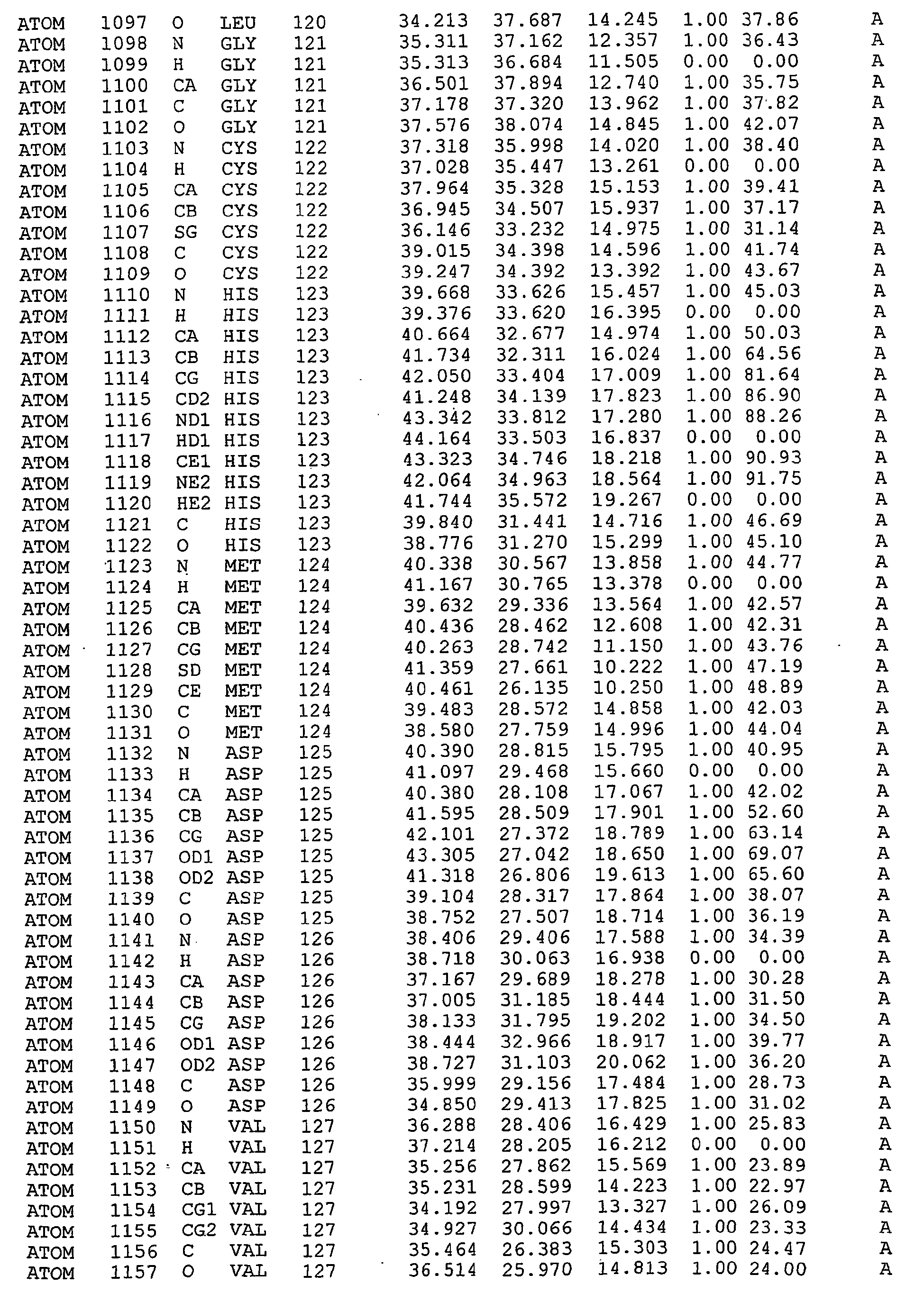

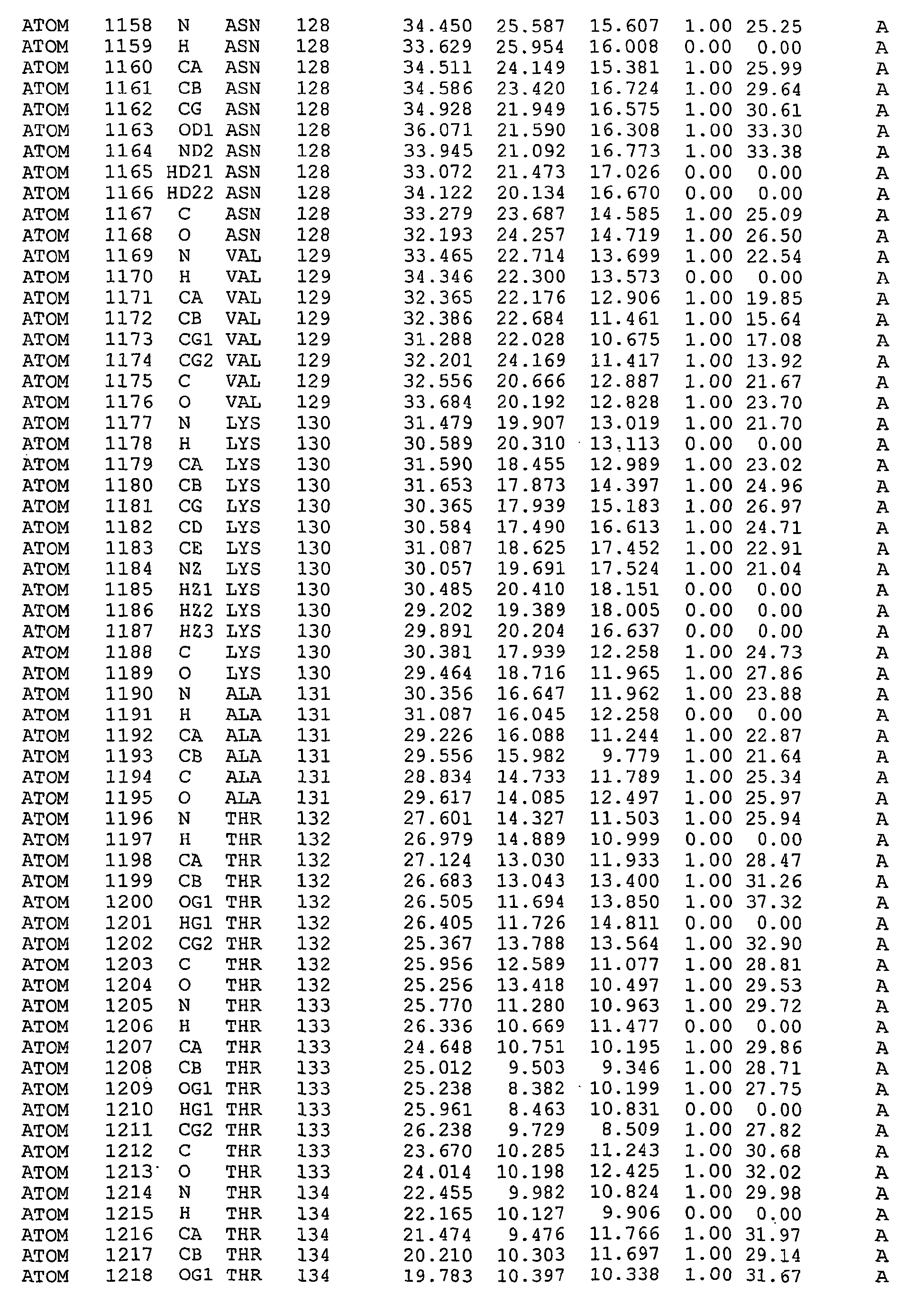

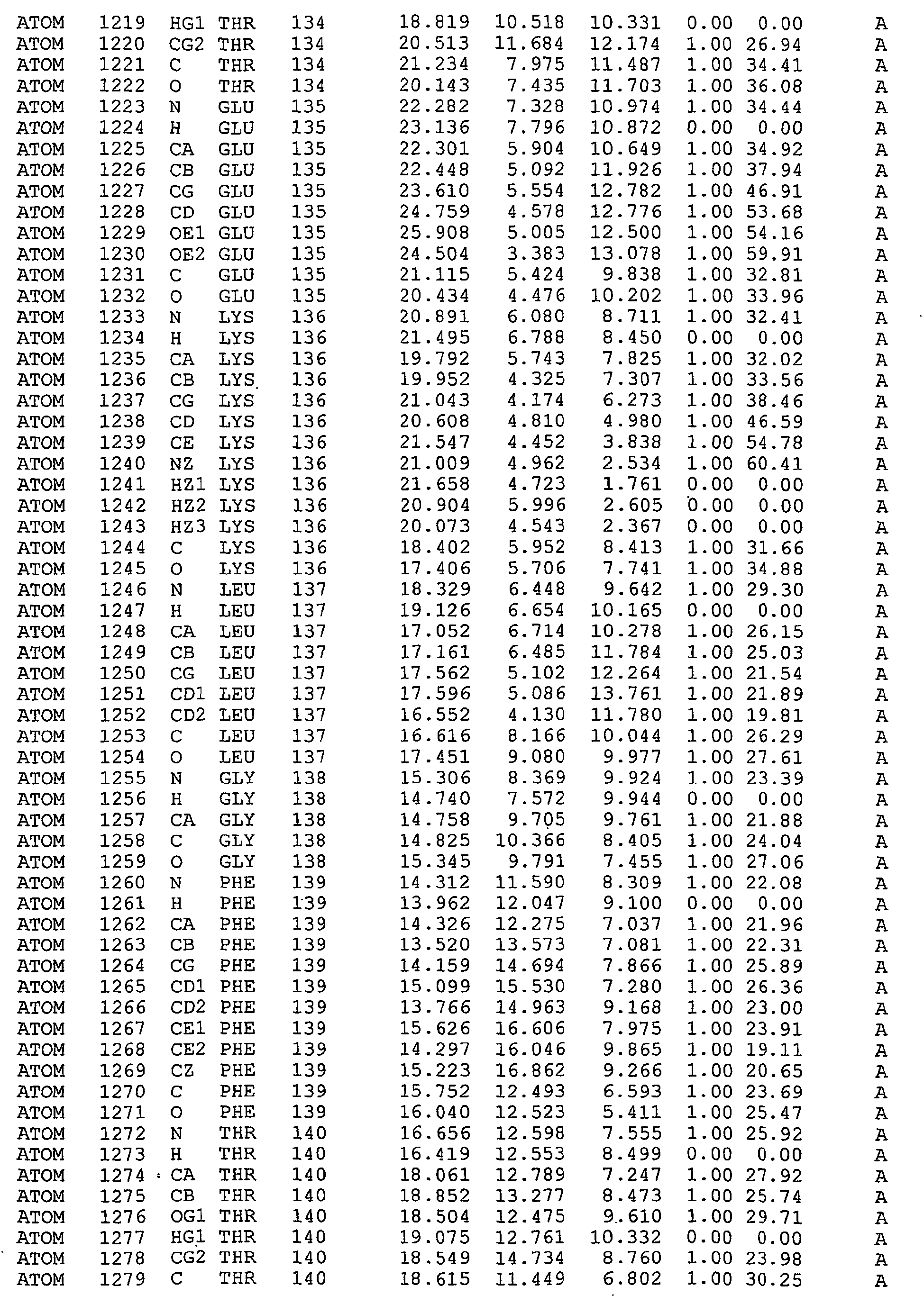

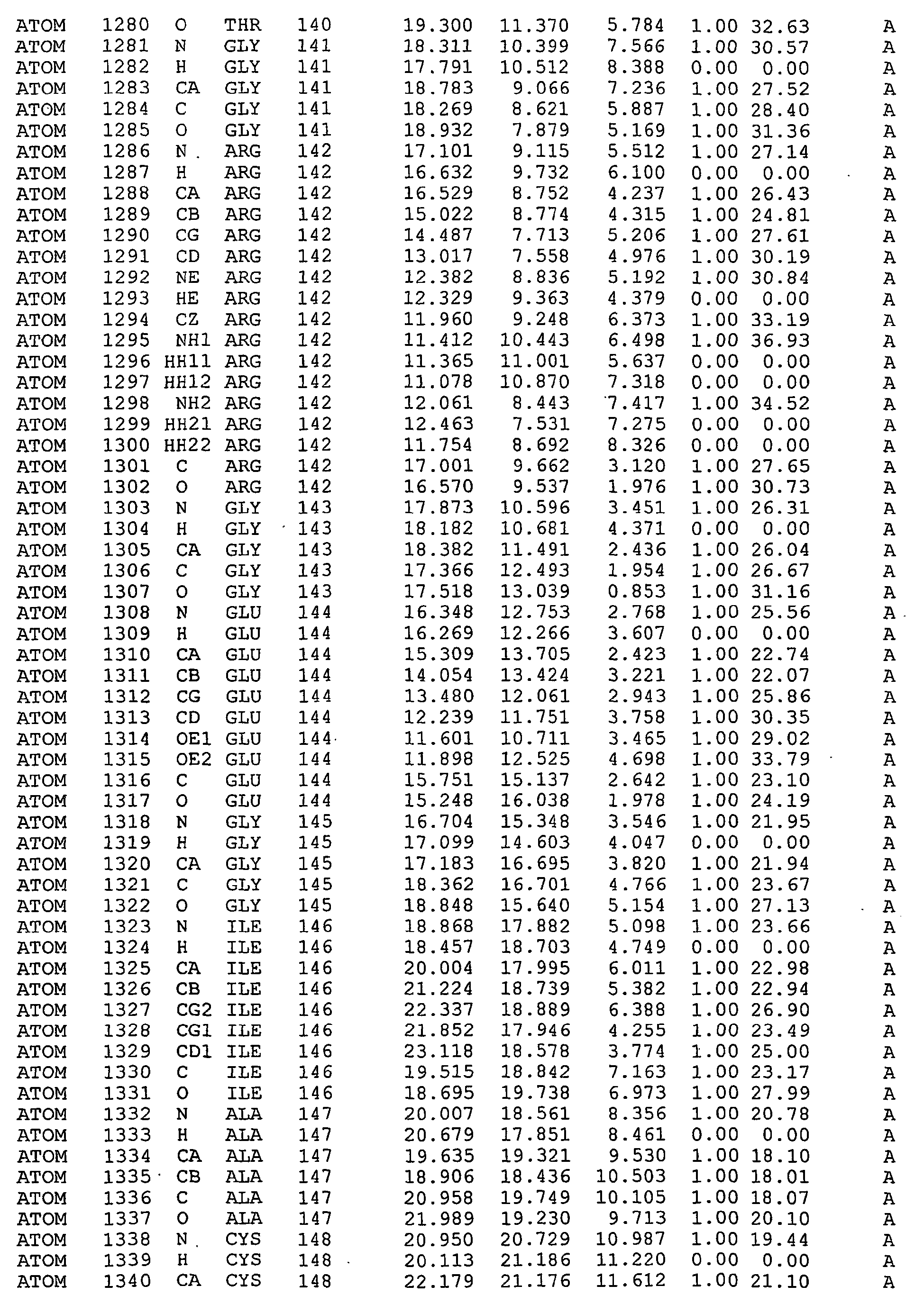

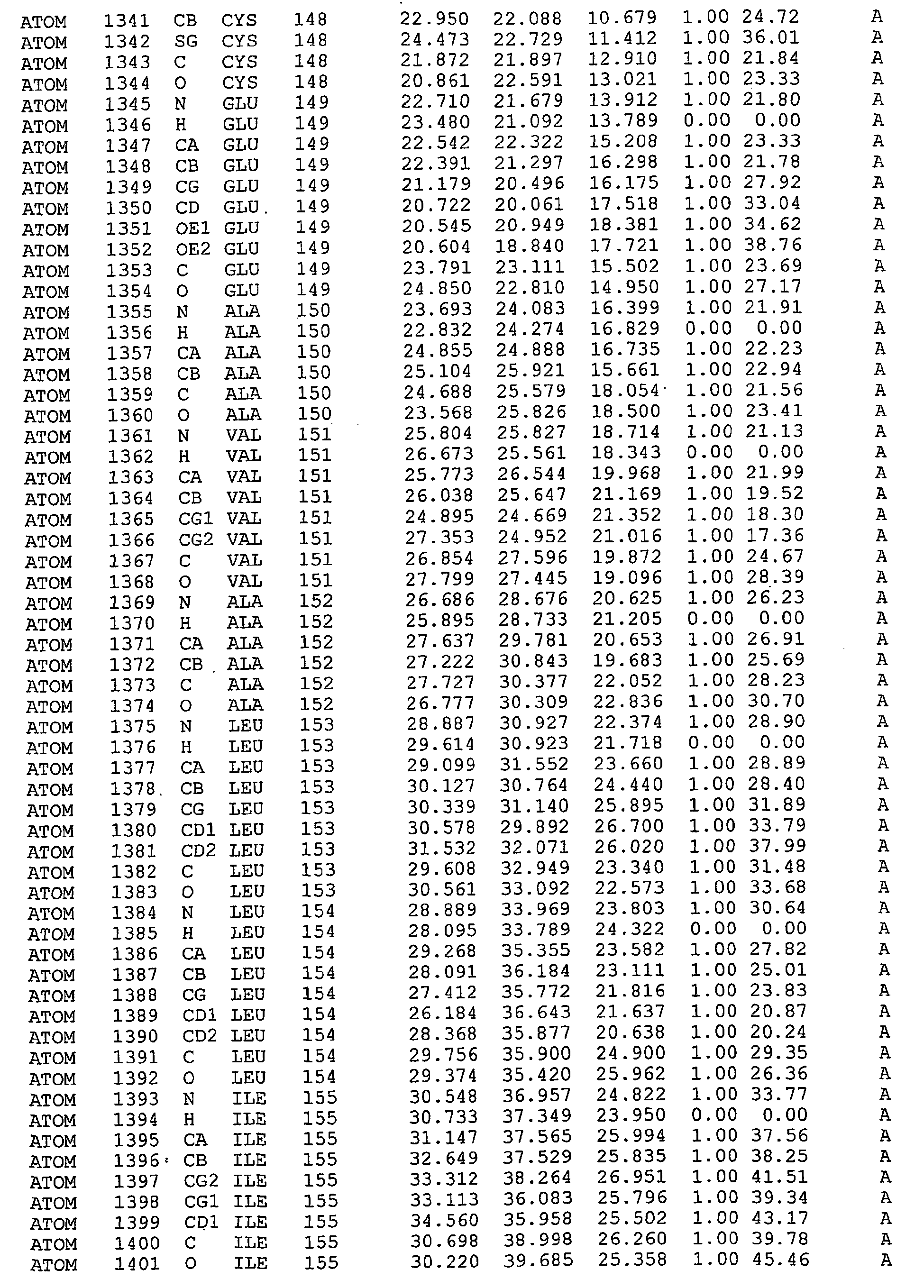

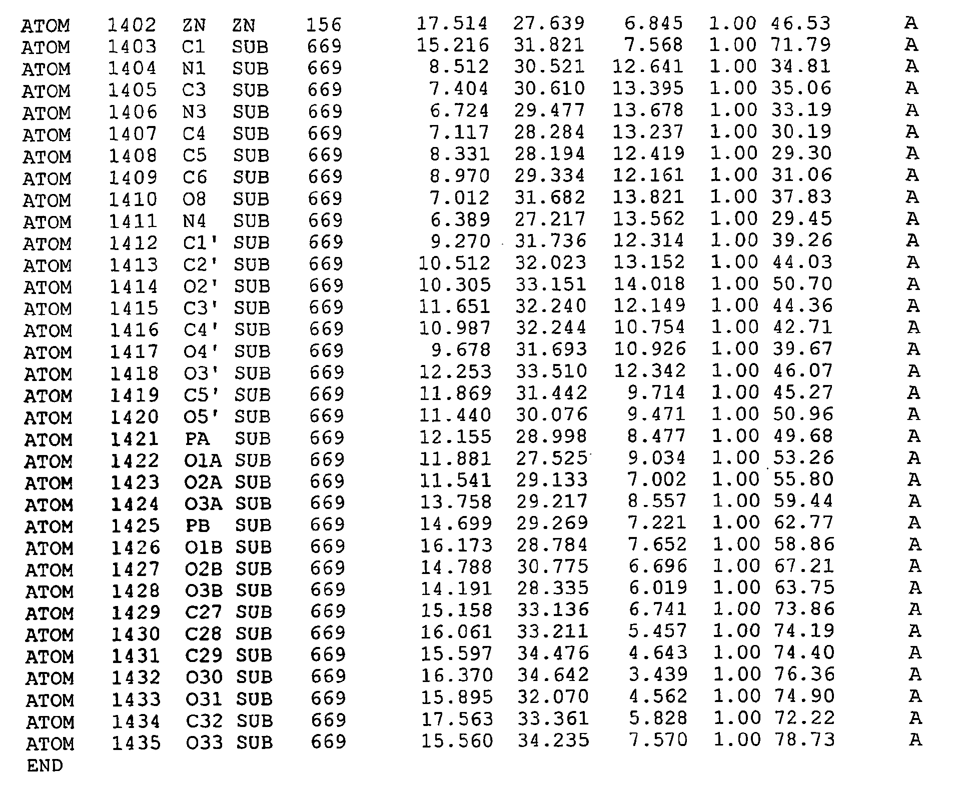

### Annex 6: coordinates of structure subs

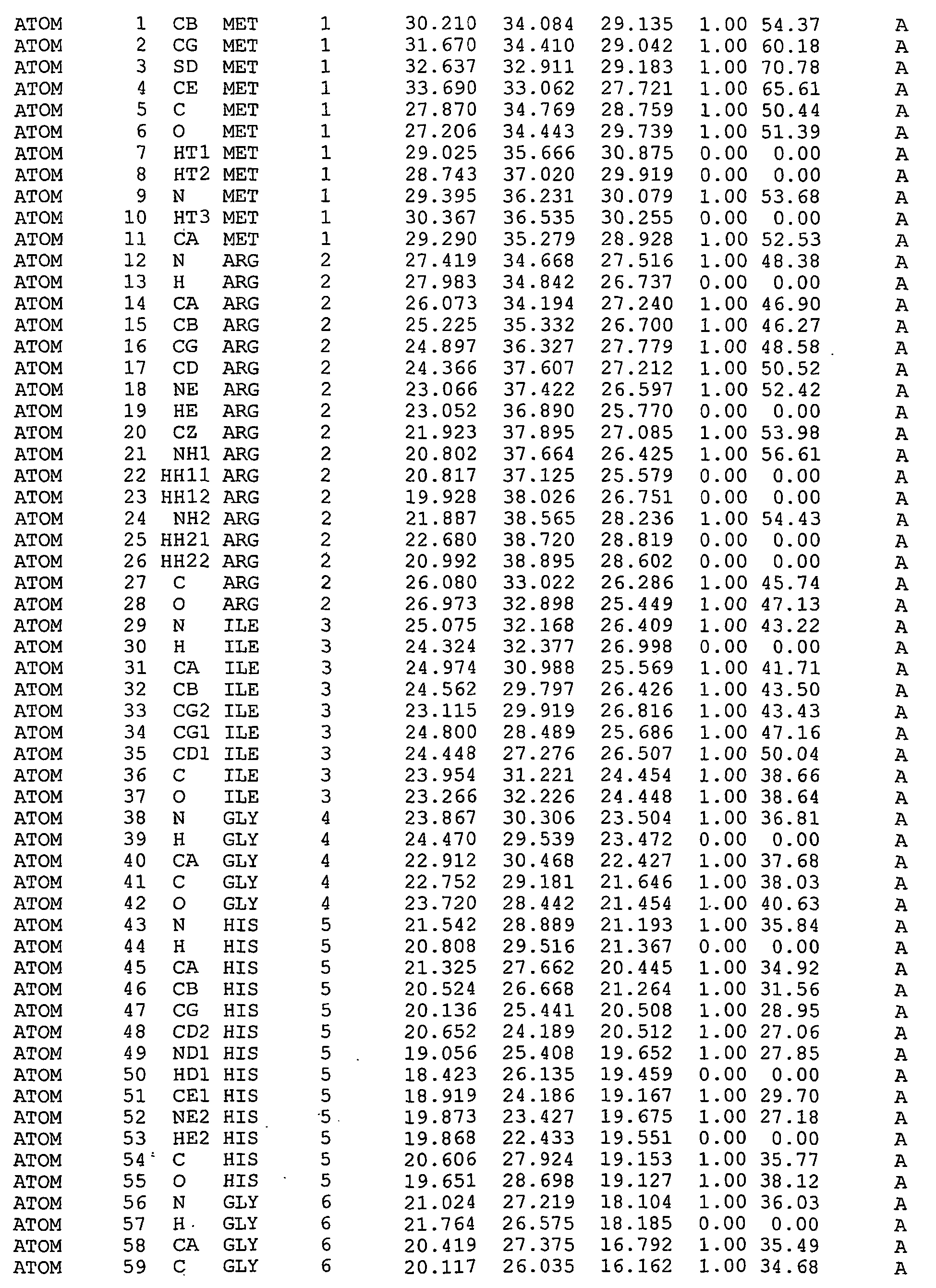

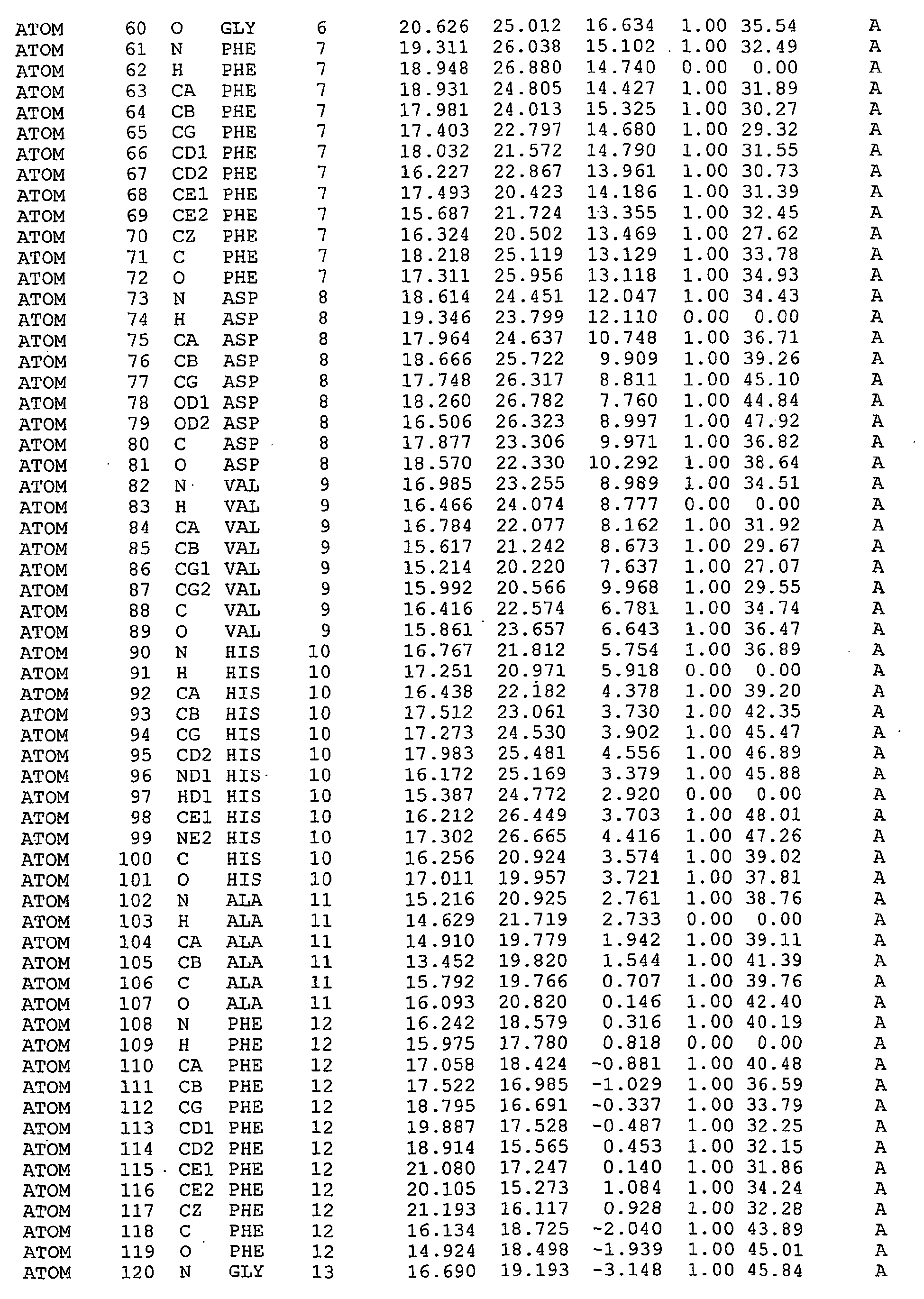

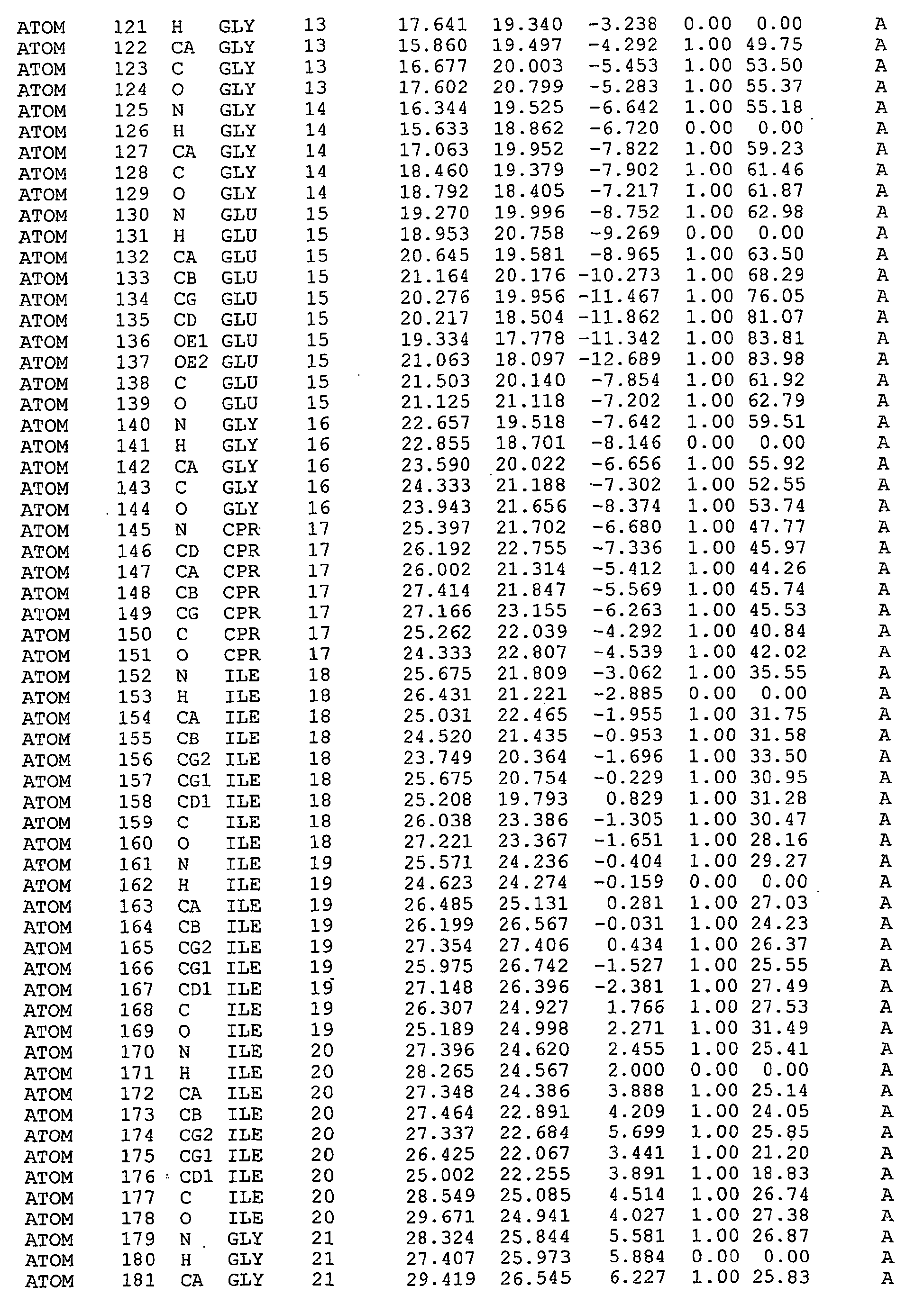

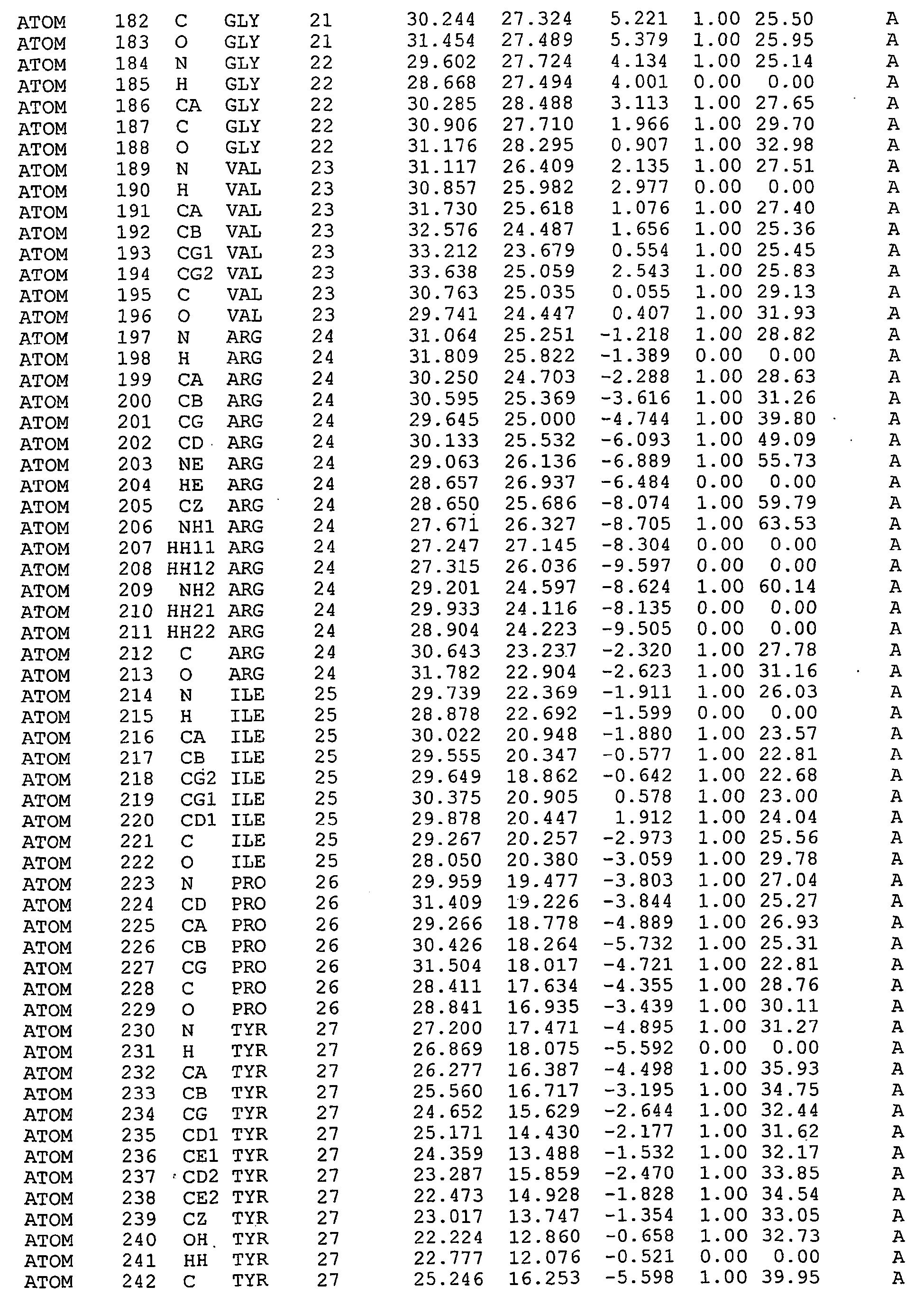

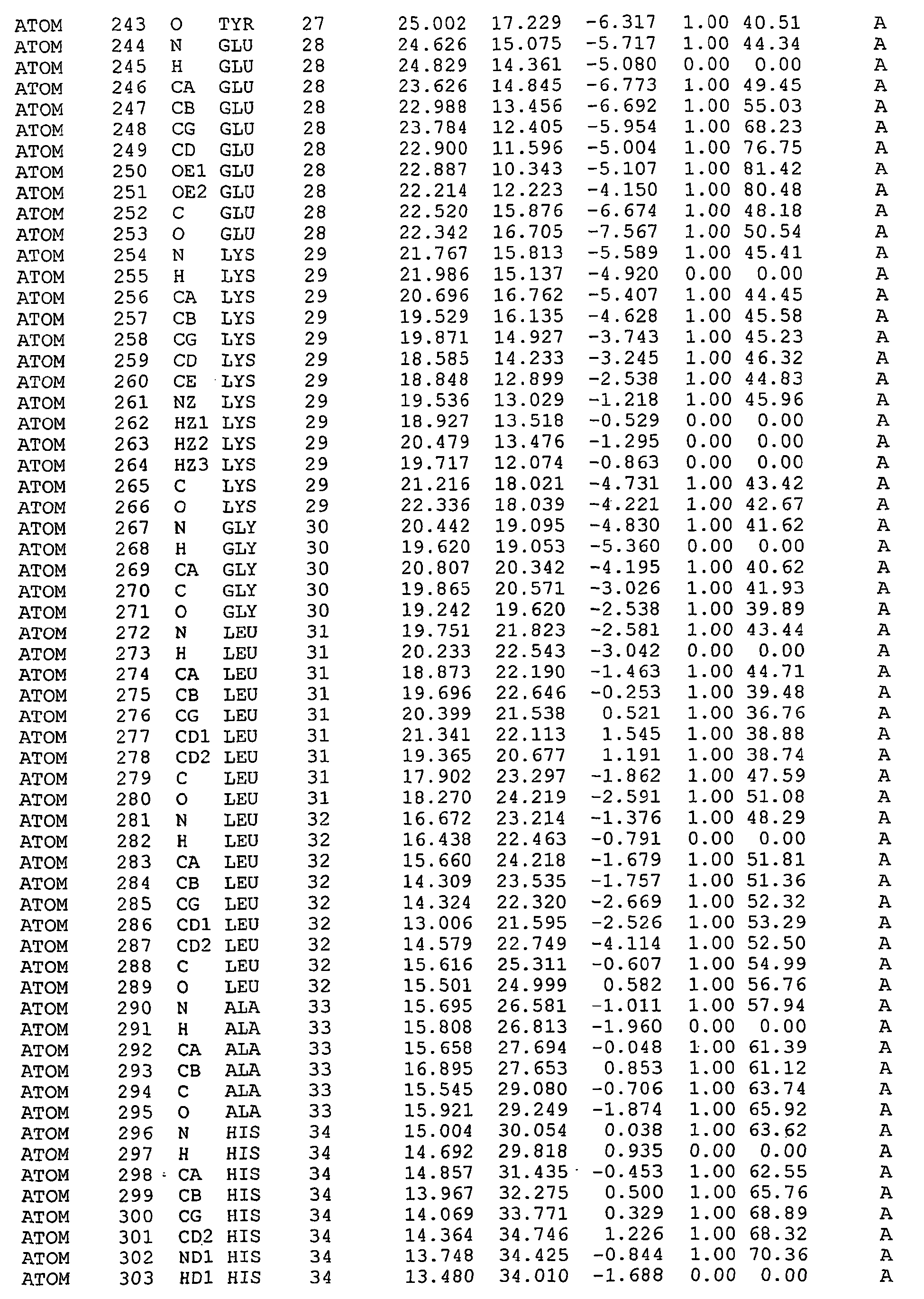

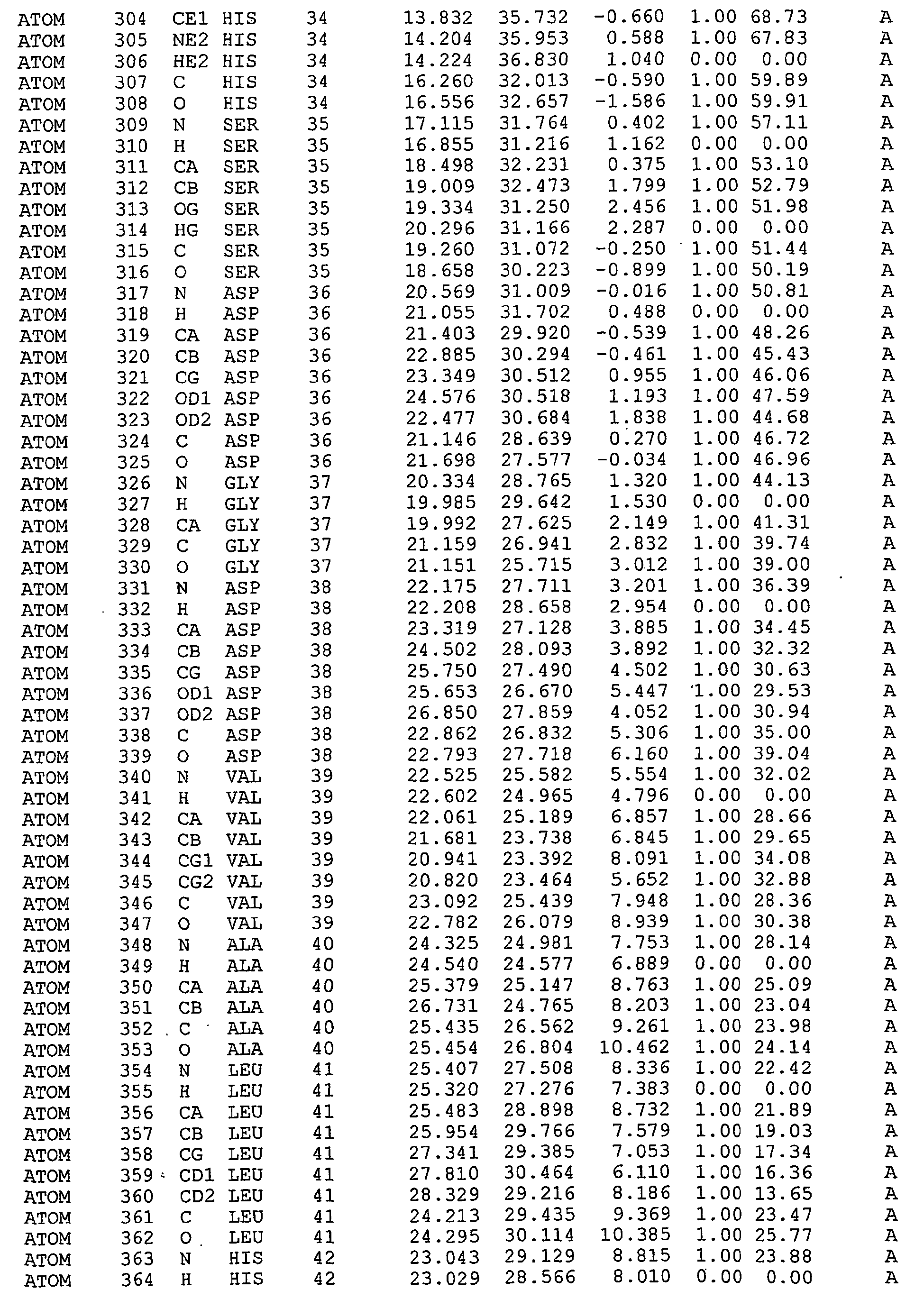

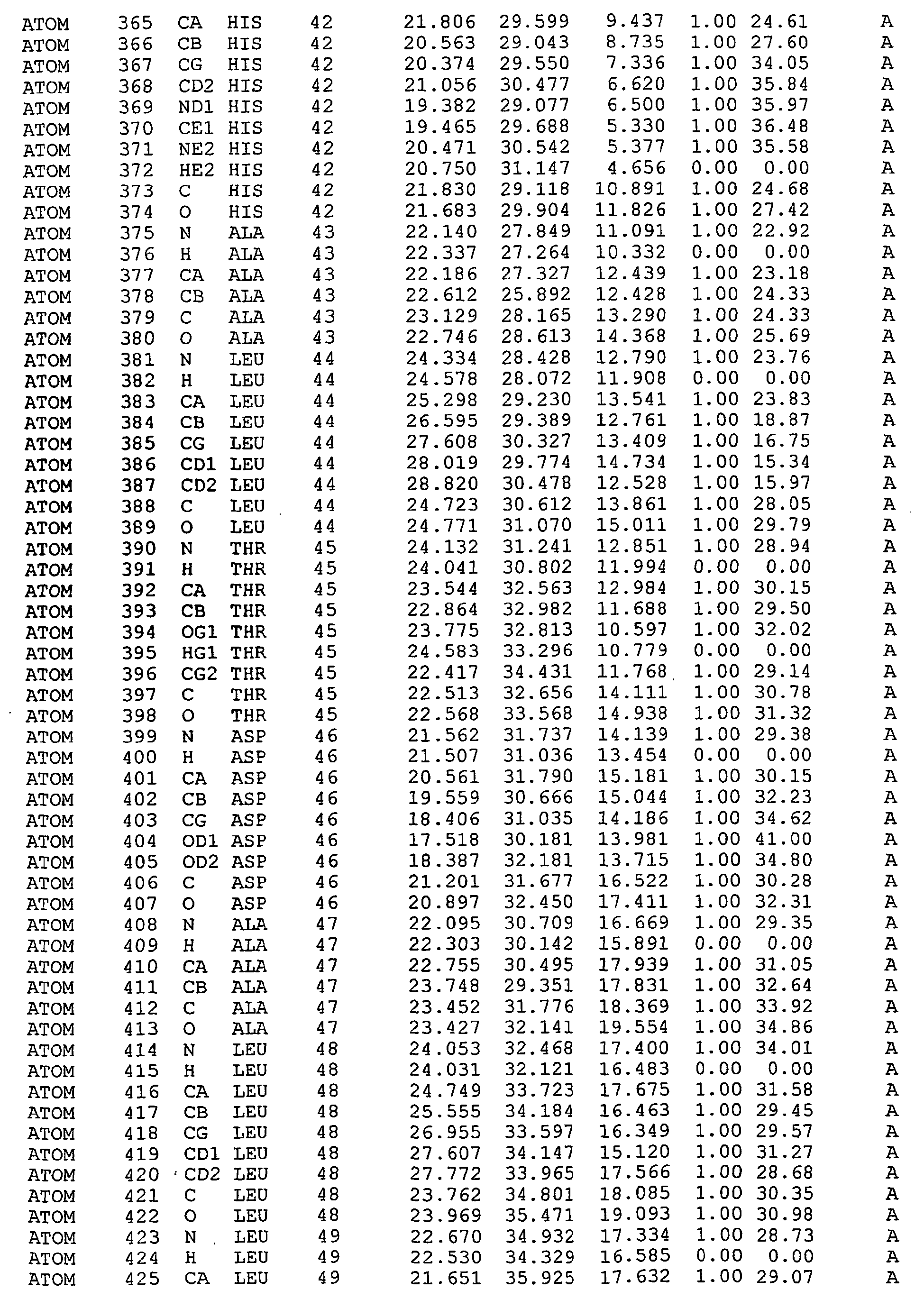

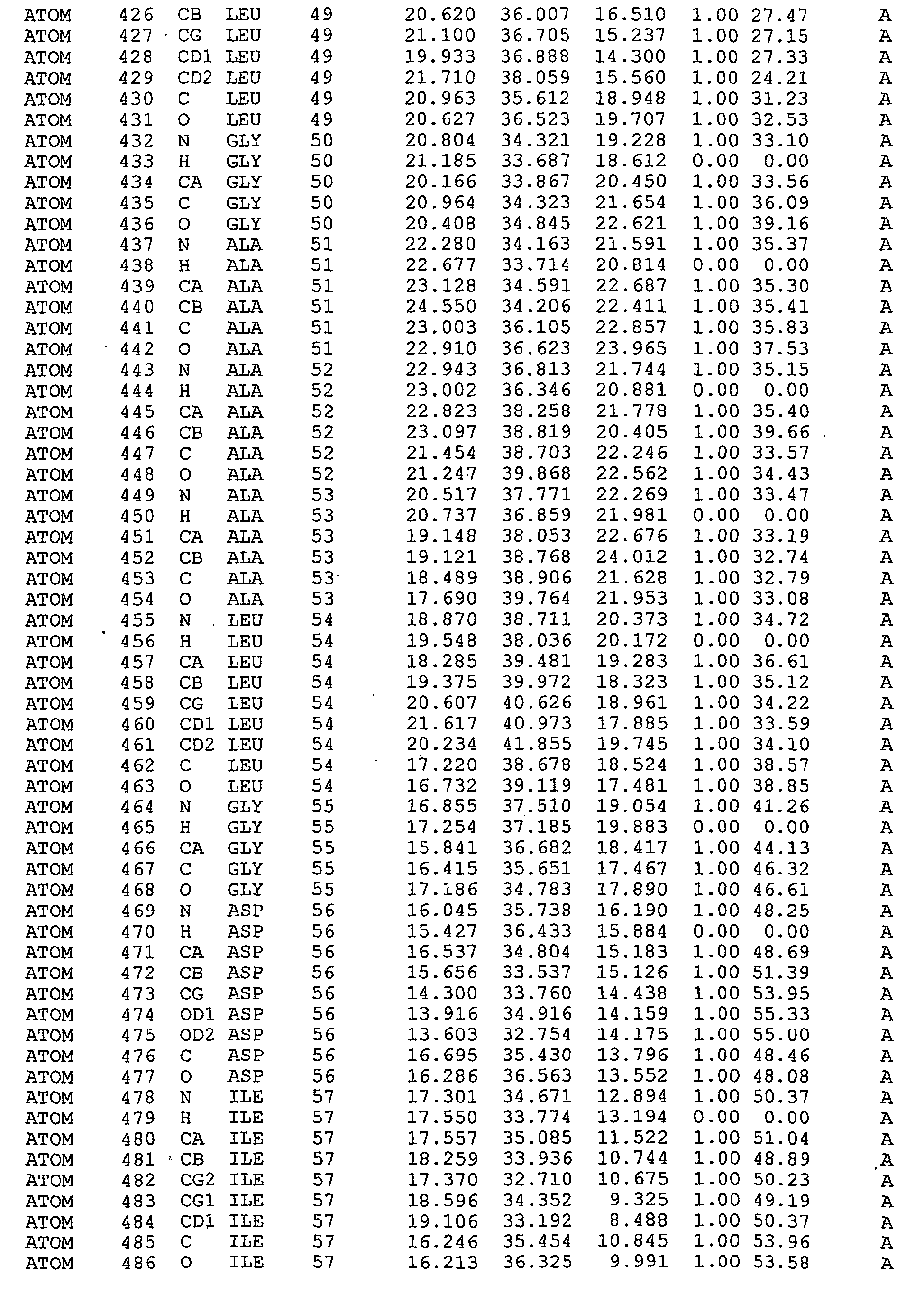

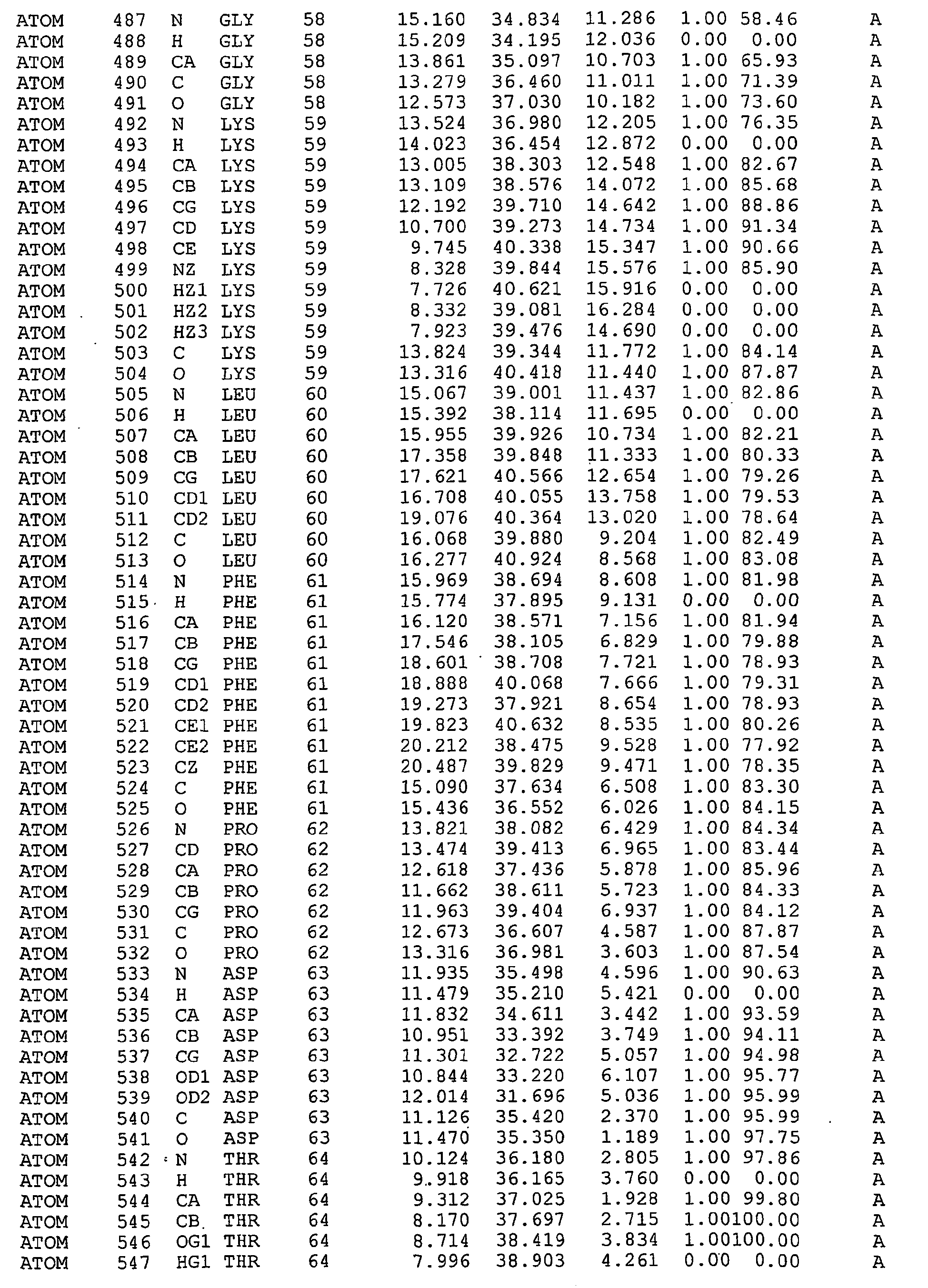

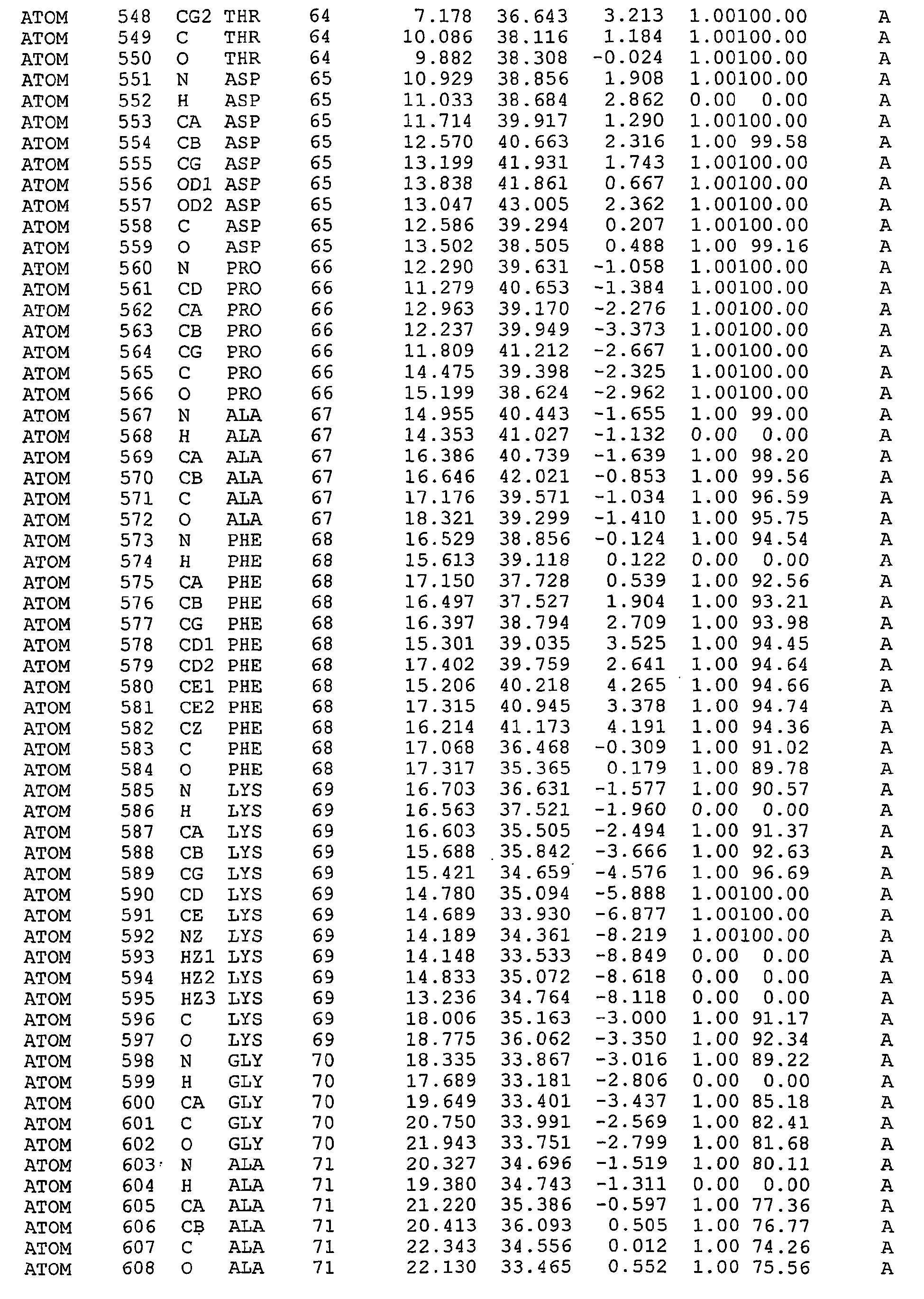

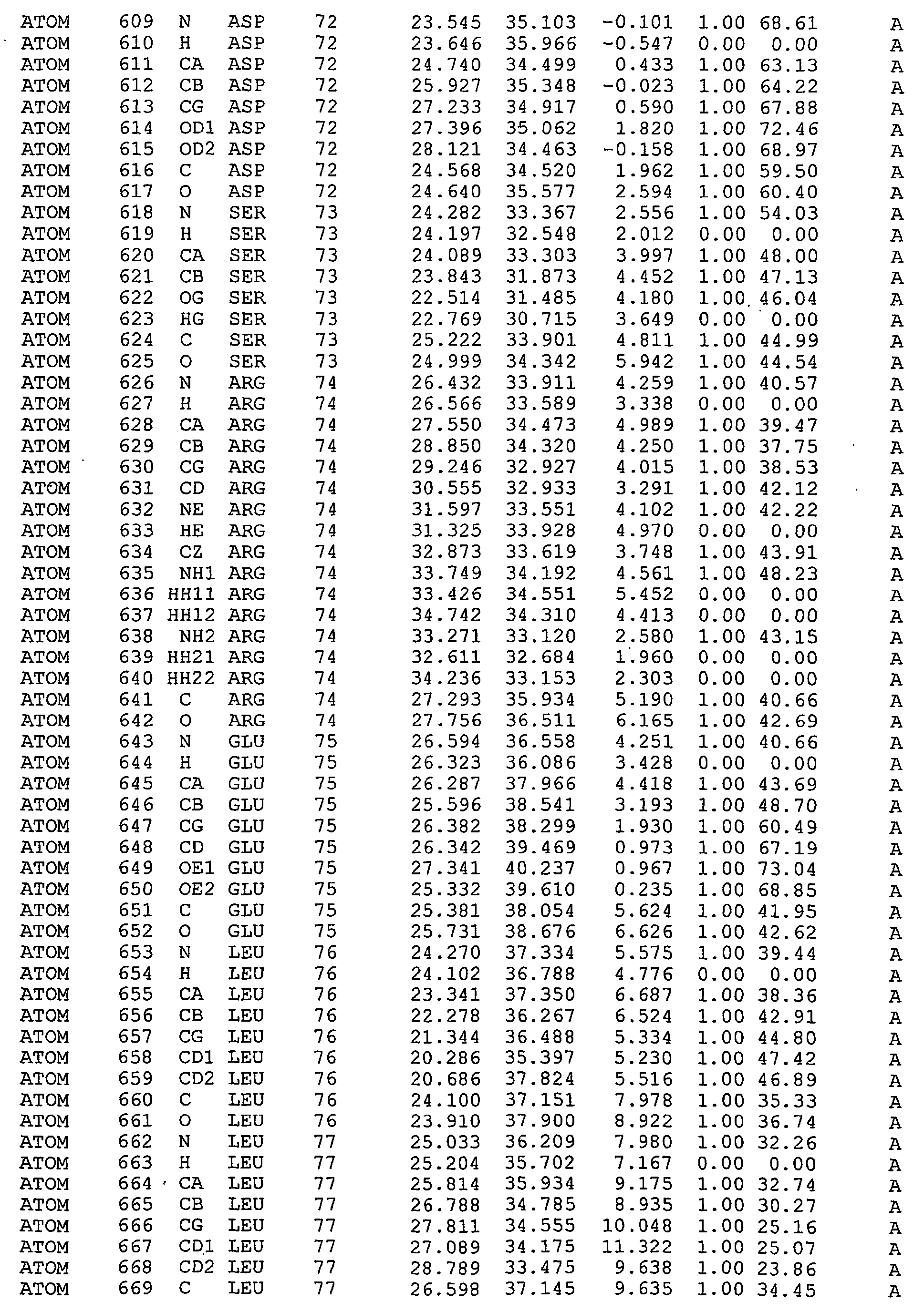

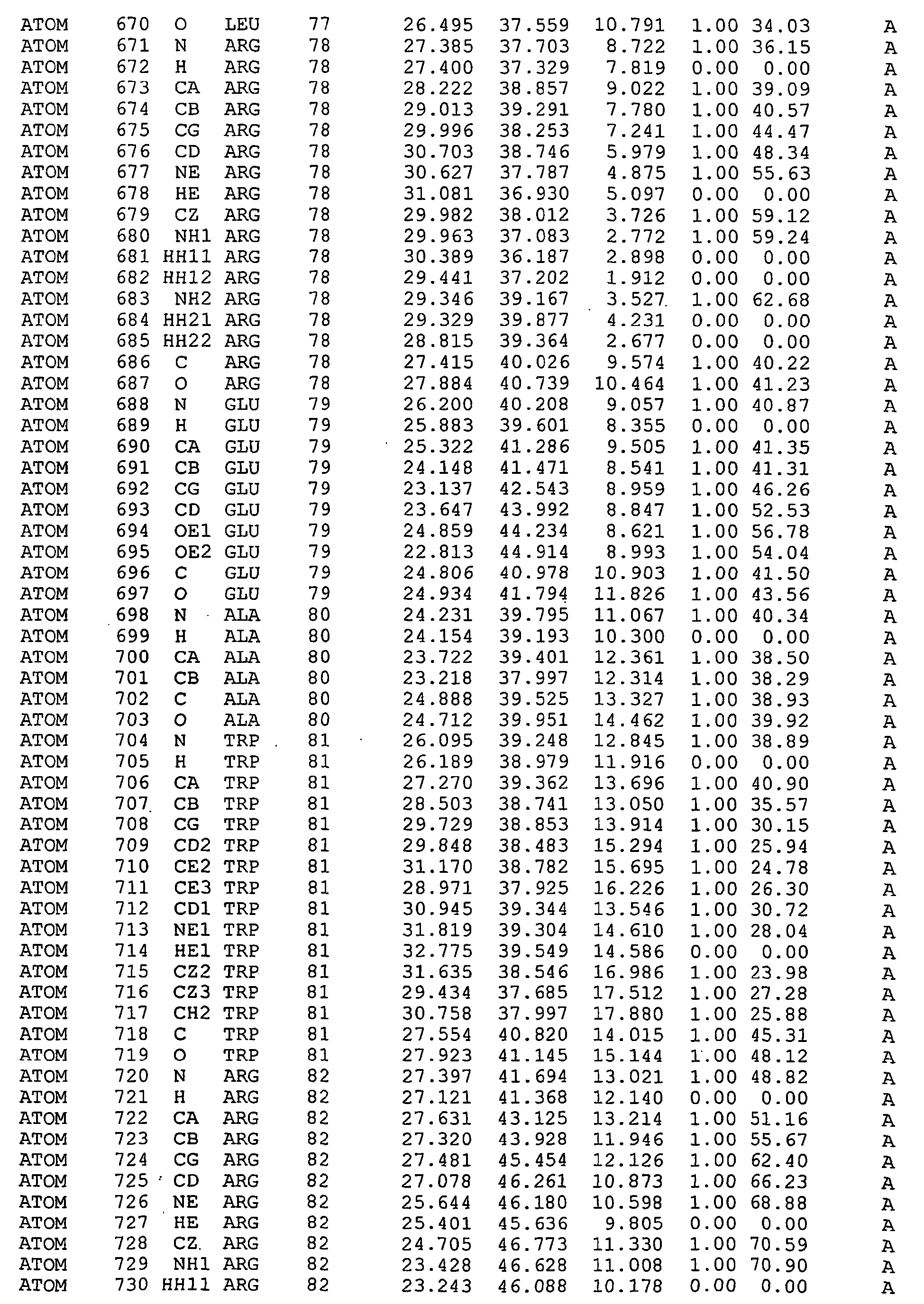

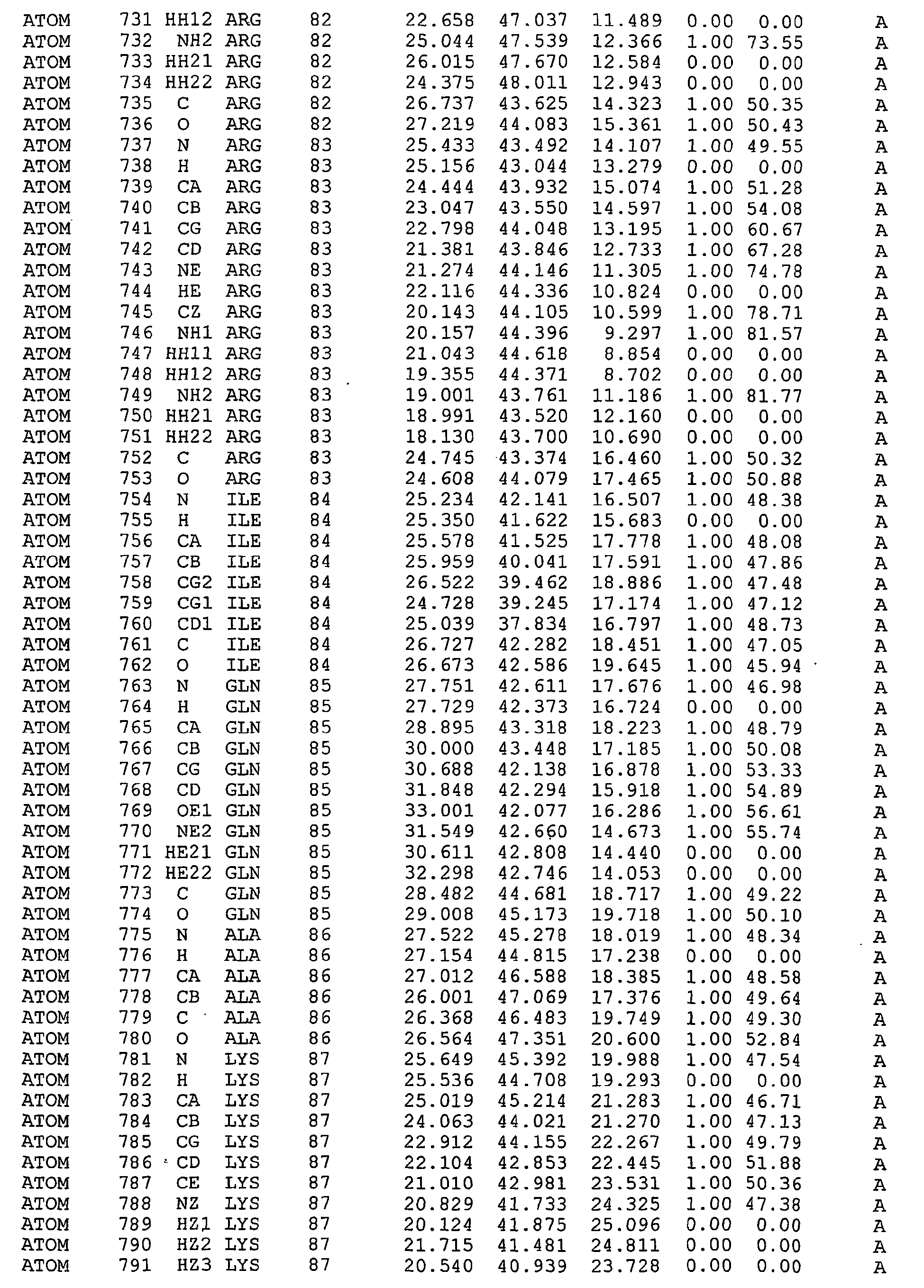

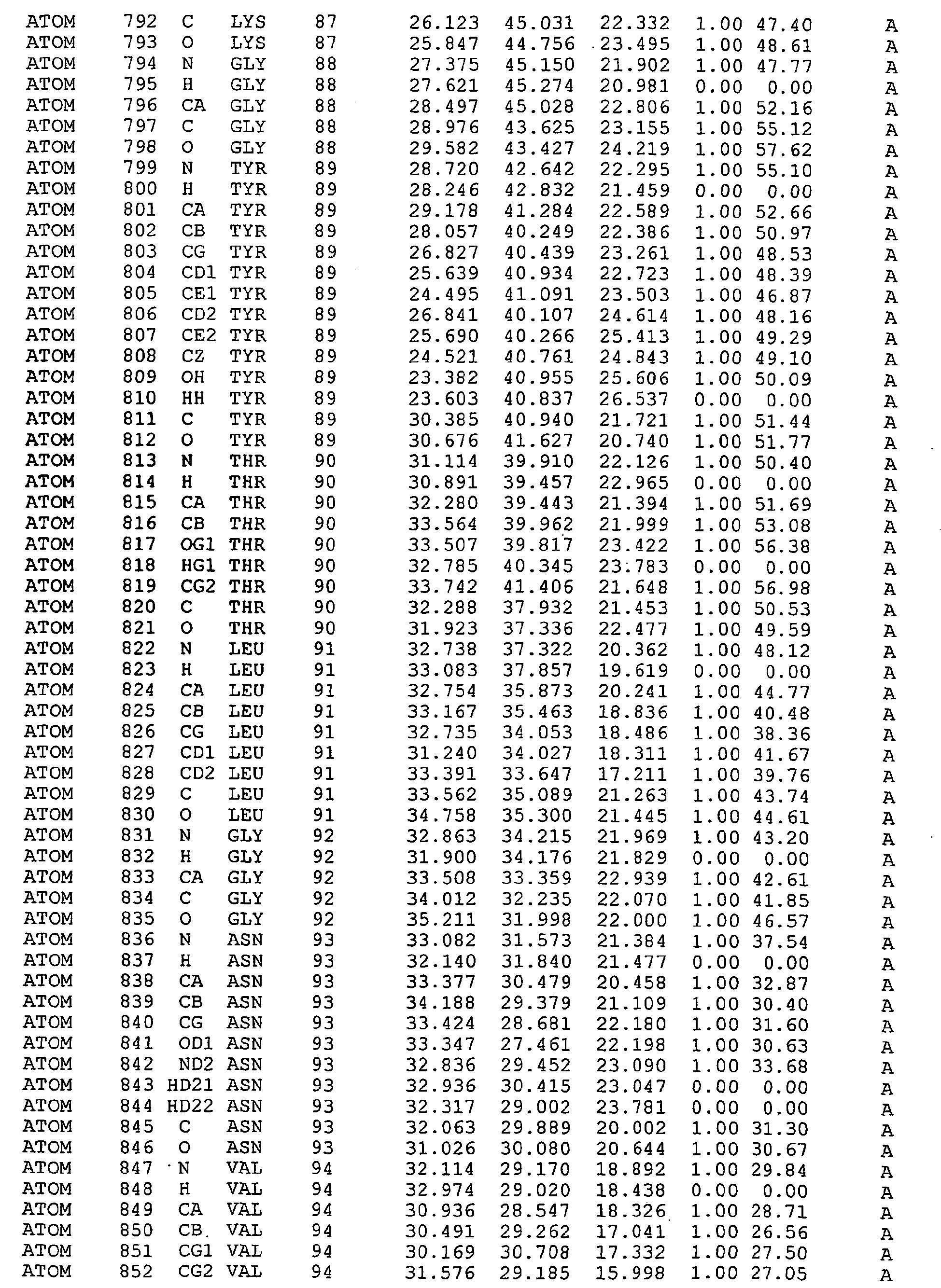

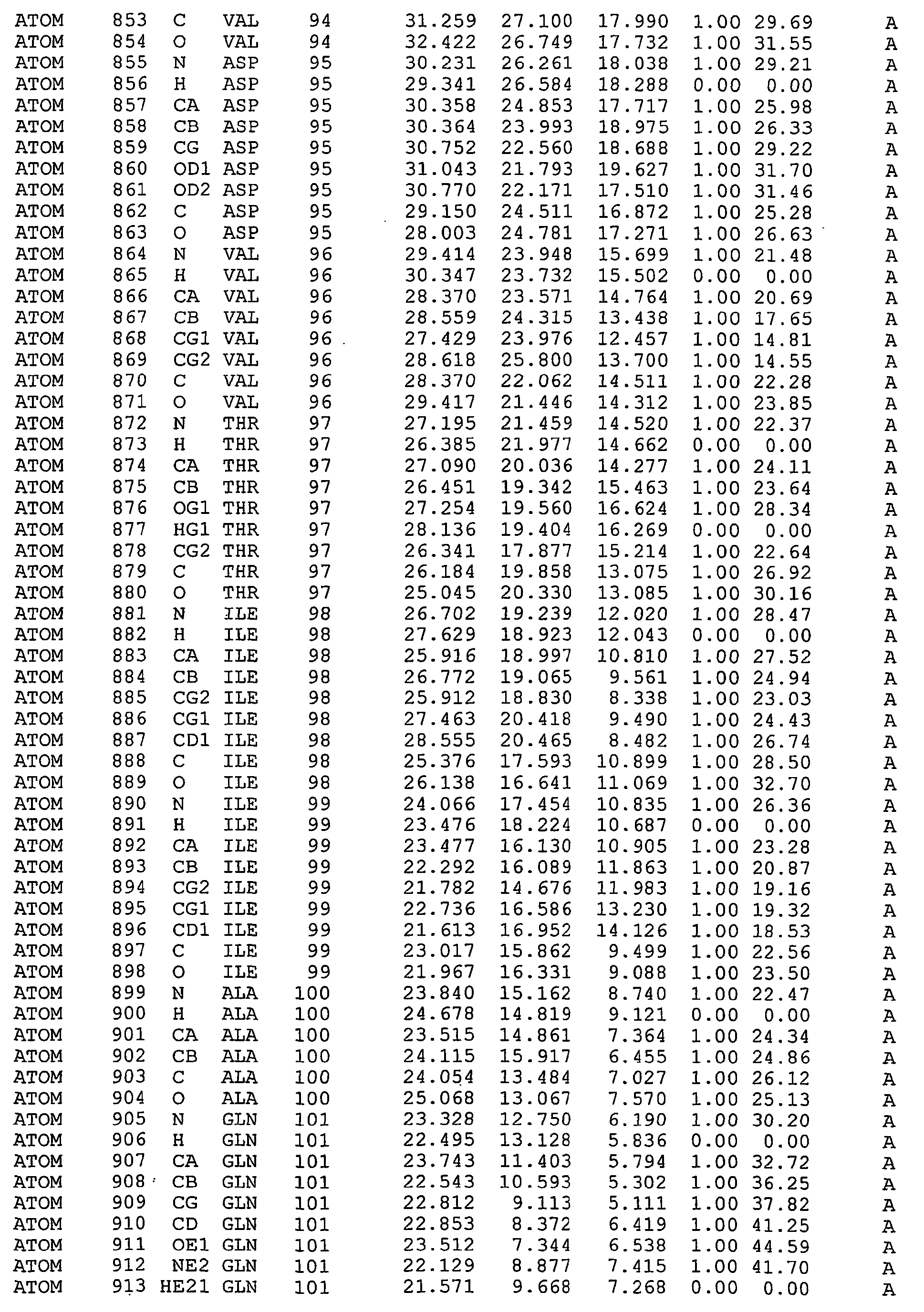

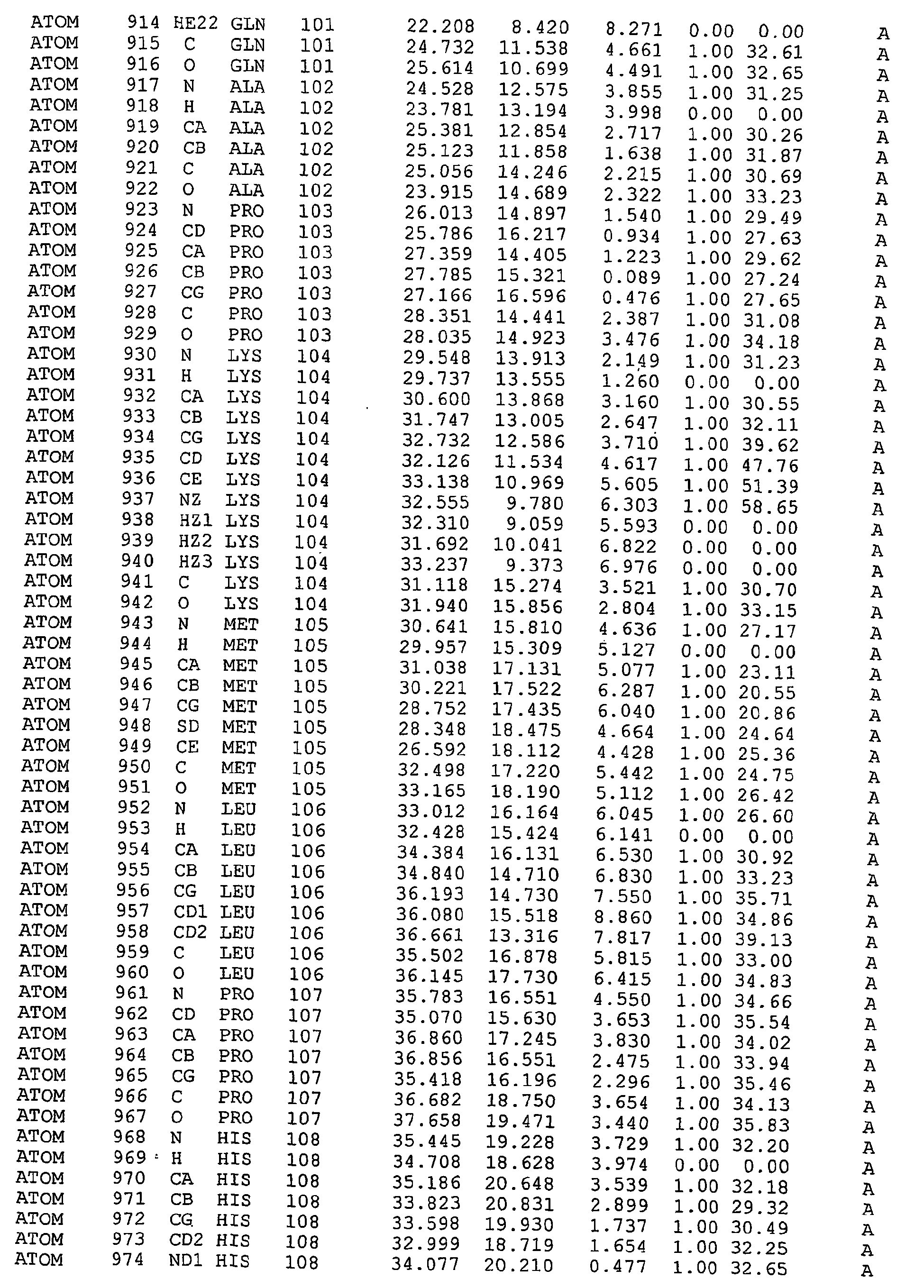

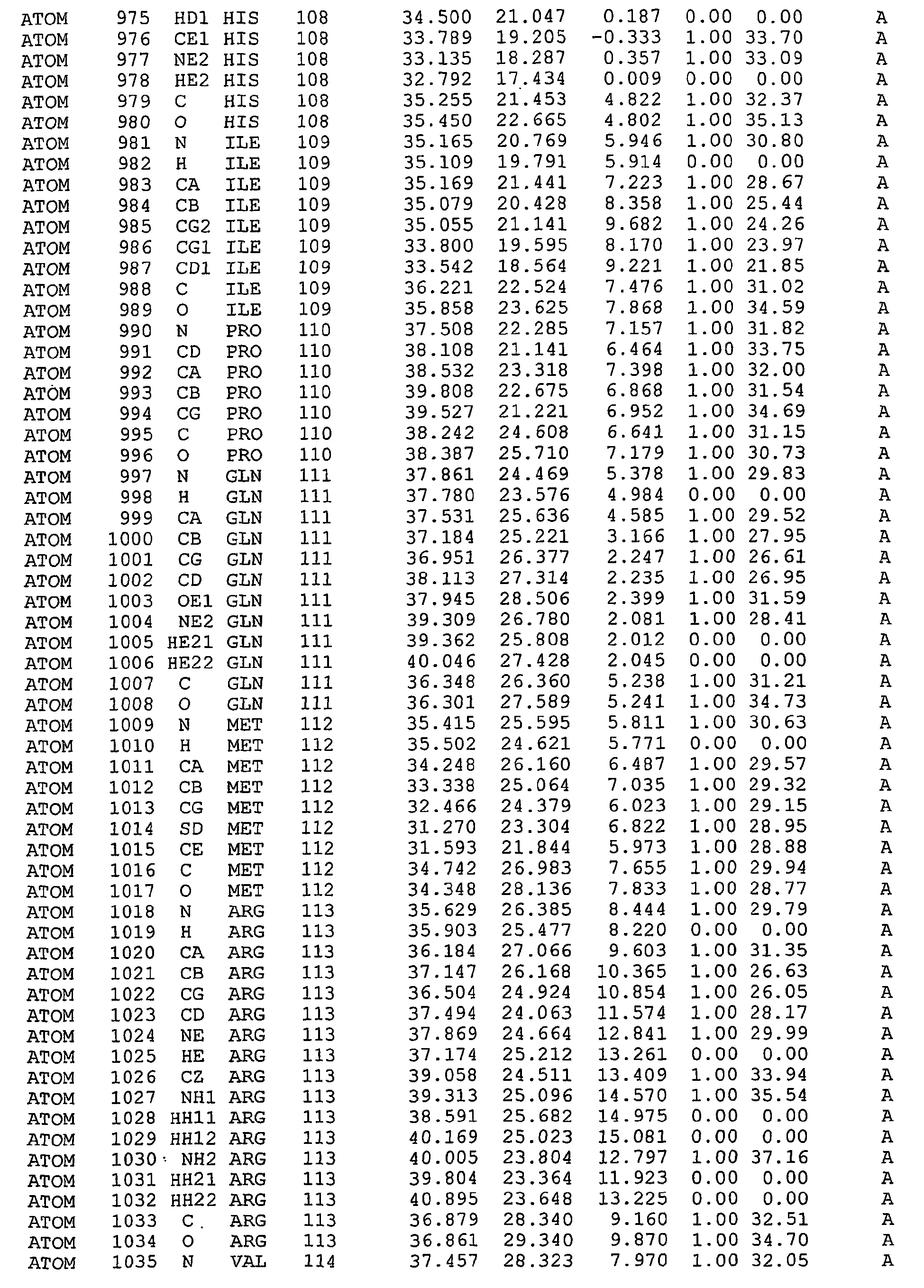

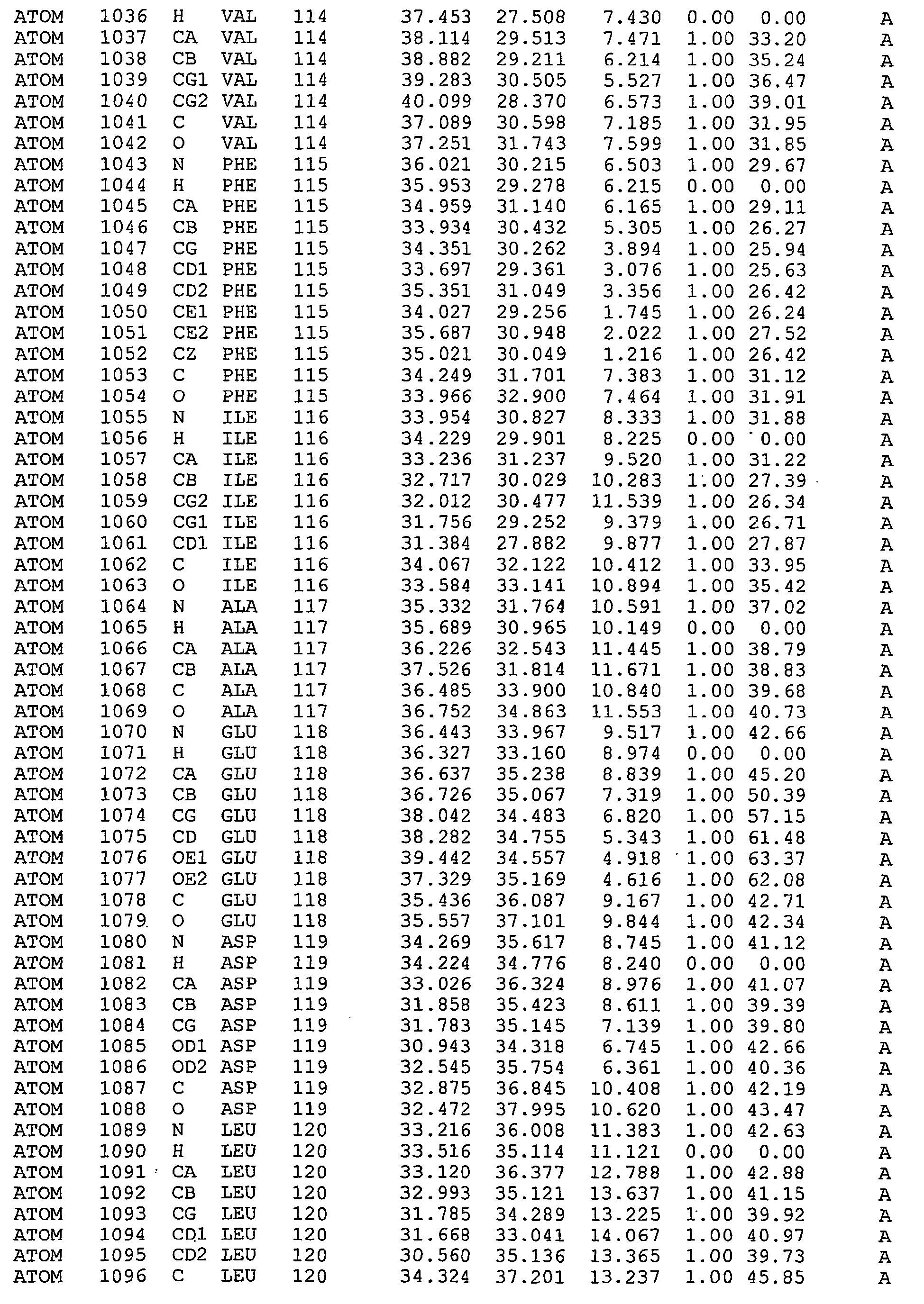

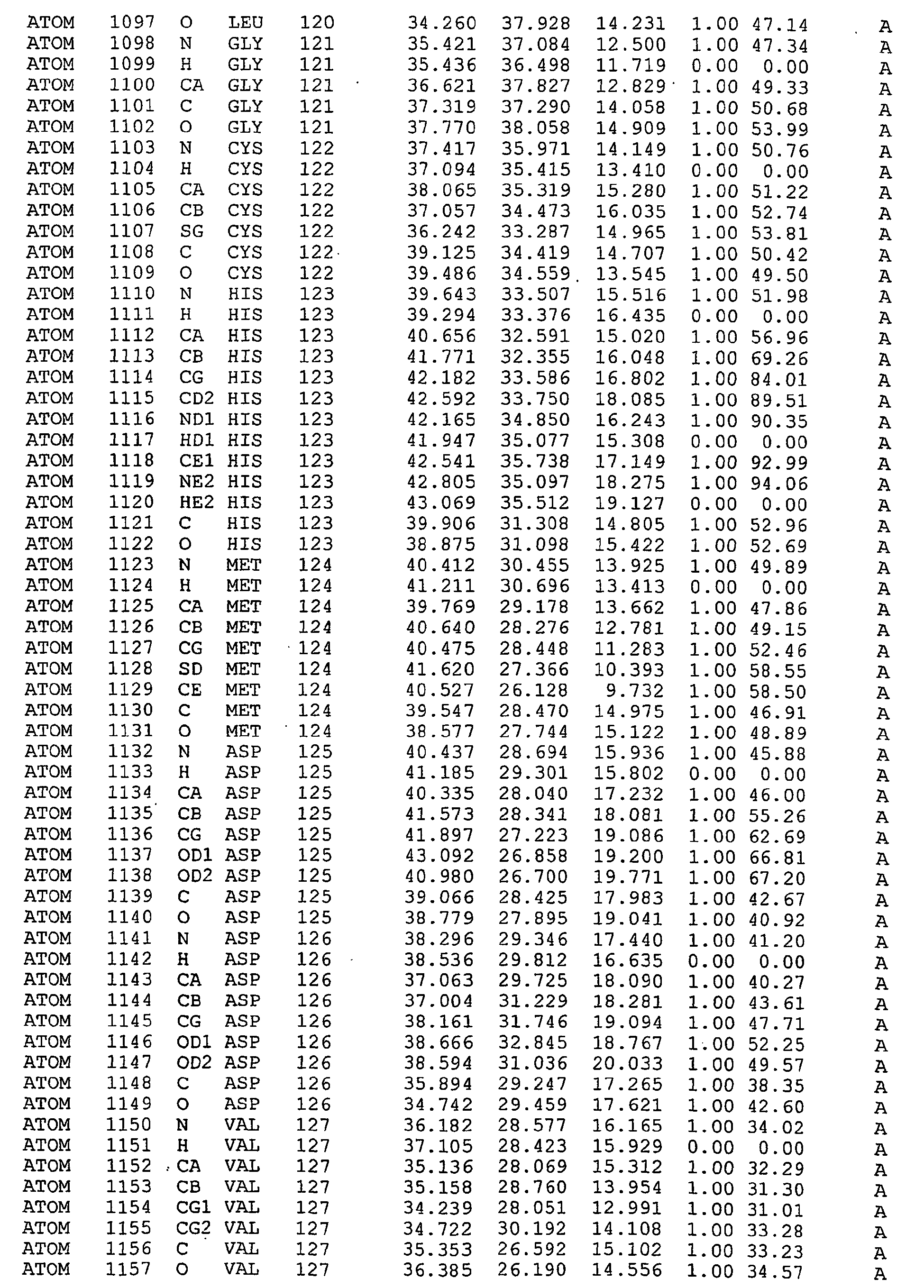

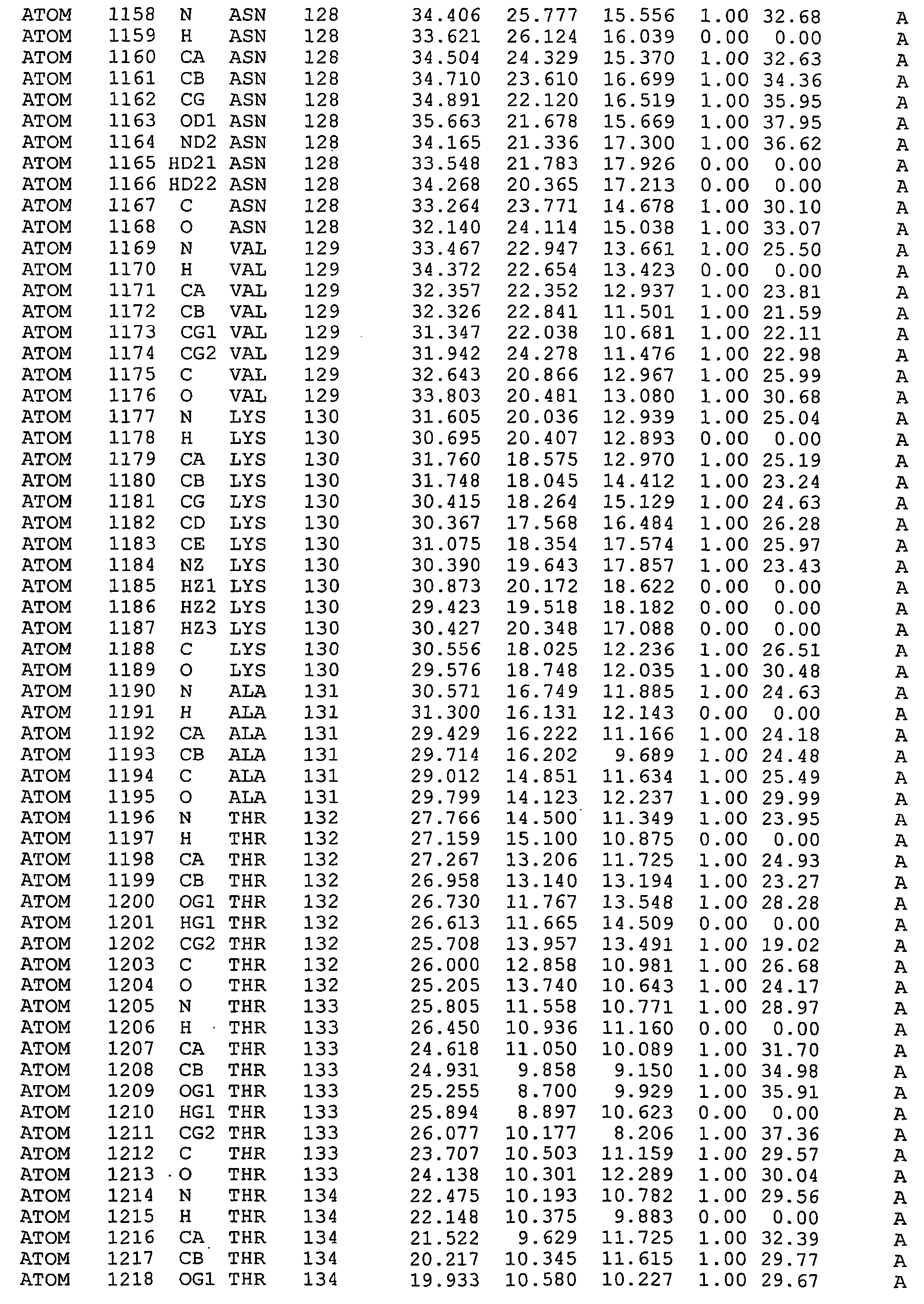

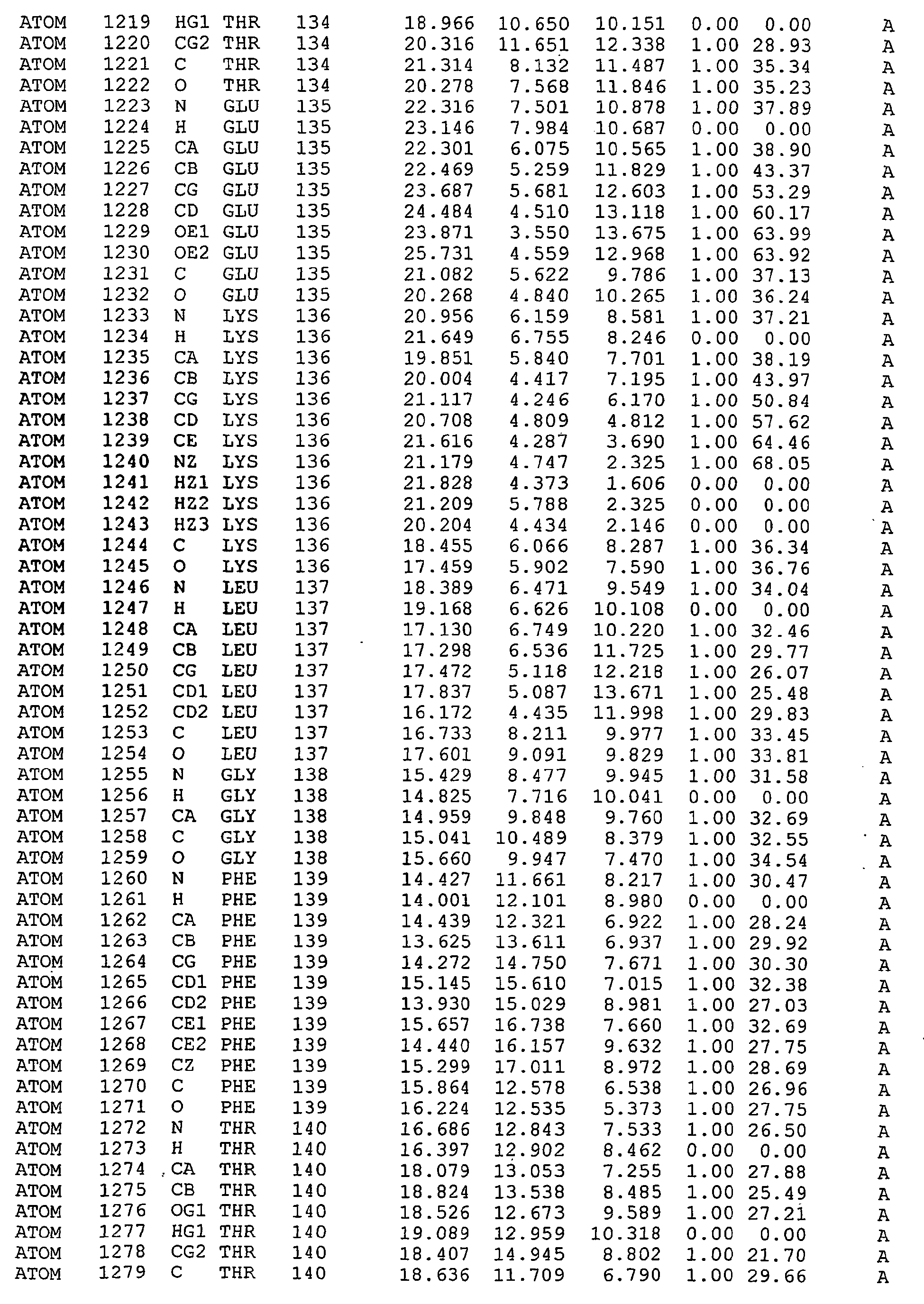

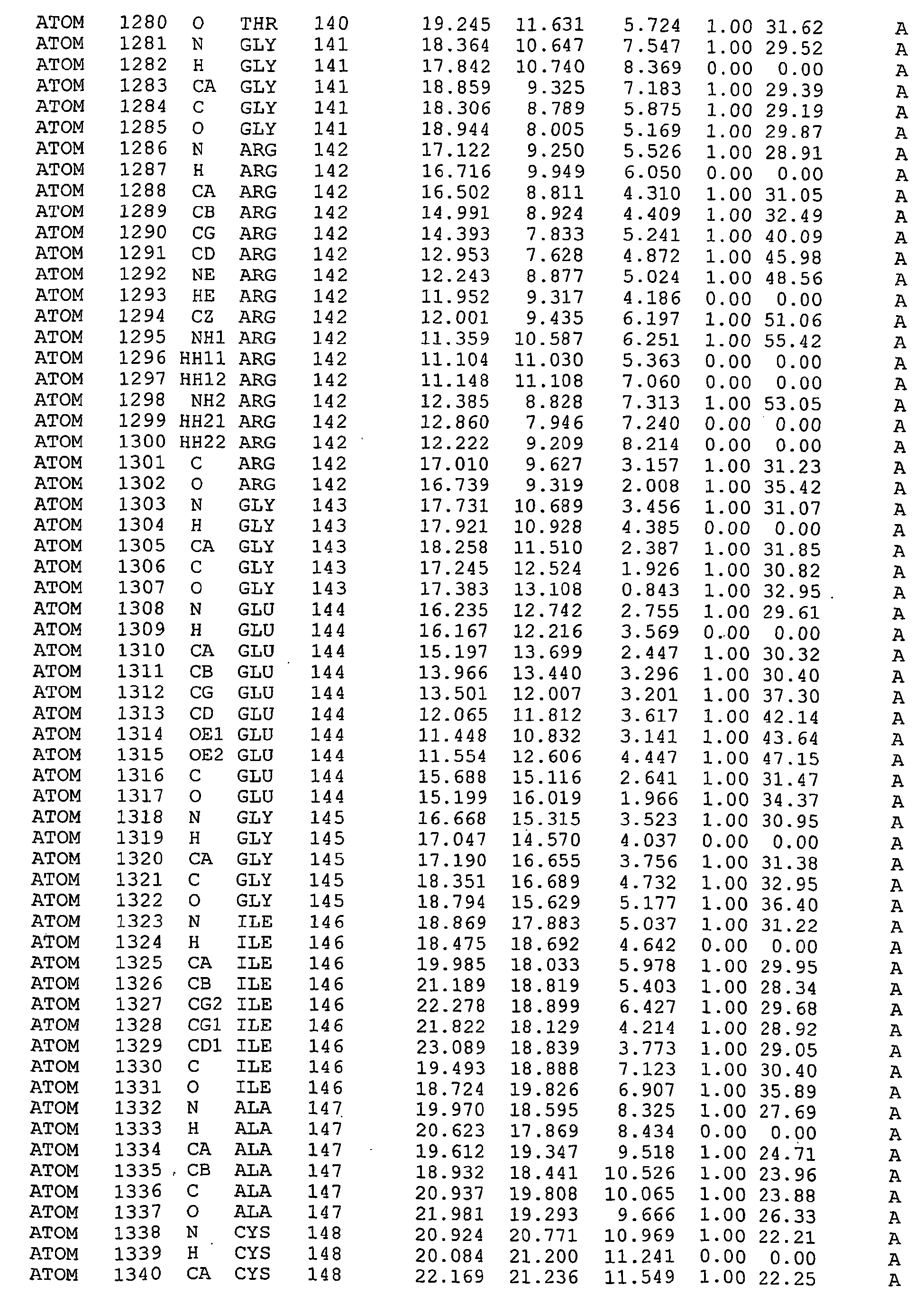

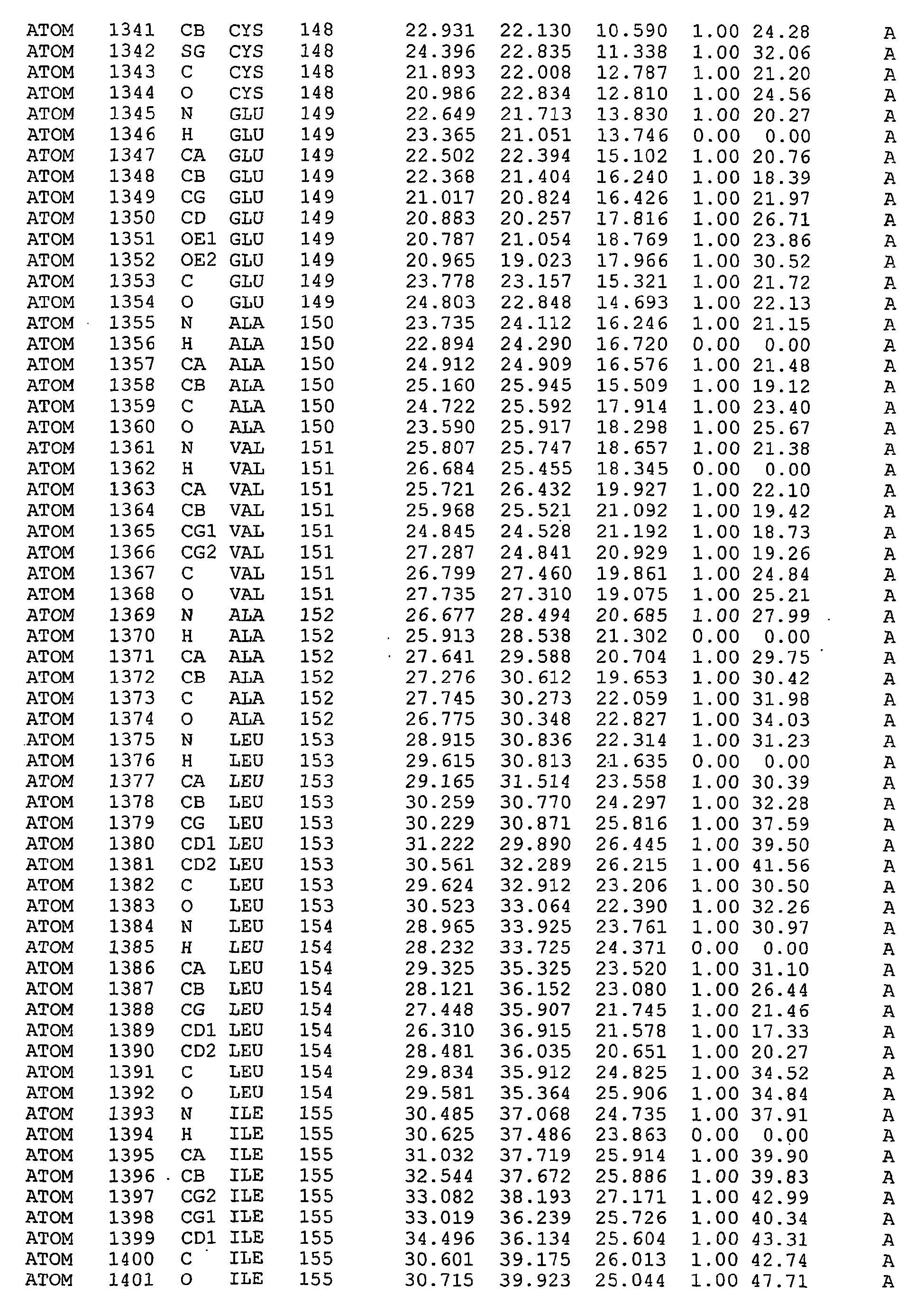

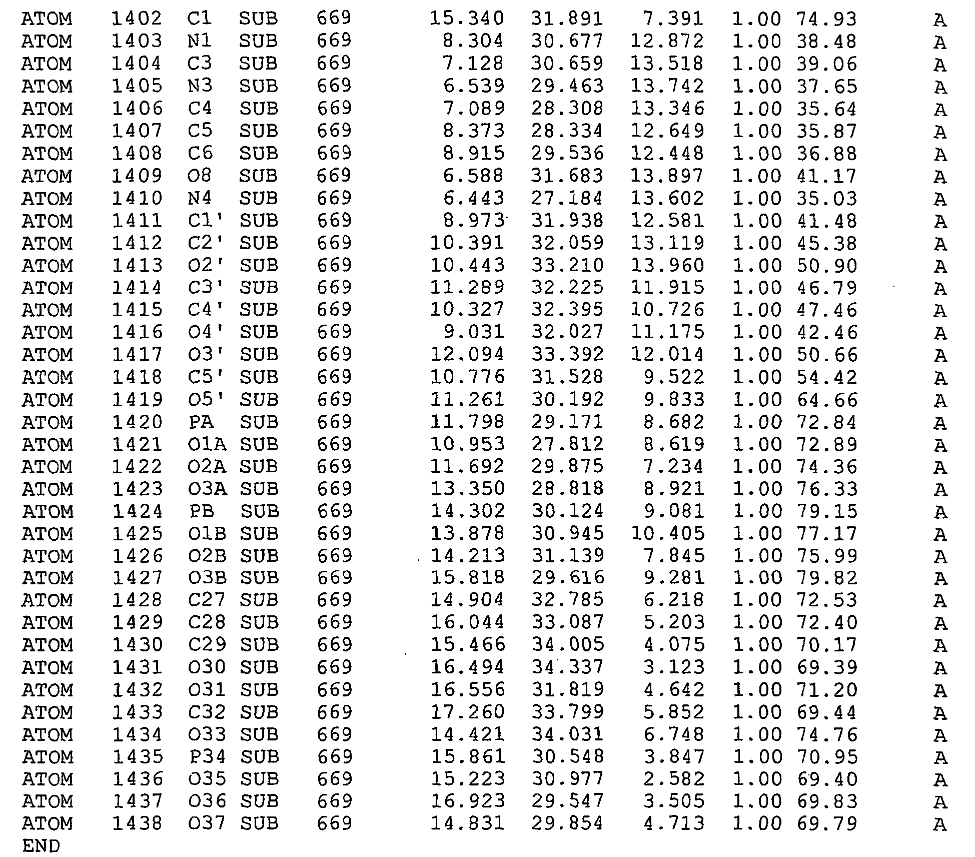

## Claims

1. A crystal which comprises the protein 2C-methyl-D-erythritol 2,4-cyclodiphosphate synthase with or without zinc, **characterized by** the cubic space group I 2(1)3 and a unit cell with a= 144.5 ± 2 Å.

2. The crystal according to claims 1 which effectively diffracts x-rays for the determination of the atomic coordinates of the protein to a resolution better than 5 Å.

3. The crystal according to claim 1 or 2 which effectively diffracts x-rays for the determination of the atomic coordinates of the protein to a resolution better than 3.5 Å.

4. The crystal according to any one of claims 1 to 3 comprising an organic compound selected from the group of 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol 2,4-cyclodiphosphate and a combination of cytidine monophosphate and 2C-methyl-D-erythritol 2,4-cyclodiphosphate.

5. A method of growing a crystal comprising the protein 2C-methyl-D-erythritol 2,4-cyclodiphosphate synthase and zinc by vapor diffusion using a reservoir solution containing 0.1 M HEPES pH 7.5 and 2 M ammonium formate.

6. Use of a crystal according to any one of claims 1 to 4 for the determination of the three-dimensional structure of the protein 2C-methyl-D-erythritol 2,4-cyclodiphosphate synthase or the three-dimensional structure of said synthase in complex with a compound selected from the group of 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol 2,4-cyclodiphosphate and a combination of cytidine monophosphate and 2C-methyl-D-erythritol 2,4-cyclodiphosphate; with or without zinc.

7. A method of using 2*C*-methyl-D-erythritol 2,4-cyclodiphosphate synthase and atomic coordinates of the three-dimensional structure of said synthase or of a complex of said synthase with a compound selected from the following group: 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol 2,4-cyclodiphosphate or a combination of cytidine monophosphate and 2C-methyl-D-erythritol 2,4-cyclodiphosphate, with or without zinc, in an inhibitor-screening assay, comprising:
(a) selecting a potential inhibitor by performing rational drug design using said atomic coordinates in conjunction with computer modelling;
(b) contacting the potential inhibitor with said synthase with or without zinc; and
(c) detecting binding of the potential inhibitor to said synthase or detecting inhibition of enzymatic activity of said synthase by the potential inhibitor;
wherein said atomic coordinates are selected from any of the three-dimensional structures represented by any one of annexes 1 to 6; or said atomic coordinates are selected from a three-dimensional structure that deviates from any one of the three-dimensional structures represented by annexes 1 to 6 by a root mean square deviation over protein backbone atoms of not more than 3 Å.

8. A method according to claim 7, wherein binding is detected by soaking the crystal with the potential inhibitor or by growing the crystal in the presence of the potential inhibitor and determining the three-dimensional structure of the complex comprising the synthase and the potential inhibitor with or without zinc.

9. A method of identifying a potential inhibitor of 2C-methyl-D-erythritol 2,4-cyclodiphosphate synthase by determining binding interactions between the potential inhibitor and a set of binding interaction sites in a binding cavity of said synthase complexed with a compound selected from the group consisting of 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol 2,4-cyclodiphosphate or a combination of cytidine monophosphate and 2C-methyl-D-erythritol 2,4-cyclodiphosphate, with or without zinc, comprising
(a) generating the binding cavity on a computer screen using atomic coordinates selected from any one of the three-dimensional structures represented by annexes 1 to 6 or using atomic coordinates selected from a three-dimensional structure that deviates from any one of the three-dimensional structures represented by annexes 1 to 6 by a root mean square deviation over protein backbone atoms of not more than 3 Å,
(b) generating potential inhibitors with their spatial structure on the computer screen, and
(c) selecting potential inhibitors that can bind to at least 3 amino acid residues without steric interference.

10. A computer-assisted method for identifying potential inhibitors of the protein 2C-methyl-D-erythritol 2,4-cyclodiphosphate synthase using a programmed computer comprising a processor, a data storage system, a data input device, and a data output device, comprising the following steps:
(a) inputting into the programmed computer through said input device data comprising:
atomic coordinates of a subset of the atoms of a complex of said protein with a compound selected from the following group: 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol 2,4-cyclodiphosphate or a combination of cytidine monophosphate and 2C-methyl-D-erythritol 2,4-cyclodiphosphate, with or without zinc, thereby generating a criteria data set;
wherein said atomic coordinates are selected from any of the three-dimensional structures represented by any one of annexes 1 to 6; or said atomic coordinates are selected from a three-dimensional structure that deviates from any one of the three-dimensional structures represented by annexes 1 to 6 by a root mean square deviation over protein backbone atoms of not more than 3 Å;
(b) comparing, using said processor, the criteria data set to a computer data base of low-molecular weight organic chemical structures stored in the data storage system; and
(c) selecting from said data base, using computer methods, a chemical structure having a portion that is structurally complementary to the criteria data set pertaining to the protein and/or structurally similar to the criteria data set pertaining to a compound of said group and being free of steric interference with the protein.

11. A computer-assisted method for identifying potential inhibitors of the protein 2C-methyl-D-erythritol 2,4-cyclodiphosphate synthase using a programmed computer comprising a processor, a data storage system, a data input device, and a data output device, comprising the following steps:
(a) inputting into the programmed computer through said input device data comprising:
atomic coordinates of a subset of the atoms of a complex of said protein with a compound selected from the following group: 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol 2,4-cyclodiphosphate or a combination of cytidine monophosphate and 2C-methyl-D-erythritol 2,4-cyclodiphosphate with or without zinc, thereby generating a criteria data set;
wherein said atomic coordinates are selected from any of the three-dimensional structures represented by any of annexes 1 to 6; or said atomic coordinates are selected from a three-dimensional structure that deviates from any one of the three-dimensional structures represented by annexes 1 to 6 by a root mean square deviation over protein backbone atoms of not more than 3 Å; and
(b) constructing, using computer methods, a model of a chemical structure having a portion that is structurally complementary to the criteria data set pertaining to the protein and/or structurally similar to the criteria data set pertaining to a compound of said group and being free of steric interference with the protein.

12. A method of identifying a candidate inhibitor capable of binding to and inhibiting the enzymatic activity of 2C-methyl-D-erythritol 2,4-cyclodiphosphate synthase, said method comprising the following steps:
(a) introducing into a computer information derived from atomic coordinates defining a conformation of the active site of said synthase or a complex of said synthase with a compound selected from the following group: 4-diphosphocytidyl-2C-methyl-D-erythritol, 4-diphosphocytidyl-2C-methyl-D-erythritol 2-phosphate, cytidine, cytidine monophosphate, cytidine diphosphate, 2C-methyl-D-erythritol or a combination of cytidine monophosphate and 2C-methyl-D-erythritol 2,4-cyclodiphosphate; with or without zinc, based on three-dimensional structure determination, whereby said program utilizes or displays on the computer screen the structures of said conformation;
wherein said atomic coordinates correspond to the atomic coordinates of any of annexes 1 to 6, or said atomic coordinates correspond to atomic coordinates that deviate from atomic coordinates of any of annexes 1 to 6 by a root mean square deviation over protein backbone atoms of not more than 3 Å;
(b) generating a three-dimensional representation of at least one of the three pockets of the active site of said synthase and/or a compound of said group by said computer program on a computer screen;
(c) superimposing a model of a candidate inhibitor on the representation of at least one pocket of the active site and/or a compound of said group;
(d) assessing the possibility of bonding and the absence of steric interference of the candidate inhibitor with the active site of the protein;
(e) incorporating said candidate compound in an activity assay of said synthase; and
(f) determining whether said candidate compound inhibits enzymatic activity of said synthase.

13. The method according to claim 12, wherein information is introduced into the computer derived from atomic coordinates of at least some of the following interactions of the active site:
Ala131# contacting the face of the cytidyl moiety;
Ala131# bonding with its carbonyl oxygen to at least one of the 2'-and 3'-hydroxyl groups of the cytidyl moiety;
Asp56 making a hydrogen bond with its carboxyl group to at least one of the 2'-and 3'-hydroxyl groups of the cytidyl moiety;
Gly58 making van der Waals contact with its Cα to at least one of the the 2'-and 3'-hydroxyl groups of the cytidyl moiety;
peptide group between Lys104# and Met105# hydrogen bonding to N3 of the cytidyl moiety;
Thr133# supporting the cytidyl moiety and hydrogen bonding with its γ-O or its backbone NH to the α-phosphate;
Lys104# contacting with its side chain the cytidyl moiety;
Leu106# contacting with its side chain the cytidyl moiety;
Leu106# hydrogen bonding with its NH to the carbonyl oxygen of the cytidyl moiety; Asp63 binding to the β-phosphate of cytidine diphosphate;
His34 hydrogen bonding with its backbone NH group to at least one oxygen atom of the P2 phosphate group of 2C-methyl-D-erythritol 2,4-cyclodiphosphate;
Ser35 hydrogen bonding with its backbone NH group to one oxygen atom of the P2 phosphate group;
Ser35 hydrogen bonding with its γ-OH to one of the oxygen atoms of the P2 phosphate group;
Leu76 making a van der Waals contact with its δ-C to the 2C-methyl group;
Ile57 making a van der Waals contact with δ-C to the 2-methyl group;
Ile57 making a van der Waals contact with γ-C to the 2-methyl group;
Phe61 hydrogen bonding with its backbone carbonyl oxygen to the 1-hydroxyl group;
Phe61 hydrogen bonding with its backbone carbonyl oxygen to the 3-hydroxyl group;
Ile57 hydrogen bonding with its backbone carbonyl oxygen to the 3-hydroxyl group;
Ile57 making van der Waals contact with its γ-C to the carbon at the 4-position;
Pro103# hydrogen bonding with its backbone carbonyl oxygen to the amino group of the cytidyl moiety;
Ala100# hydrogen bonding with its backbone carbonyl oxygen to the amino group of the cytidyl moiety;
Ala100# supporting with its backbone carbonyl oxygen the C5 position of the cytidyl moiety;
amino acids not denoted by # belonging to one subunit and those denoted by # belonging to another subunit.

14. The method according to one of claims 7 to 13, wherein the atomic coordinates are determined to a resolution of at least 4 Å.

15. The method according to one of claims 7 to 14, wherein compounds are selected that can bind to at least 5 binding sites of the synthase.

16. The method according to one of claims 7 to 14, wherein said root mean square deviation over protein backbone atoms is not more than 2 Å.

## Patentansprüche

1. Kristall, der das Protein 2C-Methyl-D-erythritol 2,4-cyclodiphosphat-Synthase mit oder ohne Zink umfasst, charakterisiert durch die kubische Raumgruppe I 2(1)3 und eine Einheitszelle mit a = 144,5 ± 2 Å.

2. Der Kristall gemäß Anspruch 1, der Röntgenstrahlung zur Bestimmung der atomaren Koordinaten des Proteins zu einer Auflösung von besser als 5 Å streut.

3. Der Kristall gemäß einem der Ansprüche 1 oder 2, der Röntgenstrahlung zur Bestimmung der atomaren Koordinaten des Proteins zu einer Auflösung von besser als 3,5 Å streut.

4. Der Kristall gemäß einem der Ansprüche 1 bis 3, umfassend eine organische Verbindung ausgewählt aus der Gruppe bestehend aus 4-Diphosphocytidyl-2C-methyl-D-erythritol, 4-Diphosphocytidyl-2C-methyl-D-erythritol 2-phosphat, Cytidin, Cytidinmonophosphat, Cytidindiphosphat, 2C-Methyl-D-erythritol- 2,4-cyclodiphosphat und eine Kombination von Cytidinmonophosphat und 2C-Methyl-D-erythritol-2,4-cyclodiphosphat.

5. Verfahren zum Züchten eines Kristalls umfassend das Protein 2C-Methyl-D- erythritol 2,4-cyclodiphosphat-Synthase und Zink durch Dampfdiffusion unter Verwendung einer Reservoirlösung, die 0.1 M HEPES pH 7.5 und 2 M Ammoniumformiat enthält.

6. Verwendung eines Kristalls gemäß einem der Ansprüche 1 bis 4 zur Bestimmung der dreidimensionalen Struktur des Proteins 2C-Methyl-D-erythritol 2,4-cyclodiphosphat-Synthase oder der dreidimensionalen Struktur der Synthase im Komplex mit einer Verbindung ausgewählt aus der Gruppe: 4-Diphosphocytidyl-2C-methyl-D-erythritol, 4-Diphosphocytidyl-2C-methyl-D-erythritol 2-phosphat, Cytidin, Cytidinmonophosphat, Cytidindiphosphat, 2C-Methyl-D-erythritol-2,4-cyclodiphosphat und eine Kombination von Cytidinmonophosphat und 2C-Methyl-D-erythritol-2,4- cyclodiphosphat, jeweils mit oder ohne Zink.

7. Verfahren zur Verwendung von 2C-Methyl-D-erythritol-2,4-cyclodiphosphat-Synthase und von Atomkoordinaten der dreidimensionalen Struktur der Synthase oder eines Komplexes der Synthase mit einer Verbindung ausgewählt aus der folgenden Gruppe: 4-Diphosphocytidyl-2C-methyl-D-erythritol, 4-Diphosphaocytidyl-2C- methyl-D-erythritol 2-phosphat, Cytidin, Cytidinmonophosphat, Cytidindiphosphat, 2C-Methyl-D-erythritol-2,4-cyclodiphosphat und eine Kombination von Cytidi monophosphat und 2C-Methyl-D-erythritol-2,4-cyclodiphosphat, mit oder ohne Zink, in einem Inhibitor-Screening Assay umfassend:
a) Auswahl eines potentiellen Inhibitors durch Rational Drug Design unter Verwendung der Atomkoordinaten in Verbindung mit Computer Modelling.
b) Zusammenbringen des potentiellen Inhibitors mit der Synthase mit oder ohne Zink; und
c) Detektion des Bindens des potentiellen Inhibitors an die Synthase oder Detektion der Inhibition der enzymatischen Aktivität der Synthase durch den potentiellen Inhibitor,
wobei die Atomkoordinaten ausgewählt werden aus einer der durch die Annexe 1 bis 6 repräsentierten dreidimensionalen Strukturen; oder die Atomkoordinaten werden ausgewählt aus einer dreidimensionalen Struktur, die von einer der durch die Annexe 1 bis 6 repräsentierten dreidimensionalen Strukturen durch eine mittlere quadratische Abweichung über Rückgratatome des Proteins von nicht mehr als 3 Å abweicht.

8. Das Verfahren gemäß Anspruch 7, wobei das Binden durch Baden des Kristalls in einem potentiellen Inhibitor oder durch Züchten des Kristalls in Gegenwart des potentiellen Inhibitors und durch Bestimmen der dreidimensionalen Struktur des Komplexes umfassend die Synthase und den potentiellen Inhibitor, mit oder ohne Zink, bestimmt wird.

9. Verfahren zur Identifizierung eines möglichen Inhibitors der 2C-Methyl-D-erythritol- 2,4-cyclodiphosphat-Synthase durch Bestimmung der Bindungswechselwirkung zwischen dem potentiellen Inhibitor und einer Menge von Bindungsstellen in einer Bindungskavität der Synthase im Komplex mit einer Verbindung ausgewählt aus der Gruppe bestehend aus 4-Diphosphocytidyl-2C-methyl-D-erythritol, 4-Diphosphocytidyl-2C-methyl-D-erythritol 2-phosphat, Cytidin, Cytidinmonophosphat, Cytidindiphosphat, 2C-Methyl-D-erythritol-2,4-cyclodiphosphat oder einer Kombination von Cytidin-monophosphat und 2C-Methyl-D-erythritol-2,4- cyclodiphosphat, mit oder ohne Zink, umfassend:
(a) Erzeugen der Bindungskavität auf einem Computerbildschirm unter Verwendung von Atomkoordinaten, die ausgewählt werden aus einer der durch die Annexe 1 bis 6 repräsentierten dreidimensionalen Strukturen oder unter Verwendung von Atomkoordinaten, die aus einer dreidimensionalen Struktur, ausgewählt werden, die von einer der durch die Annexe 1 bis 6 repräsentierten dreidimensionalen Strukturen durch eine mittlere quadratische Abweichung über Rückgratatome des Proteins von nicht mehr als 3 Å abweicht,
(b) Erzeugen von potentiellen Inhibitoren in ihrer räumlichen Struktur auf dem Computerbildschirm und
(c) Auswählen eines potentiellen Inhibitors, der an mindestens drei Aminosäurereste ohne räumliche Überlappung bindet.

10. Computer-gestütztes Verfahren zur Identifizierung eines potentiellen Inhibitors des Proteins 2C-Methyl-D-erythritol-2,4-cyclodiphosphat-Synthase unter Verwendung eines programmierten Computers umfassend einen Prozessor, ein Datenspeichersystem, ein Dateneingabegerät und ein Datenausgabegerät, wobei das Verfahren die folgenden Schritte umfasst:
(a) Eingabe von Daten in den programmierten Computer durch das Eingabegerät, wobei die Daten umfassen: Atomkoordinaten einer Untermenge der Atome eines Komplexes des Proteins mit einer Verbindung ausgewählt aus der folgenden Gruppe: 4-Diphosphocytidyl-2C-methyl-D-erythritol, 4-Diphosphocytidyl-2C-methyl-D-erythritol 2-phosphat, Cytidin, Cytidinmonophosphat, Cytidindiphosphat, 2C-Methyl-D-erythritol-2,4-cyclodiphosphat oder eine Kombination von Cytidinmonophosphat und 2C-Methyl-D-erythritol-2,4-cyclodiphosphat, mit oder ohne Zink, wodurch ein Referenzdatensatz erzeugt wird,
wobei die Atomkoordinaten ausgewählt werden aus einer der durch die Annexe 1 bis 6 repräsentierten dreidimensionalen Strukturen; oder die Atomkoordinaten werden ausgewählt aus einer dreidimensionalen Struktur, die von einer der durch die Annexe 1 bis 6 repräsentierten dreidimensionalen Strukturen durch eine mittlere quadratische Abweichung über Rückgratatome des Proteins von nicht mehr als 3 Å abweicht,
(b) Vergleichen des Referenzdatensatzes unter Verwendung des Prozessors mit einer Computerdatenbank von niedermolekularen organisch-chemischen Strukturen, die in dem Datenspeichersystem gespeichert sind und
(c) Auswahl mittels Computermethoden einer chemischen Struktur aus der Datenbank, wobei die chemische Struktur einen Teil aufweist, der strukturell komplementär zum sich auf das Protein erstreckenden Referenzdatensatz ist, und/oder der strukturell ähnlich ist zum sich auf eine Verbindung der Gruppe erstreckenden Referenzdatensatz ist, und frei von räumlicher Überlappung mit dem Protein ist.

11. Computergestütztes Verfahren zur Identifizierung eines potentiellen Inhibitors des Proteins 2C-Methyl-D-erythritol-2,4-cyclodiphosphat Synthase unter Verwendung eines programmierten Computers umfassend einen Prozessor, ein Datenspeichersystem, ein Dateneingabegerät und ein Datenausgabegerät, wobei das Verfahren die folgenden Schritte umfasst:
a) Eingabe von Daten in den programmierten Computer durch das Eingabegerät, wobei die Daten umfassen: Atomkoordinaten einer Untermenge der Atome eines Komplexes des Proteins mit einer Verbindung ausgewählt aus der folgenden Gruppe: 4-Diphosphocytidyl-2C-methyl-D-erythritol, 4-Diphosphocytidyl-2C-methyl-D-erythritol 2-phosphat, Cytidin, Cytidinmonophosphat, Cytidindiphosphat, 2C-Methyl-D-erythritol-2,4-cyclodiphosphat oder eine Kombination von Cytidinmonophosphat und 2C-Methyl-D-erythritol-2,4-cyclodiphosphat, mit oder ohne Zink, wodurch ein Referenzdatensatz erzeugt wird,
wobei die Atomkoordinaten ausgewählt werden aus einer der durch die Annexe 1 bis 6 repräsentierten dreidimensionalen Strukturen; oder die Atomkoordinaten werden ausgewählt aus einer dreidimensionalen Struktur, die von einer der durch die Annexe 1 bis 6 repräsentierten dreidimensionalen Strukturen durch eine mittlere quadratische Abweichung über Rückgratatome des Proteins von nicht mehr als 3 Å abweicht, und
b) Entwerfen eines Modells einer chemischen Struktur unter Verwendung von Computermethoden, wobei die chemische Struktur einen Teil aufweist, der strukturell komplementär zum sich auf das Protein erstreckenden Referenzdatensatz ist, und/oder der strukturell ähnlich ist zum sich auf eine Verbindung der Gruppe erstreckenden Referenzdatensatz ist, und frei von räumlicher Überlappung mit dem Protein ist.

12. Verfahren zur Identifizierung eines Inhibitor-Kandidaten, der an die 2C-Methyl-D-erythritol-2,4-cyclodiphosphat Synthase binden und ihre enzymatische Aktivität inhibieren kann, wobei das Verfahren die folgenden Schritte umfasst:
a) Eingabe von Informationen in einen Computer, die von atomaren Koordinaten abgeleitet sind, die eine Konformation des aktiven Zentrums der Synthase oder eines Komplexes der Synthase mit einer Verbindung ausgewählt aus der Gruppe: 4-Diphosphocytidyl-2C-methyl-D-erythritol, 4-Diphosphocytidyl-2C-methyl-D- erythritol 2-phosphat, Cytidin, Cytidin-monophosphat, Cytidindiphosphat, 2C- Methyl-D-erythritol-2,4-cyclodiphosphat und eine Kombination von Cytidin- monophosphat und 2C-Methyl-D-erythritol-2,4-cyclodiphosphat, mit oder ohne Zink, definieren, basierend auf dreidimensionaler Strukturanalyse, wobei das Programm die Strukturen der Konformation verwendet oder auf **einem Computerbildschirm darstellt,**
wobei die Atomkoordinaten ausgewählt werden aus einer der durch die Annexe 1 bis 6 repräsentierten dreidimensionalen Strukturen; oder die Atomkoordinaten werden ausgewählt aus einer dreidimensionalen Struktur, die von einer der durch die Annexe 1 bis 6 repräsentierten dreidimensionalen Strukturen durch eine mittlere quadratische Abweichung über Rückgratatome des Proteins von nicht mehr als 3 Å abweicht, und
b) Erzeugung einer dreidimensionalen Darstellung mindestens einer der drei Taschen des aktiven Zentrums der Synthase und/oder einer Verbindung der Gruppe durch das Computer-Programm auf einem Computerbildschirm,
c) Überlagern eines Modells eines Inhibitor-Kandidaten und der Darstellung mindestens einer Tasche des aktiven Zentrums und/oder einer Verbindung der Gruppe,
d) Abschätzen der Möglichkeit einer Bindung und der Abwesenheit räumlicher Überlappung des Inhibitor-Kandidaten mit dem aktiven Zentrum des Proteins,
e) Verwenden der Kandidaten-Verbindung in einem Aktivitätstest der Synthase; und
f) Bestimmung, ob die Kandidaten-Verbindung die enzymatische Aktivität der Synthase inhibiert.

13. Das Verfahren gemäß Anspruch 12, wobei Informationen in den Computer eingegeben werden, die von atomaren Koordinaten von zumindest einigen der folgenden Wechselwirkungen des aktiven Zentrums abgeleitet sind:
Ala131#, das eine Fläche des Cytidyl-Rests kontaktiert;
Ala131#, das über seine Carbonyl-Gruppe an mindestens eine der 2'- und 3'-Hydroxylgruppen des Cytidyl-Rests bindet;
Asp56, das mit seiner Carboxyl-Gruppe eine Wasserstoffbrücke zu mindestens einer der 2'- und 3'-Hydroxylgruppen des Cytidyl-Rests ausbildet;
Gly58, das über sein Cα-Atom zu mindestens einer der 2'- und 3'-Hydroxylgruppen des Cytidyl-Rests einen van der Waals-Kontakt ausbildet;
die Peptid-Gruppe zwischen Lys104# und Met105#, die eine Wasserstoffbrücke mit N3 des Cytidyl-Rests ausbildet;
Thr133#, das den Cytidyl-Rest stützt und über sein γ-O oder seine NH-Gruppe des Rückgrats zum α-Phosphat eine Wasserstoffbrücke ausbildet;
Lys104#, das mit seiner Seitenkette den Cytidyl-Rest kontaktiert;
Leu106#, das mit seiner Seitenkette den Cytidyl-Rest kontaktiert;
Leu106#, das mit seiner NH-Gruppe eine Wasserstoffbrücke zum Carbonyl-Sauerstoff des Cytidyl-Rests ausbildet,
Asp63, das das β-Phospat des Cytidiniphosphats bindet;
His34, das mit seiner Peptid-NH Gruppe zu mindestens einem Sauerstoffatom der P2-Phosphatgruppe des 2C-Methyl-D-erythritol 2,4-cyclodiphosphats eine Wasserstoffbrücke ausbildet;
Ser35, das mit seiner Peptid-NH Gruppe zu einem Sauerstoffatom der P2-Phosphatgruppe eine Wasserstoffbrücke ausbildet;
Ser35, das mit seiner γ-OH-Gruppe zu einem Sauerstoffatom der P2-Phosphatgruppe eine Wasserstoffbrücke ausbildet;
Leu76, das mit seinem δ-C-Atom einen van der Waals-Kontakt zur 2C-Methylgruppe ausbildet; Ile57, das mit seinem delta -C-Atom einen van der Waals-Kontakt zur 2-Methylgruppe ausbildet;
lle57, das mit seinem γ-C-Atom einen van der Waals-Kontakt zur 2-Methylgruppe ausbildet; Phe61, das mit seinem Peptid-Carbonylsauerstoff eine Wasserstoffbrücke zur 1-Hydroxyl-Gruppe ausbildet;
Phe61, das mit seinem Peptid-Carbonylsauerstoff eine Wasserstoffbrücke zur 3-Hydroxyl-Gruppe ausbildet;
Ile57, das mit seinem Peptid-Carbonylsauerstoff eine Wasserstoffbrücke zur 3-Hydroxyl-Gruppe ausbildet;
Ile57, das mit seinem γ-C-Atom einen van der Waals-Kontakt zum Kohlenstoff an Position 4 ausbildet;
Pro103#, das mit seinem Peptid-Carbonylsauerstoff eine Wasserstoffbrücke zur Aminogruppe des Cytidyl-Rests ausbildet;
Ala100#, das mit seinem Peptid-Carbonylsauerstoff eine Wasserstoffbrücke zur Aminogruppe des Cytidyl-Rests ausbildet;
Ala100#, das mit seinem Peptid-Carbonylsauerstoff die C5-Position des Cytidyl-Rests stützt;
wobei Aminosäuren, die nicht mit # gekennzeichnet sind und solche, die mit # gekennzeichnet sind, verschiedenen Untereinheiten angehören.

14. Das Verfahren gemäß einem der Ansprüche 7 bis 13, wobei die atomaren Koordinaten zu einer Auflösung von mindestens 4 Å bestimmt sind.

15. Das Verfahren gemäß einem der Ansprüche 7 bis 14, wobei Verbindungen ausgewählt werden, die an mindestens fünf Bindungsstellen der Synthase binden können.

16. Das Verfahren gemäß einem der Ansprüche 7 bis 14, wobei die atomaren Koordinaten zu einer Auflösung von mindestens 2 Å bestimmt sind.

## Revendications

1. Cristal qui comprend la protéine 2C-méthyl-D-érythritol 2,4-cyclodiphosphate synthase avec ou sans zinc, **caractérisé par** le groupe spatial cubique 1 2(1)3 et une cellule unitaire avec a = 144,5 ± 2 Å.

2. Cristal selon la revendication 1, qui diffracte efficacement les rayons X pour la détermination des coordonnées atomiques de la protéine à une résolution meilleure que 5 Å.

3. Cristal selon la revendication 1 ou 2, qui diffracte efficacement les rayons X pour la détermination des coordonnées atomiques de la protéine à une résolution meilleure que 3,5 Å.

4. Cristal selon l'une quelconque des revendications 1 à 3, comprenant un composé organique choisi dans le groupe constitué par le 4-diphosphocytidyl-2C-méthyl-D-érythritol, le 2-phosphate de 4-diphosphocytidyl-2C-méthyl-D-érythritol, la cytidine, le monophosphate de cytidine, le diphosphate de cytidine, le 2,4-cyclodiphosphate de 2C-méthyl-D-érythritol, et une combinaison de monophosphate de cytidine et de 2,4-cyclodiphosphate de 2C-méthyl-D-érythritol.

5. Procédé pour faire croître un cristal comprenant la protéine 2C-méthyl-D-érythritol 2,4-cyclodiphosphate synthase et du zinc par diffusion de vapeur en utilisant une solution de réservoir contenant du HEPES 0,1 M pH 7,5 et du formiate d'ammonium 2 M.

6. Utilisation d'un cristal selon l'une quelconque des revendications 1 à 4 pour la détermination de la structure tridimensionnelle de la protéine 2C-méthyl-D-érythritol 2,4-cyclodiphosphate synthase ou de la structure tridimensionnelle de ladite synthase complexée avec un composé choisi dans le groupe constitué par le 4-diphosphocytidyl-2C-méthyl-D-érythritol, le 2-phosphate de 4-diphosphocytidyl-2C-méthyl-D-érythritol, la cytidine, le monophosphate de cytidine, le diphosphate de cytidine, le 2,4-cyclodiphosphate de 2C-méthyl-D-érythritol, et une combinaison de monophosphate de cytidine et de 2,4-cyclodiphosphate de 2C-méthyl-D-érythritol ; avec ou sans zinc.

7. Procédé d'utilisation de 2C-méthyl-D-érythritol 2,4-cyclodiphosphate synthase et des coordonnées atomiques de la structure tridimensionnelle de ladite synthase ou d'un complexe de ladite synthase avec un composé choisi dans le groupe suivant : 4-diphosphocytidyl-2C-méthyl-D-érythritol, 2-phosphate de 4-diphosphocytidyl-2C-méthyl-D-érythritol, cytidine, monophosphate de cytidine, diphosphate de cytidine, 2,4-cyclodiphosphate de 2C-méthyl-D-érythritol, et une combinaison de monophosphate de cytidine et de 2,4-cyclodiphosphate de 2C-méthyl-D-érythritol, avec ou sans zinc, dans un essai de dépistage d'inhibiteur, comprenant :
(a) la sélection d'un inhibiteur potentiel par mise en oeuvre d'une conception de médicament rationnelle en utilisant lesdites coordonnées atomiques conjointement avec une modélisation par ordinateur ;
(b) la mise en contact de l'inhibiteur potentiel avec ladite synthase avec ou sans zinc ; et
(c) la détection de la liaison de l'inhibiteur potentiel à ladite synthase ou la détection de l'inhibition de l'activité enzymatique de ladite synthase par l'inhibiteur potentiel ;
dans lequel lesdites coordonnées atomiques sont choisies à partir de l'une quelconque des structures tridimensionnelles représentées par l'une quelconque des annexes 1 à 6 ; ou bien lesdites coordonnées atomiques sont choisies à partir d'une structure tridimensionnelle qui s'écarte de l'une quelconque des structures tridimensionnelles représentées par les annexes 1 à 6 d'un écart de la moyenne quadratique par rapport aux atomes du squelette de la protéine d'au plus 3 Å.

8. Procédé selon la revendication 7, dans lequel la liaison est détectée en imbibant le cristal avec l'inhibiteur potentiel ou en faisant croître le cristal en présence de l'inhibiteur potentiel, et en déterminant la structure tridimensionnelle du complexe comprenant la synthase et l'inhibiteur potentiel avec ou sans zinc.

9. Procédé pour identifier un inhibiteur potentiel de la 2C-méthyl-D-érythritol 2,4-cyclodiphosphate synthase par détermination d'interactions de liaison entre l'inhibiteur potentiel et un ensemble de sites d'interaction de liaison dans une cavité de liaison de ladite synthase complexée avec un composé choisi dans le groupe constitué par le 4-diphosphocytidyl-2C-méthyl-D-érythritol, le 2-phosphate de 4-diphosphocytidyl-2C-méthyl-D-érythritol, la cytidine, le monophosphate de cytidine, le diphosphate de cytidine, le 2,4-cyclodiphosphate de 2C-méthyl-D-érythritol, et une combinaison de monophosphate de cytidine et de 2,4-cyclodiphosphate de 2C-méthyl-D-érythritol, avec ou sans zinc, comprenant :
(a) la génération de la cavité de liaison sur un écran d'ordinateur par utilisation de coordonnées atomiques choisies à partir de l'une quelconque des structures tridimensionnelles représentées par les annexes 1 à 6 ou par utilisation de coordonnées atomiques choisies à partir d'une structure tridimensionnelle qui s'écarte de l'une quelconque des structures tridimensionnelles représentées par les annexes 1 à 6 d'un écart de la moyenne quadratique par rapport aux atomes du squelette de la protéine d'au plus 3 Å,
(b) la génération d'inhibiteurs potentiels avec leur structure spatiale sur l'écran d'ordinateur, et
(c) la sélection d'inhibiteurs potentiels qui peuvent se lier à au moins 3 résidus d'acides aminés sans interférence stérique.

10. Procédé assisté par ordinateur pour identifier des inhibiteurs potentiels de la protéine 2C-méthyl-D-érythritol 2,4-cyclodiphosphate synthase, utilisant un ordinateur programmé comprenant un processeur, un système de stockage de données, un dispositif d'entrée de données, et un dispositif de sortie de données, comprenant les étapes suivantes .
(a) saisie dans l'ordinateur programmé, par l'intermédiaire dudit dispositif d'entrée de données, comprenant :
des coordonnées atomiques d'un sous-ensemble des atomes d'un complexe de ladite protéine avec un composé choisi dans le groupe suivant : 4-diphosphocytidyl-2C-méthyl-D-érythritol, 2-phosphate de 4-diphosphocytidyl-2C-méthyl-D-érythritol, cytidine, monophosphate de cytidine, diphosphate de cytidine, 2,4-cyclodiphosphate de 2C-méthyl-D-érythritol, et une combinaison de monophosphate de cytidine et de 2,4-cyclodiphosphate de 2C-méthyl-D-érythritol, avec ou sans zinc, ce qui génère un ensemble de données de critères ;
lesdites coordonnées atomiques étant choisies à partir de l'une quelconque des structures tridimensionnelles représentées par l'une quelconque des annexes 1 à 6 ; ou bien lesdites coordonnées atomiques étant choisies à partir d'une structure tridimensionnelle qui s'écarte de l'une quelconque des structures tridimensionnelles représentées par les annexes 1 à 6 d'un écart de la moyenne quadratique par rapport aux atomes du squelette de la protéine d'au plus 3 Å ;
(b) comparaison, au moyen dudit processeur, de l'ensemble de données de critères à une base de données informatiques de structures chimiques organiques de faible poids moléculaire mémorisée dans le système de stockage de données ; et
(c) sélection dans ladite base de données, au moyen de procédés informatiques, d'une structure chimique ayant une partie qui est structurellement complémentaire de l'ensemble de données de critères relatif à la protéine et/ou structurellement similaire à l'ensemble de données de critères relatif à un composé dudit groupe, et qui est exempte d'interférence stérique avec la protéine.

11. Procédé assisté par ordinateur pour identifier des inhibiteurs potentiels de la protéine 2C-méthyl-D-érythritol 2,4-cyclodiphosphate synthase, utilisant un ordinateur programmé comprenant un processeur, un système de stockage de données, un dispositif d'entrée de données, et un dispositif de sortie de données, comprenant les étapes suivantes .
(a) saisie dans l'ordinateur programmé, par l'intermédiaire dudit dispositif d'entrée de données, comprenant :
les coordonnées atomiques d'un sous-ensemble des atomes d'un complexe de ladite protéine avec un composé choisi dans le groupe suivant : 4-diphosphocytidyl-2C-méthyl-D-érythritol, 2-phosphate de 4-diphosphocytidyl-2C-méthyl-D-érythritol, cytidine, monophosphate de cytidine, diphosphate de cytidine, 2,4-cyclodiphosphate de 2C-méthyl-D-érythritol, et une combinaison de monophosphate de cytidine et de 2,4-cyclodiphosphate de 2C-méthyl-D-érythritol, avec ou sans zinc, ce qui génère un ensemble de données de critères ;
lesdites coordonnées atomiques étant choisies à partir de l'une quelconque des structures tridimensionnelles représentées par l'une quelconque des annexes 1 à 6 ; ou bien lesdites coordonnées atomiques étant choisies à partir d'une structure tridimensionnelle qui s'écarte de l'une quelconque des structures tridimensionnelles représentées par les annexes 1 à 6 d'un écart de la moyenne quadratique par rapport aux atomes du squelette de la protéine d'au plus 3 Å ; et
(b) assemblage, au moyen de procédés informatiques, d'un modèle d'une structure chimique ayant une partie qui est structurellement complémentaire de l'ensemble de données de critères relatif à la protéine et/ou structurellement similaire à l'ensemble de données de critères relatif à un composé dudit groupe, et qui est exempte d'interférence stérique avec la protéine.

12. Procédé pour identifier un inhibiteur candidat capable de se lier à et d'inhiber l'activité enzymatique de la 2C-méthyl-D-érythritol 2,4-cyclodiphosphate synthase, ledit procédé comprenant les étapes suivantes :
(a) introduction, dans un ordinateur, d'informations dérivant de coordonnées atomiques définissant une conformation du site actif de ladite synthase ou d'un complexe de ladite synthase avec un composé choisi dans le groupe suivant : 4-diphosphocytidyl-2C-méthyl-D-érythritol, 2-phosphate de 4-diphosphocytidyl-2C-méthyl-D-érythritol, cytidine, monophosphate de cytidine, diphosphate de cytidine, 2C-méthyl-D-érythritol, et une combinaison de monophosphate de cytidine et de 2,4-cyclodiphosphate de 2C-méthyl-D-érythritol ; avec ou sans zinc, sur la base d'une détermination de structure tridimensionnelle, ledit programme utilisant ou affichant sur l'écran d'ordinateur les structures de ladite conformation ;
lesdites coordonnées atomiques correspondant aux coordonnées atomiques de l'une quelconque des annexes 1 à 6, ou bien lesdites coordonnées atomiques correspondant à des coordonnées atomiques qui s'écartent des coordonnées atomiques de l'une quelconque des annexes 1 à 6 d'un écart de la moyenne quadratique par rapport aux atomes du squelette de la protéine d'au plus 3 Å ;
(b) génération d'une représentation tridimensionnelle d'au moins l'une des trois poches du site actif de ladite synthase et/ou d'un composé dudit groupe par ledit programme informatique sur un écran d'ordinateur ;
(c) superposition d'un modèle d'un inhibiteur candidat sur la représentation d'au moins une poche du site actif et/ou d'un composé dudit groupe ;
(d) détermination de la possibilité de liaison et de l'absence d'interférence stérique de l'inhibiteur candidat avec le site actif de la protéine ;
(e) incorporation dudit composé candidat dans un essai d'activité de ladite synthase ; et
(f) détermination du fait que ledit composé candidat inhibe ou non l'activité enzymatique de ladite synthase.

13. Procédé selon la revendication 12, dans lequel les informations sont introduites dans l'ordinateur et dérivent de coordonnées atomiques d'au moins certaines des interactions suivantes du site actif :
Ala131# venant au contact de la face du fragment cytidyle ;
Ala131# se liant par son oxygène de carbonyle à au moins l'un des groupes 2'- et 3'-hydroxyle du fragment cytidyle ;
Asp56 formant une liaison hydrogène par son groupe carboxyle à au moins l'un des groupes 2'- et 3'-hydroxyle du fragment cytidyle ;
Gly58 réalisant un contact de van der Waals par son Cα à au moins l'un des groupes 2'- et 3'-hydroxyle du fragment cytidyle ;
le groupe peptide entre Lys104# et Met105# se liant par liaison hydrogène au N3 du fragment cytidyle ;
Thr133# supportant le fragment cytidyle et se liant par liaison hydrogène par son γ-O ou son groupe NH de squelette à l'α-phosphate ;
Lys104# venant au contact du fragment cytidyle par sa chaîne latérale ;
Leu106# venant au contact du fragment cytidyle par sa chaîne latérale ;
Leu106# se liant par liaison hydrogène par son NH à l'oxygène de carbonyle du fragment cytidyle ;
Asp63 se liant au β-phosphate du diphosphate de cytidine ;
His34 se liant par liaison hydrogène avec son groupe NH de squelette à au moins un atome d'oxygène du groupe phosphate P2 du 2,4-cyclodiphosphate de 2C-méthyl-D-érythritol ;
Ser35 se liant par liaison hydrogène par son groupe NH de squelette à un atome d'oxygène du groupe phosphate P2 ;
Ser35 se liant par liaison hydrogène par son γ-OH à l'un des atomes d'oxygène du groupe phosphate P2 ;
Leu76 réalisant un contact de van der Waals par son δ-C au groupe 2C-méthyle ;
Ile57 réalisant un contact de van der Waals par son δ-C au groupe 2-méthyle ;
Ile57 réalisant un contact de van der Waals par son γ-C au groupe 2-méthyle ;
Phe61 se liant par liaison hydrogène par son oxygène de carbonyle de squelette au groupe 1-hydroxyle ;
Phe61 se liant par liaison hydrogène par son oxygène de carbonyle de squelette au groupe 3-hydroxyle ;
Ile57 se liant par liaison hydrogène par son oxygène de carbonyle de squelette au groupe 3-hydroxyle ;
Ile57 réalisant un contact de van der Waals par son γ-C au carbone en position 4 ;
Pro103# se liant par liaison hydrogène par son oxygène de carbonyle de squelette au groupe amino du fragment cytidyle ;
Ala100# se liant par liaison hydrogène par son oxygène de carbonyle de squelette au groupe amino du fragment cytidyle ;
Ala100# supportant par son oxygène de carbonyle de squelette la position C5 du fragment cytidyle ;
les acides aminés non désignés par # appartenant à une sous-unité, et ceux désignés par # appartenant à une autre sous-unité.

14. Procédé selon l'une des revendications 7 à 13, dans lequel les coordonnées atomiques sont déterminées à une résolution d'au moins 4 Å.

15. Procédé selon l'une des revendications 7 à 14, dans lequel sont choisis des composés qui peuvent se lier à au moins 5 sites de liaison de la synthase.

16. Procédé selon l'une des revendications 7 à 14, dans lequel l'écart de la moyenne quadratique par rapport aux atomes du squelette de la protéine est d'au plus 2 Å.


### SEQUENCE LISTING

<110> Max-Planck-Gesellschaft zur Förderung der Wissenschaften
   Bacher, Adelbert
   Hecht, Stefan
   Huber, Robert
   Kaiser, Johannes
   Rohdich, Felix
<120> Crystal Structure of 2C-mehtyl-D-erythritol 2,4-cyclodiphosphate sy nthase
<130> PCT-11862
<140> PCT/EP02/05238
   <141> 2002-05-13
<150> DE 101 23 597.6
   <151> 2001-05-15
<150> US 60/293,875
   <151> 2001-05-25
<160> 28
<170> PatentIn version 3.1
<210> 1
   <211> 36
   <212> DNA
   <213> Artificial Sequence
<220>
   <223> PCR primer
<400> 1
   gagaaggatc catgcgaatt ggacacggtt ttgacg 36
<210> 2
   <211> 33
   <212> DNA
   <213> Artificial Sequence
<220>
   <223> PCR primer
<400> 2
   tattatctgc agccttgcgg tttaccgtgg agg 33
<210> 3
   <211> 40
   <212> DNA
   <213> Artificial Sequence
<220>
   <223> PCR primer
<400> 3
   cctgacggat ccatgcgaat tggacacggt ttttcagtac 40
<210> 4
   <211> 33
   <212> DNA
   <213> Artificial Sequence
<220>
   <223> PCR primer
<400> 4
   tatcaactgc agtcattttg ttgccttaat gag 33
<210> 5
   <211> 50
   <212> DNA
   <213> Artificial Sequence
<220>
   <223> PCR primer
<400> 5
   cctgacggat ccatgcgaat tggacacggt tttgacgtat cggcctttgg 50
<210> 6
   <211> 33
   <212> DNA
   <213> Artificial Sequence
<220>
   <223> PCR primer
<400> 6
   tatcaactgc agtcattttg ttgccttaat gag 33
<210> 7
   <211> 63
   <212> DNA
   <213> Artificial Sequence
<220>
   <223> PCR primer
<400> 7
   cgcattcctt acgaaaaagg attgctggcg cattctgatg gcgacgtggc gctctctgcg 60
   ttg 63
<210> 8
   <211> 59
   <212> DNA
   <213> Artificial Sequence
<220>
   <223> PCR primer
<400> 8
   gcctttggcg gtgaaggccc aattatcatt ggtggcgtac gcattcctta cgaaaaagg 59
<210> 9
   <211> 57
   <212> DNA
   <213> Artificial Sequence
<220>
   <223> PCR primer
<400> 9
   cctgacggat ccatgcgaat tggacacggt tttgacgtac atgcctttgg cggtgaa 57
<210> 10
   <211> 170
   <212> PRT
   <213> Streptomyces sp.
<400> 10
   Met Gly Ser Glu Ser Ser Gly Thr Ala Glu Leu Pro Pro Leu Pro Gln
   1               5                   10                  15
   Val Gly Ile Gly Thr Asp Ile His Ala Phe Glu Asp Gly Arg Glu Leu
               20                  25                  30
   Trp Cys Ala Gly Leu Lys Trp Glu Gly Glu Gly Thr Gly Leu Ala Gly
           35                  40                  45
   His Ser Asp Ala Asp Val Val Ala His Ala Ala Cys Asn Ala Leu Phe
       50                  55                  60
   Ser Ala Ala Gly Ala Gly Asp Leu Gly Gln His Phe Gly Thr Gly Arg
   65                  70                  75                  80
   Pro Glu Trp Ser Gly Ala Ser Gly Val Thr Leu Leu Ala Glu Ala Ala
                   85                  90                  95
   Arg Ile Val Arg Ala Arg Gly Phe Arg Ile Gly Asn Val Ala Val Gln
               100                 105                 110
   Val Val Gly Ala Arg Pro Lys Ile Gly Lys Arg Arg Asp Glu Ala Glu
           115                 120                 125
   Ala Val Leu Ser Ala Ala Val Gly Ala Pro Val Ser Val Ser Gly Ala
       130                 135                 140
   Thr Thr Asp Gly Leu Gly Phe Pro Gly Arg Gly Glu Gly Leu Met Ala
   145                 150                 155                 160
   Val Ala Thr Ala Leu Val Val Arg Gly Ala
                   165                 170
<210> 11
   <211> 159
   <212> PRT
   <213> Mycobacterium tuberculosis
<400> 11
   Met Asn Gln Leu Pro Arg Val Gly Leu Gly Thr Asp Val His Pro Ile
   1               5                   10                  15
   Glu Pro Gly Arg Pro Cys Trp Leu Val Gly Leu Leu Phe Pro Ser Ala
               20                  25                  30
   Asp Gly Cys Ala Gly His Ser Asp Gly Asp Val Ala Val His Ala Leu
           35                  40                  45
   Cys Asp Ala Val Leu Ser Ala Ala Gly Leu Gly Asp Ile Gly Glu Val
       50                  55                  60
   Phe Gly Val Asp Asp Pro Arg Trp Gln Gly Val Ser Gly Ala Asp Met
   65                  70                  75                  80
   Leu Arg His Val Val Val Leu Ile Thr Gln His Gly Tyr Arg Val Gly
                   85                  90                  95
   Asn Ala Val Val Gln Val Ile Gly Asn Arg Pro Lys Ile Gly Trp Arg
               100                 105                 110
   Arg Leu Glu Ala Gln Ala Val Leu Ser Arg Leu Leu Asn Ala Pro Val
           115                 120                 125
   Ser Val Ser Ala Thr Thr Thr Asp Gly Leu Gly Leu Thr Gly Arg Gly
       130                 135                 140
   Glu Gly Leu Ala Ala Ile Ala Thr Ala Leu Val Val Ser Leu Arg
   145                 150                 155
<210> 12
   <211> 158
   <212> PRT
   <213> Haemophilus influenzae
<400> 12
   Met Ile Arg Ile Gly His Gly Phe Asp Val His Ala Phe Gly Glu Asp
   1               5                   10                  15
   Arg Pro Leu Ile Ile Gly Gly Val Glu Val Pro Tyr His Thr Gly Phe
               20                  25                  30
   Ile Ala His Ser Asp Gly Asp Val Ala Leu His Ala Leu Thr Asp Ala
           35                  40                  45
   Ile Leu Gly Ala Ala Ala Leu Gly Asp Ile Gly Lys Leu Phe Pro Asp
       50                  55                  60
   Thr Asp Met Gln Tyr Lys Asn Ala Asp Ser Arg Gly Leu Leu Arg Glu
   65                  70                  75                  80
   Ala Phe Arg Gln Val Gln Glu Lys Gly Tyr Lys Ile Gly Asn Val Asp
                   85                  90                  95
   Ile Thr Ile Ile Ala Gln Ala Pro Lys Met Arg Pro His Ile Asp Ala
               100                 105                 110
   Met Arg Ala Lys Ile Ala Glu Asp Leu Gln Cys Asp Ile Glu Gln Val
           115                 120                 125
   Asn Val Lys Ala Thr Thr Thr Glu Lys Leu Gly Phe Thr Gly Arg Gln
       130                 135                 140
   Glu Gly Ile Ala Cys Glu Ala Val Ala Leu Leu Ile Arg Gln
   145                 150                 155
<210> 13
   <211> 159
   <212> PRT
   <213> Escherichia coli
<400> 13
   Met Arg Ile Gly His Gly Phe Asp Val His Ala Phe Gly Gly Glu Gly
   1               5                   10                  15
   Pro Ile Ile Ile Gly Gly Val Arg Ile Pro Tyr Glu Lys Gly Leu Leu
               20                  25                  30
   Ala His Ser Asp Gly Asp Val Ala Leu His Ala Leu Thr Asp Ala Leu
           35                  40                  45
   Leu Gly Ala Ala Ala Leu Gly Asp Ile Gly Lys Leu Phe Pro Asp Thr
       50                  55                  60
   Asp Pro Ala Phe Lys Gly Ala Asp Ser Arg Glu Leu Leu Arg Glu Ala
   65                  70                  75                  80
   Trp Arg Arg Ile Gln Ala Lys Gly Tyr Thr Leu Gly Asn Val Asp Val
                   85                  90                  95
   Thr Ile Ile Ala Gln Ala Pro Lys Met Leu Pro His Ile Pro Gln Met
               100                 105                 110
   Arg Val Phe Ile Ala Glu Asp Leu Gly Cys His Met Asp Asp Val Asn
           115                 120                 125
   Val Lys Ala Thr Thr Thr Glu Lys Leu Gly Phe Thr Gly Arg Gly Glu
       130                 135                 140
   Gly Ile Ala Cys Glu Ala Val Ala Leu Leu Ile Lys Ala Thr Lys
   145                 150                 155
<210> 14
   <211> 158
   <212> PRT
   <213> Vibrio cholerae
<400> 14
   Met Ile Arg Ile Gly His Gly Phe Asp Val His Arg Phe Gly Gly Glu
   1               5                   10                  15
   Gly Pro Ile Ile Ile Gly Gly Val Lys Ile Pro Tyr Glu Gln Gly Leu
               20                  25                  30
   Ile Ala His Ser Asp Gly Asp Val Ala Leu His Ala Leu Ser Asp Ala
           35                  40                  45
   Leu Leu Gly Ala Ile Ala Ala Gly Asp Ile Gly Arg His Phe Pro Asp
       50                  55                  60
   Thr Asp Asp Lys Trp Lys Gly Ala Asp Ser Arg Glu Leu Leu Lys Asp
   65                  70                  75                  80
   Val Tyr Arg Arg Val Lys Ala Gln Gly Tyr Val Leu Gly Asn Ala Asp
                   85                  90                  95
   Val Thr Ile Ile Ala Gln Ala Pro Lys Met Ala Pro Tyr Ile Gln Ala
               100                 105                 110
   Met Cys Ala Ala Ile Ala Glu Asp Leu Glu Thr Asp Leu Gly Asn Ile
           115                 120                 125
   Asn Val Lys Ala Thr Thr Thr Glu Lys Leu Gly Phe Thr Gly Arg Lys
       130                 135                 140
   Glu Gly Ile Ala Cys Glu Ala Val Val Leu Leu Arg Lys Ala
   145                 150                 155
<210> 15
   <211> 157
   <212> PRT
   <213> Pseudomonas aeruginosa
<400> 15
   Met Arg Ile Gly His Gly Tyr Asp Val His Arg Phe Gly Glu Gly Asp
   1               5                   10                  15
   Phe Ile Thr Leu Gly Gly Val Arg Ile Pro His Lys His Gly Leu Val
               20                  25                  30
   Ala His Ser Asp Gly Asp Val Leu Leu His Ala Leu Ser Asp Ala Leu
           35                  40                  45
   Leu Gly Ala Ala Ala Leu Gly Asp Ile Gly Lys His Phe Pro Asp Thr
       50                  55                  60
   Asp Pro Arg Phe Lys Gly Ala Asp Ser Arg Ala Leu Leu Arg His Val
   65                  70                  75                  80
   Val Ala Ile Val Ala Glu Lys Gly Trp Lys Val Gly Asn Val Asp Ala
                   85                  90                  95
   Thr Ile Val Ala Gln Ala Pro Lys Met Ala Pro His Ile Glu Thr Met
               100                 105                 110
   Arg Gly Leu Ile Ala Glu Asp Leu Gly Val Ala Val Asp Gln Val Asn
           115                 120                 125
   Val Lys Ala Thr Thr Thr Glu Arg Leu Gly Phe Thr Gly Arg Glu Glu
       130                 135                 140
   Gly Ile Ala Val His Ala Val Ala Leu Leu Met Ala Arg
   145                 150                 155
<210> 16
   <211> 158
   <212> PRT
   <213> Bacillus subtilis
<400> 16
   Met Phe Arg Ile Gly Gln Gly Phe Asp Val His Gln Leu Val Glu Gly
   1               5                   10                  15
   Arg Pro Leu Ile Ile Gly Gly Ile Glu Ile Pro Tyr Glu Lys Gly Leu
   20 25 30
   Leu Gly His Ser Asp Ala Asp Val Leu Leu His Thr Val Ala Asp Ala
   35 40 45
   Cys Leu Gly Ala Val Gly Glu Gly Asp Ile Gly Lys His Phe Pro Asp
   50 55 60
   Thr Asp Pro Glu Phe Lys Asp Ala Asp Ser Phe Lys Leu Leu Gln His
   65 70 75 80
   Val Trp Gly Ile Val Lys Gln Lys Gly Tyr Val Leu Gly Asn Ile Asp
   85 90 95
   Cys Thr Ile Ile Ala Gln Lys Pro Lys Met Leu Pro Tyr Ile Glu Asp
   100 105 110
   Met Arg Lys Arg Ile Ala Glu Gly Leu Glu Ala Asp Val Ser Gln Val
   115 120 125
   Asn Val Lys Ala Thr Thr Thr Glu Lys Leu Gly Phe Thr Gly Arg Ala
   130 135 140
   Glu Gly Ile Ala Ala Gln Ala Thr Val Leu Ile Gln Lys Gly
   145 150 155
<210> 17
   <211> 160
   <212> PRT
   <213> Neisseria meningitidis
<400> 17
   Met Thr Asn Ile Arg Ile Gly Gln Gly Tyr Asp Val His Gln Leu Thr
   1 5 10 15
   Glu Gly Arg Lys Leu Ile Leu Gly Gly Val Glu Ile Pro Phe Glu Lys
   20 25 30
   Gly Leu Leu Gly His Ser Asp Ala Asp Ala Leu Leu His Ala Val Thr
   35 40 45
   Asp Ala Leu Leu Gly Ala Ala Gly Leu Gly Asp Ile Gly Ser His Phe
   50 55 60
   Pro Asp Thr Ala Ala Glu Phe Lys Asp Ala Asp Ser Arg Val Leu Leu
   65 70 75 80
   Arg Ala Ala Tyr Gln Ser Val Gln Ala Gln Gly Trp Gln Ala Val Asn
   85 90 95
   Val Asp Thr Thr Val Ile Ala Gln Lys Pro Lys Leu Ala Pro His Ile
   100 105 110
   Pro Gln Met Arg Ala Asn Ile Ala Ala Asp Leu Gly Ile Asp Ile Ser
   115 120 125
   Cys Val Asn Ile Lys Gly Lys Thr Asn Glu Lys Leu Gly Tyr Leu Gly
   130 135 140
   Arg Met Glu Gly Ile Glu Ala Gln Ala Ala Val Leu Leu Val Arg Ile
   145 150 155 160
<210> 18
   <211> 176
   <212> PRT
   <213> Xylella fastidiosa
<400> 18
   Met Ser Gly Leu Phe Arg Gly Arg Val Met Thr Gly Leu Asn Val Arg
   1 5 10 15
   Ile Gly Gln Gly Tyr Asp Val His Ala Phe Gly Pro Gly Asp His Val
   20 25 30
   Met Leu Gly Gly Val Arg Val Pro His Arg Cys Gly Val Leu Ala His
   35 40 45
   Ser Asp Gly Asp Val Ile Leu His Ala Leu Cys Asp Ala Met Leu Gly
   50 55 60
   Ala Leu Gly Leu Gly Asp Ile Gly Gln His Phe Pro Pro Ser Asp Val
   65 70 75 80
   Arg Trp Lys Gly Ala Asp Ser Gly Val Phe Val Arg His Cys His Leu
   85 90 95
   Leu Leu Arg Glu Arg Asp Trp Cys Val Gly Asn Val Asp Ala Thr Ala
   100 105 110
   Ile Cys Glu Ser Pro Lys Ile Ala Pro Tyr Val Asn Ala Met Arg Glu
   115 120 125
   Gly Ile Ala Arg Leu Leu Glu Val Gln Pro Glu Cys Val Ser Val Lys
   130 135 140
   Ala Thr Thr Ser Glu Gly Leu Gly Phe Ile Gly Arg Gly Glu Gly Leu
   145 150 155 160
   Ala Ala Gln Val Val Val Leu Leu Tyr Arg Leu Asn Gly Val Val Val
   165 170 175
<210> 19
   <211> 161
   <212> PRT
   <213> Synechocystis sp.
<400> 19
   Met Thr Ala Leu Arg Ile Gly Asn Gly Tyr Asp Ile His Arg Leu Val
   1               5                   10                  15
   Gly Asp Arg Pro Leu Ile Leu Gly Gly Val Thr Ile Ala His His Leu
               20                  25                  30
   Gly Leu Asp Gly His Ser Asp Ala Asp Val Leu Thr His Ala Leu Met
           35                  40                  45
   Asp Ala Leu Leu Gly Ala Leu Ser Leu Gly Asp Ile Gly His Tyr Phe
       50                  55                  60
   Pro Pro Ser Asp Ala Arg Trp Gln Gly Ala Asp Ser Leu Lys Leu Leu
   65                  70                  75                  80
   Ala Gln Val His Gln Leu Ile Leu Glu Arg Gly Trp Arg Ile Asn Asn
                   85                  90                  95
   Leu Asp Asn Val Ile Val Ala Glu Gln Pro Lys Leu Lys Pro His Ile
               100                 105                 110
   Gln Ala Met Lys Glu Asn Leu Ala Lys Val Leu Thr Ile Asp Pro Asp
           115                 120                 125
   Leu Ile Gly Ile Lys Ala Thr Thr Asn Glu Arg Leu Gly Pro Thr Gly
       130                 135                 140
   Arg Glu Glu Gly Ile Ala Ala Tyr Ser Val Ala Leu Leu Ile Lys Glu
   145                 150                 155                 160
   Gly
<210> 20
   <211> 161
   <212> PRT
   <213> Buchnera sp.
<400> 20
   Met Arg Ile Gly Tyr Gly Phe Asp Leu His Ala Phe Gly Ser Thr Lys
   1               5                   10                  15
   Pro Leu Ile Ile Gly Gly Val Leu Ile Pro Cys Glu Lys Gly Leu Ile
               20                  25                  30
   Ala His Ser Asn Gly Asp Leu Leu Ile His Ser Leu Ile Asp Ala Leu
           35                  40                  45
   Leu Gly Ala Thr Ala Met Gly Asp Ile Gly Ser Phe Phe Pro Ser Asn
       50                  55                  60
   Asn Tyr Ile Tyr Lys Asn Ile Asp Ser Arg Ile Leu Leu Lys Lys Ile
   65                  70                  75                  80
   Trp Asn Lys Ile Ile Leu Leu Asn Tyr Asp Ile Cys Asn Ile Asp Ile
                   85                  90                  95
   Thr Ile Ile Ala Glu Tyr Pro Lys Met Leu Pro Tyr Ile Phe Phe Met
               100                 105                 110
   Arg Ser Asn Leu Ser Leu Asp Leu Asn Thr Glu Ile Asp Asn Ile Ser
           115                 120                 125
   Ile Lys Ser Thr Thr Ser Lys Lys Ile Gly Cys Ile Gly Arg Lys Glu
       130                 135                 140
   Ala Ile Ala Cys His Ser Ile Val Met Leu Ile Lys Asn Lys Lys Cys
   145                 150                 155                 160
   Ile
<210> 21
   <211> 156
   <212> PRT
   <213> Aquifex aeolicus
<400> 21
   Met Glu Leu Arg Ile Gly Phe Gly Phe Asp Ser His Glu Phe Val Glu
   1               5                   10                  15
   Gly Lys Leu Leu Ile Leu Gly Gly Val Glu Ile Glu Lys Asp Tyr Gly
   20 25 30
   Leu Lys Gly His Ser Asp Gly Asp Ala Leu Leu His Ala Ile Thr Asp
   35 40 45
   Ala Ile Leu Gly Ala Leu Gly Glu Arg Asp Ile Gly Glu Ile Phe Lys
   50 55 60
   Asp Thr Asp Pro Arg Trp Lys Asn Ala Pro Ser Arg Ile Phe Leu Glu
   65 70 75 80
   Lys Ala Leu Glu Val Met Ser Glu Lys Gly Phe Asn Ile Ser Asn Ile
   85 90 95
   Asp Cys Val Ile Val Ala Asp Arg Pro Lys Ile Ala Pro His Lys Glu
   100 105 110
   Arg Ile Lys Glu Ser Leu Ser Lys Leu Leu Gly Ile Pro Lys Glu Arg
   115 120 125
   Ile Ser Leu Lys Gly Lys Arg Arg Glu Gly Phe Cys Glu Gly Asn Gly
   130 135 140
   Leu Val Cys Met Cys Thr Val Leu Leu Val Lys Met
   145 150 155
<210> 22
   <211> 231
   <212> PRT
   <213> Arabidopsis thaliana
<400> 22
   Met Ala Thr Ser Ser Thr Gln Leu Leu Leu Ser Ser Ser Ser Leu Phe
   1 5 10 15
   His Ser Gln Ile Thr Lys Lys Pro Phe Leu Leu Pro Ala Thr Lys Ile
   20 25 30
   Gly Val Trp Arg Pro Lys Lys Ser Leu Ser Leu Ser Cys Arg Pro Ser
   35 40 45
   Ala Ser Val Ser Ala Ala Ser Ser Ala Val Asp Val Asn Glu Ser Val
   50 55 60
   Thr Ser Glu Lys Pro Thr Lys Thr Leu Pro Phe Arg Ile Gly His Gly
   65 70 75 80
   Phe Asp Leu His Arg Leu Glu Pro Gly Tyr Pro Leu Ile Ile Gly Gly
   85 90 95
   Ile Val Ile Pro His Asp Arg Gly Cys Glu Ala His Ser Asp Gly Asp
   100 105 110
   Val Leu Leu His Cys Val Val Asp Ala Ile Leu Gly Ala Leu Gly Leu
   115 120 125
   Pro Asp Ile Gly Gln Ile Phe Pro Asp Ser Asp Pro Lys Trp Lys Gly
   130 135 140
   Ala Ala Ser Ser Val Phe Ile Lys Glu Ala Val Arg Leu Met Asp Glu
   145 150 155 160
   Ala Gly Tyr Glu Ile Gly Asn Leu Asp Ala Thr Leu Ile Leu Gln Arg
   165 170 175
   Pro Lys Ile Ser Pro His Lys Glu Thr Ile Arg Ser Asn Leu Ser Lys
   180 185 190
   Leu Leu Gly Ala Asp Pro Ser Val Val Asn Leu Lys Ala Lys Thr His
   195 200 205
   Glu Lys Val Asp Ser Leu Gly Glu Asn Arg Ser Ile Ala Ala His Thr
   210 215 220
   Val Ile Leu Leu Met Lys Lys
   225 230
<210> 23
   <211> 165
   <212> PRT
   <213> Thermotoga maritima
<400> 23
   Met Glu Ser Asp Pro Met Phe Ile Gly Phe Gly Tyr Asp Arg His Pro
   1 5 10 15
   Leu Val Glu Gly Arg Arg Leu Val Leu Ala Gly Val Glu Ile Asp Ala
   20 25 30
   Pro Leu Gly Ser Leu Gly His Ser Asp Gly Asp Val Leu Ser His Ala
   35 40 45
   Ile Ile Asp Ala Leu Leu Gly Ala Gly Cys Leu Gly Asp Ile Gly Thr
   50 55 60
   Trp Phe Pro Glu Thr Lys Glu Tyr Lys Asp Ala Asn Ser Leu Asp Leu
   65 70 75 80
   Leu Lys Glu Thr Val Lys Ile Leu Glu Glu Arg Gly Phe Ser Val Val
   85 90 95
   Asn Val Asp Ala Thr Val Val Ala Ser Ile Val Lys Leu Ser Pro Tyr
   100 105 110
   Arg Glu Lys Ile Val Glu Asn Leu Lys Ser Ala Leu Glu Thr Ser Arg
   115 120 125
   Val Asn Val Lys Phe Lys Ser Gly Asn Thr Leu Gly Phe Glu Gly Glu
   130 135 140
   Glu Arg Gly Ile Ser Ala Tyr Ala Val Cys Leu Val Glu Glu Lys Gly
   145 150 155 160
   Cys Thr Lys Ser Thr
   165
<210> 24
   <211> 161
   <212> PRT
   <213> Deinococcus radiodurans
<400> 24
   Met Thr Gly Leu Pro Phe Arg Ile Gly Tyr Gly Glu Asp Ala His Arg
   1               5                   10                  15
   Leu Ala Pro Gly Leu Pro Leu Val Leu Gly Gly Val Ala Ile Pro His
               20                  25                  30
   Ala Glu Leu Gly Ala Val Ala His Ser Asp Gly Asp Ala Val Leu His
           35                  40                  45
   Ala Val Ala Asp Ala Leu Leu Ser Gly Leu Ala Leu Gly Asp Ile Gly
       50                  55                  60
   Gln Tyr Phe Pro Asp Thr Ala Ala Glu Trp Lys Gly Met Asp Ser Arg
   65                  70                  75                  80
   Arg Ile Leu Ala Lys Ala Leu Glu Leu Val Glu Glu Arg Gly Tyr Arg
                   85                  90                  95
   Pro Val Asn Val Ala Leu Val Val Thr Leu Asp Arg Pro Lys Leu Gly
               100                 105                 110
   Pro Leu Arg Ala Asp Ile Ala Ala Ser Val Ala Glu Leu Leu Gly Leu
           115                 120                 125
   Pro Ala Gly Glu Val Gly Val Ser Phe Lys Thr Ser Glu Gly Leu Ala
       130                 135                 140
   Leu Glu His Val Gln Thr Arg Val Thr Val Leu Leu Gly Arg Val Asp
   145                 150                 155                 160
   Asp
<210> 25
   <211> 240
   <212> PRT
   <213> Plasmodium falciparum
<400> 25
   Met Phe Leu Lys Gly Tyr Thr Ser Asn Val Val Leu Ile Ile Leu Thr
   1               5                   10                  15
   Phe Phe Ile Leu Leu Thr Lys Glu Glu Lys Asn Ile Lys Asn Asn Ile
               20                  25                  30
   Ser Gly Tyr Cys Phe Leu Asn Phe Gly Leu Lys Lys Asn Ala Ile Ile
           35                  40                  45
   Lys Lys Arg Glu Lys Gln Asn Leu Lys Leu Phe Cys Tyr Asn Gly Ile
       50                  55                  60
   Arg Ile Gly Gln Gly Tyr Asp Ile His Lys Ile Lys Val Leu Asp Glu
   65                  70                  75                  80
   Glu Tyr Asn Thr Tyr Ala Asn Asn Asp Phe Asn Lys Asn Glu Gln Ser
                   85                  90                  95
   Phe Lys Thr Leu Thr Leu Gly Gly Val Lys Ile Asn Asn Val Leu Val
               100                 105                 110
   Leu Ser His Ser Asp Gly Asp Ile Ile Tyr His Ser Ile Val Asp Ser
           115                 120                 125
   Ile Leu Gly Ala Leu Gly Ser Leu Asp Ile Gly Thr Leu Phe Pro Asp
       130                 135                 140
   Lys Asp Glu Lys Asn Lys Asn Lys Asn Ser Ala Ile Phe Leu Arg Tyr
   145                 150                 155                 160
   Ala Arg Leu Leu Ile Tyr Lys Lys Asn Tyr Asp Ile Gly Asn Val Asp
                   165                 170                 175
   Ile Asn Val Ile Ala Gln Val Pro Lys Ile Ser Asn Ile Arg Lys Asn
               180                 185                 190
   Ile Ile Lys Asn Ile Ser Thr Val Leu Asn Ile Asp Glu Ser Gln Ile
           195                 200                 205
   Ser Val Lys Gly Lys Thr His Glu Lys Leu Gly Val Ile Gly Glu Lys
       210                 215                 220
   Lys Ala Ile Glu Cys Phe Ala Asn Ile Leu Leu Ile Pro Lys Asn Ser
   225                 230                 235                 240
<210> 26
   <211> 178
   <212> PRT
   <213> Chlamydia muridarum
<400> 26
   Met Ser Glu Pro Ser Ser Cys Phe Val Leu Pro Asp Pro Glu Trp Ile
   1               5                   10                  15
   Tyr Arg Val Gly Ile Gly Gln Asp Ser His Arg Phe Leu Pro Ser Asp
               20                  25                  30
   Asn Pro Lys Pro Cys Val Leu Gly Gly Ile Ile Phe Glu Asn Thr Pro
           35                  40                  45
   Gly Phe Glu Ala Asn Ser Asp Gly Asp Val Val Phe His Ala Ile Cys
       50                  55                  60
   Asn Ala Phe Ser Ser Val Thr His Gln Val Ile Leu Gly Ala Leu Ala
   65                  70                  75                  80
   Asp Glu Leu Leu Lys Ala Lys Gly Ile Ser Asp Ser Ala Val Tyr Leu
                   85                  90                  95
   Lys Glu Ala Val Ala Ser Leu Lys Pro Thr Gln Lys Ile Ser His Leu
               100                 105                 110
   Ala Ile Thr Ile Glu Gly Gln Arg Pro Lys Leu Leu Pro Lys Ile Ser
           115                 120                 125
   Ala Met Arg Glu Lys Ile Ala Glu Ile Leu Arg Ile Ser Leu Asp Ser
       130                 135                 140
   Val Asn Ile Thr Ala Thr Ser Gly Glu Gly Leu Thr Ser Met Gly Gln
   145                 150                 155                 160
   Gly His Gly Val Gln Cys Phe Cys Val Leu Thr Val Met Glu Phe Cys
                   165                 170                 175
   Pro Arg
<210> 27
   <211> 483
   <212> DNA
   <213> Escherichia coli
<400> 27
   atgcgaattg gacacggttt tgacgtacat gcctttggcg gtgaaggccc aattatcatt     60
   ggtggcgtac gcattcctta cgaaaaagga ttgctggcgc attctgatgg cgacgtggcg    120
   tctctccatg cgttgaccga tgcattgctt ggcgcggcgg cgctggggga tatcggcaag    180
   ctgttcccgg ataccgatcc ggcatttaaa ggtgccgata gccgcgagct gctacgcgaa    240
   gcctggcgtc gtattcaggc gaagggttat acccttggca acgtcgatgt cactatcatc    300
   gctcaggcac cgaagatgtt gccgcacatt ccacaaatgc gcgtgtttat tgccgaagat    360
   ctcggctgcc atatggatga tgttaacgtg aaagccacta ctacggaaaa actgggattt    420
   accggacgtg gggaagggat tgcctgtgaa gcggtggcgc tactcattaa ggcaacaaaa    480
   tga                                                                  483
<210> 28
   <211> 160
   <212> PRT
   <213> Escherichia coli
<400> 28
   Met Arg Ile Gly His Gly Phe Asp Val His Ala Phe Gly Gly Glu Gly
   1               5                   10                  15
   Pro Ile Ile Ile Gly Gly Val Arg Ile Pro Tyr Glu Lys Gly Leu Leu
               20                  25                  30
   Ala His Ser Asp Gly Asp Val Ala Ser Leu His Ala Leu Thr Asp Ala
           35                  40                  45
   Leu Leu Gly Ala Ala Ala Leu Gly Asp Ile Gly Lys Leu Phe Pro Asp
       50                  55                  60
   Thr Asp Pro Ala Phe Lys Gly Ala Asp Ser Arg Glu Leu Leu Arg Glu
   65                  70                  75                  80
   Ala Trp Arg Arg Ile Gln Ala Lys Gly Tyr Thr Leu Gly Asn Val Asp
                   85                  90                  95
   Val Thr Ile Ile Ala Gln Ala Pro Lys Met Leu Pro His Ile Pro Gln
               100                 105                 110
   Met Arg Val Phe Ile Ala Glu Asp Leu Gly Cys His Met Asp Asp Val
           115                 120                 125
   Asn Val Lys Ala Thr Thr Thr Glu Lys Leu Gly Phe Thr Gly Arg Gly
       130                 135                 140
   Glu Gly Ile Ala Cys Glu Ala Val Ala Leu Leu Ile Lys Ala Thr Lys
   145                 150                 155                 160